# EUROPEAN PATENT APPLICATION

(11) **EP 4 745 142 A1**
(43) Date of publication of application: **20.05.2026**
(21) Application number: 24838906.6
(22) Date of filing: 12.07.2024
(51) Int. Cl.: C07D 487/04, C07D 471/04, C07D 401/14, A61K 31/404, A61K 31/495, A61K 31/395, A61P 29/00, A61P 35/00, A61P 37/00

(54) **AROMATIC COMPOUND, PHARMACEUTICAL COMPOSITION CONTAINING SAME, AND USE THEREOF**

(30) Priority: 13.07.2023 CN 202310859092
(71) Applicant: Increland, Sichuan 610000 (CN)
(72) Inventor: ZHANG, Dengyou, Chengdu, Sichuan 610000 (CN); ZHANG, Yangxue, Chengdu, Sichuan 610000 (CN); LI, Ping, Chengdu, Sichuan 610000 (CN); ZHOU, Ziyang, Chengdu, Sichuan 610000 (CN)
(74) Representative: Goodwin Procter (UK) LLP
(86) International application number: PCT/CN2024/105291
(87) International publication number: WO 2025/011655

(57) **Abstract**

Disclosed in the present invention are an aromatic compound, a pharmaceutical composition containing same, and a use thereof. The aromatic compound is a compound having formula (I), and can effectively degrade IRAK4 or otherwise inhibit IRAK4 activity. The compound and the pharmaceutical composition of the present invention have good prospects for application in IRAK4-mediated diseases including immune diseases, tumors, Alzheimer's disease, and fibrotic diseases, providing a new choice for clinically screening and/or preparing drugs for diseases associated with IRAK4 activity.

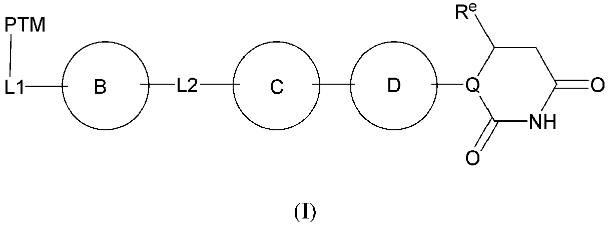

## Description

### Cross-Reference to Related Application

The present application claims priority to Chinese patent application with the application number of 202310859092.8 filed with the China National Intellectual Property Administration on 13 July 2023 and entitled "AROMATIC COMPOUND, PHARMACEUTICAL COMPOSITION CONTAINING SAME, AND USE THEREOF", the entire content of which is incorporated herein by reference.

### Technical Field

The present invention belongs to the field of pharmaceutical synthesis, and in particular, to an aromatic compound, a pharmaceutical composition comprising the same and use thereof.

### Background

Protein degradation is a highly regulated and essential process for maintaining cellular homeostasis. Ubiquitin-proteasome pathway (UPP) functions in vivo to selectively identify and remove excess proteins, and degrade misfolded or abnormal proteins. Ubiquitin molecules are covalently linked to terminal lysine residues by E3 ubiquitin ligases, thereby marking proteins for degradation by proteasomes into small peptides, and finally digesting the small peptides into their constituent amino acids, which are then used as building blocks for new proteins. The UPP plays a central role in multiple cellular processes and, if defective or unbalanced, causes the pathogenesis of a variety of diseases. The UPP is central to regulating almost all cellular processes, including antigen processing, apoptosis, organelle biogenesis, cell cycle, DNA transcription and repair, differentiation and development, immune response and inflammation, neural and muscle degeneration, morphogenesis of neural networks, regulation of cell surface receptors, ion channels and secretory pathways, responses to stress and extracellular regulators, ribosome biogenesis and viral infection. Defective proteasomal degradation is implicated in a variety of clinical conditions, including, e.g., Alzheimer's disease, Parkinson's disease, Huntington's disease, myodystrophy, cardiovascular disease, and cancer.

Protein degradation targeted chimera (PROTAC) is an effective means for degrading pathogenic proteins. Small molecules capable of binding to target proteins are linked to small molecules capable of binding to E3 ligases including, e.g., CRBN, VHL, MDM2, and DRAF to form heterobifunctional molecules, and the target protein is ubiquitinated by simulating the ubiquitin-proteasome pathway (UPP), thereby degrading the target protein by the proteasome. Compared with small molecule inhibitors, a potential advantage of the protein degradation targeted chimera is that it can disable all functions of pathogenic proteins.

At present, more than 600 E3 ubiquitin ligases have been found to promote in vivo ubiquitination of different proteins, which can be classified into four families: HECT domain E3 family, U-box E3 family, monomeric RING E3 family, and multi-subunit E3 family. Cereblon (CRBN) ligase is a most widely used E3 ligase of PROTAC technology. As a 442-amino acid protein, the Cereblon belongs to a Cullin RING E3 ubiquitin ligase, forms a Cullin-4-RING E3 ubiquitin ligase (CRL4) complex, and interacts with adaptor protein damaged DNA binding protein 1 (DDB1). In a CRL4 complex, CRBN acts as a substrate-specific receptor. Known CRBN ligands include thalidomide and other derived immunomodulatory imide drugs. After the CRBN binds to the ligand, E3 ubiquitin ligase activity of the CRBN is re-regulated, thereby increasing recruitment of transcription factors Ikaros and Aiolos, and triggering subsequent ubiquitination and proteasomal degradation. At present, the CRBN, as an E3 ligase in PROTAC, has been successfully used to target more than 30 different proteins, including proteins implicated in various cancers (Sun X. et al., 2019), proteins implicated in immune dysfunction (Bassi et al., 2018), proteins implicated in neurodegenerative disease (Silva et al., 2019), and hepatitis C virus proteins (de Wispelaere et al., 2019). Most CRBN-targeted PROTACs employ pomalidomide, 4-hydroxythalidomide, alkyl-linked thalidomide derivatives, or lenalidomide derivatives. However, it is possible to develop better CRBN ligands. These new CRBN ligands will provide more options for the development of the PROTAC technology.

IRAK4 belongs to a serine/threonine kinase and is a key protein in mediating Toll-like receptor (TLR) signals of interleukin-1 (IL-1) receptor family (IL-1, IL-18, and IL-33 receptors) and pathogen recognition. Studies show that when foreign pathogens and inflammatory stress are recognized, under the action of extracellular ligands, interleukin-1 receptors or TLR receptors recruit the adapter protein myeloid differentiation primary response protein (Myd88), which then forms a complex with the IRAK4, thereby activating NF-κB light chain enhancer and activator protein 1 (AP-1), producing various inflammatory factors by cells, such as tumor necrosis factor α (TNFα) and IL-6, and inducing various immune diseases, such as psoriasis, hidradenitis suppurativa, atopic dermatitis, rheumatoid arthritis, and systemic lupus erythematosus. In addition, the IRAK4 has been verified to be implicated in lymphocytic leukemia and lymphoma, Alzheimer's disease, and fibrosis. Therefore, the IRAK4 is an attractive target for drug development.

At present, major pharmaceutical companies, including, e.g., Prizer, Gilead, Bayer, and Curis, have successively promoted the entry of IRAK4 small molecule inhibitors into clinical trials for, e.g., hematological tumor, psoriasis, rheumatoid arthritis, enteritis, and systemic lupus erythematosus. The IRAK4 inhibitor PF-06650833 studied by Pfizer has entered phase II clinical trial. Early clinical results show that PF-06650833 has good safety, and its efficacy shows that PF-06650833 can inhibit IRAK4-mediated inflammatory pathway. Such clinical data fully demonstrates that the IRAK4 is a clinically proven drug target and has the potential to treat a variety of diseases.

Recent studies show that not only does the kinase activity of IRAK4 mediate the inflammatory signaling pathways, but also the IRAK4 protein skeleton can activate some inflammatory signaling pathways. In human skin fibroblasts, inhibition of IRAK4 by ATP-competitive small molecules cannot effectively inhibit the release of IL-6 and TNF-α stimulated by IL-1β. In addition, knockout of the IRAK4 can effectively eliminate inflammatory responses mediated by IL-1, IL-8, and TLR ligands. Therefore, ATP-competitive small molecule inhibitors cannot completely eliminate the inflammatory signaling pathways mediated by IRAK4 proteins. htus it can be seen that targeting IRAK4 by small molecule inhibitors has its therapeutic limitations.

Protein degradation targeted chimera (PROTAC) is an effective means for degrading pathogenic proteins. Small molecules capable of binding to target proteins are linked to small molecules capable of binding to E3 ligases including, e.g., CRBN, VHL, MDM2, and DRAF to form heterobifunctional molecules, and the target protein is ubiquitinated by simulating the ubiquitin-proteasome pathway (UPP), thereby degrading the target protein by the proteasome. Compared with small molecule inhibitors, a potential advantage of the protein degradation targeted chimera is that it can disable all functions of pathogenic proteins. In addition, GSK scientists have demonstrated that the IRAK4 proteins can be degraded by binding IRAK4 small molecule inhibitors with ligands of E3 ligases CRBN and VHL through linker fragments to form the protein degradation targeting chimera (PROTAC). At the same time, Kymera and Avinas have designed corresponding PROTAC for the IRAK4. These emerging technologies provide a new therapeutic means for targeting the IRAK4.

### Summary of the Invention

An object of the present invention is to solve the defects of the prior art, and provide an IRAK4-targeted PROTAC drug.

The present invention provides a compound represented by formula I, or an enantiomer thereof, a diastereomer thereof, a racemate thereof, a mixture comprising the same, or a deuterated compound thereof, or a pharmaceutically acceptable salt thereof: 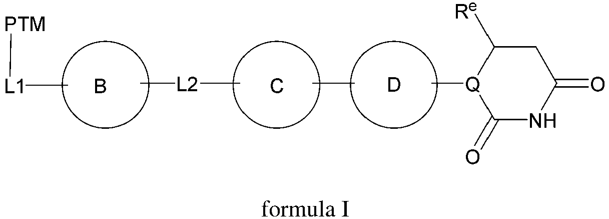 wherein
PTM is selected from 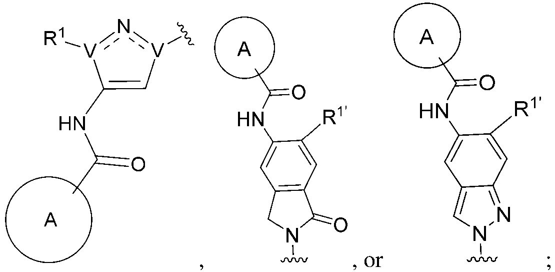
the ring D is selected from 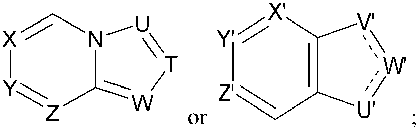
and when the ring D is selected from 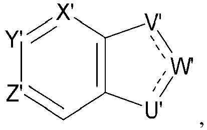 the PTM is not selected from 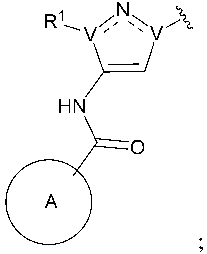
Q is selected from CR^{2a} or N;
V' is selected from C or N;
U' is selected from CR² or NR²;
W' is selected from CR³ or N;
X' is selected from C, CR⁴, or N;
Y' is selected from C, CR⁵, or N;
Z' is selected from C, CR⁶, or N;
U is selected from C, CR^{U}, or N;
W is selected from C, CR^{W}, or N;
T is selected from C, CR^{T}, or N;
X is selected from C, CR^{X}, or N;
Y is selected from C, CR^{Y}, or N;
Z is selected from C, CR^{Z}, or N;
-̅ -̅ -̅ represents a single bond or a double bond;
V is selected from C or N;
R¹ and R^{1'} are each independently selected from hydrogen, deuterium, halogen, cyano, substituted or unsubstituted C1-C6 linear or branched alkyl, substituted or unsubstituted C1-C6 linear or branched alkoxy, substituted or unsubstituted 4-10-membered heterocyclyl, substituted or unsubstituted C5-C12 bridged heterocyclyl, substituted or unsubstituted C3-C10 heterospirocyclyl, substituted or unsubstituted C1-C6 linear or branched alkylamino, substituted or unsubstituted 3-10-membered cycloalkyl, substituted or unsubstituted unsaturated 3-10-membered cyclic hydrocarbon radical, or substituted or unsubstituted C2-C6 linear or branched unsaturated hydrocarbon radical; wherein the substutuents are independently selected from deuterium, halogen, hydroxyl, cyano, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, or 4-10-membered heterocyclyl; and the heterocyclyl comprises 1-4 heteroatoms selected from oxygen, sulfur, and nitrogen;
ring A is independently selected from 0-4 R⁷-substituted: 5-10-membered heteroaromatic rings or 5-6-membered heteroaromatic ring-5-6-membered heteroaromatic rings; and the heteroaryl comprises 1-4 heteroatoms selected from oxygen, sulfur, and nitrogen;
R^{2a}, R², R³, R⁴, R⁵, R⁶, R^{U}, R^{W}, R^{T}, R^{X}, R^{Y}, R^{Z}, R^{e}, and R⁷ are each independently selected from hydrogen, deuterium, halogen, cyano, hydroxyl, amino, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, substituted or unsubstituted C1-C6 linear or branched alkanoyl, or substituted or unsubstituted 3-10-membered cycloalkanoyl; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, 5-10-membered heteroaromatic ring, 3-10-membered cycloalkyl, C1-C6 linear or branched alkyl-C(=O)-, or 3-10-membered cycloalkyl-C(=O)-; and the heterocyclyl, the bridged heterocyclyl, the heterospirocyclyl, and the heteroaromatic ring each comprise 1-4 heteroatoms selected from oxygen, sulfur, and nitrogen;
L1 is selected from N.A., 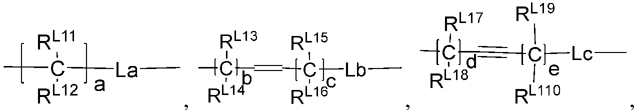 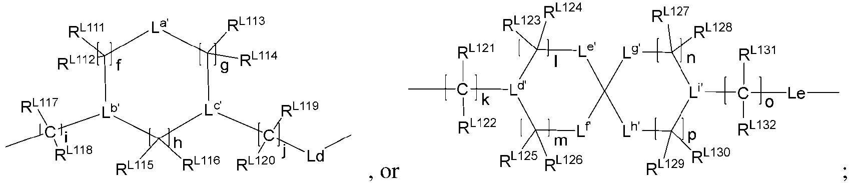
a, b, c, d, e, f, g, h, i, j, k, l, m, n, o, and p are each independently selected from 0-6;
La, Lb, Lc, Ld, Le, L^{a'}, L^{e'}, L^{f'}, L^{g'}, and L^{h'} are each independently selected from N.A., O, S, - N(R⁸)-, -C(=O)-R⁹-, or -SO₂R¹⁰-; R⁸ is selected from hydrogen, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, -R¹¹COR¹²-, -R¹³OCOR¹⁴-, -R¹⁵COOR¹⁶-, - R¹⁷NR¹⁸COR¹⁹-, -R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴-, or 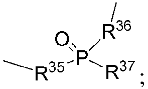 the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, or 5-10-membered heteroaromatic ring; the heterocyclyl, the bridged heterocyclyl, the heterospirocyclyl, and the heteroaromatic ring each comprise 1-4 heteroatoms selected from oxygen, sulfur, and nitrogen; the R⁸ can form a substituted or unsubstituted 3-10-membered ring with R^{L111}, R^{L112} R^{L113} R^{L114}, R^{L123}, R¹²⁴, R^{L125}, R^{L126}, R^{L127}, R^{L128}, R^{L129}, and R^{L130} respectively through C, N, O, and S; and the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, - R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴- 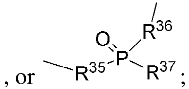
R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹, R³², R³³, R³⁴, R³⁵, R³⁶, and R³⁷ are each independently selected from N.A., hydrogen, deuterium, halogen, cyano, hydroxyl, amino, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, substituted or unsubstituted C1-C6 linear or branched alkanoyl, or substituted or unsubstituted 3-10-membered cycloalkanoyl; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, or 5-10-membered heteroaromatic ring; and the heterocyclyl, the bridged heterocyclyl, the heterospirocyclyl, and the heteroaromatic ring each comprise 1-4 heteroatoms selected from oxygen, sulfur, and nitrogen;
L^{b'}, L^{c'}, L^{d'}, and L^{i'} are each independently selected from N or CR³⁸;
R³⁸, R^{L11}, R^{L12}, R^{L13}, R^{L14}, R^{L15}, R^{L16}, R^{L17}, R^{L18}, R^{L19}, R^{L110}, R^{L111}, R^{L112}, R^{L113}, R^{L114}, R^{L115}, R^{L116}, R^{L117}, R^{L118}, R^{L119}, R^{L120}, R^{L121}, R^{L122}, R^{L123}, R^{L124}, R^{L25}, R^{L126}, R^{L127}, R^{L128}, R^{L129}, R^{L130}, R^{L131}, and R^{L132}are each independently selected from hydrogen, deuterium, halogen, cyano, hydroxyl, amino, substituted or unsubstituted 4-10-membered heterocyclyl, substituted or unsubstituted C5-C12 bridged heterocyclyl, substituted or unsubstituted C3-C10 **heterospirocyclyl,** substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, - R¹¹COR¹²-, -R¹³OCOR¹⁴-, -R¹⁵COOR¹⁶-, -R¹⁷NR¹⁸COR¹⁹-, -R²⁰CONR²¹R²²-, -R²³SO₂R²⁴-, - R²⁵OSO₂R²⁶-, -R²⁷SO₂OR²⁸-, -R²⁹NR³⁰SO₂R³¹-, -R³²SO₂NR³³R³⁴-, or 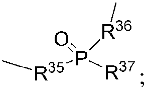
the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, or 5-10-membered heteroaromatic ring; the heterocyclyl, the bridged heterocyclyl, the heterospirocyclyl, and the heteroaromatic ring each comprise 1-4 heteroatoms selected from oxygen, sulfur, and nitrogen; the R³⁸ can form a substituted or unsubstituted 3-10-membered ring with the R^{L111}, R^{L112}, R^{L113}, R^{L114}, R^{L115}, R^{L116}, R^{L117}, R^{L118}, R^{L119}, R^{L120}, R^{L121}, R^{L122}, R^{L123}, R^{L124}, R^{L125}, R^{L126}, R^{L127}, R^{L128}, R^{L129}, R^{L130}, R^{L131}, and R^{L132} through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, - R¹³OCOR¹⁴-, -R¹⁵COOR¹⁶-, -R¹⁷NR¹⁸COR¹⁹-, -R²⁰CONR²¹R²²-, -R²³SO₂R²⁴-, -R²⁵OSO₂R²⁶-, - R²⁷SO₂OR²⁸-, -R²⁹NR³⁰SO₂R³¹-, -R³²SO₂NR³³R³⁴-, or 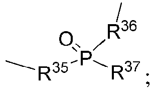 any two radicals among the R^{L111}, R^{L112}, R^{L113}, R^{L114}, R^{L115}, R^{L116}, R^{L117}, R^{L118}, R^{L119}, and R^{L120} can form a substituted or unsubstituted 3-12-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, -R²⁰CONR²¹R²²-, -R²³SO₂R²⁴-, - R²⁵OSO₂R²⁶-, -R²⁷SO₂OR²⁸-, -R²⁹NR³⁰SO₂R³¹-, -R³²SO₂NR³³R³⁴-, or 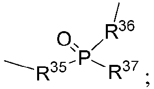 any two radicals among the R^{L121}, R^{L122}, R^{L123}, R^{L124}, R^{L125}, and R^{L126} can form a substituted or unsubstituted 3-10-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, -R²⁰CONR²¹R²²-, -R²³SO₂R²⁴-, - R²⁵OSO₂R²⁶-, -R²⁷SO₂OR²⁸-, -R²⁹NR³⁰SO₂R³¹-, -R³²SO₂NR³³R³⁴-, or 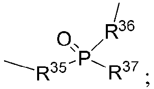 any two radicals among the R^{L127}, R^{L128}, R^{L129}, R^{L130}, R^{L131}, and R^{L132} can form a substituted or unsubstituted 3-10-membered ring through C, N, O, or S; and the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, -R²⁰CONR²¹R²²-, -R²³SO₂R²⁴-, - R²⁵OSO₂R²⁶-, -R²⁷SO₂OR²⁸-, -R²⁹NR³⁰SO₂R³¹-, -R³²SO₂NR³³R³⁴-, or 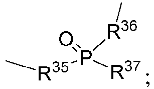
ring B is selected from N.A., substituted or unsubstituted C3-C12 cycloalkyl, substituted or unsubstituted C3-C12 heterocyclyl, substituted or unsubstituted C3-C12 bridged heterocyclyl, substituted or unsubstituted C3-C12 heterospirocyclyl, substituted or unsubstituted 6-10-membered aromatic ring, or substituted or unsubstituted 5-10-membered heteroaromatic ring; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, - R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴- 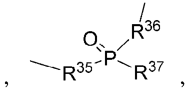 4-10-membered heterocyclyl, C5-C12 bridged heterocyclyl, C3-C10 heterospirocyclyl, 6-10-membered aromatic ring, or 5-10-membered heteroaromatic ring; and the heterocyclyl, the bridged heterocyclyl, the heterospirocyclyl, and the heteroaromatic ring each comprise 1-4 heteroatoms selected from oxygen, sulfur, and nitrogen;
L2 is selected from N.A., 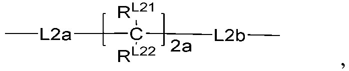 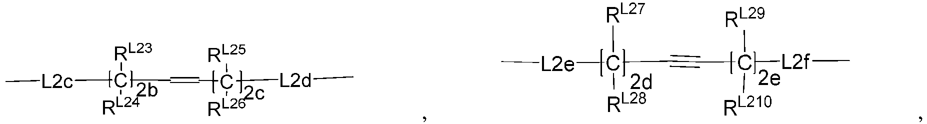 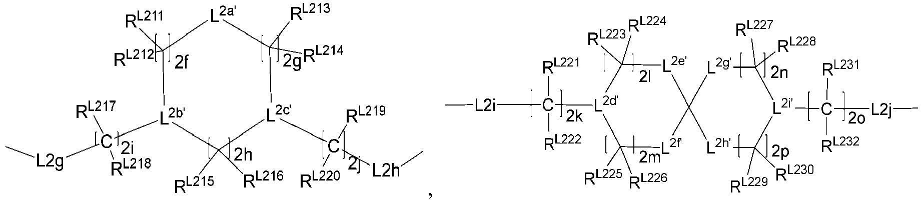 , or 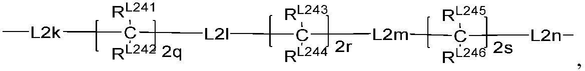
2a, 2b, 2c, 2d, 2e, 2f, 2g, 2h, 2i, 2j, 2k, 2l, 2m, 2n, 2o, 2p, 2q, 2r, and 2s are each independently selected from 0-6;
L2a, L2b, L2c, L2d, L2e, L2f, L2g, L2h, L2i, L2j, L2k, L2l, L2m, L2n, L^{2a'}, L^{2e'}, L^{2f'}, L^{2g'}, and L^{2h'} are independently selected from N.A., O, S, NR³⁹, COR⁴⁰, or -SO₂R⁴¹;
L^{2b'}, L^{2c'}, L^{2d'}, and L^{2i'} are selected from N or CR⁴⁴;
R³⁹, R⁴⁰, R⁴¹, R⁴⁴, R^{L21}, R^{L22}, R^{L23}, R^{L24}, R^{L25}, R^{L26}, R^{L27}, R^{L28}, R^{L29}, R^{L210}, R^{L21}, R^{L22}, R^{L23}, R^{L24}, R^{L25}, R^{L26}, R^{L27}, R^{L28}, R^{L29}, R^{L210}, R^{L211}, R^{L212}, R^{L213}, R^{L214}, R^{L223}, R^{L224}, R^{L225}, R^{L226}, R^{L227}, R^{L228}, R^{L229}, R^{L230}, R^{L241}, R^{L242}, R^{L243}, R^{L244}, R^{L245}, and R^{L246} are each independently selected from hydrogen, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, -R¹¹COR¹²-, - R¹³OCOR¹⁴-, -R¹⁵COOR¹⁶-, -R¹⁷NR¹⁸COR¹⁹-, -R²⁰CONR²¹R²²-, -R²³SO₂R²⁴-, -R²⁵OSO₂R²⁶-, - R²⁷SO₂OR²⁸-, -R²⁹NR³⁰SO₂R³¹-, -R³²SO₂NR³³R³⁴-, or 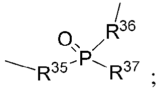 the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, or 5-10-membered heteroaromatic ring; the heterocyclyl, the bridged heterocyclyl, heterospirocyclyl, and the heteroaromatic ring each comprise 1-4 heteroatoms selected from oxygen, sulfur, and nitrogen; the R³⁹ can form a substituted or unsubstituted 3-10-membered ring with the R^{L21}, R^{L22}, R^{L23}, R^{L24}, R^{L25}, R^{L26}, R^{L27}, R^{L28}, R^{L29}, R^{L210}, R^{L211}, R^{L212}, R^{L213}, R^{L214}, R^{L223}, R^{L224}, R^{L225}, R^{L226}, R^{L227}, R^{L228}, R^{L229}, and R^{L230} respectively through C, N, O, and S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, - R¹⁷NR¹⁸COR¹⁹-, -R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴-, or 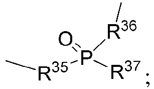 any two radicals among the R^{L211}, R^{L212}, R^{L213}, R^{L214}, R^{L215}, R^{L216}, R^{L217}, R^{L218}, R^{L219}, and R^{L220} can form a substituted or unsubstituted 3-12-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, - R¹⁵COOR¹⁶-, -R¹⁷NR¹⁸COR¹⁹-, -R²⁰CONR²¹R²²-, -R²³SO₂R²⁴-, -R²⁵OSO₂R²⁶-, -R²⁷SO₂OR²⁸-, - R²⁹NR³⁰SO2R³¹-, -R³²SO₂NR³³R³⁴-, or 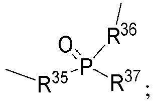 any two radicals among the R^{L221}, R^{L222}, R^{L223}, R^{L224}, R^{L225}, and R^{L226} can form a substituted or unsubstituted 3-10-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, - R¹⁵COOR¹⁶-, -R¹⁷NR¹⁸COR¹⁹-, -R²⁰CONR²¹R²²-, -R²³SO₂R²⁴-, -R²⁵OSO₂R²⁶-, -R²⁷SO₂OR²⁸-, - R²⁹NR³⁰SO2R³¹-, -R³²SO₂NR³³R³⁴-, or 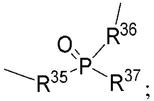 any two radicals among the R^{L227}, R^{L228} R^{L229}, R^{L230}, R^{L231}, and R^{L232} can form a substituted or unsubstituted 3-10-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, - R¹⁵COOR¹⁶-, -R¹⁷NR¹⁸COR¹⁹-, -R²⁰CONR²¹R²²-, -R²³SO₂R²⁴-, -R²⁵OSO₂R²⁶-, -R²⁷SO₂PR²⁸-, - R²⁹NR³⁰SO2R³¹-, -R³²SO₂NR³³R³⁴-, or 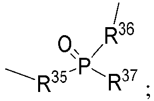 or the R⁴⁴ can form a substituted or unsubstituted 3-10-membered ring with the R^{L211}, R^{L212}, R^{L213}, R^{L214}, R^{L223}, R^{L224}, R^{L225}, R^{L226}, R^{L227}, R^{L228}, R^{L229}, R^{L230}, R^{L231}, and R^{L232} through C, N, O, or S; and the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, - R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴-, or 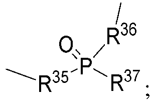
ring C is selected from N.A., substituted or unsubstituted C3-C12 cycloalkyl, substituted or unsubstituted C3-C12 heterocyclyl, substituted or unsubstituted C3-C12 bridged heterocyclyl, substituted or unsubstituted C3-C12 heterospirocyclyl, substituted or unsubstituted 6-10-membered aromatic ring, or substituted or unsubstituted 5-10-membered heteroaromatic ring; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, C1-C6 linear or branched haloalkyl, C2-C6 linear or branched unsaturated halohydrocarbon radical, C1-C6 linear or branched haloalkoxy, 3-10-membered cycloalkoxy, C1-C6 linear or branched alkylamino, 3-10-membered cycloalkylamino, -R¹¹COR¹²-, -R¹³OCOR¹⁴-, - R¹⁵COOR¹⁶-, -R¹⁷NR¹⁸COR¹⁹-, -R²⁰CONR²¹R²²-, -R²³SO₂R²⁴-, -R²⁵OSO₂R²⁶-, -R²⁷SO₂OR²⁸-, - R²⁹NR³⁰SO2R³¹-, -R³²SO₂NR³³R³⁴-, 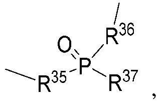 4-10-membered heterocyclyl, C5-C12 bridged heterocyclyl, C3-C10 heterospirocyclyl, 6-10-membered aromatic ring, or 5-10-membered heteroaromatic ring; and the heterocyclyl, the bridged heterocyclyl, the heterospirocyclyl, and the heteroaromatic ring each comprise 1-4 heteroatoms selected from oxygen, sulfur, and nitrogen; 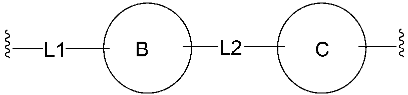 is selected from: 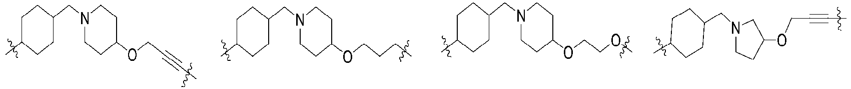 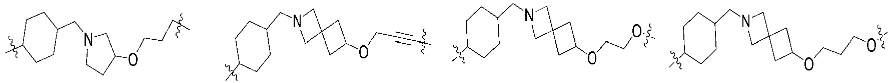 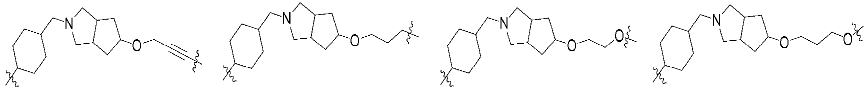 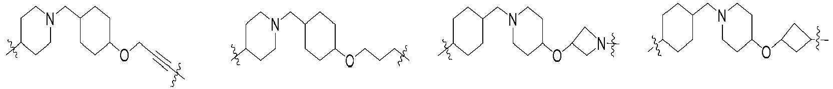 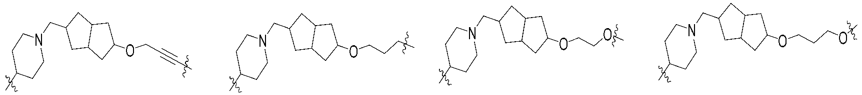 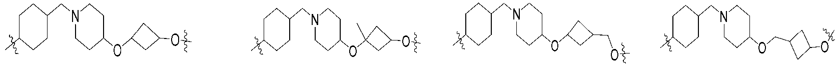 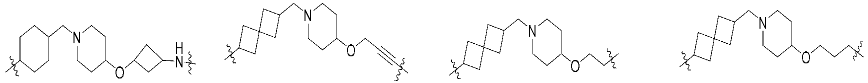 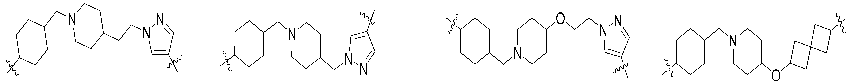 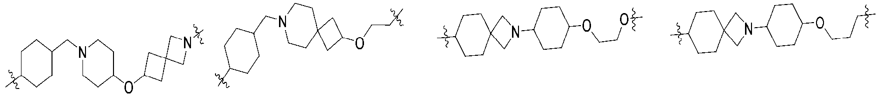 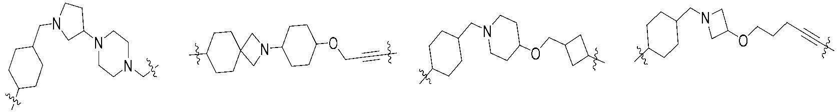 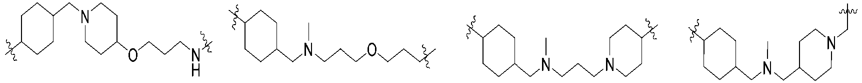 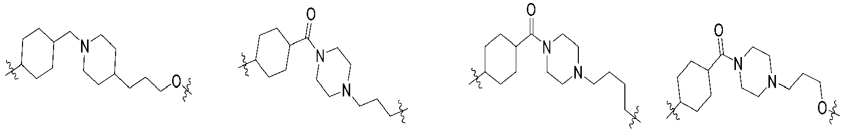 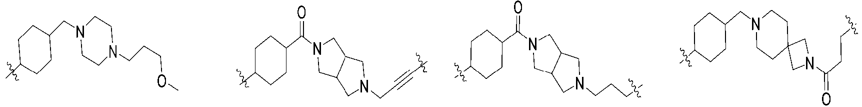 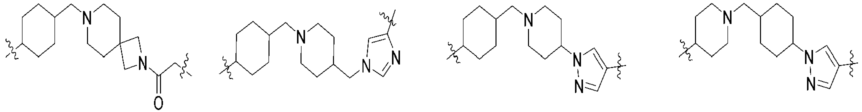 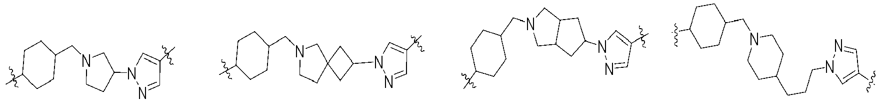 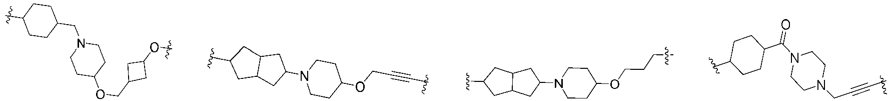 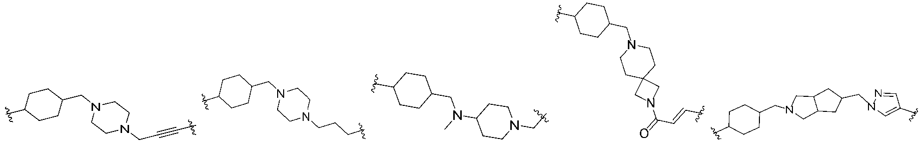 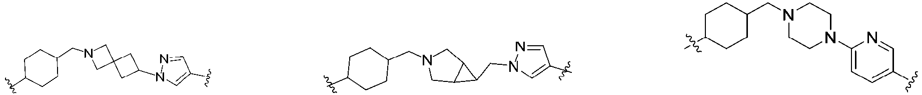 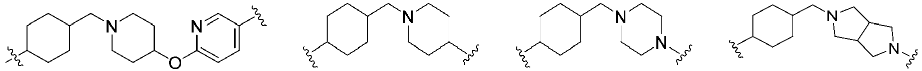 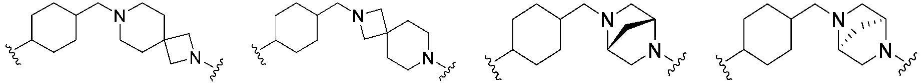 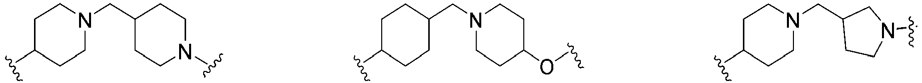 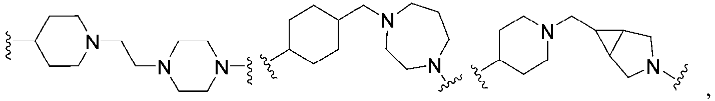 a single cis-trans isomer thereof or a mixture comprising the same;
for example, 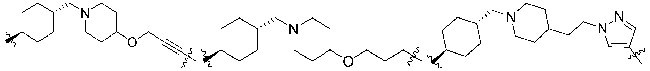 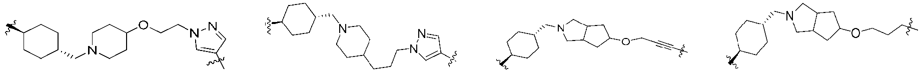 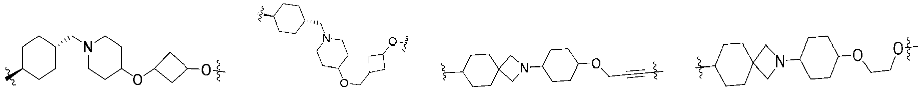 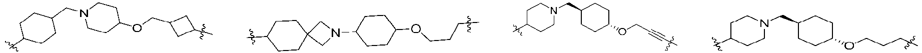 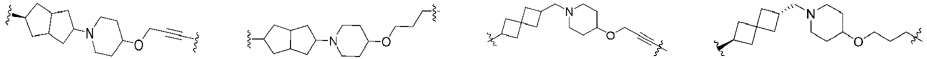 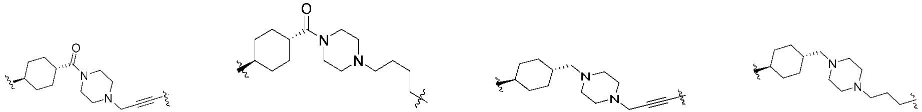 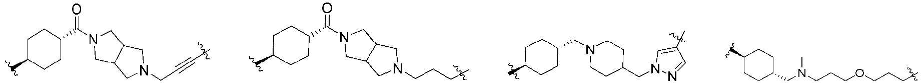 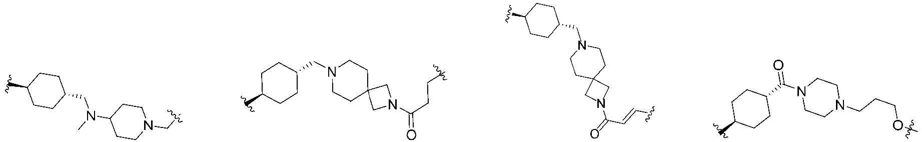 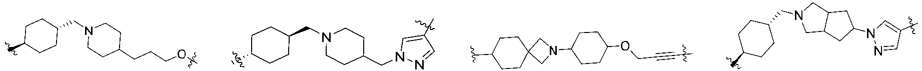 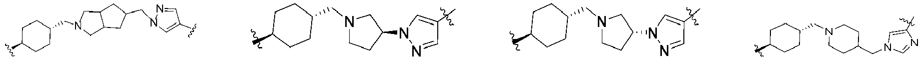 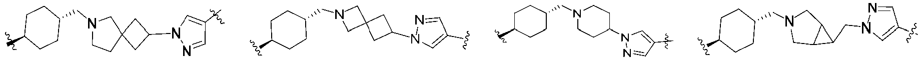 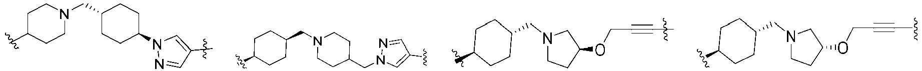 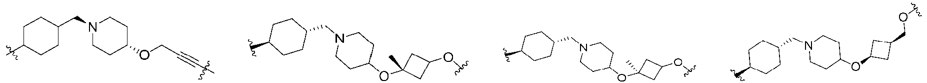 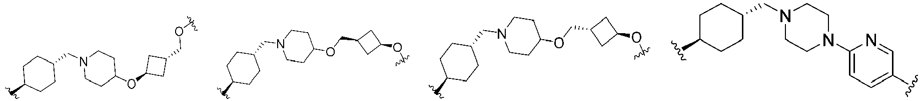 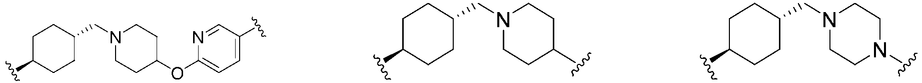 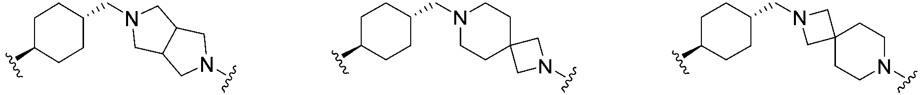 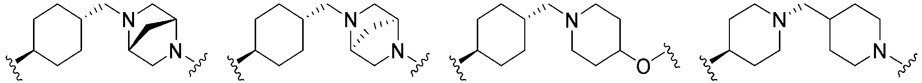 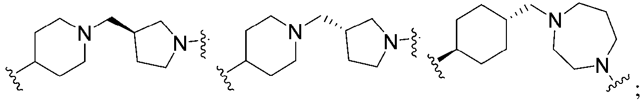
each of the halogen is independently fluorine, chlorine, bromine, or iodine, for example, fluorine or chlorine;
C1-C6 alkyl among each of the substituted or unsubstituted C1-C6 linear or branched alkyl is independently methyl, ethyl, n-propyl, isopropyl, n-butyl, isobutyl, tert-butyl, sec-butyl, n-pentyl, isopentyl, neopentyl, and hexyl; for example, methyl, ethyl, or propyl;
3-10-membered cycloalkyl among each of the substituted or unsubstituted 3-10-membered cycloalkyl is independently cyclopropyl, cyclobutyl, cyclopentyl, cyclobutyl, 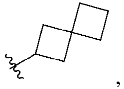 or 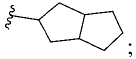
4-10-membered heterocyclyl among each of the substituted or unsubstituted 4-10-membered heterocyclyl is independently 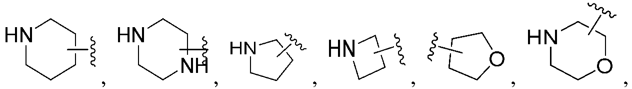 or 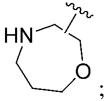 for example, 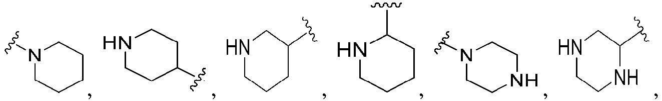 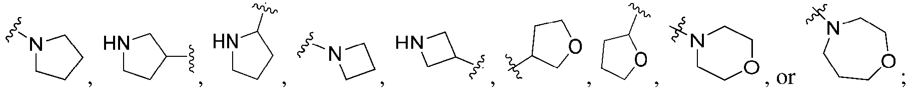
C3-C10 heterospirocyclyl among each of the substituted or unsubstituted C3-C10 heterospirocyclyl is independently 2-azaspirocyclo[3.3]heptyl, 7-azaspirocyclo[3.5]nonyl, 2-azaspirocyclo[3.5]nonyl, 2,7-diazaspirocyclo[3.5]nonyl, 6-azaspirocyclo[3.4]octyl, 4-oxa-7-azaspirocyclo[2.5]octyl, 5-oxa-8-azaspirocyclo[3.5]nonyl, 2-oxa-6-azaspirocyclo[3.3]heptyl, 2-oxa-6-azaspirocyclo[3.4]octyl, or 4,7-diazaspirocyclo[2.5]octyl; for example, 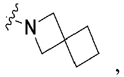 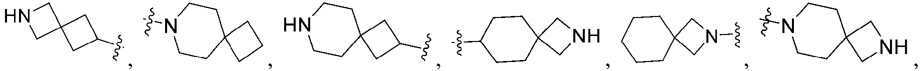 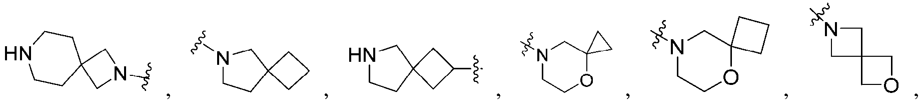 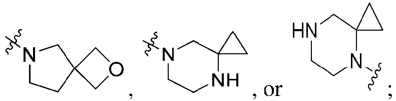
C5-C12 bridged heterocyclyl among each of the substituted or unsubstituted C5-C12 bridged heterocyclyl is independently octahydrocyclopenta[C]pyrrolyl, octahydropyrrolo[3,4-c]pyrrolyl, 3-azabicyclo[3.1.0]hexyl, 2-oxa-5-azabicyclo[2.2.1]heptyl, or 8-oxa-3-azabicyclo[3.2.1]octyl; for example, 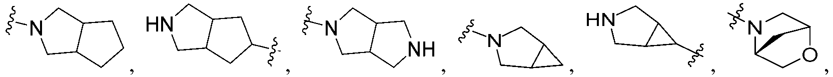 or 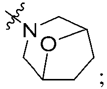 and
5-10-membered heteroaromatic ring among each of the substituted or unsubstituted 5-10-membered heteroaromatic ring is independently

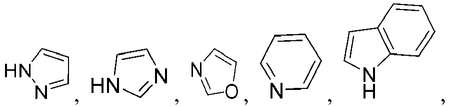

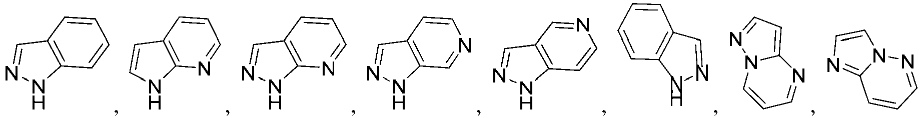

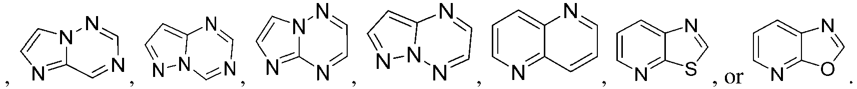

In some embodiments of the present invention, the ring D is selected from 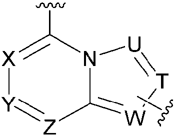 or

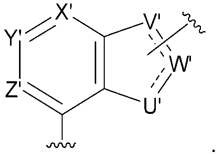

In some embodiments of the present invention, the compound of formula I is as shown in formula Ia: 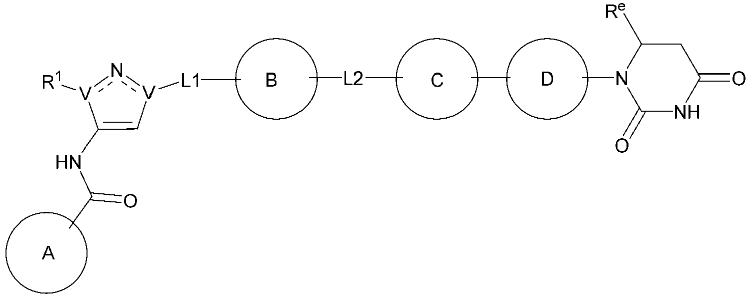 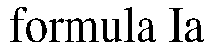 wherein
the ring D is selected from 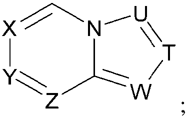
U is selected from N, W is selected from C or CR^{W}, and T is selected from C or CR^{T};
or the W is selected from N, the U is selected from C or CR^{U}, and the T is selected from C or CR^{T};
at most one of X, Y, and Z is N;
R¹ is selected from hydrogen, deuterium, halogen, cyano, or substituted or unsubstituted C1-C6 linear or branched alkyl; wherein the substituents are independently selected from deuterium, halogen, hydroxyl, C1-C6 linear or branched alkoxy, or 3-10-membered cycloalkoxy; and a number of the substituents is 1, 2, or 3; for example, the R¹ is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, or cyclopropoxymethyl; 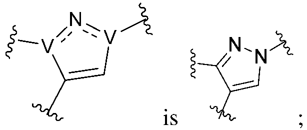
the R^{U}, R^{W}, R^{T}, R^{X}, R^{Y}, R^{Z}, and R^{e} are each independently selected from hydrogen, halogen, cyano, substituted or unsubstituted C1-C6 linear or branched alkyl, or substituted or unsubstituted C1-C6 linear or branched alkoxy; for example, H, F, or methyl;
the ring A is independently selected from rings below: 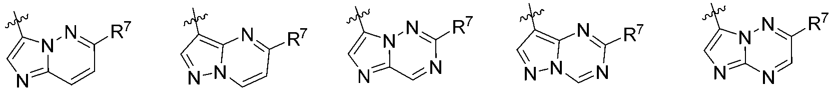 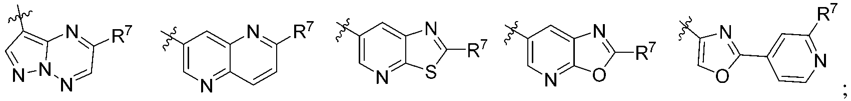
the R⁷ is selected from:

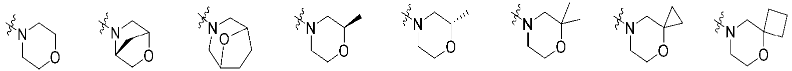

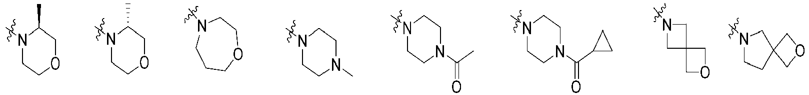

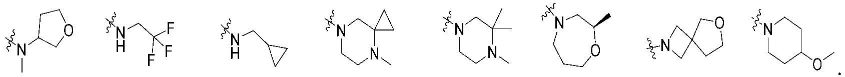

In some preferred embodiments of the present invention:
the ring D is selected from: 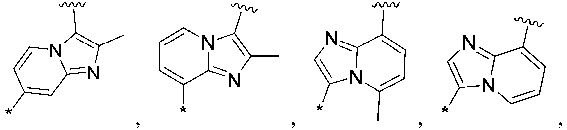 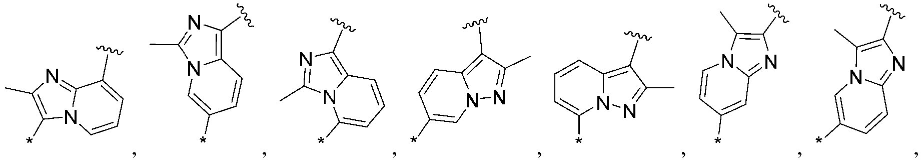 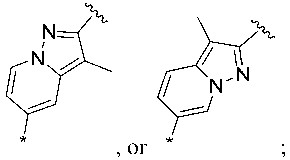 wherein * terminuses are each linked to the ring C; 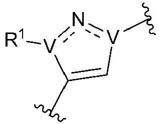 is 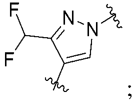
the ring A is selected from: 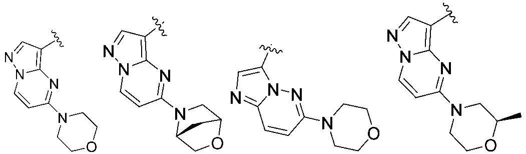 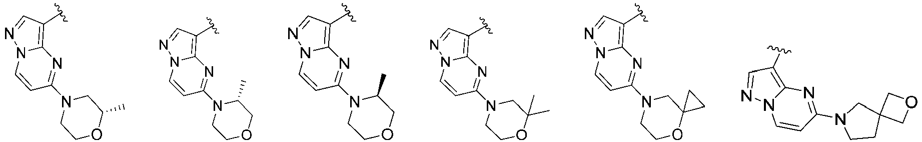 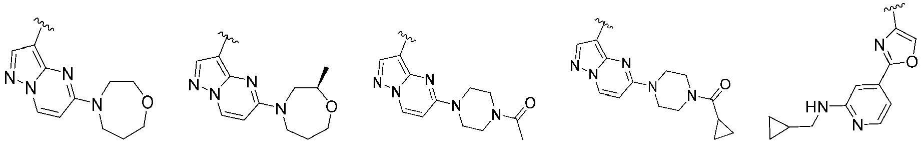 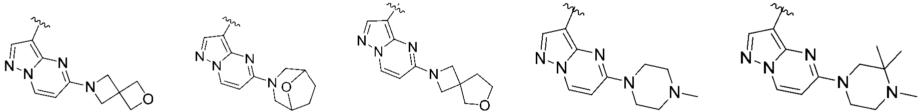 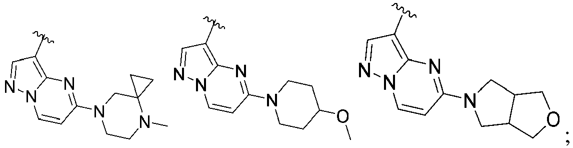 and
the R^{e} is hydrogen or methyl.

Further, the compound represented by formula Ia is as shown below: 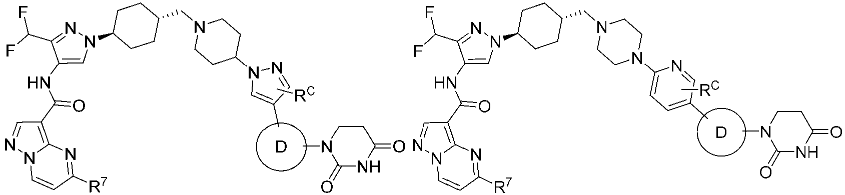 or 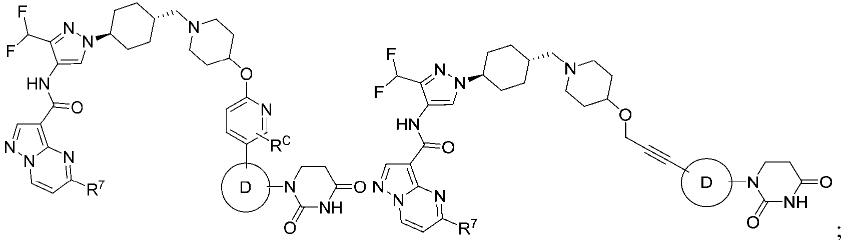 wherein
the R⁷ is selected from 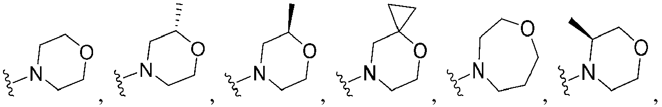 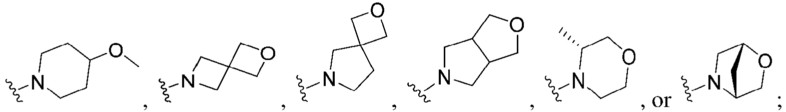 and
the R^{C} is selected from hydrogen, deuterium, halogen, cyano, hydroxyl, amino, C1-C6 linear or branched alkyl, C2-C6 linear or branched unsaturated hydrocarbon radical, C1-C6 linear or branched alkoxy, 3-10 membered cycloalkoxy, or C1-C6 linear or branched alkylamino.

In some specific embodiments of the present invention, the compound is selected from structures below:

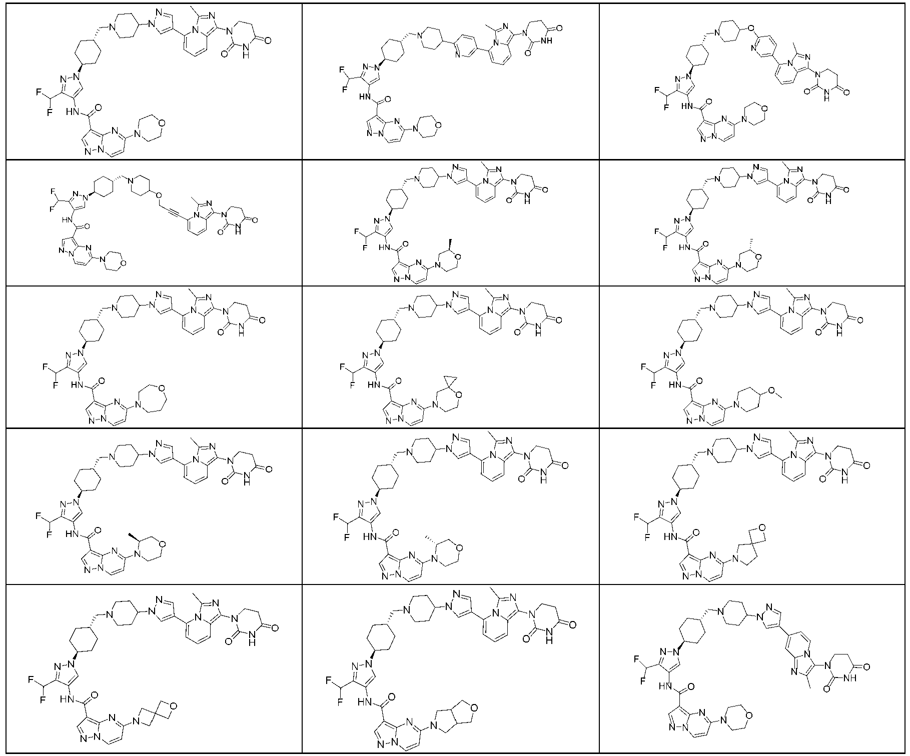

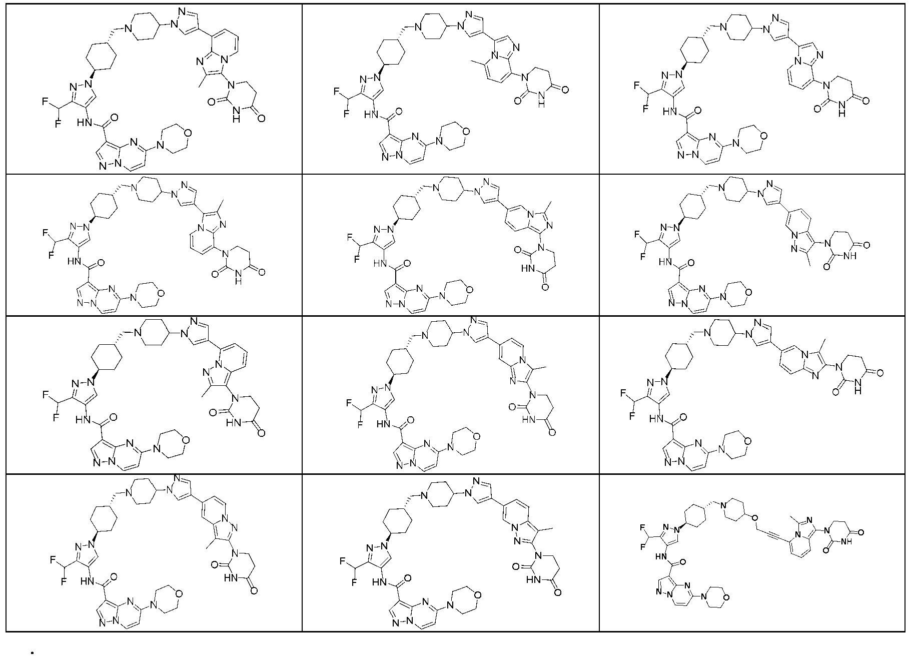

In some preferred embodiments of the present invention, the compound of formula I is as shown in formula Ib or formula Ic: 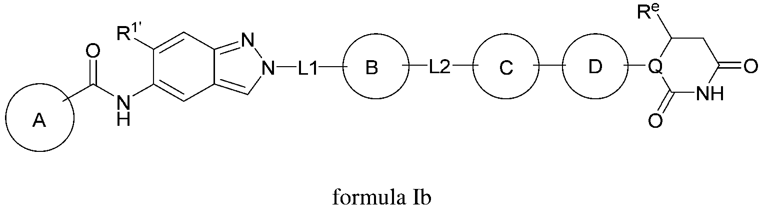 or 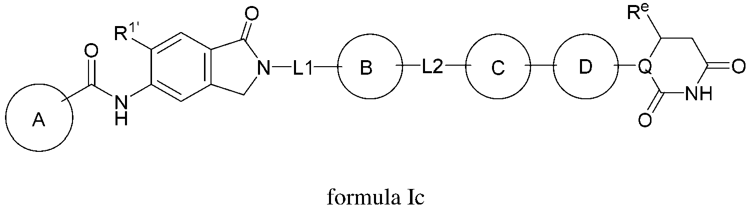 wherein
the ring D is selected from 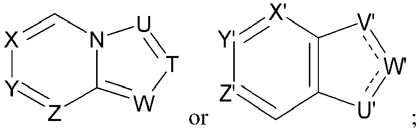
Q is selected from CR^{2a} or N;
U is selected from N, W is selected from C or CR^{W}, and T is selected from C or CR^{T};
or the W is selected from N, the U is selected from C or CR^{U}, and the T is selected from C or CR^{T};
at most one of X, Y, and Z is N;
V' is selected from N or C, U' is selected from CR² or NR², and W' is selected from CR³ or N;
X' is selected from CR⁴ or N; Y' is selected from CR⁵ or N; Z' is selected from C, CR⁶, or N; and at most one of X', Y', and Z' is N;
R^{1'} is selected from hydrogen, deuterium, halogen, cyano, substituted or unsubstituted C1-C6 linear or branched alkyl, substituted or unsubstituted C1-C6 linear or branched alkoxy, substituted or unsubstituted 4-10-membered heterocyclyl, substituted or unsubstituted C5-C12 bridged heterocyclyl, substituted or unsubstituted C3-C10 heterospirocyclyl, or substituted or unsubstituted C1-C6 linear or branched alkylamino; wherein the substutuents are independently selected from deuterium, halogen, hydroxyl, C1-C6 linear or branched alkyl, C1-C6 linear or branched alkoxy, 3-10-membered cycloalkoxy, 4-10-membered heterocyclyl, 3-10-membered cycloalkyl, C1-C6 linear or branched alkyl-C(=O)-, or 3-10-membered cycloalkyl-C(=O)-; and a number of the substituents is 1, 2, or 3;
for example, the R^{1'} is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, or cyclopropoxymethyl; and for another example, the R^{1'} is selected from: 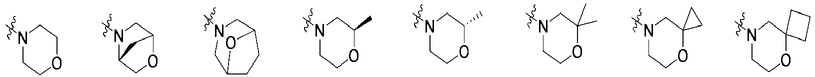 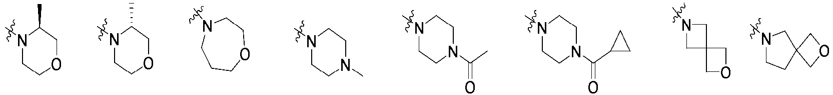 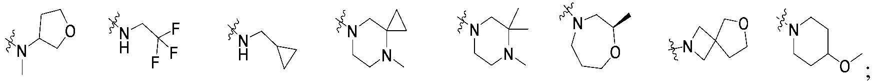
the R^{2a}, R², R³, R⁴, R⁵, R⁶, R^{U}, R^{W}, R^{T}, R^{X}, R^{Y}, R^{Z}, and R^{e} are each independently selected from hydrogen, halogen, cyano, substituted or unsubstituted C1-C6 linear or branched alkyl, substituted or unsubstituted C1-C6 linear or branched alkoxy, or substituted or unsubstituted 3-10-membered cycloalkyl; for example, H, F, Cl, cyclopropyl, cyclobutyl, ethyl, n-propyl, isopropyl, monofluoromethyl, difluoromethyl, trifluoromethyl, cyano, methoxy, ethoxy, or methyl;
the ring A is independently selected from rings below: 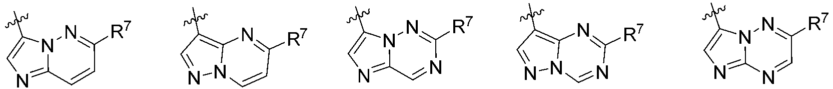 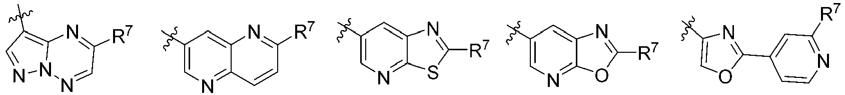 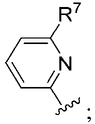
the R⁷ is selected from hydrogen, deuterium, halogen, cyano, substituted or unsubstituted C1-C6 linear or branched alkyl, substituted or unsubstituted C1-C6 linear or branched alkoxy, substituted or unsubstituted 4-10-membered heterocyclyl, substituted or unsubstituted C5-C12 bridged heterocyclyl, substituted or unsubstituted C3-C10 heterospirocyclyl, or substituted or unsubstituted C1-C6 linear or branched alkylamino; wherein the substutuents are independently selected from deuterium, halogen, hydroxyl, C1-C6 linear or branched alkyl, C1-C6 linear or branched alkoxy, 3-10-membered cycloalkoxy, 4-10-membered heterocyclyl, 3-10-membered cycloalkyl, C1-C6 linear or branched alkyl-C(=O)-, or 3-10-membered cycloalkyl-C(=O)-; and a number of the substituents is 1, 2, or 3;
for example, the R⁷ is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, or cyclopropoxymethyl; and for another example, the R⁷ is selected from:

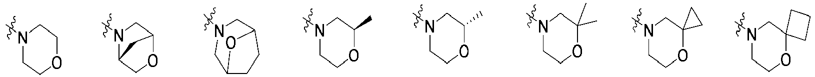

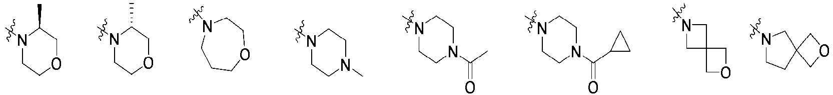

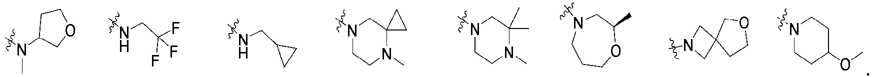

In some preferred embodiments, the ring D is selected from 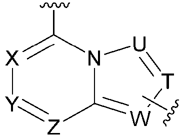 or

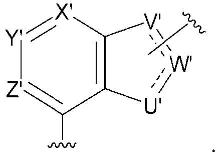

In some preferred embodiments of the present invention:
the ring D is selected from: 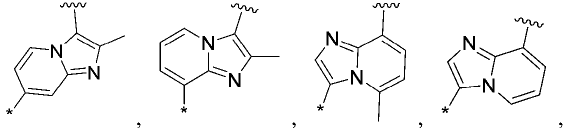 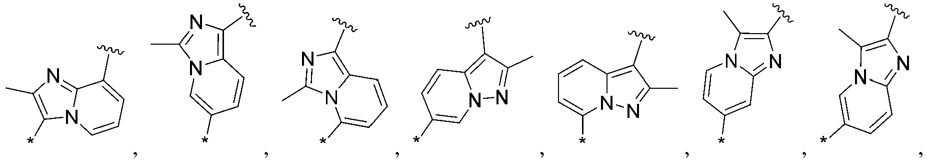 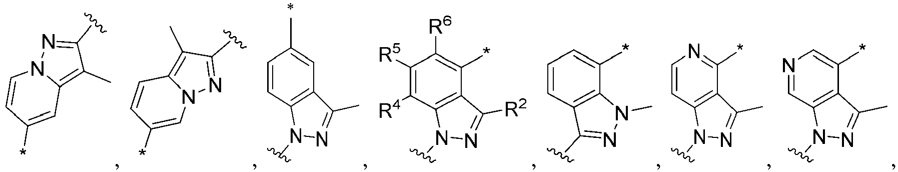 or 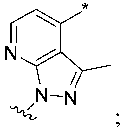 wherein * terminuses are each linked to the ring C; wherein the R², R⁴, R⁵, and R⁶ are each independently selected from hydrogen, halogen, cyano, substituted or unsubstituted C1-C6 linear or branched alkyl, substituted or unsubstituted C1-C6 linear or branched alkoxy, or substituted or unsubstituted 3-10-membered cycloalkyl; and preferably, the R², R⁴, R⁵, and R⁶ are each independently selected from H, F, Cl, cyclopropyl, cyclobutyl, ethyl, n-propyl, isopropyl, monofluoromethyl, difluoromethyl, trifluoromethyl, cyano, methoxy, ethoxy, or methyl; and
the ring A is selected from:

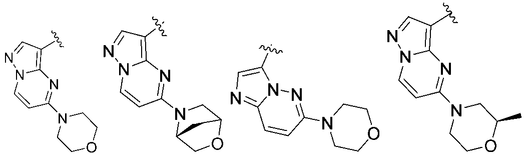

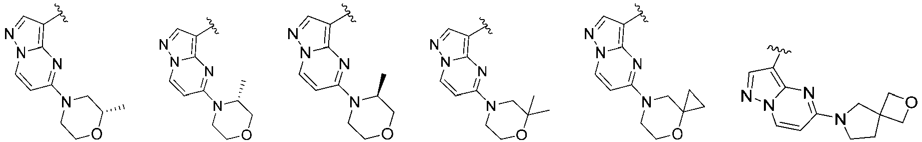

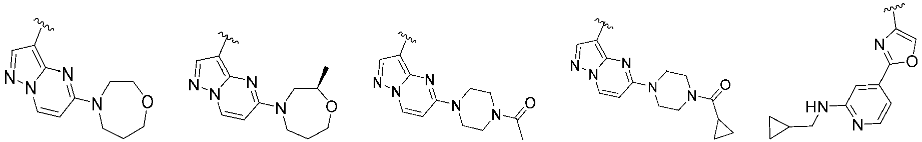

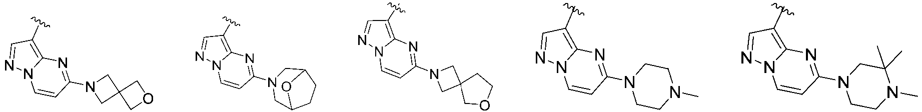

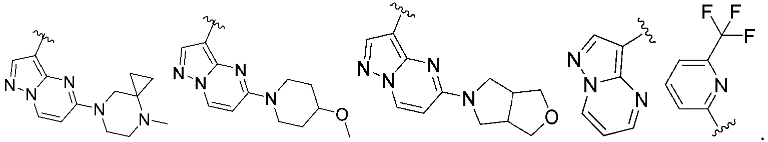

Further, the compound represented by formula Ib is as shown below: 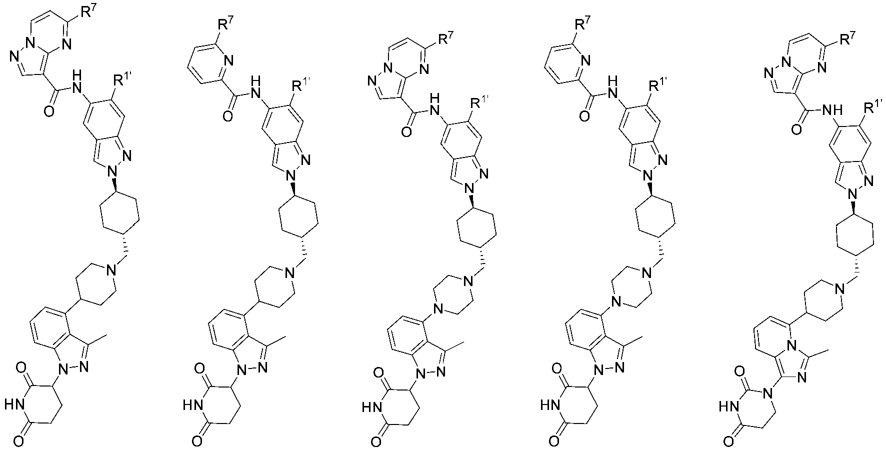 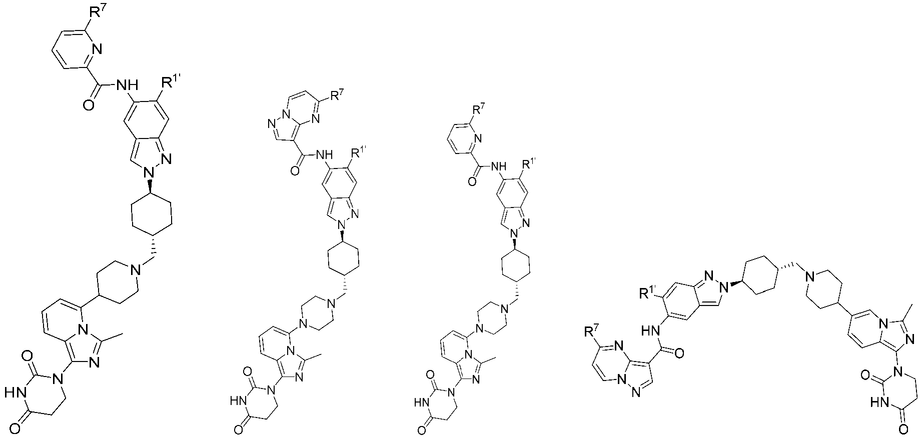 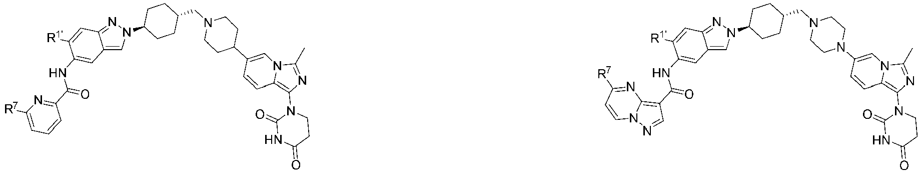 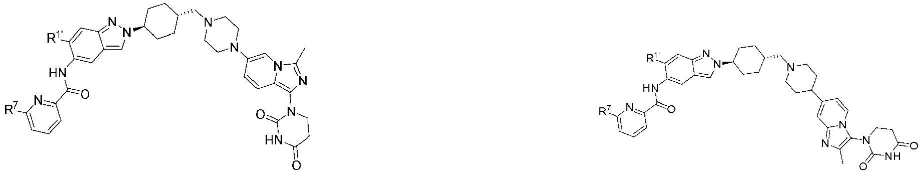 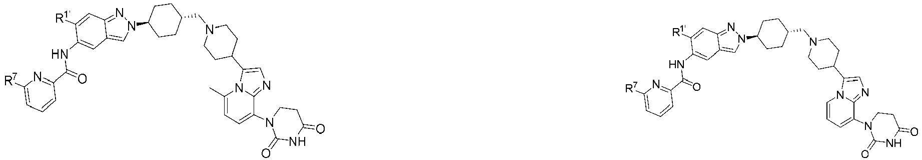 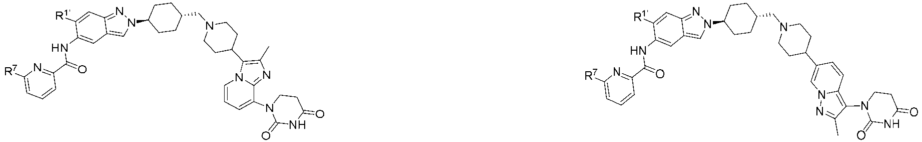 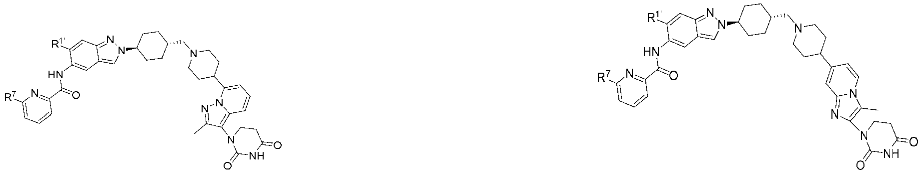 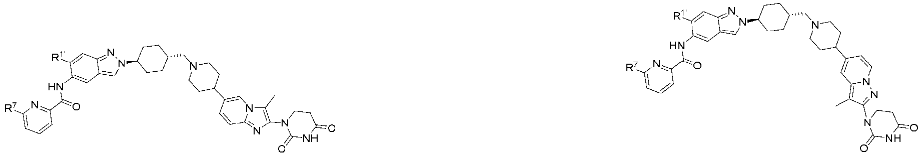 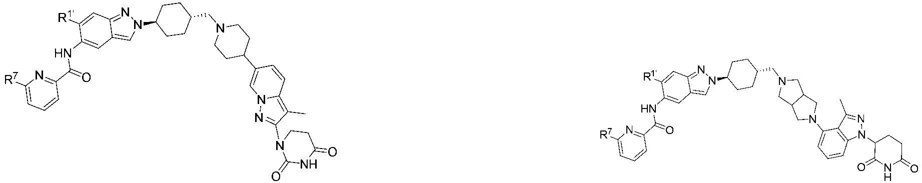 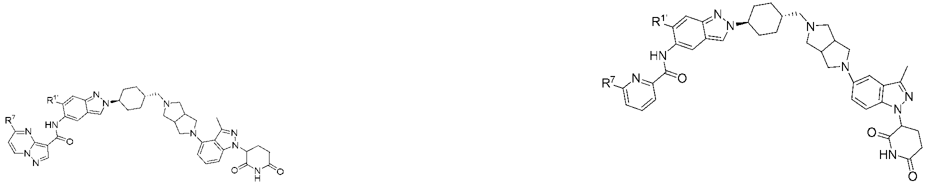 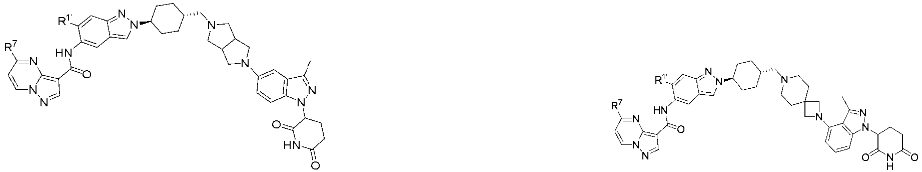 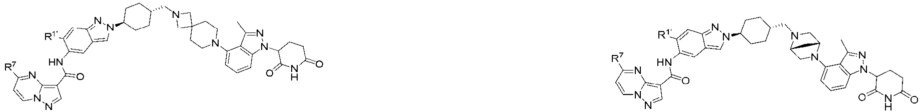 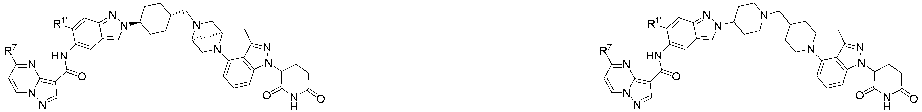 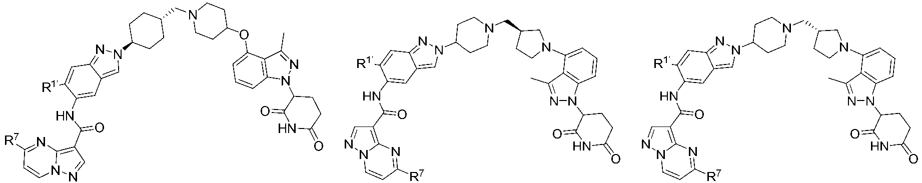 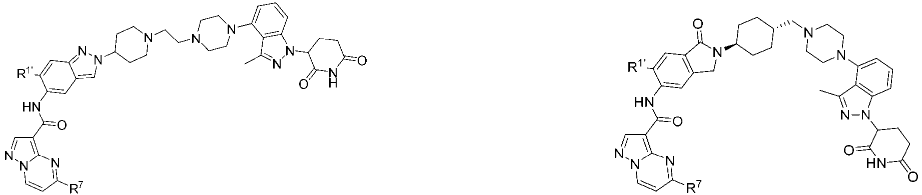 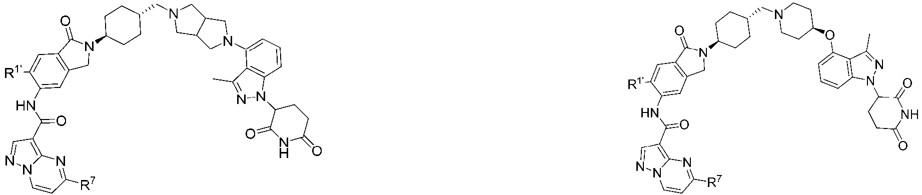 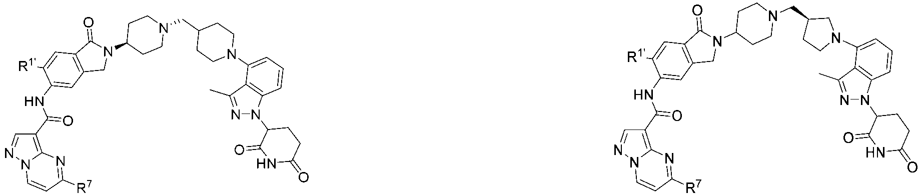 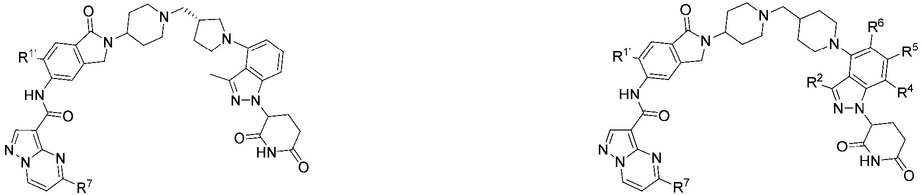 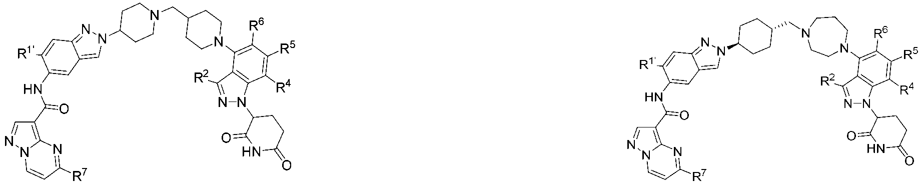 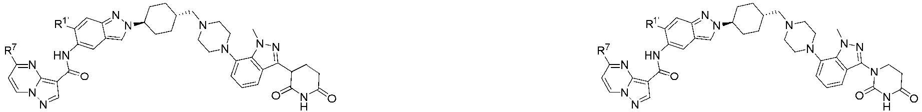 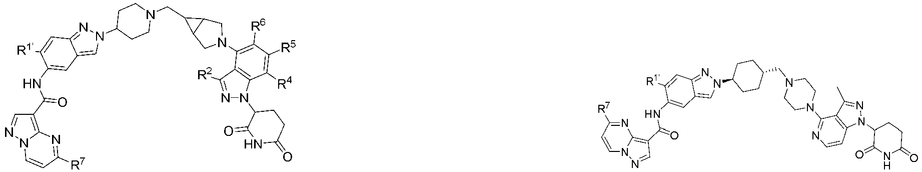 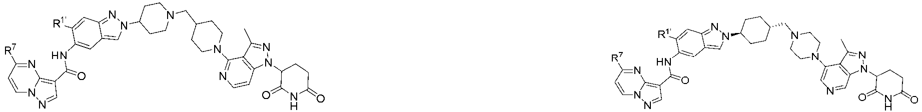 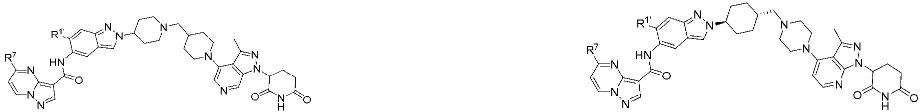 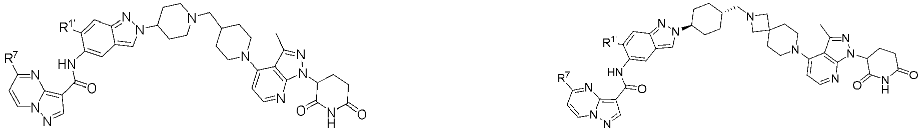 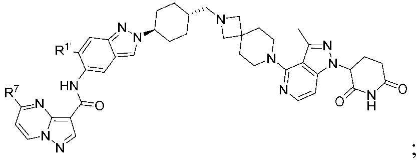 wherein
the R², R⁴, R⁵, and R⁶ are each independently selected from H, F, Cl, cyclopropyl, cyclobutyl, ethyl, n-propyl, isopropyl, monofluoromethyl, difluoromethyl, trifluoromethyl, cyano, methoxy, ethoxy, or methyl;
the R^{1'} is selected from hydrogen, deuterium, methyl, ethyl, propyl, hydroxymethyl, methoxy, ethoxy, trifluoromethyl, difluoromethyl, monofluoromethyl, methoxymethyl, amino, monomethylamino, dimethylamino, deuteromonomethylamino, deuterodimethylamino, 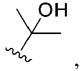 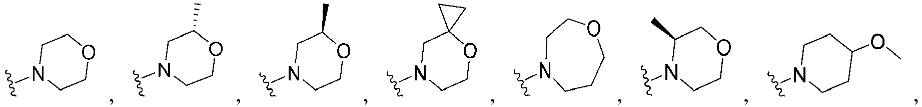 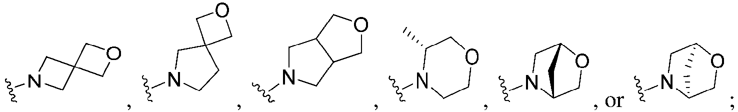 and
the R⁷ is selected from hydrogen, deuterium, methyl, ethyl, propyl, trifluoromethyl, difluoromethyl, monofluoromethyl, 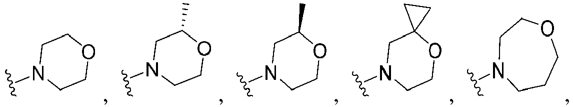 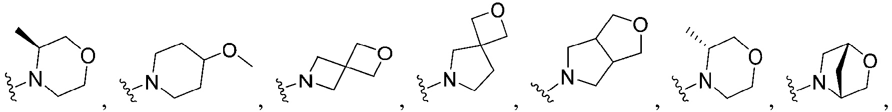 or

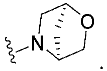

In some preferred embodiments of the present invention, the compound is selected from compounds below:

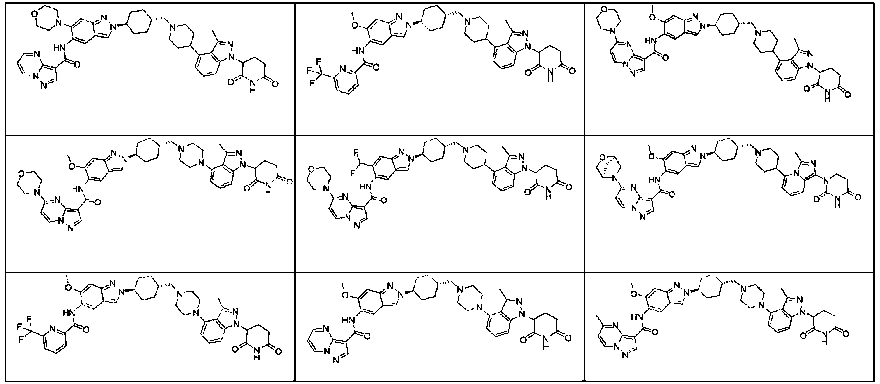

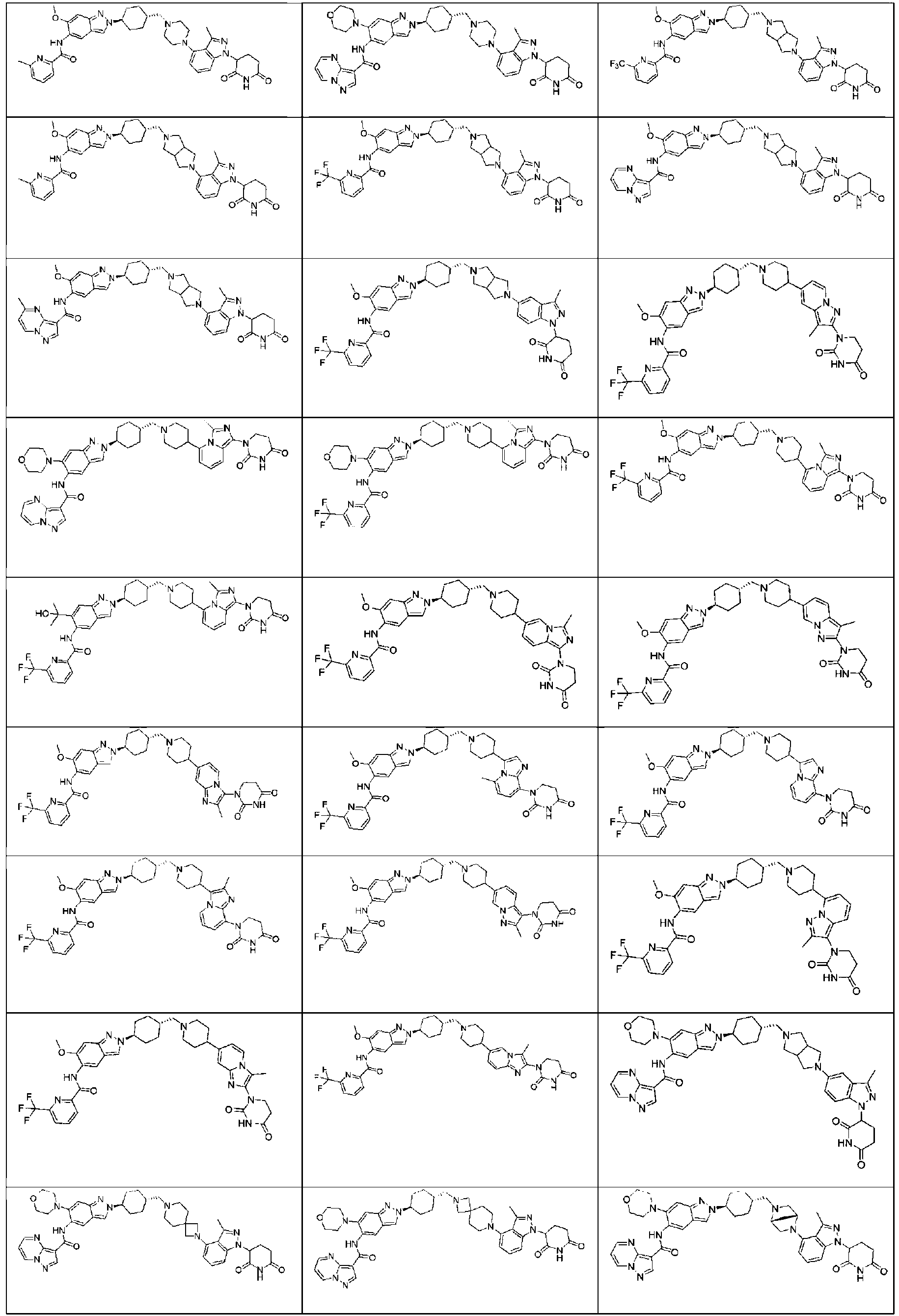

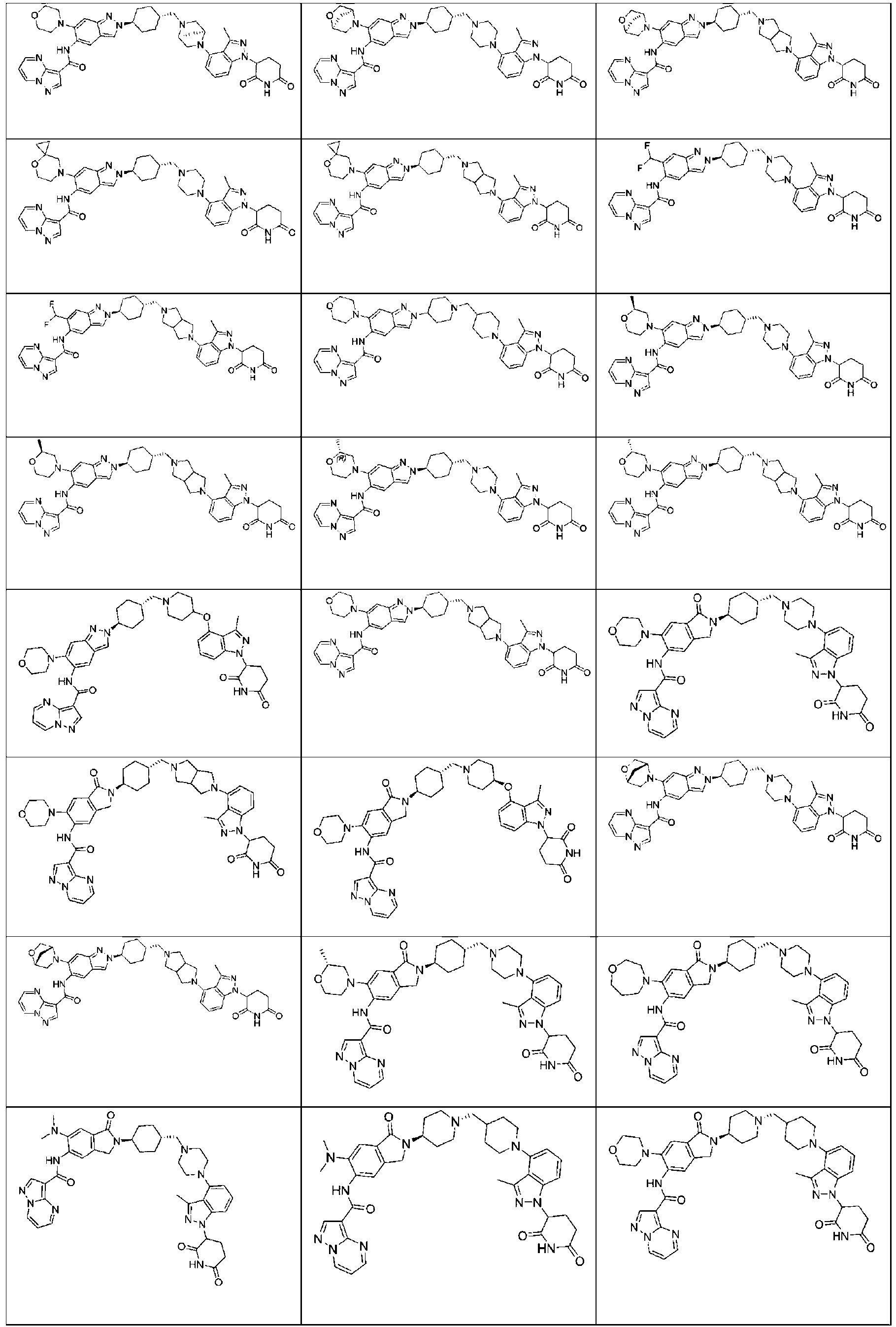

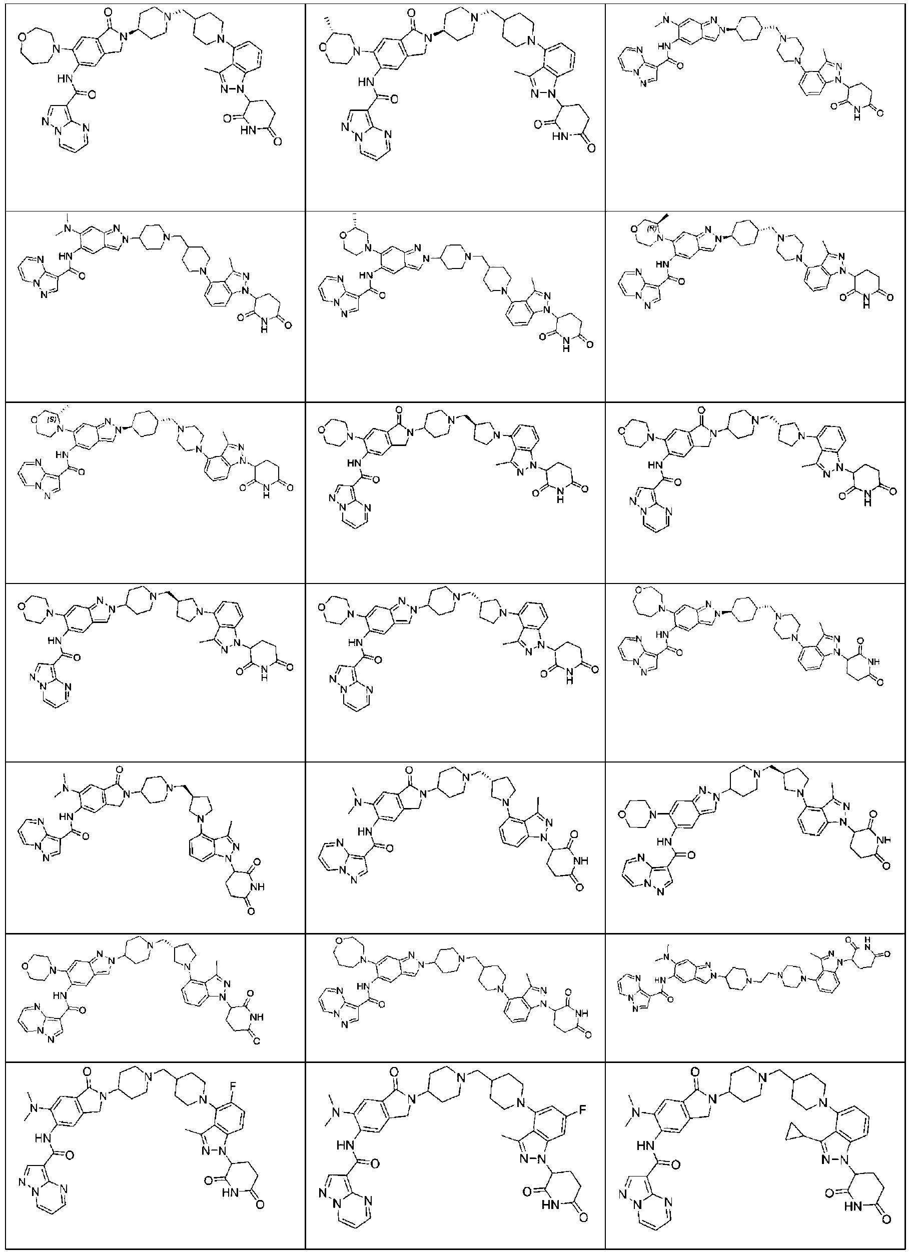

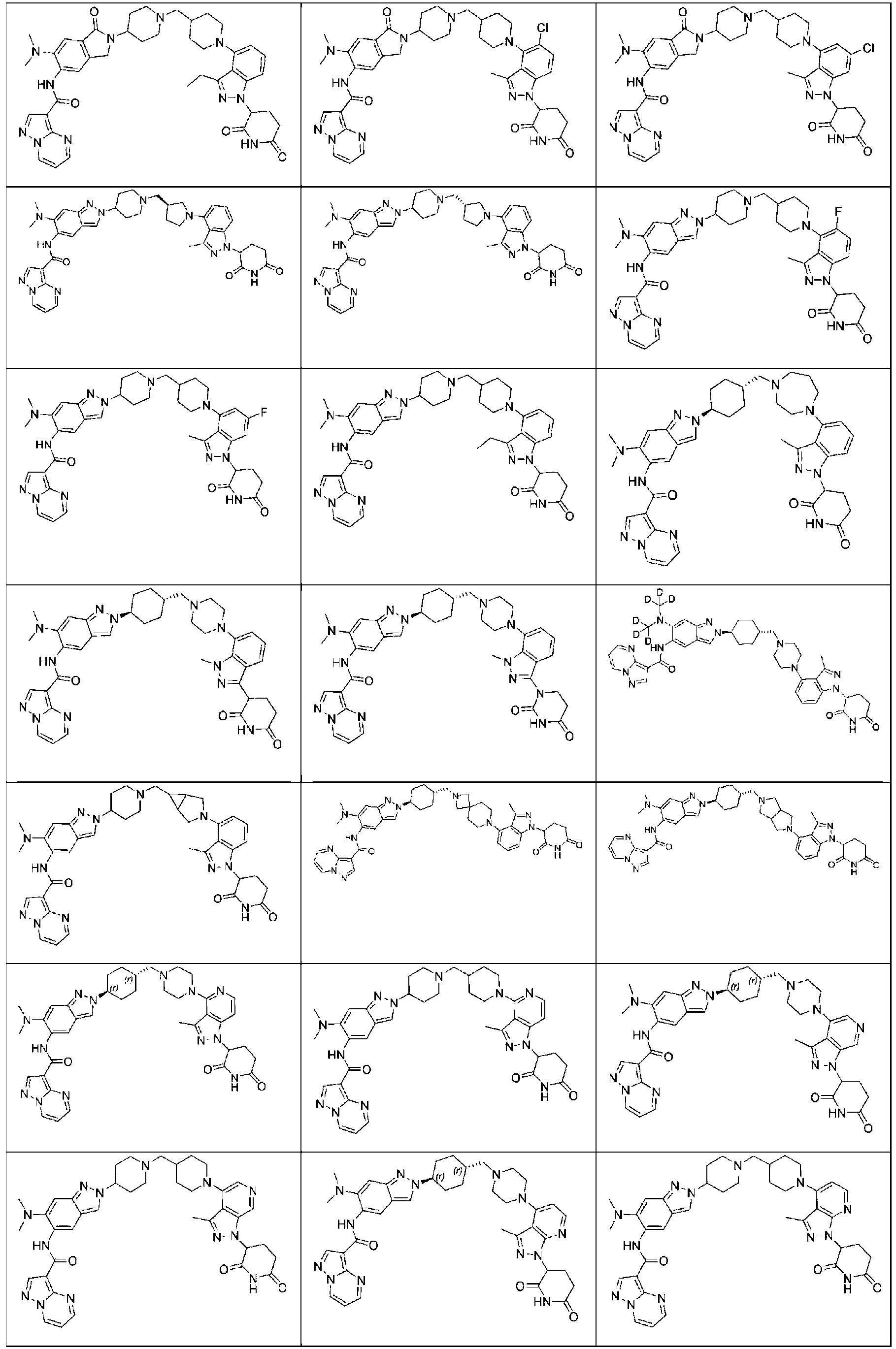

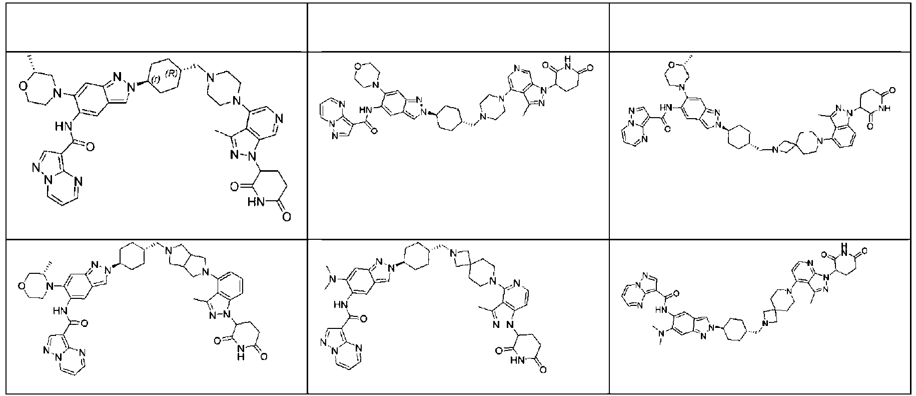

The present invention further provides use of the above compound, or the enantiomer thereof, the diastereomer thereof, the racemate thereof, the mixture comprising the same, or the pharmaceutically acceptable salt thereof for the manufacture of a medicament; the medicament being a medicament for treating and preventing one or more diseases associated with or mediated by an interleukin-1 receptor-associated kinase 4 (IRAK4) signaling pathway, an interleukin-6 (IL-6) receptor, and a tumor necrosis factor α (TNFα), or the medicament being a medicament for treating and/or preventing an autoimmune disease and/or a cancer or a proliferative disease.

Further, in the above use
the disease includes a cancer, a neurodegenerative disease, a viral disease, an autoimmune disease, an inflammatory disease, a genetic disease, a hormone-related disease, a metabolic disorder, an organ transplantation-related disease, an immunodeficiency disease, an osteoclastic disease, a proliferative disease, an infectious disease, thrombin-induced platelet aggregation, a liver disease, a lesion caused by T cell activation, and a cardiovascular disease;
the cancer or the proliferative disorder may be selected from brain cancer, kidney cancer, liver cancer, bladder cancer, breast cancer, gastric cancer, ovarian cancer, colon cancer, rectal cancer, esophageal cancer, lung cancer, prostate cancer, pancreatic cancer, vaginal cancer, cervical cancer, testicular cancer, urogenital tract cancer, laryngeal cancer, skin cancer, bone cancer, thyroid cancer, sarcoma, glioblastoma, neuroblastoma, multiple myeloma, head and neck cancer, epidermoid carcinoma, large cell carcinoma, non-small cell lung cancer, lymphoma, Hodgkin's or non-Hodgkin's lymphoma, seminoma, melanoma, leukemia, diffuse large B-cell lymphoma, ABC DLBCL, chronic lymphocytic leukemia, chronic lymphocytic lymphoma, primary exudative lymphoma, Burkitt lymphoma/leukemia, acute lymphocytic leukemia, B-cell lymphocytic leukemia, lymphoplasmacytic lymphoma, Waldenström macroglobulinemia, splenic marginal zone lymphoma, plasmacytoma or intravascular large B cell lymphoma; epidermal hyperproliferative disease, psoriasis, prostatic hyperplasia, IL-1 driven disease, and MyD88 driven disease;
the MyD88-driven disease may be selected from ABC DLBCL, Waldenström macroglobulinemia, Hodgkin's lymphoma, primary cutaneous T-cell lymphoma, and chronic lymphocytic leukemia;
the neurodegenerative disease may be selected from Alzheimer's disease, Parkinson's disease, amyotrophic lateral sclerosis, Huntington's disease, cerebral ischemia, traumatic neurodegenerative disease, and graft-versus-host disease; and
the inflammatory disease may be selected from ocular allergy, conjunctivitis, keratoconjunctivitis sicca, phlebitis-conjunctivitis, allergic rhinitis, hemolytic anemia, aplastic anemia, pure red cell anemia, idiopathic thrombocytopenic purpura, or acne; or another inflammatory disease caused by an autoimmune response is selected from systemic lupus erythematosus, rheumatoid arthritis, polychondritis, scleroderma, Wegener's granulomatosis, dermatomyositis, chronic active hepatitis, myasthenia gravis, Stephen-Johnson syndrome, idiopathic steatorrhea, ulcerative colitis, Crohn's disease or other autoimmune inflammatory bowel diseases, irritable bowel syndrome, celiaca, periodontitis, hyaline membrane disease, kidney disease, glomerular disease, alcoholic liver disease, endocrine ophthalmopathy, Graves disease, sarcoidosis, alveolar catarrh, chronic hypersensitivity pneumonia, multiple sclerosis, primary biliary cirrhosis, uveitis, Sjögren's syndrome, uveitis, keratoconjunctivitis, interstitial fibrosis, psoriatic arthritis, systemic juvenile idiopathic arthritis, nephritis, diverticulitis, interstitial cystitis, glomerulonephritis, pancreatitis, hereditary periodic fever syndrome, asthma, acute lung injury, acute respiratory distress syndrome, eosinophilia, hypersensitivity response, anaphylaxis, sinusitis, chronic obstructive pulmonary disease, lung disease, cystic fibrosis, appendicitis, atopic dermatitis, allergy, blepharitis, bronchiolitis, bronchitis, bursitis, cervicitis, cholangitis, cholecystitis, chronic graft rejection, conjunctivitis, cystitis, dacryoadenitis, dermatitis, dermatomyositis, encephalitis, endocarditis, endometritis, enteritis, epididymitis, fasciitis, fibrositis, gastritis, gastroenteritis, allergic purpura, hepatitis, hidradenitis suppurativa, immunoglobulin A nephropathy, interstitial lung disease, laryngitis, mastitis, meningitis, myelitis, myocarditis, myositis, nephritis, oophoritis, orchitis, osteitis, tympanitis, pancreatitis, mumps, pericarditis, peritonitis, pharyngitis, pleurisy, phlebitis, pneumonia, polymyositis, enteritis, prostatitis, pyelonephritis, rhinitis, salpingitis, sinusitis, stomatitis, synovitis, tendinitis, tonsillitis, vaginitis, vasculitis, vulvitis, pelada, herpetiform dermatitis, subcutaneous dermatitis, vitiligo, hypersensitivity vasculitis, urticaria, bullous pemphigus, pemphigus vulgaris, pemphigus foliaceus, epidermolysis bullosa, acute and chronic gout, chronic gouty arthritis, psoriasis, psoriatic arthritis, rheumatoid arthritis, juvenile rheumatoid arthritis, or osteoarthritis.

The compounds and derivatives provided in the present invention can be named according to the nomenclature system of the IUPAC (International Union of Pure and Applied Chemistry) or the CAS (Chemical Abstracts Service, CoLumbus, OH).

Definitions of terms used in the present invention: Unless otherwise stated, an initial definition provided for a radical or term herein is applicable to the radical or term throughout the specification; and for terms that are not specifically defined herein, meanings that can be given to them by a person skilled in the art should be provided based on the disclosure and context.

The "substitution" refers to the replacement of a hydrogen atom in a molecule with another different atom or molecule.

The "can be further substituted" means that the "substitution" may, but does not have to, occur, and the description includes occurrence or non-occurrence.

Minimum and maximum contents of carbon atoms in a hydrocarbon radical are indicated by prefixes, for example, a prefix C_{a-b} alkyl indicates any alkyl containing "a" to "b" carbon atoms. Therefore, for example, a "C₁₋₄ alkyl" refers to an alkyl containing 1-4 carbon atoms.

The "alkyl" mentioned in the present invention refers to a saturated hydrocarbon chain having a specified number of member atoms. For example, a C₁-₆ alkyl refers to an alkyl radical having 1 to 6 member atoms, such as 1 to 4 member atoms. The alkyl radical may be linear or branched. A representative branched alkyl radical has one, two, or three branched chains. The alkyl radical may be optionally substituted with one or more substituents as defined herein. The alkyl includes methyl, ethyl, propyl (n-propyl and isopropyl), butyl (n-butyl, isobutyl, and tert-butyl), pentyl (n-pentyl, isopentyl, and neopentyl), and hexyl. The alkyl radical may also be a moiety of an additional radical, wherein the additional radical is, for example, a C₁-₆ alkoxy.

The "alkylene" mentioned in the present invention refers to a divalent saturated aliphatic hydrocarbon radical having a specified number of carbon atoms. A "Cₐ-_{b} alkylene" refers to an alkylene radical having a to b carbon atoms. The alkylene radical includes a branched and linear hydrocarbon radical. For example, a "C₁-₆ alkylene" is intended to include, e.g., methylene, ethylidene, propylidene, 2-methylpropylidene, dimethylethylidene, and pentylidene. Therefore, the term "propylidene" may be a structure as illustrated below: 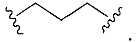 Similarly, the term "dimethylbutylidene" may be any one of structures as illustrated below: 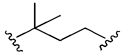 or

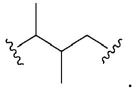

The "alkenyl" mentioned in the present invention refers to a linear or branched hydrocarbon radical having a specified number of carbon atoms and at least 1 unsaturated ethenyl site (>C=C<). For example, a C_{a-b} alkenyl refers to an alkenyl radical having a to b carbon atoms, and is intended to include, for example, ethenyl, propenyl, isopropenyl, 1,3-butadienyl, and the like.

The "alkynyl" mentioned in the present invention refers to a linear monovalent hydrocarbon radical or a branched monovalent hydrocarbon radical comprising at least one triple bond. The term "alkynyl" is further intended to include those hydrocarbon radicals each having one triple bond and one double bond. For example, a C₂-₆ alkynyl is intended to include, e.g., ethynyl and propynyl.

The "halogen" mentioned in the present invention is fluorine, chlorine, bromine, or iodine.

The "haloalkyl" or "halogenated alkyl" mentioned in the present invention means that a hydrogen atom in an alkyl can be substituted with one or more halogen atoms. For example, a C₁₋₄ haloalkyl refers to an alkyl having a hydrogen atom substituted with one or more halogen atoms and comprising 1-4 carbon atoms, and further includes, for example, trifluoromethyl, difluoromethyl, and the like.

A substituent such as the "=O" or "=S" mentioned in the present invention means that two hydrogen atoms or lone pair electrons are substituted with an oxygen atom or a sulfur atom to form a double bond.

The "-OR," "-NRR," or the like mentioned in the present invention means that a radical R is linked to an oxygen atom or a nitrogen atom by a single bond.

In the "-C(O)R," "-S(O)₂R," "-P(O)RR," or the like mentioned in the present invention, an oxygen atom is linked to a carbon atom, a sulfur atom, or a phosphorus atom by a double bond, and an R is linked to a carbon atom or a sulfur atom by a single bond. In the "-C(O)NRR," "-S(O)₂NRR," or the like mentioned in the present invention, an oxygen atom is linked to a carbon atom or a sulfur atom by a double bond, a nitrogen atom is linked to a carbon atom or a sulfur atom by a single bond, and an R is linked to a nitrogen atom by a single bond. In the "-NRC(O)R," "-NRS(O)₂R," or the like mentioned in the present invention, an R is linked to a nitrogen atom by a single bond, another R is linked to a carbon atom or a sulfur atom by a single bond, a nitrogen atom is linked to a carbon atom or a sulfur atom by a single bond, and an oxygen atom is linked to a carbon atom or a sulfur atom by a double bond.

The "cycloalkyl" mentioned in the present invention refers to a saturated or partially saturated cyclic radical having a plurality of carbon atoms, no ring heteroatoms, and a monocyclic ring or polycyclic rings (including a fused, bridged, spiro, and adamantane system). For a polycyclic system having aromatic and nonaromatic rings without ring heteroatoms, the term "cycloalkyl" (e.g., 5,6,7,8-tetrahydronaphthalen-5-yl) is applicable when the point of attachment is at a nonaromatic carbon atom. The term "cycloalkyl" includes a cycloalkenyl radical, such as cyclohexenyl. Examples of the cycloalkyl include, for example, adamantyl, cyclopropyl, cyclobutyl, cyclohexyl, cyclopentyl, cyclooctyl, cyclopentenyl, and cyclohexenyl. Examples of the cycloalkyl including a polybicycloalkyl ring system are bicyclohexyl, bicyclopentyl, bicyclooctyl, and the like. Two such polybicycloalkyl structures are illustrated and named below: 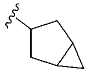 bicyclohexyl and 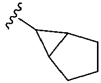 bicyclohexyl. The adamantyl includes, but is not limited to, a structure below: 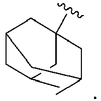 Specific examples of the bridged ring (bridged cycloalkyl) of the present invention include, e.g., 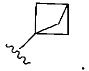 Specific examples of the spiro-ring of the present invention include, e.g.,

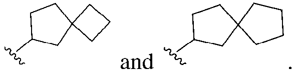

The "heterocyclic ring" or the "heterocyclyl" mentioned in the present invention refers to a saturated ring or an unsaturated non-aromatic ring comprising at least one heteroatom; wherein the heteroatom refers to, e.g., a nitrogen atom, an oxygen atom, and a sulfur atom. Usually, this refers to a monovalent saturated or partially unsaturated monocyclic or bicyclic ring system with a plurality of ring atoms, preferably a monovalent saturated or partially unsaturated monocyclic or bicyclic ring system with 3 to 9 ring atoms, and comprises 1, 2, or 3 ring heteroatoms selected from N, O, and S, and remaining ring atoms of carbon. A bicyclic ring is composed of two rings that share two ring atoms, i.e., a bridge separating the two rings is a single bond or a chain of one or two ring atoms. Examples of the monocyclic saturated heterocycyl are oxetanyl, azetidinyl, pyrrolidinyl, 2-oxo-pyrrolidin-3-yl, tetrahydrofuranyl, tetrahydro-thienyl, pyrazolidinyl, imidazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples of the bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, and examples of the partially unsaturated heterocycloalkyl are dihydrofuranyl, imidazolinyl, tetrahydro-pyridyl, or dihydropyranyl. Some other examples include, but are not limited to: 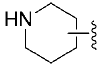 (for example, 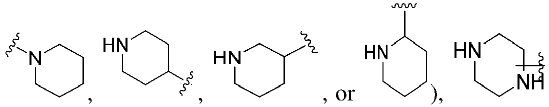 (for example, 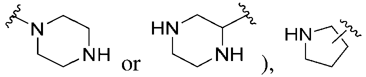 (for example, 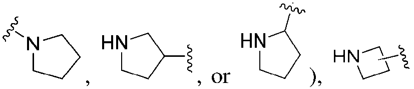 (for example, 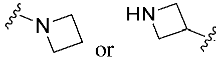 ), 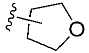 (for example, 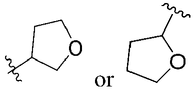 ), 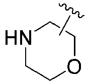 (for example, 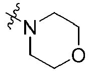 ), 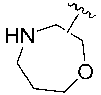 (for example, 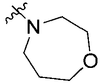 ). For another example, examples of the bicyclic ring include octahydrocyclopentano[C]pyrrolyl (for example, 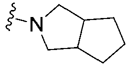 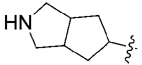 ), octahydropyrrolo[3,4-C]pyrrolyl (for example, 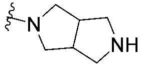 ), and 3-azabicyclo[3.1.0]hexyl (for example, 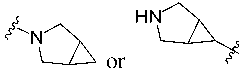 ).

The "heterospirocyclyl" or the "heterospirocyclic ring" mentioned in the present invention refers to a radical comprising at least one heteroatom and having 1 carbon atom thereof shared by two rings. The heterospirocyclyl comprises 1-4 heteroatoms selected from oxygen, sulfur, and nitrogen; including a saturated or partially unsaturated non-aromatic heterospirocyclyl. For example, a 4-10-membered heterospirocyclyl may be 2-azaspirocyclo[3.3]heptyl (for example, 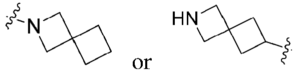 ), 7-azaspirocyclo[3.5]nonyl (for example, 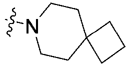 or 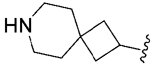 ), 2-azaspirocyclo[3.5]nonyl (for example, 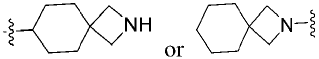 ), 2,7-diazaspirocyclo[3.5]nonyl (for example, 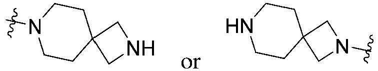 ), 6-azaspirocyclo[3.4]octyl (for example, 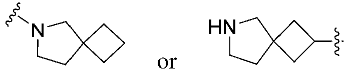 ), 4-oxa-7-azaspirocyclo[2.5]octyl (for example, 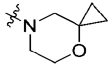 ), 5-oxa-8-azaspirocyclo[3.5]nonyl (for example, 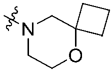 ), 2-oxa-6-azaspirocyclo[3.3]heptyl (for example, 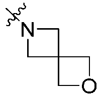 ), 2-oxa-6-azaspirocyclo[3.4]octyl (for example, 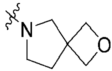 ), or 4,7-diazaspirocyclo[2.5]octyl (for example, 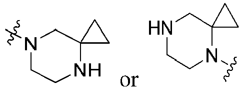 ).

The "bridged heterocyclyl" or the "bridged heterocyclic ring" mentioned in the present invention refers to a radical comprising at least one heteroatom and having two non-adjacent carbon atoms or heteroatoms shared by two rings. The bridged heterocyclyl comprises 1-4 heteroatoms selected from oxygen, sulfur, and nitrogen, including saturated or partially unsaturated non-aromatic bridged heterocyclyl; for example, 2-oxa-5-azabicyclo[2.2.1]heptyl (for example, 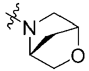 ) or 8-oxa-3-azabicyclo[3.2.1]octyl (for example, 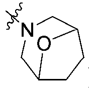 ).

The "aromatic ring" or the "aryl" mentioned in the present invention refers to an aromatic hydrocarbon radical having a plurality of carbon atoms. The aryl generally includes a monocyclic, bicyclic, or tricyclic aryl. Further, the term "aryl" as used herein refers to an aromatic substituent that may be a monoaromatic ring or fused polyaromatic rings. Non-limiting examples include phenyl, naphthyl, or tetrahydronaphthyl.

The "heteroaromatic ring" or the "heteroaryl" mentioned in the present invention refers to an unsaturated aromatic ring comprising at least one heteroatom; wherein the heteroatom refers to, e.g., a nitrogen atom, an oxygen atom, or a sulfur atom, generally including an aromatic monocyclic or bicyclic hydrocarbon that comprises a plurality of ring atoms, with one or more ring atoms thereof selected from O, N, and S, preferably one to three heteroatoms. Representative heteroaryl includes, for example: pyridyl, indolyl, quinoxalinyl, quinolinyl, isoquinolinyl, benzothienyl, benzofuranyl, benzothienyl, benzopyranyl, benzothiapyranyl, furanyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl, and benzoxazolyl. Some other examples include, but are not limited to

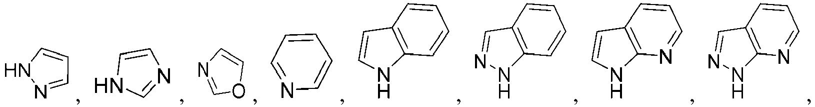

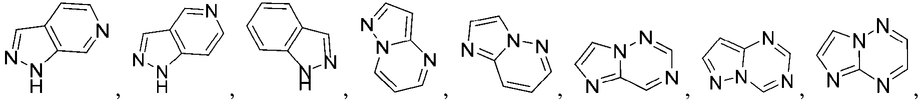

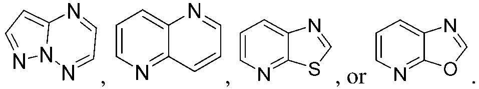

The "stereoisomer" includes an enantiomer and a diastereomer;

The term "pharmaceutically acceptable" means that a carrier, a vehicle, a diluent, an adjuvant, and/or a formed salt is generally chemically or physically compatible with other components constituting a pharmaceutical dosage form, and is physiologically compatible with a receptor.

The terms "salt" and "pharmaceutically acceptable salt" refer to an acidic and/or basic salt of the above compound or a stereoisomer thereof formed with an inorganic and/or organic acid and base, further include a zwitterionic salt (inner salt), and still further include a quaternary ammonium salt, such as an alkylammonium salt. These salts may be obtained directly from final isolation and purification of the compound, or may be obtained by mixing the above compound or the stereoisomer thereof with a certain amount of an acid or base appropriately (for example, equivalent). These salts may be obtained by precipitation in a solution and collection by filtration, or may be obtained by recycling after solvent evaporation, or may be obtained by reaction in an aqueous medium and then lyophilization. The salt mentioned in the present invention may be hydrochloride, sulfate, citrate, besylate, hydrobromide, hydrofluoride, phosphate, acetate, propionate, succinate, oxalate, malate, succinate, fumarate, maleate, tartrate, or trifluoroacetate of the compound.

In some embodiments, one or more compounds of the present invention may be used in combination with each other. The compounds of the present invention are optionally used in combination with any other active agent for the manufacture of a medicament or a pharmaceutical composition for regulating a cell function or treating a disease. If a group of compounds are used, these compounds may be administered to a subject concurrently, separately, or sequentially.

Obviously, based on the above contents of the present invention, according to the common technical knowledge and customary means in the art, without departing from the above basic technical idea of the present invention, various other forms of modifications, replacements, or alterations may be further made.

The beneficial effects of the present invention are:
the present invention discloses a compound of formula I, which can effectively degrade the IRAK4 or inhibit the IRAK4 activity in other ways, and has very good application prospects in treating IRAK4-mediated diseases including, e.g., immune diseases (such as psoriasis, hidradenitis suppurativa, atopic dermatitis, rheumatoid arthritis, systemic lupus erythematosus, alcoholic liver disease, autoimmune liver disease, or acne), tumors (such as multiple myeloma, lymphocytic leukemia, and lymphoma), Alzheimer's disease, and fibrosis.

Compared with a positive compound (patent No. WO2020113233 of Kymera Therapeutics, Inc.), the compound of the present invention has advantages in the following aspects, including: inhibitory effect against R848-induced IL-6 secretion levels in PBMC cells, inhibitory effect against LPS+IL-1β-induced IL-6 secretion levels in PBMC cells, effect on IRAK4 protein degradation in THP1 cells, stability in liver microsomes, therapeutic effect on IMQ-induced psoriatic skin thickening and ear thickening, and oral bioavailability. Therefore, the compound disclosed in the present invention provides a new option for clinical selection and/or preparation of a medicament for treating a disease associated with the IRAK4 activity.

### Description of Drawings

FIG. 1 shows a therapeutic effect of a compound of the present invention on IMQ-induced psoriatic skin thickening; and
FIG. 2 shows a significant therapeutic effect of a compound of the present invention on IMQ-induced psoriatic ear thickening.

### Detailed Description

The present invention is further introduced below with reference to embodiments, but is not limited to the scope of the embodiments. Experimental methods in the following embodiments without clear indication of specific conditions are carried out based on conventional methods and conditions, or are selected based on to product specifications.

Raw materials and devices employed in the specific embodiments of the present invention are all known products, and are obtained by purchasing commercially available products.

Unless otherwise specified in the embodiments, the reaction temperature is room temperature, which refers to 20-25°C. All temperatures are expressed in °C (Celsius).

The overnight is 14±1 h.

High performance liquid chromatography (HPLC) conditions: Waters high performance liquid chromatograph (e2695/e2487). Analytical high performance liquid chromatography conditions: C18 column (3.5 µm, 4.6×75 mm), UV detection wavebands: 220 and 254 nm, elution conditions: gradient elution with 5-95% acetonitrile (containing 0.1% V/V TFA or 10 mmol NH₄CO₃) for 10 min.

GiLson GX-281 reversed-phase preparative chromatograph or the Biotage IsoLera One flash purification system is used for reversed-phase purification.

Bruker Avance III 400 or 600 NMR spectrometer is used for NMR measurements with NMR shifts (δ) given in a unit of 10⁻⁶ (ppm). The solvents are, e.g., deuterated dimethyl sulfoxide (DMSO-d6), deuterated chloroform (CDCL3), and deuterated methanol (CD3OD), and the internal standard is tetramethylsilane (TMS).

Known starting materials, reagents, and solvents of the present invention may be synthesized using or according to known methods in the art, or may be purchased from, e.g., Chengdu Jinshan Chemical Reagent Co., Ltd., Shanghai Bide Technology Co., Ltd., and Shanghai Titan Technology Co., Ltd.

Known starting materials, reagents, and solvents of the present invention may be synthesized using or according to known methods in the art, or may be purchased from, e.g., Chengdu Jinshan Chemical Reagent Co., Ltd., Shanghai Bide Technology Co., Ltd., and Shanghai Titan Technology Co., Ltd.

In the above discussion and the following embodiments, the following abbreviations have the following meanings. An abbreviation, if not defined, has generally accepted meaning. MPLC is preparative medium pressure liquid chromatography; TLC is thin layer chromatography; MeOH is methanol; EtOH is ethanol; DMAP refers to 4-dimethylaminopyridine; DMF is N,N-dimethylformamide; DMA is N,N-dimethylacetamide; EA is ethyl acetate; THF is tetrahydrofuran; DMSO is dimethyl sulfoxide; DCM is dichloromethane; DCE is dichloroethane; MTBE is methyl tert-butyl ether; Boc2O is ditert-butyl dicarbonate; Boc is tert-butyloxycarbonyl; SEMCl is 2-(trimethylsilyl)ethoxymethyl chloride; SEM is 2-(trimethylsilyl)ethoxymethyl; CbzCl is benzyloxyformyl chloride; Cbz is benzyloxyformyl; FmocCl is 9-fluorenylmethyl chloroformate; Fmoc is 9-fluorenylmethoxyformyl; MsCl is methanesulfonyl chloride; Ms is methanesulfonyl; TBSCl is tert-butyldimethylchlorosilane; TBS is tert-butyldimethylchlorosilyl; TBDPSCl is tert-butyldiphenylchlorosilane; TBDPS is tert-butyldiphenylsilyl; TBAF is tetrabutylammonium fluoride; NBS is N-bromosuccinimide; TFA is trifluoroacetic acid; DBU is 1,8-diazabicycloundec-7-ene; DIPEA is N,N-diisopropylethylamine; TEA is triethylamine; HATU is 2-(7-azabenzotriazole)-N,N,N',N'-tramethyluronium hexafluorophosphate; HBTU is 2-(benzotriazole)-N,N,N',N'-tetramethyluronium hexafluorophosphate; and NMM is N-methylmorpholine.

### Examples

### Synthesis of intermediate IM-1

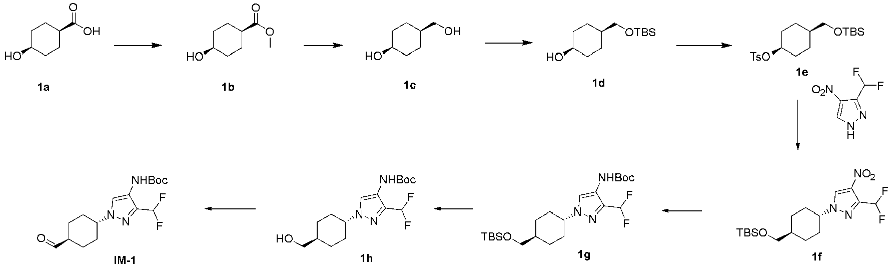

### Step 1: Synthesis of intermediate 1b

Compound 1a (850 g, 3.47 mmol) was added to methanol, 1/200 concentrated sulfuric acid was added, and the mixture was refluxed at 65°C for reaction for 12 h. After the reaction was completed, a saturated sodium carbonate solution was added. The mixture was adjusted to a pH of 7, filtered, and concentrated. 2,000 mL of EA was added. The mixture was extracted with saturated brine three times (400 mL×3), dried over anhydrous sodium sulfate, and concentrated to give the compound 1b (900 g), which was directly added to the next step.

### Step 2: Synthesis of intermediate 1c

The compound 1b (900 g) was added to THF (6,000 mL), and 150 g of lithium aluminum hydride was added batchwise at -20°C. After the reaction was completed, based on lithium aluminum hydride, equivalent water, equivalent 15% aqueous sodium hydroxide solution, and 3×aqueous solution were added sequentially to quench the reaction. The mixture was filtered and concentrated to give the compound 1c (410 g).

### Step 3: Synthesis of intermediate 1d

In an ice-water bath, the compound 1c (400 g) was added to DCM (6,000 mL), and a solution of 521 g of TBSCl in DCM was added to compound 4 (251 g) dropwise. The mixture was kept at room temperature for reaction for 12 h. After the reaction was completed, the mixture was concentrated, and washed with water (2,000 mL) 3 times. The aqueous phase was washed with DCM twice. The organic phase was extracted with 1,000 mL of saturated brine once, dried over anhydrous sodium sulfate, and concentrated to give the compound 1d (737 g).

### Step 4: Synthesis of intermediate 1e

The compound 1d (720 g, 2.95 mol) was dissolved in DCM (10 L), a solution of 940 g of TsCl in dichloromethane (2 eq) was rapidly added dropwise, then, a solution of DMAP in DCM was slowly added dropwise, and the mixture was kept at 30°C for reaction for 12 h. After the reaction was completed, 2 L of water was added, the mixture was extracted with 2 L of DCM twice. The organic phase was extracted with 10% citric acid once, washed with saturated NaHCO₃ once, washed with saturated brine once, dried over anhydrous sodium sulfate, filtered, concentrated, and subjected to silica gel column chromatography to give compound 6 (760 g).

### Step 5: Synthesis of intermediate 1f

3-(difluoromethyl)-4-nitro-1H-pyrazole (73.64 g) was added to DMF (1 L) and cesium carbonate (1.4 eq), and the mixture was kept for reaction for 10 min. The compound 1e (180 g) was added, and the mixture was kept at 60°C for reaction for 12 h. After 2,500 mL of water was added, the mixture was extracted with 500 mL of petroleum ether 3 times, and washed with 500 mL of water twice. Petroleum ether was washed with 500 mL of saturated brine once. The mixture was dried over anhydrous sodium sulfate, concentrated, and passed through a chromatographic column, to give 52.6 g of the compound 1f.

### Step 6: Synthesis of intermediate 1g

Compound 8 (52.6 g) was added to 500 mL of methanol, 1 eq of triethylamine, and (Boc)₂O (3 eq). The mixture was stirred at room temperature for 10 min, and 0.05 eq of palladium carbon was added. After hydrogen replacement 3 times, the mixture was kept at room temperature for reaction overnight. After the reaction was completed, the mixture was filtered and concentrated to give compound 9 (62 g).

### Step 7: Synthesis of intermediate 1h

The compound 1g (62 g) was added to 300 mL of THF, and HF.py (2 eq) was added dropwise in an ice-water bath. The mixture was naturally warmed, and stirred for 12 h. After the reaction was completed, the mixture was extracted with water and EA 3 times. The organic phase was washed with saturated sodium bicarbonate once, washed with saturated brine once, dried over anhydrous sodium sulfate, concentrated, and passed through a chromatographic column to give the compound (39.6 g). ESI-LCMS: m/z 346 [M+1], 1.45 min.

### Step 8: Synthesis of intermediate IM-1

The compound 1h (39.6 g) was added to 250 mL of DCM, DMP (1.3 eq) was added batchwise in an ice-water bath, and the mixture was stirred for 3 h. After the reaction was completed, the mixture was adjusted with saturated sodium bicarbonate to a pH of 7, filtered, layered, dried over anhydrous sodium sulfate, concentrated, and passed through a chromatographic column to give compound 1 (31 g). ESI-LCMS: m/z 344 [M+1],1.55 min.

### Synthesis of intermediate IM-2

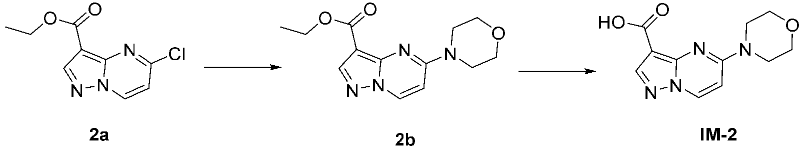

### Step 1: Synthesis of intermediate 2b

Ethyl 5-chloropyrazolo[1,5-a]pyrimidine-3-carboxylate (2.25 g, 10 mmol) was dissolved in 10 mL of acetonitrile, then morpholine (1.31 g, 15 mmol) and DIPEA (2.58 g, 20 mmol) were added, and the mixture was heated under reflux for 2 h. After the reaction was completed, 10 ml of water was added to precipitate a solid. After cooling, the mixture was filtered and the filter cake was rinsed with water. The solid was collected, and dried to give 2.55 g of ethyl 5-morpholinopyrazolo[1,5-a]pyrimidine-3-carboxylate. LCMS (ESI) m/z: [M+1]=277.

### Step 2: Synthesis of intermediate IM-2

Ethyl 5-morpholinopyrazolo[1,5-a]pyrimidine-3-carboxylate (2.55 g, 9.2 mmol) was dissolved in 20 ml of methanol, then a solution of sodium hydroxide (800 mg) in water (10 ml) was added, and the mixture was stirred at room temperature for 16 h. After the reaction was completed, 1 M dilute hydrochloric acid was added to adjust the mixture to a pH of about 5-6 and precipitate a solid. The organic solvent was removed under reduced pressure, and then the mixture was filtered. The solid was washed with water, collected, and dried to give 2.01 g of 5-morpholinopyrazolo[1,5-a]pyrimidine-3-carboxylic acid. LCMS (ESI) m/z: [M+1]=249.

Synthesis of intermediates IM-3 to IM-13: preparation methods for the intermediates IM-3 to IM-13 are similar to the preparation of the intermediate IM-2, as shown in Table 1.

**Table 1 Compound No. and structure**

| Compound No. | Structure | Compound No. | Structure |
|---|---|---|---|
| IM-3 | 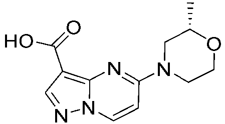 | IM-9 | 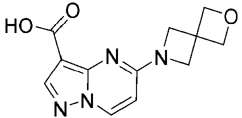 |
| IM-4 | 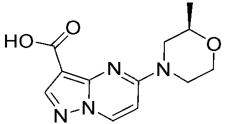 | IM-10 | 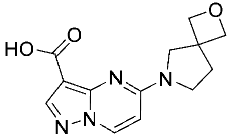 |
| IM-5 | 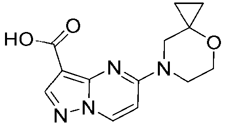 | IM-11 | 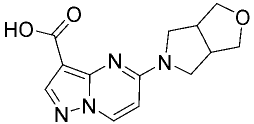 |
| IM-6 | 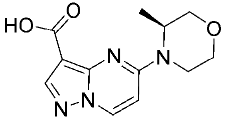 | IM-12 | 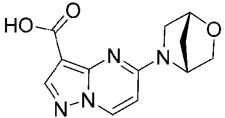 |
| IM-7 | 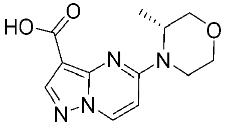 | IM-13 | 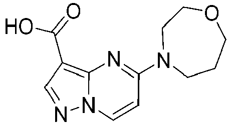 |
| IM-8 | 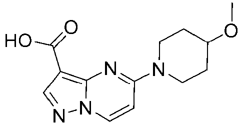 | IM-14 | 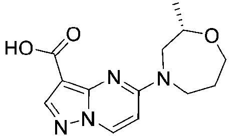 |

### Synthesis of intermediate IM-15

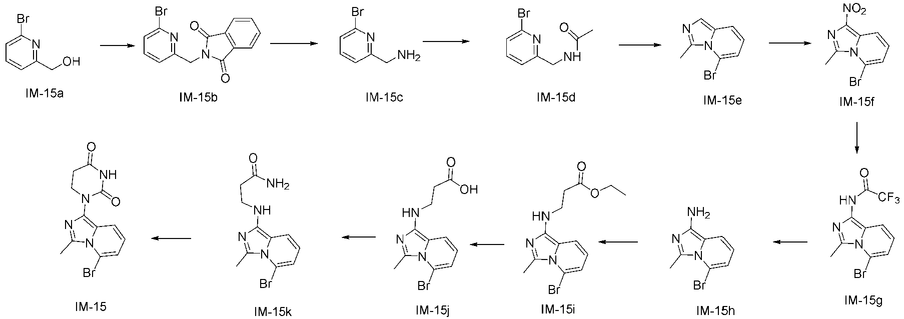

### Step 1: Synthesis of intermediate IM-15b

IM-15a (15 g), triphenylphosphine (31 g), and phthalimide (12.9 g) were dissolved in 200 mL of THF, and diisopropyl azodicarboxylate (23.5 mL) was slowly added at 0°C. After the addition was completed, the mixture was kept at room temperature for reaction overnight, concentrated, and subjected to column chromatography to give a yellow solid (20 g). LCMS (ESI) m/z: [M+1]=316.9.

### Step 2: Synthesis of intermediate IM-15c

The IM-15b (20 g) was dissolved in 200 mL of ethanol, and 10 mL of hydrazine hydrate was added. The mixture was heated, refluxed for 5 h, cooled to room temperature, and filtered. The filtrate was concentrated, and extracted with EA. The organic layer was successively washed with water and saturated brine, dried over anhydrous sodium sulfate, and concentrated under reduced pressure to give a crude product, which was directly used in the next step without separation and purification. LCMS (ESI) m/z: [M+1]=186.9.

### Step 3: Synthesis of intermediate IM-15d

The IM-15c (10g) was dissolved in 100 mL of dichloromethane, 11 mL of triethylamine was added, the mixture was cooled, and 6 mL of acetic anhydride was added dropwise at 0°C. After the addition was completed, the mixture was kept at room temperature for reaction for 2 h. 20 mL of water was added, and the mixture was extracted with dichloromethane. The organic layer was collected, successively washed with saturated brine, dried over anhydrous sodium sulfate, and concentrated under reduced pressure to give 8 g of crude product, which was directly used in the next step without separation and purification. LCMS (ESI) m/z: [M+1]=228.9.

### Step 4: Synthesis of intermediate IM-15e

The IM-15d (8 g) was dissolved in 60 mL of phosphorus oxychloride, and refluxed overnight. The reaction mixture was concentrated, and ice blocks were added to the residue. The mixture was neutralized with a saturated sodium carbonate solution, and extracted with ethyl acetate. The organic layer was successively washed with saturated brine, dried over anhydrous sodium sulfate, and concentrated under reduced pressure to give a crude product, which was subjected to column chromatography, to give 5 g of the product. LCMS (ESI) m/z: [M+1]=210.9.

### Step 5: Synthesis of intermediate IM-15f

The IM-15e (5 g) was dissolved in 50 mL of concentrated sulfuric acid, 1.8 mL concentrated nitric acid was added dropwise in an ice bath, and the mixture was kept for reaction overnight. Ice blocks were slowly added to the reaction mixture, to precipitate a yellow solid. After suction filtration, the yellow solid was washed with water, and suction-filtered, to collect the filter cake, which was dried under vacuum or reduced pressure to give the target product. LCMS (ESI) m/z: [M+1]=255.9.

### Step 6: Synthesis of intermediate IM-15g

The IM-15f (2 g) was dissolved in 20 mL of trifluoroacetic acid, 2.7 mL of trifluoroacetic anhydride and zinc powder (2.55 g) were added successively, and the mixture was kept at room temperature for reaction. After the reaction was completed, the reaction mixture was concentrated, and the residue was extracted with EA. The organic layer was successively washed with water and saturated brine, dried over anhydrous sodium sulfate, and concentrated under reduced pressure to give a crude product, which was subjected to column chromatography, to give 1.8 g of the target product. LCMS (ESI) m/z: [M+1]=321.9.

### Step 7: Synthesis of intermediate IM-15h

The IM-15g (1.5 g) was dissolved in 20 mL methanol, 1.3 g potassium carbonate was added, and heated under reflux until the reaction was completed. After suction filtration, the filtrate was concentrated to give the target product, which was used in the next step without purification. LCMS (ESI) m/z: [M+1]=225.9.

### Step 8: Synthesis of intermediate IM-15i

The IM-15h (1 g) was dissolved in 10 mL of ethanol, 2 mL of trifluoromethanesulfonic acid and 4.8 mL of ethyl acrylate were successively added, and the mixture was heated under reflux. After the reaction was completed, the mixture was concentrated, and extracted with EA. The organic layer was successively washed with a saturated aqueous sodium carbonate solution and saturated brine, dried over anhydrous sodium sulfate, and concentrated under reduced pressure to give a crude product, which was subjected to column chromatography, to give 900 mg of the target product. LCMS (ESI) m/z: [M+1]=327.2.

### Step 9: Synthesis of intermediate 15j

The IM-15i (900 mg) was dissolved in 6 mL of MeOH/THF/H₂O(V/V/V=4/1/1), 165 mg of sodium hydroxide was added, and the mixture was kept at room temperature for reaction. After the reaction was completed, the mixture was concentrated and evaporated to remove the organic solvent, the aqueous phase was adjusted to a pH of 4 to precipitate a white solid, and the mixture was suction-filtered to collect the filter cake, which was dried to give 800 mg of a white solid. LCMS (ESI) m/z: [M+1]=298.0.

### Step 10: Synthesis of intermediate IM-15k

The IM-15j (800 mg) was dispersed in 10 mL of dioxane, and 255 mg of ammonium bicarbonate, 0.33 mL of pyridine, and 703 mg of Boc anhydride were added successively. After the addition was completed, the mixture was heated to 60°C for reaction for 3 h, and concentrated. Water was added to precipitate a white solid. The mixture was suction-filtered, and dried to give 750 mg of the target product. LCMS (ESI) m/z: [M+1]=297.0.

### Step 11: Synthesis of intermediate IM-15

The IM-15k (500 mg), 1.36 g of carbonyldiimidazole, and 1.64 g of cesium carbonate were dispersed in 5 mL of acetonitrile. The mixture was refluxed, concentrated after the reaction was completed, and extracted with dichloromethane. The organic layer was successively washed with saturated brine, dried over anhydrous sodium sulfate, and concentrated under reduced pressure to give a crude product, which was subjected to column chromatography, to give 360 mg of the target product. LCMS (ESI) m/z: [M+1]=323.1.

### Synthesis of intermediate IM-16

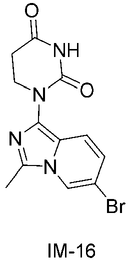

Preparation method for the intermediate IM-16 is similar to the preparation method for the intermediate IM-15.

### Synthesis of intermediate IM-17

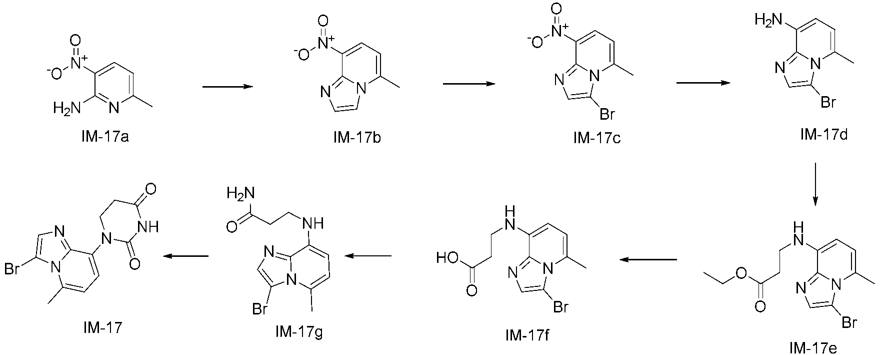

### Step 1: Synthesis of intermediate IM-17b

The IM-17a (5 g) was dissolved in 30 mL of DME, and 3.1 g of dichloroacetaldehyde was added. The mixture was heated for reaction for 6 h, and suction-filtered, to collect a solid, thus giving 5.2 g of a yellow solid. LCMS (ESI-MS): 178 [M+H]⁺

### Step 2: Synthesis of intermediate IM-17c

The IM-17b was dissolved in 50 mL of DMF, and 4.98 g of NBS was added batchwise. The mixture was kept at room temperature for reaction for 2 h, and suction-filtered to give 4.8 g of a yellow solid. LCMS (ESI-MS): 256 [M+H]⁺.

### Step 3: Synthesis of intermediate IM-17d

The IM-17c (4.8 g) was dissolved in MeOH/H₂O (V/V=3/1), and 10 g of ammonium chloride and 10.5 g of iron powder were added. The mixture was kept under nitrogen protection at 80°C for reaction for 2 h, and suction-filtered. The filtrate was concentrated, and the residue was extracted with EA. The organic layers were combined, successively washed with saturated brine, dried over anhydrous sodium sulfate, and concentrated under reduced pressure. The residue was slurried with dichloromethane to give a white solid. LCMS (ESI-MS): 226 [M+H]⁺; 1HNMR(400 MHz, CD₃OD) δ 7.44 (s, 1H), 6.48 (d,J=7.6 Hz, 1H), 6.40 (d, J=7.5 Hz, 1H), 2.94 (s, 3H).

### Steps 4 to 7: Synthesis of intermediate IM-17

The steps 4 to 7 are similar to the steps 8 to 11 of the preparation for the IM-15. LCMS (ESI-MS): 323.0 [M+H]⁺.

### Synthesis of intermediate IM-18

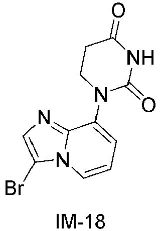

The preparation method for the intermediate IM-18 is similar to the preparation method for the intermediate IM-17.

### Synthesis of intermediate IM-19

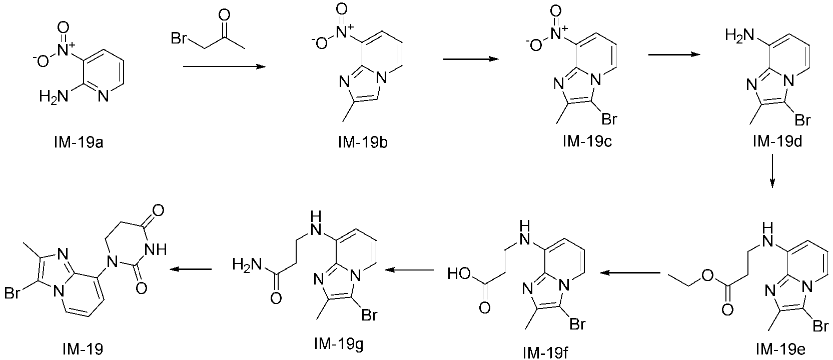

### Step 1: Synthesis of intermediate IM-19b

IM-17a (5 g) was dissolved in 30 mL of DME, and 5.9 g of 1-bromopropan-2-one was added. The mixture was heated for reaction for 6 h, and suction-filtered, to collect a solid, thus giving 5.2 g of a yellow solid.

### Step 2: Synthesis of intermediate IM-19c

The IM-19b (4 g) was dissolved in 40 mL of DMF, 4.8 g of NBS was added batchwise, the mixture was kept at room temperature for reaction for 2 h, 500 mL of water was added to the reaction mixture, and the mixture was suction-filtered to give 5 g of a yellow solid.

### Step 3: Synthesis of intermediate IM-19d

The IM-19c (5 g) was dissolved in 100 mL of MeOH/H₂O (V/V=3/1), and 11 g of ammonium chloride and 12 g of iron powder were added. The mixture was kept under nitrogen protection at 80°C for reaction for 2 h, and suction-filtered. The filtrate was concentrated, and the residue was extracted with EA. The organic layers were combined, successively washed with saturated brine, dried over anhydrous sodium sulfate, and concentrated under reduced pressure. The residue was subjected to column chromatography to give the target product. LCMS (ESI-MS): 226 [M+H]⁺; ¹HNMR (400 MHz, DMSO) δ 7.48 (d, J=6.6 Hz, 1H), 6.77 (t, J=7.0 Hz, 1H), 6.31 (d, J=7.4 Hz, 1H), 5.71 (s, 2H), 2.32 (s, 3H).

### Steps 4 to 7: Synthesis of intermediate IM-19

The steps 4 to 7 are similar to the steps 8 to 11 of the preparation for the IM-15. LCMS (ESI-MS): 323.0 [M+H]⁺

### Synthesis of intermediate IM-20

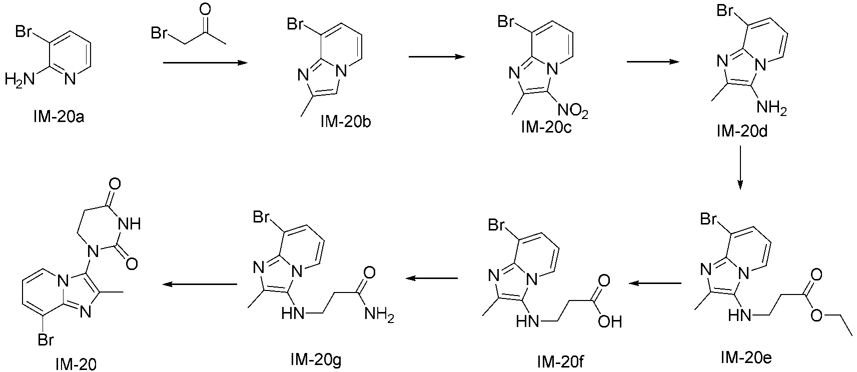

The preparation method for the intermediate IM-20 is similar to the preparation method for the intermediate IM-19. LCMS (ESI-MS): 323.0 [M+H]⁺

### Synthesis of intermediate IM-21

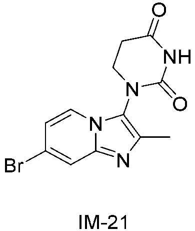

Preparation method for the intermediate IM-21 is similar to the preparation method for the intermediate IM-19. LCMS (ESI-MS): 323.0 [M+H]⁺

### Synthesis of intermediate IM-22

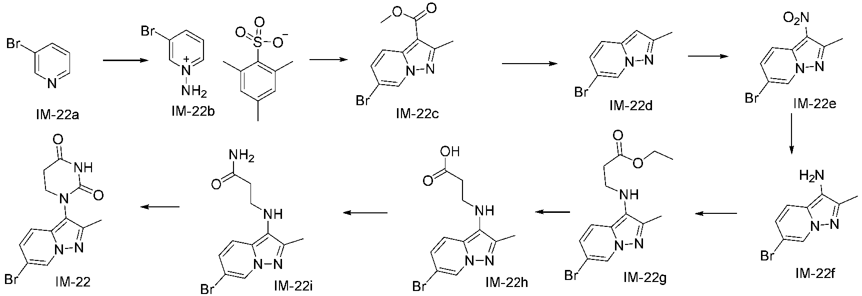

### Step 1: Synthesis of intermediate IM-22b

Compound MSH (31.00 g) was added to DCM (150 mL), and compound IM-22a (15 g) was slowly added. The reaction mixture was stirred overnight, monitored by LCMS, and concentrated to give the crude compound IM-22b.

### Step 2: Synthesis of intermediate IM-22c

The crude product from the last step was added to DMF (150 mL), and potassium carbonate (26 g) and methyl but-2-ynoate (18 g) were added in an ice bath. The mixture was stirred at room temperature overnight, monitored by LCMS, concentrated, and subjected to column chromatography, to give the compound IM-22c (5 g). ESI-LCMS: m/z 268.9 [M+H]⁺.

### Step 3: Synthesis of intermediate IM-22d

The IM-22c (5 g) was dissolved in 25 mL of acetic acid, 10 mg of an aqueous solution of hydrobromic acid (48%) was added, and the mixture was heated to 100°C overnight. After the reaction was completed, the mixture was extracted with ethyl acetate. The organic layer was successively washed with saturated brine, dried over anhydrous sodium sulfate, and concentrated under reduced pressure to give a crude product, which was subjected to column chromatography, to give 3.5 g of the target product. ESI-LCMS: m/z 210.9 [M+H]⁺.

### Steps 4 to 9: Synthesis of intermediate IM-22

The steps 4 to 9 of the preparation for the intermediate IM-22 are similar to the steps 2 to 7 of the preparation for the intermediate IM-20. ESI-LCMS: m/z 323.0 [M+H]⁺.

### Synthesis of intermediate IM-23

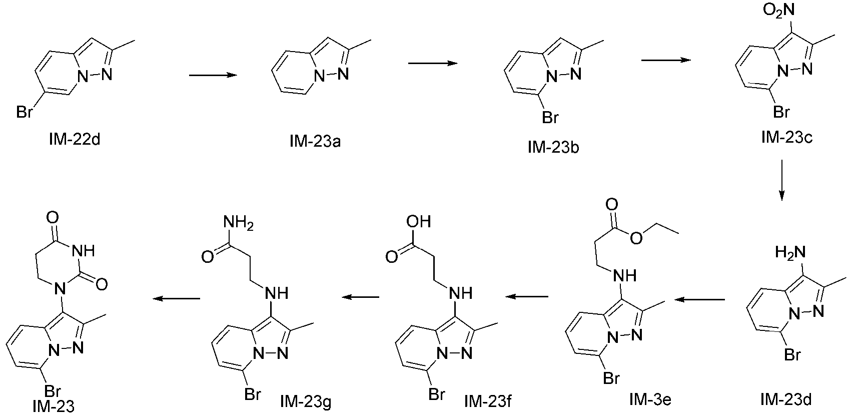

### Step 1: Synthesis of intermediate IM-23a

The IM-22d (1.5 g) was dissolved in 10 mL of methanol, and triethylamine (1.1 mL) and 150 mg palladium carbon were added. After replacement under a hydrogen balloon 3 times, the mixture was kept at room temperature for reaction overnight, and suction-filtered. The filtrate was concentrated and subjected to column chromatography to give the target product (600 mg).

### Step 2: Synthesis of intermediate IM-23b

The IM-23a (600 mg) was dissolved in 6 mL of anhydrous tetrahydrofuran, the mixture was cooled to -78°C, and 4.3 mL (1.6 M) of n-butyllithium was added dropwise. After the dropwise addition was completed, the mixture was kept for reaction for 30 min, 2.2 g of 1,2-dibromotetrachloroethane was added, and the reaction was continued for additional 30 min. The mixture was slowly heated to room temperature, water was added, and the mixture was extracted with ethyl acetate. The organic layer was successively washed with saturated brine, dried over anhydrous sodium sulfate, and concentrated under reduced pressure to give a crude product, which was subjected to column chromatography, to give 700 mg of the target product. ESI-LCMS: m/z 210.9 [M+H]⁺.

### Steps 3 to 8: Synthesis of intermediate IM-23

The steps 3 to 8 of the preparation for the intermediate IM-23 are similar to the steps 2 to 7 of the preparation for the intermediate IM-20. ESI-LCMS: m/z 323.0 [M+H]⁺.

### Synthesis of intermediate IM-24

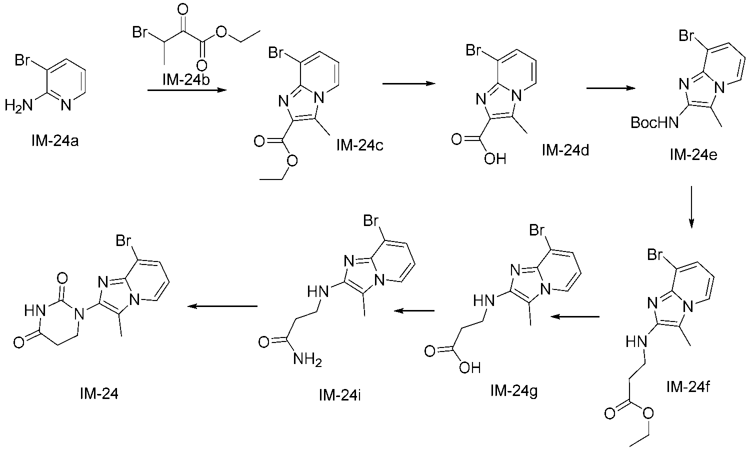

### Step 1: Synthesis of intermediate IM-24c

IM-24a (4 g) was dissolved in 30 mL of DME, and 3.7 g of IM-24b was added. The mixture was heated for reaction for 6 h, and suction-filtered to collect a solid, which was redissolved in 30 mL of methanol. The mixture was heated to 80°C for reaction overnight, and concentrated to give the target compound. LCMS (ESI-MS): 285 [M+H]⁺

### Step 2: Synthesis of intermediate IM-24d

The IM-24c (4.9 g) was dissolved in 30 mL of tetrahydrofuran, 15 mL of methanol, and 7.5 mL of water. 1.3 g of lithium hydroxide was added. The mixture was kept at room temperature for reaction for 3 h, concentrated, evaporated to remove the organic solvent, and adjusted to a pH of 4-5 to precipitate a solid, which was suction-filtered, to collect the filter cake, thus giving 2.9 g of a white solid. LCMS (ESI-MS): 255 [M+H]⁺.

### Step 3: Synthesis of intermediate IM-24e

The IM-24d was dissolved in 30 mL of tert-butanol, 4.4 g of DIEA and 6.1 g of DPPA were added, and the mixture was heated to 80°C under nitrogen for reaction overnight. 50 mL of water was added to the reaction mixture, and the mixture was extracted with ethyl acetate. The organic layer was successively washed with saturated brine, dried over anhydrous sodium sulfate, and concentrated under reduced pressure to give a crude product, which was subjected to column chromatography, to give 950 mg of the target product as a white solid. LCMS (ESI-MS): 326 [M+H]⁺, ¹HNMR(400 MHz, CD₃OD) δ 8.11 (d, J=7.2 Hz, 1H), 7.67 (d, J=1.4 Hz, 1H), 7.11 (dd, J=7.2, 2.0 Hz, 1H), 2.40 (s, 3H), 1.54 (s, 9H).

### Steps 4 to 7: Synthesis of intermediate IM-24

The steps 4 to 7 are similar to the steps 8 to 11 of the preparation for the IM-15. LCMS (ESI-MS): 323.0 [M+H]⁺

### Synthesis of intermediate IM-25

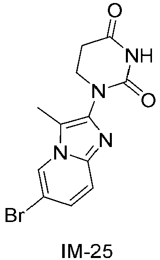

Preparation method for the intermediate IM-25 is similar to the preparation method for the intermediate IM-24. LCMS (ESI-MS): 323.0 [M+H]⁺

### Synthesis of intermediate IM-26

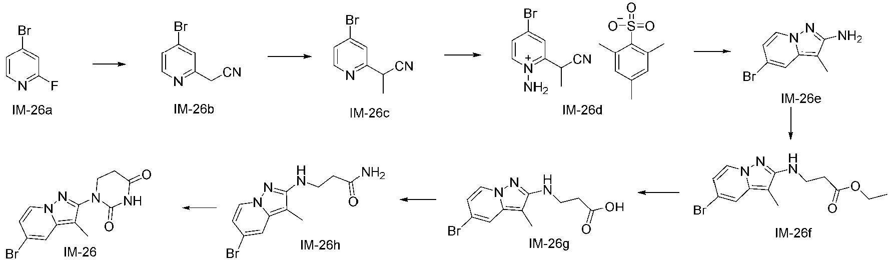

### Step 1: Synthesis of intermediate IM-26b

15 mL of acetonitrile was added to 200 mL of anhydrous tetrahydrofuran, the mixture was cooled to -78°C, n-butyllithium (178 mL, 1.6 M) was added, the reaction mixture was kept at - 78°C for reaction for 45 min, and IM-26a (20 g) was slowly added. After the addition was completed, the reaction mixture was heated to a temperature of -30°C for reaction for 2 h. The reaction mixture was quenched with a saturated ammonium chloride solution, 200 mL of water was added, and the mixture was extracted with ethyl acetate. The organic layer was successively washed with saturated brine, dried over anhydrous sodium sulfate, and concentrated under reduced pressure, to give a crude product, which was subjected to column chromatography, to give 16 g of the target product. LCMS (ESI-MS): 196.9 [M+H]⁺

### Step 2: Synthesis of intermediate IM-26c

16 g was dissolved in 100 mL of DMF, NaH (6.5 g, 60%) was added batchwise in an ice-water bath under nitrogen protection, and the mixture was stirred for 15 min, 15.4 mL of dimethyl sulfate. After the addition was completed, the reaction mixture was heated to room temperature for reaction overnight, and water was added to quench the reaction. The mixture was extracted with ethyl acetate. The organic layer was successively washed with saturated brine, dried over anhydrous sodium sulfate, and concentrated under reduced pressure to give a crude product, which was subjected to column chromatography, to give 14 g of the target product. LCMS (ESI-MS): 210.9 [M+H]⁺

### Step 3: Synthesis of intermediate IM-26d

The synthesis in the step 3 is similar to the step 1 of the preparation for the intermediate IM-22.

### Step 4: Synthesis of intermediate IM-26e

The IM-26d (20 g) was dissolved in 100 mL of methanol, and K₂CO₃ (13 g) was added. The mixture was stirred at room temperature overnight, and concentrated. Water was added to the residue, and the resulting mixture was extracted with dichloromethane. The organic layer was successively washed with saturated brine, dried over anhydrous sodium sulfate, and concentrated under reduced pressure, to give a crude product, which was subjected to column chromatography, to give 9.7 g of the target product. LCMS (ESI-MS): 225.9 [M+H]⁺

### Steps 5 to 8: Synthesis of intermediate IM-26

The steps 5 to 8 are similar to the steps 8 to 11 of the preparation for the IM-15. LCMS (ESI-MS): 323.0 [M+H]⁺

### Synthesis of intermediate IM-27

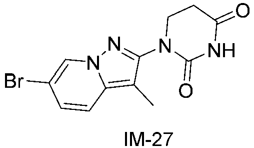

Preparation method for the intermediate IM-27 is similar to the preparation method for the intermediate IM-26. LCMS (ESI-MS): 323.0 [M+H]⁺

### Synthesis of intermediate IM-28

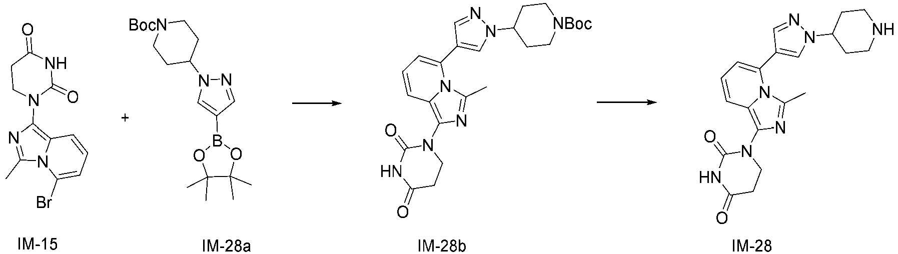

### Step 1: Synthesis of intermediate IM-28b

The IM-15 (500 mg), IM-28a (876 mg), and K₃PO₄ (985 mg) were dispersed in 5 mL of dioxane and 1 mL of water. Pd(PPh₃)₄ (89.4 mg) was added under nitrogen protection. After nitrogen replacement 3 times, the mixture was heated at 90°C for 18 h, and concentrated. The residue was subjected to column chromatography to give the target product (420 mg). LCMS (ESI-MS): 494.2 [M+H]⁺

### Step 2: Synthesis of intermediate IM-28

The IM-28b was added to 4 M dioxane hydrochloride solution. After the raw materials disappeared, and the solvent was removed using a rotary evaporator, to give the IM-28, which was directly used in the next reaction without purification. LCMS (ESI) m/z: [M+1]=394.2.

Synthesis of intermediates IM-29 to IM-42: preparation methods for the intermediates IM-29 to IM-42 are similar to the preparation of the intermediate IM-28, as shown in Table 2.

**Table 2 Compound No. and structure**

| Compound No. | Structure | Compound No. | Structure |
|---|---|---|---|
| IM-29 | 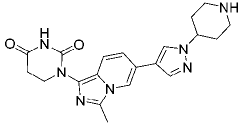 | IM-36 | 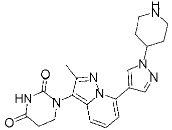 |
| IM-30 | 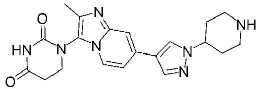 | IM-37 | 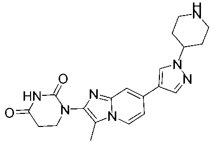 |
| IM-31 | 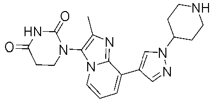 | IM-38 | 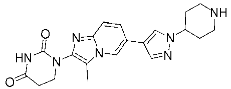 |
| IM-32 | 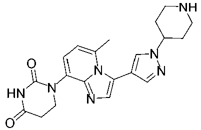 | IM-39 | 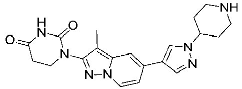 |
| IM-33 | 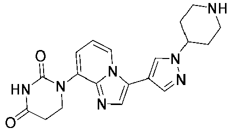 | IM-40 | 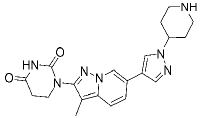 |
| IM-34 | 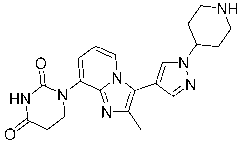 | IM-41 | 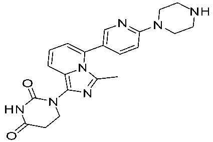 |
| IM-35 | 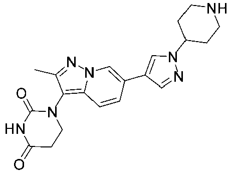 | IM-42 | 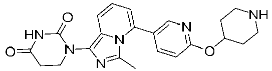 |

### Synthesis of intermediate IM-43

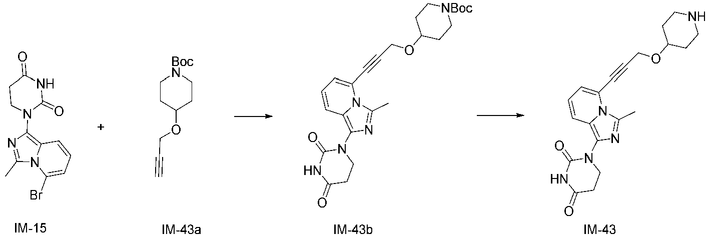

### Step 1: Synthesis of intermediate 43b

The IM-15 (150 mg) was dissolved in 5 ml of DMF, and then N-Boc-4-propargyloxypiperidine (124 mg), bis(triphenylphosphine)palladium(II) chloride (33 mg), and cesium carbonate (306 mg) were added. Then, the reaction system was protected under nitrogen, and stirred at 100°C overnight. After the reaction was completed, the mixture was extracted with ethyl acetate and water, and then the aqueous phase was washed with ethyl acetate twice. The organic phase was collected, washed with saturated brine once, and then dried over anhydrous sodium sulfate. The solvent was removed using a rotary evaporator. The residue was purified by column chromatography to give the target compound. LCMS (ESI) m/z: [M+1]=482.2.

### Step 2: Synthesis of intermediate IM-43

The IM-43b was dissolved in dioxane, and then 4 M dioxane hydrochloride solution was added. After the raw materials disappeared, the solvent was removed using a rotary evaporator to give IM-43 hydrochloride, which was used for the reaction in the next step without purification. LCMS (ESI) m/z: [M+1]=382.2.

### Synthesis of intermediate IM-44

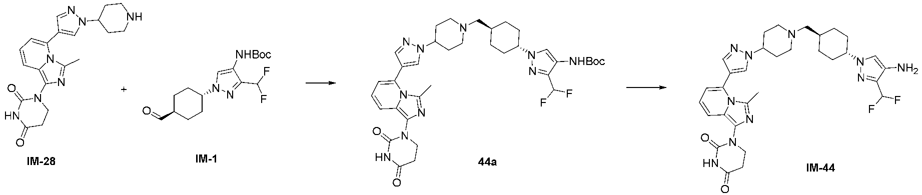

### Step 1: Synthesis of intermediate IM-44a

The IM-1 (700 mg) was dissolved in DMF/THF (1.0 mL/15.0 mL), the reaction mixture was cooled to -10°C, the IM-28 (776 mg) and TEA (412 mg) were added, and the mixture was stirred under argon protection for 0.5 h. Then, HOAc (367 mg) and NaBH(OAc)3 (1.3 g) were added to the reaction mixture. The reaction mixture was stirred for 3 h, quenched with water, and extracted with ethyl acetate. The organic layers were combined, washed with saturated brine, then dried over anhydrous sodium sulfate, concentrated, and separated through a silica gel column (DCM/MeOH=100/1 to 20/1) to give a white solid with a yield of 77%. LCMS (ESI) m/z: [M+1]=721.4.

### Step 2: Synthesis of intermediate IM-44

IM-44a (1 g) was dissolved in 10 mL of dioxane, and a dioxane hydrochloride solution (4 M) was added. The reaction mixture was stirred at room temperature for 5 h. After the reaction was completed, the reaction mixture was concentrated to give the target product, which was directly used for the reaction in the next step without purification. LCMS (ESI) m/z: [M+1]=621.3.

Synthesis of intermediates IM-45 to IM-58b: preparation methods for the intermediates IM-45 to IM-58b are similar to the preparation for the intermediate IM-44, as shown in Table 3.

**Table 3 Compound No. and structure**

| Compound No. | Structure | Compound No. | Structure |
|---|---|---|---|
| IM-45 | 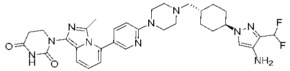 | IM-53 | 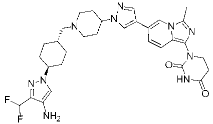 |
| IM-46 | 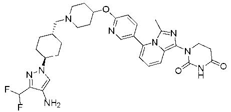 | IM-54 | 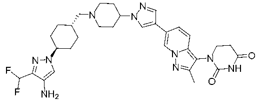 |
| IM-47 | 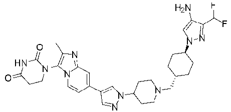 | IM-55 | 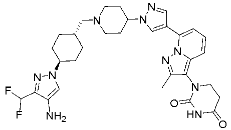 |
| IM-48 | 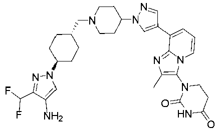 | IM-56 | 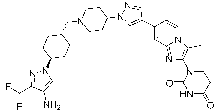 |
| IM-49 | 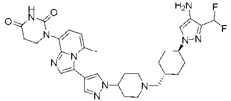 | IM-57 | 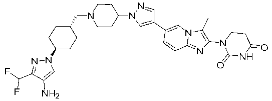 |
| IM-50 | 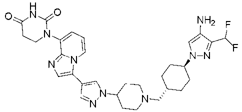 | IM-58a | 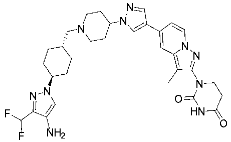 |
| IM-51 | 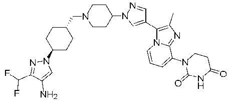 | IM-58b | 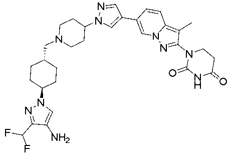 |
| IM-52 | 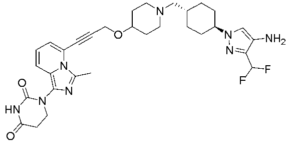 | | |

### Synthesis of intermediate IM-59

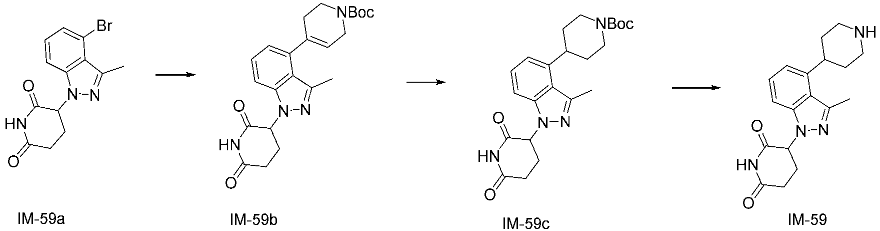

### Step 1: Synthesis of intermediate IM-59b

IM-59a (2 g), N-Boc-1,2,5,6-tetrahydropyridine-4-boronic acid pinacol ester (1.5 g), and K₂CO₃ (1.72 g) were dispersed in 20 mL of dioxane and 5 mL of water. Under nitrogen protection, [1,1'-bis(diphenylphosphino)ferrocene]dichloropalladium(II) (725 mg) was added. Air in the reaction system was replaced with nitrogen, and the mixture was kept under nitrogen protection at 90°C for reaction for 18 h. 20 mL of dioxane was added to the reaction mixture. After full ultrasonic dispersion, the mixture was suction-filtered through celite, and the filtrate was concentrated. The residue was dispersed in 20 mL of acetonitrile, CDI (5 g) was added, and the mixture was refluxed at 80°C. After the reaction was completed, the mixture was concentrated, and subjected to column chromatography to give 900 mg of the target product. LCMS (ESI) m/z: [M+1]=425.3.

### Step 2: Synthesis of intermediate IM-59c

The IM-59b (900 mg) was dissolved in 15 mL of ethanol, and 100 mg of Pd/C was added. The mixture was heated under hydrogen to 60°C for reaction for 18 h, and suction-filtered through celite. The filtrate was concentrated to give 700 mg of the target product, which was directly used in the next step without separation and purification. LCMS (ESI) m/z: [M+1]=427.2.

### Step 3: Synthesis of intermediate IM-59

The IM-59c (500 mg) was dissolved in 5 mL of dioxane, and a dioxane hydrochloride solution (4 M) was added. The reaction mixture was stirred at room temperature for 5 h. After the reaction was completed, the reaction mixture was concentrated to give the target product, which was directly used for the reaction in the next step without purification. LCMS (ESI) m/z: [M+1]=327.2.

Synthesis of intermediates IM-60 to IM-72: preparation methods for the intermediates IM-60 to IM-72 are similar to the preparation of the intermediate IM-59, as shown in Table 4.

**Table 4 Compound No. and structure**

| Compound No. | Structure | Compound No. | Structure |
|---|---|---|---|
| IM-60 | 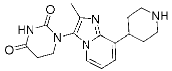 | IM-67 | 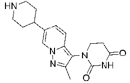 |
| IM-61 | 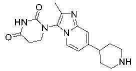 | IM-68 | 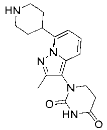 |
| IM-62 | 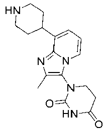 | IM-69 | 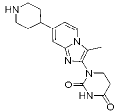 |
| IM-63 | 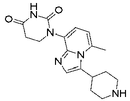 | IM-70 | 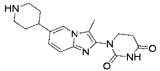 |
| IM-64 | 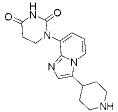 | IM-71 | 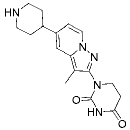 |
| IM-65 | 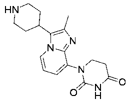 | IM-72 | 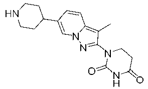 |
| IM-66 | 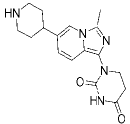 | | |

### Synthesis of intermediate IM-73

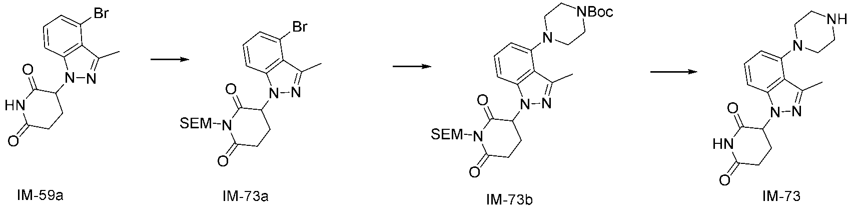

### Step 1: Synthesis of intermediate IM-73a

The IM-59a (5 g) was dissolved in 50 mL of tetrahydrofuran, the mixture was cooled in an ice-water bath, sodium hydride (931 mg) was added bathwise, the mixture was stirred in an ice-water bath for 10 min, and then SEM-C1 (3 g) was added. After the addition was completed, the mixture was warmed to room temperature for reaction overnight. The reaction was quenched with a saturated ammonium chloride solution. The mixture was extracted with ethyl acetate. The organic layer was successively washed with saturated brine, dried over anhydrous sodium sulfate, and concentrated under reduced pressure to give a crude product, which was subjected to column chromatography, to give 5.6 g of the target product. LCMS (ESI-MS): 452.1 [M+H]⁺

### Step 2: Synthesis of intermediate IM-73b

The IM-73a (3 g), Cs₂CO₃ (4.3 g), and Ruphos (609 mg) were dispersed in 30 mL of dioxane. Pd₂(dba)₃ (607 mg) was added under nitrogen protection, air in the reaction system was replaced with nitrogen, and the mixture was kept under nitrogen protection at 100°C for reaction for 18 h. 20 mL of dioxane was added to the reaction mixture. After full ultrasonic dispersion, the reaction mixture was suction-filtered through celite, the filtrate was concentrated, and the residue was subjected to column chromatography to give 1.8 g of the target product. LCMS (ESI) m/z: [M+1]=558.7.

### Step 3: Synthesis of intermediate IM-73

The IM-73b (500 mg) was dissolved in 8 mL of dichloromethane, TFA (2 mL) was added, and the reaction mixture was stirred at room temperature for 4 h. After the reaction was completed, the reaction mixture was concentrated to give the target product, which was directly used for the reaction in the next step without purification. LCMS (ESI) m/z: [M+1]=328.2.

### Synthesis of intermediate IM-74

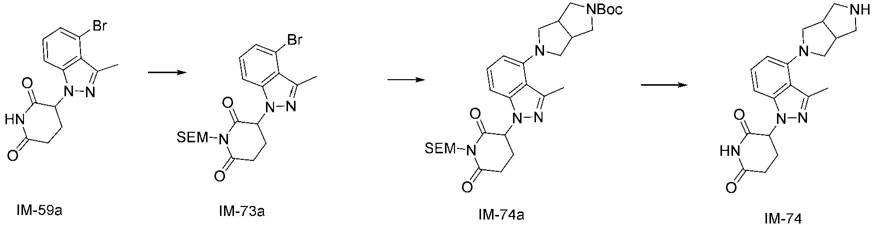

Preparation method for the synthesis of the intermediate IM-74 is similar to the synthesis of the IM-73. LCMS (ESI) m/z: [M+1]=354.2.

Synthesis of intermediates IM-75 to IM-81: preparation methods for the intermediates IM-75 to IM-81 are similar to the preparation of the intermediate IM-73, as shown in Table 5.

**Table 5 Compound No. and structure**

| Compound No. | Structure | Compound No. | Structure |
|---|---|---|---|
| IM-75 | 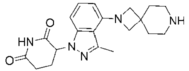 | IM-79 | 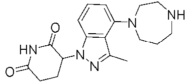 |
| IM-76 | 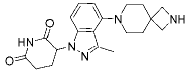 | IM-80 | 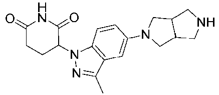 |
| IM-77 | 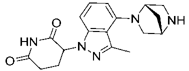 | IM-81 | 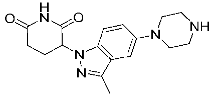 |
| IM-78 | 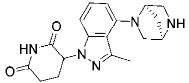 | | |

### Synthesis of intermediate IM-82

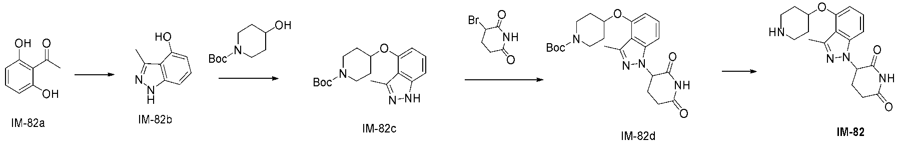

### Step 1: Synthesis of IM-82b

2,6-dihydroxyacetophenone (5.6 g, 36.83 mmol) was dissolved in 56 mL of ethylene glycol, 28 mL of hydrazine hydrate was added, and the reaction mixture was stirred at 160°C for 3 h. After the reaction was completed, the mixture was extracted with ethyl acetate. The organic layer was washed with water, dried over sodium sulfate, filtered, concentrated, and subjected to column chromatography (PE/EA=10/1 to 5/1), to give the target product, with a yield of 77%. LCMS (ESI) m/z: [M+1]=149.2.

### Step 2: Synthesis of IM-82c

3-methyl-1H-indazol-4-ol (4.2 g, 28.37 mmol) was dissolved in 42 mL of tetrahydrofuran. Tert-butyl 4-hydroxypiperidine-1-carboxylate ( 6.28 g, 31.22 mmol), PPh₃ (9.67 g, 36.87 mmol), and DIAD (8.6 g, 42.53 mmol) were added successively. After the addition was completed, the reaction mixture was refluxed overnight, and suction-filtered. The filtrate was concentrated, and subjected to column chromatography (PE/EA=10/1 to 1/1) to give 4.5 g of the product, with a yield of 48%. LCMS (ESI) m/z: [M+1]=332.19.

### Step 3: Synthesis of IM-82d

The compound IM-82c (4.5 g, 13.59 mmol) was dissolved in DMSO/THF (v/v=25 mL/25 mL). NaH (1.63 g, 67.92 mmol) was added batchwise under nitrogen protection at 0°C, the mixture was stirred for 30 min, and then a solution of KI (1.8 g, 10.86 mmol) in DMSO (20 mL) and a solution of 3-bromopiperidine-2,6-dione (5.19 g, 27.18 mmol) in tetrahydrofuran (20 mL) were added. The mixture was gradually warmed to room temperature, and stirred for 1 h. After the reaction was completed, the temperature was lowered by 0-10°C. The mixture was adjusted with 2M HCI to a pH of 6-7, and extracted with ethyl acetate. The organic layer was washed with saturated brine, dried over sodium sulfate, filtered, concentrated, and subjected to column chromatography (PE/EA=3/1) to give 2.5 g of the target product, with a yield of 42%. LCMS (ESI) m/z: [M+1]=443.22.

### Step 4: Synthesis of IM-82

The IM-82d (100 mg) was dissolved in 4 mL of dichloromethane, TFA (1 mL) was added, and the reaction mixture was stirred at room temperature for 4 h. After the reaction was completed, the reaction mixture was concentrated to give the target product, which was directly used for the reaction in the next step without purification. LCMS (ESI) m/z: [M+1]=343.17.

### Synthesis of intermediate IM-83

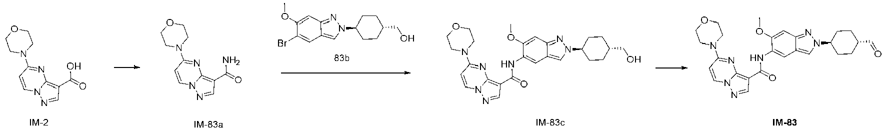

### Step 1: Synthesis of intermediate IM-83a

The IM-2 (2 g), pyridine (1.3 g), and ammonium bicarbonate (1.3 g) were dispersed in 15 mL of dioxane, and Boc₂O (2.6 g) was added. After the addition was completed, the reaction mixture was heated to 60°C for reaction for 3 h, diluted with 45 mL of water, and suction-filtered. The filter cake was washed with a little dioxane to collect a solid, which was dried to give 1.85 g of a white solid. LCMS (ESI) m/z: [M+1]=248.1.

### Step 2: Synthesis of intermediate IM-83c

IM-83b (1.56 g, preparation method derived from WO2020/264499), the IM-83a (1.25 g), Cs₂CO₃ (3.60 g), and Xantphos (532 mg) were dispersed in 20 mL of dioxane. Under nitrogen protection, Pd₂(dba)₃ (421 mg) was added, air in the reaction system was replaced with nitrogen, and the mixture was kept under nitrogen protection at 80°C for reaction for 72 h. 20 mL of dioxane was added to the reaction mixture. After full ultrasonic dispersion, the reaction mixture was suction-filtered through celite, the filtrate was concentrated, and the residue was subjected to column chromatography to give 320 mg of the target product. LCMS (ESI) m/z: [M+1]=506.2.

### Step 3: Synthesis of intermediate IM-83

The compound IM-83c (100 mg) was dissolved in 3 mL of DCM, DMP (1.3 eq) was added in an ice-water bath, and the mixture was stirred for 3 h. After the reaction was completed, the mixture was adjusted with saturated sodium bicarbonate to a pH of 7, filtered, layered, dried over anhydrous sodium sulfate, concentrated, and passed through a chromatographic column to give compound IM-80 (80 mg). LCMS (ESI) m/z: [M+1]=504.2.

Synthesis of intermediates IM-84 to IM-87: preparation methods for the intermediates IM-84 to IM-87 are similar to the preparation of the intermediate IM-83, as shown in Table 6.

**Table 6 Compound No. and structure**

| Compound No. | Structure | Compound No. | Structure |
|---|---|---|---|
| IM-84 | 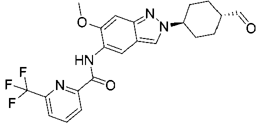 | IM-86 | 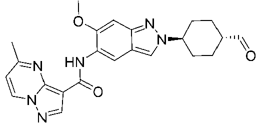 |
| IM-85 | 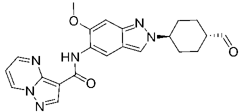 | IM-87 | 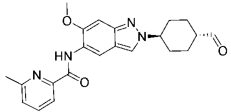 |

### Synthesis of intermediate IM-88

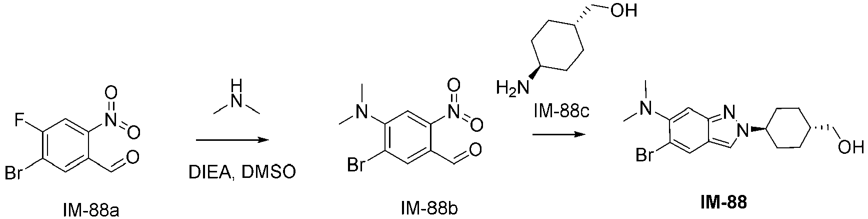

### Step 1: Synthesis of IM-88b

5-bromo-4-fluoro-2-nitrobenzaldehyde (15 g) and dimethylamine hydrochloride (9.86 g) were dissolved in 150 mL of DMSO, diisopropylethylamine (15.63 g) was added, and the reaction mixture was kept at 90°C for reaction overnight. After the reaction was completed, the mixture was diluted with water (600 mL), and extracted with EA (100 mL*3). The organic phase was washed with saturated brine, dried over anhydrous sodium sulfate, concentrated, dried, and purified by column chromatography (PE/DCM=5/1), to give 7.53 g of the target product. LCMS (ESI) m/z: [M+1]=272.98.

### Step 2: Synthesis of IM-88

The IM-88b (2.5 g) was dissolved in 30 mL of isopropanol, and then IM-85c (1.43 g) was added. The mixture was kept under nitrogen protection at 80°C for reaction for four h, and then naturally cooled to 25°C. Tributylphosphine (5.56 g) was then added to the reaction mixture, and the mixture was kept under nitrogen protection at 80°C for reaction for 16 h. After the reaction was completed, the organic solvent was rotarily evaporated. The residue was diluted with water (100 mL), and extracted with EA (50 mL*3). The organic phase was washed with saturated brine, dried over anhydrous sodium sulfate, filtered, concentrated, and purified by column chromatography (PE/EA=1:1), to give 4.5 g of the product. LCMS (ESI) m/z: [M+1]=352.1.

Synthesis of intermediates IM-89 to IM-98: preparation methods for the intermediates IM-89 to IM-98 are similar to the preparation of the intermediate IM-88, as shown in Table 7.

**Table 7 Compound No. and structure**

| Compound No. | Structure | Compound No. | Structure |
|---|---|---|---|
| IM-89 | 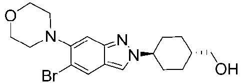 | IM-94 | 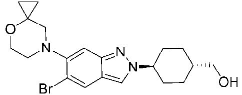 |
| IM-90 | 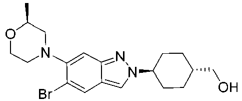 | IM-95 | 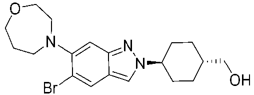 |
| IM-91 | 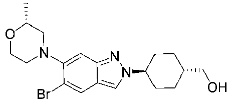 | IM-96 | 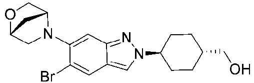 |
| IM-92 | 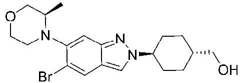 | IM-97 | 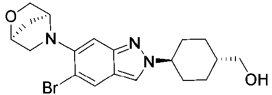 |
| IM-93 | 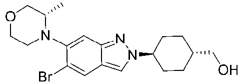 | IM-98 | 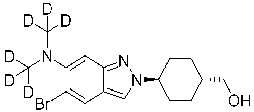 |

### Synthesis of intermediate IM-99

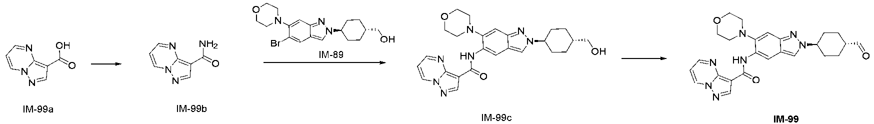

### Step 1: Synthesis of IM-99b

Pyrazolo[1,5-a]pyrimidine-3-carboxylic acid (1.5 g, 9.20 mmol) was dissolved in 15 mL of 1,4-dioxane, and stirred until the mixture became clear. Pyridine (0.727 g, 9.19 mmol), BOC anhydride (3.01 g, 13.79 mmol), and NH₄HCO₃ (1.45 g, 18.34 mmol) were added. The reaction mixture was stirred in a water bath at room temperature for 12 h. After the reaction was completed, the mixture was filtered through celite, washed with water, washed with 1,4-dioxane, and dried in a vacuum drying oven to constant weight to give the target product, with a yield of 67%. LCMS (ESI) m/z: [M+1]=163.19.

### Step 2: Synthesis of IM-99c

The IM-99b (1 g, 6.17 mmol), IM-86 (2.21 g, 5.60 mmol), CS₂CO₃ (4.38 g, 13.44 mmol), and Xantphos (0.649 g, 1.12 mmol) were dissolved in 19 mL of 1,4-dioxane. Finally, Pd₂(dba)₃ (0.513 g, 0.56 mmol) was rapidly added. After nitrogen replacement 5 times, the mixture was kept at 100°C for reaction for 16 h. After the reaction was completed, the mixture was filtered through celite, spin dried under vacuum, and purified by column chromatography (DCM/MeOH=100/1 to 20/1), to give 1.168 g of the target product, with a yield of 61.47%. LCMS (ESI) m/z: [M+1]=476.49.

### Step 3: Synthesis of IM-99

Oxalyl chloride (0.434 g, 3.42 mmol) was added to DCM (18 mL). In a dry-ice/ethanol bath at -70°C, DMSO (0.544 g, 6.96 mmol)/DCM (1 mL) solution was added dropwise, the mixture was stirred for 0.5 h, IM-95c (1.1 g, 2.28 mmol)/DCM (7 mL) solution was added, the mixture was stirred for 30 min, and DIPEA (1.47 g, 11.37 mmol)/DCM (2 mL) was added. The mixture was stirred for 15-30 min, and analyzed with acetonitrile by LCMS. After the reaction was completed, water (20 mL) was added, and the mixture was extracted with DCM (30 mL*3). The organic layer was washed with saturated brine, dried over anhydrous sodium sulfate, filtered, and concentrated to give 1 g of the target product, with a yield of 93.46%. LCMS (ESI) m/z: [M+1]=476.49.

Synthesis of intermediates IM-100 to IM-109: preparation methods for the intermediates IM-100 to IM-109 are similar to the preparation of the intermediate IM-99, as shown in Table 8.

**Table 8 Compound No. and structure**

| Compound No. | Structure | Compound No. | Structure |
|---|---|---|---|
| IM-100 | 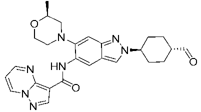 | IM-105 | 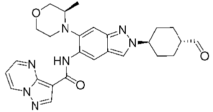 |
| IM-101 | 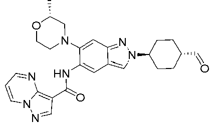 | IM-106 | 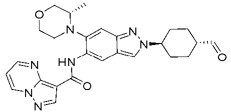 |
| IM-102 | 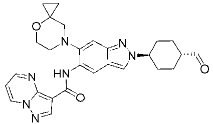 | IM-107 | 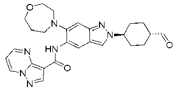 |
| IM-103 | 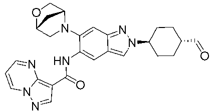 | IM-108 | 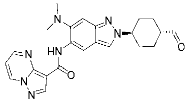 |
| IM-104 | 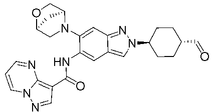 | IM-109 | 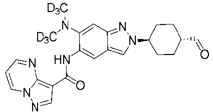 |

### Synthesis of intermediate IM-110

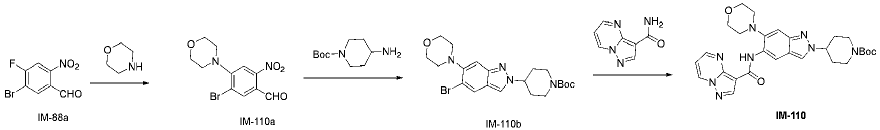

### Step 1: Synthesis of IM-110a

5-bromo-4-fluoro-2-nitrobenzaldehyde (35.6 g, 143.43 mmol) and morpholine (50.1 g, 575.06 mmol) were dissolved in 445 mL of DMSO, and the reaction mixture was kept for reaction at 80°C for 1 h. After the reaction was completed, the mixture was diluted with water (890 mL), and extracted with EA (900 mL*3). The organic phase was washed with saturated brine, dried over anhydrous sodium sulfate, concentrated, dried, and purified by column chromatography (PE/EA=10/1 to 1/4), to give 35 g of the product, with a yield of 77.6%. LCMS (ESI) m/z: [M+1]=316.12.

### Step 2: Synthesis of IM-110b

IM-110a (7 g, 22.22 mmol) was dissolved in 231 mL of isopropanol, and then tert-butyl 4-aminopiperidine-1-carboxylate (5.34 g, 26.66 mmol) was added. The mixture was kept under nitrogen protection at 80°C for reaction for four h, and then naturally cooled to 25°C. Then, tributylphosphine (13.48 g, 66.63 mmol) was added to the reaction mixture, and the mixture was kept under nitrogen protection at 80°C for reaction for 16 h. After the reaction was completed, the organic solvent was rotarily evaporated. The residue was diluted with water (200 mL), and extracted with EA (200 mL*3). The organic phase was washed with saturated brine, dried over anhydrous sodium sulfate, filtered, concentrated, and purified by column chromatography (PE/EA=10:1 to PE/EA=5/1), to give 10 g of the product , with a yield of 96.7%. LCMS (ESI) m/z: [M+1]=467.29.

### Step 3: Synthesis of IM-110

The IM-110b (2 g, 4.30 mmol), pyrazolo[1,5-a]pyrimidine-3-carboxamide (0.77 g, 4.75 mmol), Cs₂CO₃ (3.36 g, 10.31 mmol), and Xantphos (0.50 g, 0.864 mmol) were dissolved in 20 mL of 1,4-dioxane. Finally, Pd₂(dba)₃ (0.39 g,

0.426 mmol) was rapidly added, and after nitrogen replacement 5 times, the reaction mixture was kept at 100°C for reaction for 16 h. After the reaction was completed, the mixture was filtered through celite, spin dried, and purified by column chromatography (DCM/MeOH=100:1 to DCM/MeOH=40:1), to give 156 mg of the product, with a yield of 6.8%. LCMS (ESI) m/z: [M+1]=547.63.

Synthesis of intermediates IM-111 to IM-114: preparation methods for the intermediates IM-111 to 114 are similar to the preparation for the intermediate IM-105, as shown in Table 9.

**Table 9 Compound No. and structure**

| Compound No. | Structure | Compound No. | Structure |
|---|---|---|---|
| IM-111 | 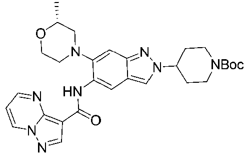 | IM-113 | 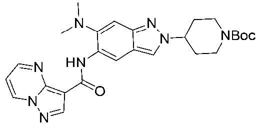 |
| IM-112 | 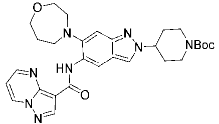 | IM-114 | 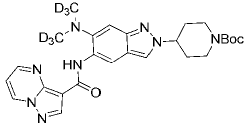 |

### Synthesis of intermediate IM-115

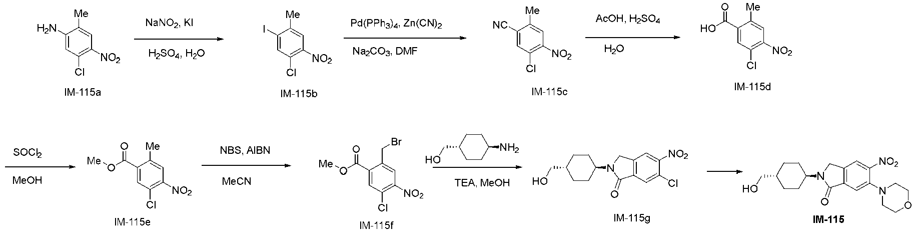

### Step 1: Synthesis of intermediate IM-115b

A solution of 5-chloro-2-methyl-4-nitroaniline (10 g, 53.6 mmol) in sulfuric acid (3 M, 100 mL) was placed in an ice bath at 0°C, and a solution of sodium nitrite (3.70 g, 53.6 mmol) in water (10 mL) was slowly added dropwise (in approximately 1 h). After the dropwise addition was completed, the mixture was stirred in an ice bath for 10 min, and an aqueous solution of potassium iodide (10.7 g, 64.3 mmol) was added. The mixture was kept in an ice bath at a reaction temperature of 0°C and stirred for 1 h, then slowly warmed to room temperature of 25°C, and further stirred at room temperature of 25°C for additional 1 h. TLC detection showed that the reaction was completed. The reaction mixture was diluted with water (500 mL), and extracted with ethyl acetate three times (3×200 mL). The organic layers were combined, washed with a saturated sodium chloride solution, dried over anhydrous sodium sulfate, filtered, concentrated under reduced pressure, and purified by silica gel column chromatography (petroleum ether:ethyl acetate=1:0 to 100:1) to give 1-chloro-5-iodo-4-methyl-2-nitrobenzene (11.6 g, 72% yield) as a yellow solid. ¹H NMR (400 MHz, DMSO-d) δ 8.24 (s, 1H), 8.04 (s, 1H), 2.43 (s, 3H).

### Step 2: Synthesis of intermediate IM-115c

1-chloro-5-iodo-4-methyl-2-nitrobenzene (11.6 g, 39.2 mmol) was dissolved in a DMF solution (80 mL), tetrakis(triphenylphosphine)palladium (4.53 g, 3.92 mmol) and sodium carbonate (8.31 g, 78.4 mmol) were added, and then zinc cyanide solid (2.76 g, 23.5 mmol) was added. The mixture was vacuumized, and was, after nitrogen replacement three times, stirred under nitrogen protection at 50°C for 24 h. TLC monitoring showed that the reaction was completed. The reaction mixture was diluted with water (500 mL), and extracted with ethyl acetate three times (3×300 mL). The organic layers were combined, washed with a saturated sodium chloride solution, dried over anhydrous sodium sulfate, filtered, concentrated under reduced pressure, and purified by silica gel column chromatography (petroleum ether:ethyl acetate=1:0 to 80:1) to give 5-chloro-2-methyl-4-nitrobenzonitrile (5.99 g, 78% yield) as a yellow solid. ¹H NMR (400 MHz, CDCl₃) δ 7.82-7.79 (m, 2H), 2.64 (s, 3H).

### Step 3: Synthesis of intermediate IM-115d

5-chloro-2-methyl-4-nitrobenzonitrile (1 g, 5 mmol) was dissolved in a glacial acetic acid solution (10 mL), water (10 mL) and concentrated sulfuric acid (10 mL) were added, and the mixture was heated to 120°C for reaction overnight. The reaction was stopped after TLC monitoring showed that the reaction was completed. After cooled to room temperature, the reaction mixture was diluted with water (100 mL), to precipitate a solid, which was suction-filtered and dried to give 5-chloro-2-methyl-4-nitrobenzoic acid (0.86 g, yield 80%) as a gray white solid. ¹H NMR (400MHz, CDCl₃) δ 8.22 (1H), 7.76 (s, 1H), 2.70 (s, 3H).

### Step 4: Synthesis of intermediate IM-115e

5-chloro-2-methyl-4-nitrobenzoic acid (0.90 g, 4.15 mmol) was dissolved in a methanol solution (10 mL), and a thionyl chloride solution (0.3 mL, 4.15 mmol) was slowly added dropwise (in approximately 1 h). The mixture was then heated to 80°C and refluxed overnight. The reaction was stopped after TLC monitoring showed that the reaction was completed. The reaction mixture was distilled under reduced pressure until the solvent was evaporated to dryness, then dichloromethane (10 mL) and a saturated sodium bicarbonate solution (20 mL) were added, and the mixture was extracted with dichloromethane three times (3×50 mL). The organic layers were combined, washed with a saturated sodium chloride solution, dried over anhydrous sodium sulfate, filtered, concentrated under reduced pressure, and purified by silica gel column chromatography to give methyl 5-chloro-2-methyl-4-nitrobenzoate (0.84 g, yield 87%) as a light white solid. ¹H NMR (400 MHz, CDCI₃): δ 8.08 (s, 1H), 7.73 (s, 1H), 3.95 (s, 3H), 2.64 (s, 3H).

### Step 5: Synthesis of intermediate IM-115f

Methyl 5-chloro-2-methyl-4-nitrobenzoate (3.1 g, 13.5 mmol) was dissolved in an acetonitrile solution (60 mL), and NBS (2.88 g, 16.2 mmol) and AIBN (0.11 g, 0.68 mmol) were added. The mixture was then heated to 70°C under nitrogen protection, and refluxed for 16 h. The reaction was stopped after TLC monitoring showed that the reaction was completed. The reaction mixture was distilled under reduced pressure until the solvent was evaporated to dryness, ethyl acetate (100 mL) and water (100 mL) were added, and the mixture was extracted with ethyl acetate three times (3×100 mL). The organic layers were combined, washed with a saturated sodium chloride solution, dried over anhydrous sodium sulfate, filtered, concentrated under reduced pressure, and purified by silica gel column chromatography to give methyl 2-bromomethyl-5-chloro-4-nitrobenzoate (3.80 g, yield 91 %) as a yellow solid. ¹H NMR (400MHz, CDCl₃) δ 8.15 (s, 1H), 7.99 (s, 1H), 4.92 (s, 2H), 4.01 (s,3H).

### Step 6: Synthesis of intermediate IM-115g

Methyl 2-bromomethyl-5-chloro-4-nitrobenzoate (3.1 g, 13.5 mmol) was dissolved in a methanol solution (40 mL), (4-aminocyclohexyl)carbinol (2.01 g, 15.6 mmol) was added, and then a triethylamine solution (3.61 mL, 25.9 mmol) was added. The mixture was heated to 80°C under nitrogen protection and refluxed for 16 h. The reaction was stopped after TLC monitoring showed that the reaction was completed. The reaction mixture was distilled under reduced pressure until the solvent was evaporated to dryness, and then the residue was purified by silica gel column chromatography to give a yellow solid (2.40 g, yield 57%). ¹H NMR (400 MHz, CDCl₃) δ 8.01 (s, 1H), 7.91 (s, 1H), 4.44 (s, 2H), 4.25 (tt, J=3.6, 12.1 Hz, 1H), 3.53 (d, J=6.2 Hz, 2H), 2.01-1.93 (m, 4H), 1.62-1.55 (m, 2H), 1.55-1.49 (m, 1H), 1.46 (s, 1H), 1.28-1.17 (m, 2H).

### Step 7: Synthesis of intermediate IM-115

The IM-115g (4.5 g) and diisopropylethylamine (7.2 mL) were dissolved in 45 mL of DMSO, morpholine (2.42 mL) was added to the reaction mixture, and the mixture was heat to 90°C for reaction for 12 h. The reaction mixture was diluted with 150 mL of water, and extracted with ethyl acetate. The organic layer was collected, successively washed with water and saturated sodium chloride, dried over anhydrous sodium sulfate, and concentrated under reduced pressure. The crude product was subjected to column chromatography (PE/EA=1/1) to give 3.3 g. LCMS (ESI) m/z: [M+1]=376.2.

Synthesis of intermediates IM-116 to IM-122: preparation methods for the intermediates IM-116 to 122 are similar to the preparation for the intermediate IM-115, as shown in Table 10.

**Table 10 Compound No. and structure**

| Compound No. | Structure | Compound No. | Structure |
|---|---|---|---|
| IM-116 | 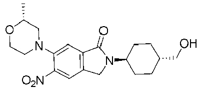 | IM-120 | 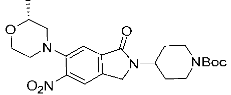 |
| IM-117 | 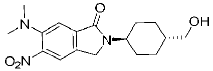 | IM-121 | 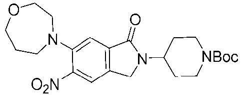 |
| IM-118 | 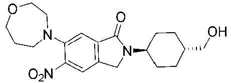 | IM-122 | 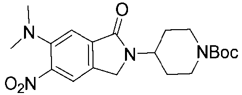 |
| IM-119 | 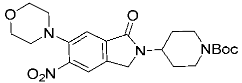 | | |

### Synthesis of intermediate IM-123

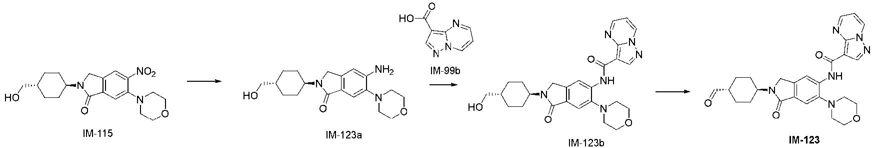

### Step 1: Synthesis of intermediate IM-123a

The IM-115 (5 g) was dissolved in 50 mL of MeOH/H₂O (4/1), iron powder (7.4 g) and ammonium chloride (7.1 g) were successively added, and the mixture was heated under reflux at 70°C for reaction for 4 h. LC-MS detection showed that the raw material reaction was completed. The reaction mixture was filtered through celite, and the filter cake was washed with DCM/MeOH (10/1) solution. The filtrate was collected, and concentrated under reduced pressure. The residue was extracted with DCM. The organic layer was collected, washed with saturated brine, dried over sodium sulfate, and concentrated under reduced pressure to give 4 g of the crude product, which was used directly in the next step without purification. LCMS (ESI) m/z: [M+1]=346.2.

### Step 2: Synthesis of intermediate IM-123b

The IM-99b (2.36 g) was dissolved in 30 mL of acetonitrile, N-methylimidazole (3.57 g) was added, the mixture was cooled to 0 °C, TCFH (4.26 g) was added, the mixture was stirred for 10 min, and the IM-123a (5.00 g) was added. After the addition was completed, the mixture was kept at room temperature for reaction for 2 h, and water was added to quench the reaction. The mixture was evaporated to remove the organic solvent and extracted with ethyl acetate. The organic layer was collected, dried over anhydrous sodium sulfate, and concentrated under reduced pressure. The residue was separated by column chromatography to give the target compound IM-123b (700 mg). LCMS (ESI) m/z: [M+1]=491.24.

### Step 3: Synthesis of intermediate IM-123

Preparation method for the IM-123 is similar to the step 3 of the preparation for the intermediate IM-99. LCMS (ESI) m/z: [M+1]=489.24.

Synthesis of intermediates IM-124 to IM-126: preparation methods for the intermediates IM-124 to 126 are similar to the preparation for the intermediate IM-123, as shown in Table 11.

**Table 11 Compound No. and structure**

| Compound No. | Structure | Compound No. | Structure |
|---|---|---|---|
| IM-124 | 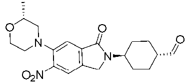 | IM-126 | 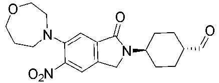 |
| IM-125 | 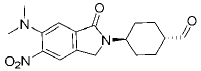 | | |

### Synthesis of intermediate IM-127

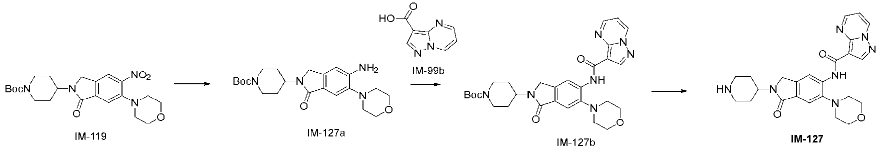

### Step 1: Synthesis of intermediate 127a

The IM-119 (3.5 g) was dissolved in 50 mL of MeOH/H₂O (4/1), iron powder (4.4 g) and ammonium chloride (4.2 g) were successively added, and the mixture was heated under reflux at 70°C for reaction overnight. LC-MS detection showed that the raw material reaction was completed. The reaction mixture was filtered through celite, and the filter cake was washed with DCM/MeOH (10/1) solution. The filtrate was collected, and concentrated under reduced pressure. The residue was extracted with DCM. The organic layer was collected, washed with saturated brine, dried over sodium sulfate, and concentrated under reduced pressure to give 2.68 g of the crude product, which was used directly in the next step without purification. LCMS (ESI) m/z: [M+1]=417.2.

### Step 2: Synthesis of intermediate 127b

The IM-99b (1.1 g) was dissolved in 30 mL of acetonitrile, N-methylimidazole (2.04 g) was added, the mixture was cooled to 0 °C, TCFH (2.27 g) was added, the mixture was stirred for 10 min, and the IM-127a (2.6 g) was added. After the addition was completed, the mixture was kept at 40°C for reaction for 2 h, and suction-filtered. The filter cake was successively washed with water, washed with acetonitrile, and dried to give 2.6 g of the product. LCMS (ESI) m/z: [M+1]=562.2.

### Step 3: Synthesis of intermediate 127

The IM-127b (200 mg) was dissolved in 4 mL of DCM, the mixture was cooled to 0°C, and 1 mL TFA was added. The mixture was warmed, and stirred for 2 h. LC-MS detection showed that the reaction was completed. The mixture was concentrated, and the residue was directly used in the next step. LCMS (ESI) m/z: [M+1]=462.2.

Synthesis of intermediates IM-128 to IM-130: preparation methods for the intermediates IM-128 to 130 are similar to the preparation for the intermediate IM-127, as shown in Table 12.

**Table 12 Compound No. and structure**

| Compound No. | Structure | Compound No. | Structure |
|---|---|---|---|
| IM-128 | 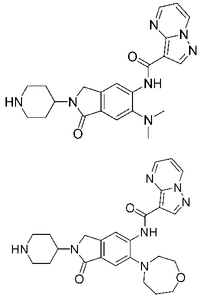 | IM-130 | 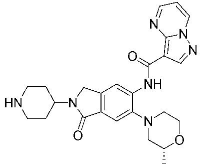 |
| IM-129 | 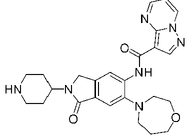 | | |

### Synthesis of intermediate IM-131

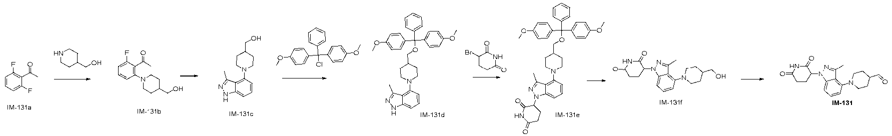

### Step 1: Synthesis of intermediate IM-131b

IM-131a (3 g) and 4-hydroxymethylpiperidine (3.3 g) were dissolved in NMP (30 mL), K₂CO₃ (5.3 g) was added at room temperature, and the mixture was kept at 120°C for reaction for 16 h. Water (100 ml) was added, and the mixture was extracted with ethyl acetate (50 mL*3). The organic phases were combined, washed with saturated brine (100 mL), dried, and filtered. The resulting crude product was spin dried, and purified by normal-phase HPLC (PE to EA:PE=3:1) to give the IM-130b (3.8 g). LCMS (ESI) m/z: [M+1]=252.1.

### Step 2: Intermediate IM-131c

The IM-131b (1 g) was dissolved in ethylene glycol (10 mL), hydrazine hydrate (1 mL, 20.615 mmol) was added, and the mixture was microwaved at 220°C for reaction for 2 h. LCMS showed that the reaction was completed. Water (50 mL) was added, and the mixture was extracted with ethyl acetate (60 mL*3). The organic phases were combined, dried, filtered, spin dried, and purified by reverse-phase HPLC (mobile phase: TFA (0.05% in water)/MeCN, approximately 60% product yield) to give the IM-131c. LCMS (ESI) m/z: [M+1]=246.1.

### Step 3: Synthesis of intermediate IM-131d

The IM-131c (780 mg) was dissolved in pyridine (10 mL), DMTr-Cl (1.12 g) was added in an ice bath, and the mixture was slowly heated to room temperature for reaction for 1 h. LCMS showed that the reaction was completed. The mixture was spin dried to remove the solvent, and purified by normal-phase HPLC (PE to PE:EA=5:1) to give 1 g of the target compound. LCMS (ESI) m/z: [M+1]=548.2.

### Step 4: Synthesis of intermediate IM-131e

The IM-131d was dissolved in THF (10 mL)and DMSO (10 mL), NaH (208.14 mg) was added in an ice bath, the mixture was kept in the ice bath for reaction for 0.5 h, and then KI (230.35 mg, 1.388 mmol) and 3-bromopiperidine-2,6-dione (832.62 mg, 4.336 mmol) were added. The mixture was slowly heated to room temperature, and kept at room temperature for reaction for 2 h. LCMS showed that the reaction was completed. Ice water was added to quench the reaction, and the mixture was extracted with ethyl acetate. The organic phases were combined, dried, filtered, and spin dried to give the target product, which was directly used for the reaction in the next step. LCMS (ESI) m/z: [M+1]=659.3.

### Step 5: Synthesis of intermediate IM-131f

The IM-131e (1.1 g, 1.670 mmol) was dissolved in DCM (6 mL), TFA (2 mL, 26.118 mmol) was added at room temperature, and the mixture was stirred at room temperature for 2 h. LCMS showed that the reaction was completed. The solvent was spin dried, and purified by reverse-phase HPLC (mobile phase: TFA (0.05% in water)/MeCN, approximately 55% product yield) to give 130 mg. LCMS (ESI) m/z: [M+1]=357.1.

### Step 6: Synthesis of intermediate IM-131

The IM-131f (50 mg, 0.140 mmol) was dissolved in MeCN (3 mL), IBX 2-iodoxybenzoic acid (50.96 mg, 0.182 mmol) was added, and the mixture was heated to 65°C for reaction for 2 h. LCMS showed that the reaction was completed. The mixture was filtered, and the filtrate was spin dried to give the crude product (50 mg, 0.085 mmol, 60.34%), which was directly used for the reaction in the next step. LCMS (ESI) m/z: [M+1]=355.2.

Synthesis of intermediates IM-132 to IM-139: preparation methods for the intermediates IM-132 to 139 are similar to the preparation for the intermediate IM-131, as shown in Table 13.

**Table 13 Compound No. and structure**

| Compound No. | Structure | Compound No. | Structure |
|---|---|---|---|
| IM-132 | 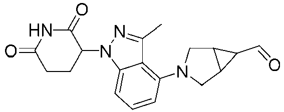 | IM-136 | 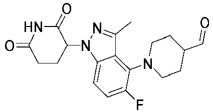 |
| IM-133 | 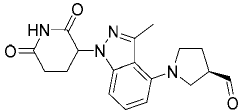 | IM-137 | 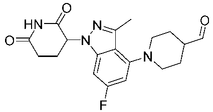 |
| IM-134 | 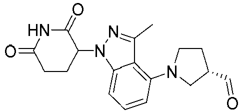 | IM-138 | 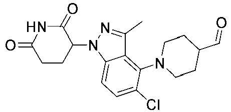 |
| IM-135 | 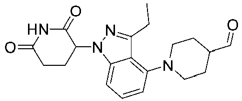 | IM-139 | 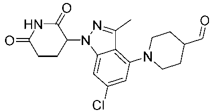 |

### Synthesis of intermediate IM-140

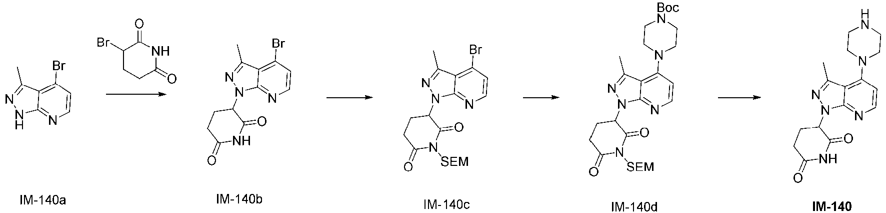

### Step 1: Synthesis of intermediate IM-140b

Compound IM-140a (5 g) was dissolved in DMSO/THF (v/v=25 mL/25 mL), NaH (3.77 g) was added batchwise under nitrogen protection at 0°C, and the mixture was stirred for 30 min. Then KI (3 g) was added, and a solution of 3-bromopiperidine-2,6-dione (9 g) in 20 mL of DMSO and tetrahydrofuran solution (20 mL) were added dropwise. The mixture was gradually warmed to room temperature and stirred for 1 h. After the reaction was completed, the temperature was lowered by 0-10°C. The mixture was adjusted with 2M HCI to a pH=6-7, and extracted with ethyl acetate. The organic layer was washed with saturated brine, dried over sodium sulfate, filtered, concentrated, and subjected to column chromatography to give 3 g of the target product. LCMS (ESI) m/z: [M+1]=323.0.

### Step 2: Synthesis of intermediate IM-140c

The IM-140b (3 g) was dissolved in 40 mL of DMF, the mixture was cooled in an ice-water bath, and DBU (4.7 g) and SEMCl (2.3 g) were added successively. After the addition was completed, the mixture was heat to 40°C for reaction overnight. The mixture was extracted with ethyl acetate. The organic layer was successively washed with saturated brine, dried over anhydrous sodium sulfate, and concentrated under reduced pressure to give a crude product, which was subjected to column chromatography, to give 2.8 g of the target product. LCMS (ESI-MS): 453.1 [M+H]⁺

### Step 3: Synthesis of intermediate IM-140d

The IM-140c (1.5 g), Cs₂CO₃ (2.2 g), and Ruphos (300 mg) were dispersed in 20 mL of dioxane. Pd₂(dba)₃ (300 mg) was added under nitrogen protection, air in the reaction system was replaced with nitrogen, and the mixture was kept under nitrogen protection at 100°C for reaction for 18 h. 20 mL of dioxane was added to the reaction mixture. After full ultrasonic dispersion, the reaction mixture was suction-filtered through celite, the filtrate was concentrated, and the residue was subjected to column chromatography to give 800 mg of the target product. LCMS (ESI) m/z: [M+1]=559.2.

### Step 4: Synthesis of intermediate IM-140

The IM-140d (100 mg) was dissolved in 2 mL of dichloromethane, TFA (1 mL) was added, and the reaction mixture was stirred at room temperature for 4 h. After the reaction was completed, the reaction mixture was concentrated to give the target product, which was directly used for the reaction in the next step without purification. LCMS (ESI) m/z: [M+1]=329.2.

Synthesis of intermediates IM-141 to IM-145: preparation methods for the intermediates IM-141 to 145 are similar to the preparation for the intermediate IM-140, as shown in Table 14.

**Table 14 Compound No. and structure**

| Compound No. | Structure | Compound No. | Structure |
|---|---|---|---|
| IM-141 | 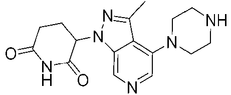 | IM-142 | 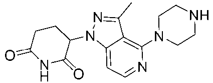 |
| IM-143 | 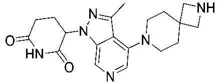 | IM-144 | 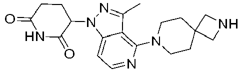 |
| IM-145 | 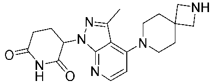 | | |

### Synthesis of intermediate IM-146

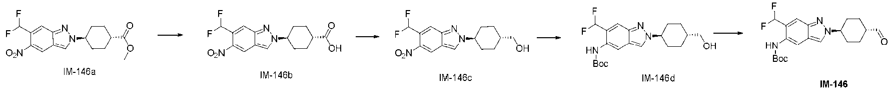

### Step 1: Synthesis of intermediate IM-146b

IM-146a (2 g, preparation method from WO2020/264499) was dissolved in 20 mL MeOH, 20 mL of THF and 10 mL of water. LiOH (375 mg) was added for reaction overnight. The mixture was concentrated and evaporated to remove the organic solvent. The aqueous phase was adjusted to a pH of 4, to precipitate a yellow solid, suction-filtered, and dried, to give 1.5 g of the target product. LCMS (ESI) m/z: [M+1]=340.3.

### Step 2: Synthesis of intermediate IM-146c

The IM-146b (1.49 g) and NMM (470 mg) were dissolved in 20 mL of THF, and isobutyl chloroformate (643 mg) was added dropwise under nitrogen protection at 0°C. After the addition was completed, the mixture was stirred for 30 min, suction-filtered, and washed with THF. The filtrate was collected. An aqueous solution of NaBH₄ (335 mg) was added dropwise at 0°C. The mixture was kept for reaction for 1 h, and extracted with ethyl acetate. The organic layer was successively washed with saturated brine, dried over anhydrous sodium sulfate, and concentrated under reduced pressure, to give the crude product, which was subjected to column chromatography, to give 1.35 g of the target product. LCMS (ESI-MS): 326.2 [M+H]⁺

### Step 3: Synthesis of intermediate IM-146d

20 mL of methanol, triethylamine (629 mg), and (Boc)₂O (2.72 g) were added to the compound IM-146c (1.35 g). The mixture was stirred at room temperature for 10 min, and 0.05 eq of palladium carbon was added. After hydrogen replacement 3 times, the mixture was kept at room temperature for reaction overnight. After the reaction was completed, the mixture was filtered and concentrated, and the residue was subjected to column chromatography to give 1.64 g of the target product. LCMS (ESI-MS): 396.2 [M+H]⁺

### Step 4: Synthesis of intermediate IM-146

The compound IM-146d (100 mg) was dissolved in 3 mL of DCM, DMP (135 mg) was added in an ice-water bath, and the mixture was stirred for 3 h. After the reaction was completed, the mixture was adjusted with saturated sodium bicarbonate to a pH of 7, filtered, layered, dried over anhydrous sodium sulfate, concentrated, and passed through a chromatographic column to give compound IM-146 (78 mg). LCMS (ESI) m/z: [M+1]=394.2.

### Synthesis of intermediate IM-147

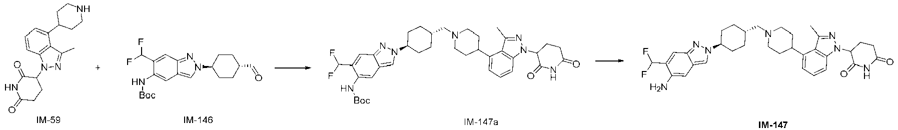

### Step 1: Synthesis of intermediate IM-147a

The IM-59 (500 mg) was dissolved in DMF/THF (1.0 mL/10.0 mL), the reaction mixture was cooled to -10°C, the IM-146 (600 mg) and TEA (300 mg) were added, and the mixture was stirred under argon protection for 0.5 h. Then, HOAc (262 mg) and NaBH(OAc)₃ (928 mg) were added to the reaction mixture. The reaction mixture was stirred for 3 h, quenched with water, and extracted with ethyl acetate. The organic layers were combined, washed with saturated brine, then dried over anhydrous sodium sulfate, concentrated, and separated through a silica gel column (DCM/MeOH=100/1 to 20/1), to give 568 mg of the target compound. LCMS (ESI) m/z: [M+1]=704.3.

### Step 2: Synthesis of intermediate IM-144

The IM-147a (500 mg) was dissolved in 8 mL of dichloromethane, TFA (2 mL) was added, and the reaction mixture was stirred at room temperature for 4 h. After the reaction was completed, the reaction mixture was concentrated to give the target product, which was directly used for the reaction in the next step without purification. LCMS (ESI) m/z: [M+1]=604.4.

Synthesis of intermediates IM-148 to IM-160: preparation methods for the intermediates IM-148 to IM-160 are similar to the preparation of the intermediate IM-147, as shown in Table 15.

**Table 15 Compound No. and structure**

| Compound No. | Structure | Compound No. | Structure |
|---|---|---|---|
| IM-148 | 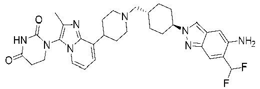 | IM-155 | 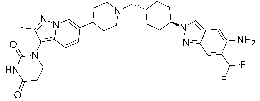 |
| IM-149 | 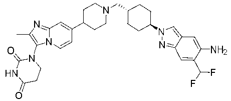 | IM-155 | 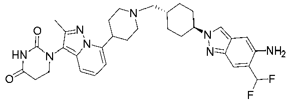 |
| IM-150 | 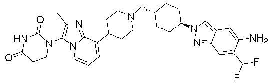 | IM-156 | 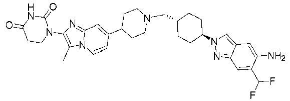 |
| IM-151 | 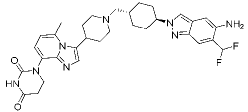 | IM-157 | 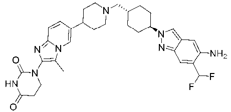 |
| IM-152 | 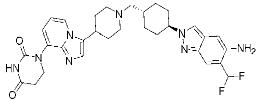 | IM-158 | 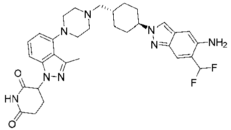 |
| IM-153 | 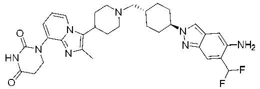 | IM-159 | 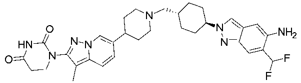 |
| IM-154 | 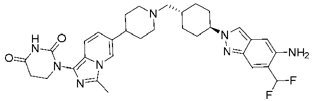 | IM-160 | 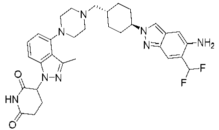 |

### Synthesis of intermediate IM-161

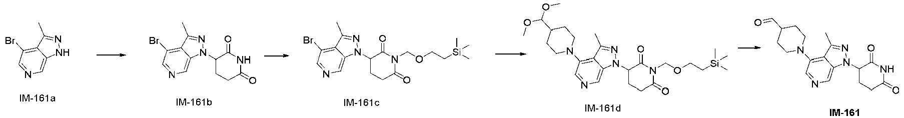

### Step 1: Synthesis of intermediate IM-161b

IM-161a (1 g) was dissolved in THF/DMSO=1:1 (10 mL), NaH (0.94 g) was added at 0-10°C, the mixture was stirred for half an hour, and then KI (0.63 g, 3.773 mmol) and a solution of 3-bromopiperidine-2,6-dione (1.81 g) in THF/DMSO=1:1 (10 mL) were added.

After the LCMS detection showed that the reaction of the IM-161a was completed, the reaction mixture was quenched with a saturated NH₄Cl solution (20 mL), and extracted with ethyl acetate (10 mL*3). The organic layer was collected, washed with saturated sodium chloride twice, dried over anhydrous sodium sulfate, concentrated, and weighed to give 1.45 g of the product. LCMS (ESI) m/z: [M+1]=322.9.

### Step 2: Synthesis of intermediate IM-161c

The IM-161b (1.45 g) was dissolved in DMF (15 mL), and DBU (1.006 mL, 6.731 mmol) and SEMCl (0.953 mL, 5.384 mmol) were added. After the addition was completed, the reaction mixture was stirred at 40°C for 4 h. LCMS showed that the raw materials were consumed. The mixture was diluted with 5× water, and extracted with ethyl acetate three times. The organic phase was collected, then washed with water twice, further washed with saturated brine twice, dried over anhydrous sodium sulfate, filtered, and concentrated. The crude product was subjected to column chromatography (PE/EA=1:1) to give 845 mg of the product. LCMS (ESI) m/z: [M+1]=452.70.

### Step 3: Synthesis of intermediate IM-161d

The IM-161c (470 mg), 4-(dimethoxymethyl)-piperidine (248.13 mg), Cs₂CO₃ (846.20 mg), and RuPhos (96.96 mg) were dissolved in anhydrous 1,4-dioxane (10 mL). After nitrogen replacement 3 times, Pd₂(dba)₃ (95.13 mg, 0.104 mmol) was finally added under nitrogen protection. After replacement 3 times, the mixture was heated under reflux at 100°C, stirred for 4 h, extracted with EA, washed with NaCl, concentrated, and subjected to column chromatography (PE/EA=1/1-EA), to give 100 mg of the product as an oil. LCMS (ESI) m/z: [M+1]=532.66.

### Step 3: Synthesis of intermediate IM-161

The reactant IM-161d (100 mg, 0.188 mmol) was added to a 25 mL eggplant-shaped flask, and dissolved in DCM (1.5 mL). 0.5 mL of TFA was added at 0°C. The mixture was stirred overnight, and concentrated. Dichloromethane and water were added, and the mixture was layered. The organic phase was washed with a saturated sodium bicarbonate solution and a NaCl solution, dried over sodium sulfate, and concentrated to give the product. LCMS (ESI) m/z: [M+1]=356.2.

**The synthesis of the compounds of the present invention is provided below.**

### Example 1: Synthesis of TM-1

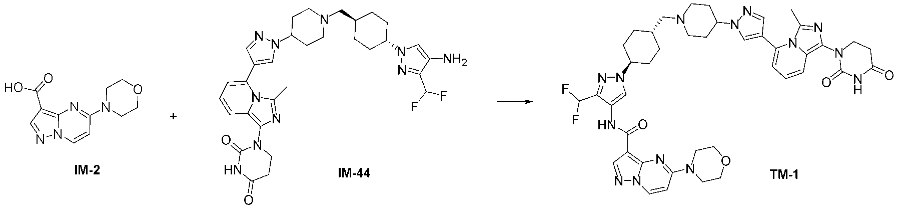

The compound IM-2 (80 mg) was added to acetonitrile (2 mL), NMI (82 mg) and TCFH (115 mg) were added, the mixture was stirred at room temperature for 15 min, and then compound 5 (160 mg) dissolved in DMF was added. The mixture was stirred at room temperature overnight, monitored by LCMS, concentrated, diluted with water to precipitate a solid, and filtered. The solid was subjected to preparative reverse-phase HPLC to give the compound TM-1 (32.9 mg). LCMS (ESI) m/z: [M+1]=851.4.

Synthesis in Examples 2 to 24: preparation methods for TM-2 to TM-24 in the Examples are similar to the preparation for the intermediate TM-1, as shown in Table 16.

**Table 16 Compound No. and structure**

| Example No. | Compound No. | Structure | Characterization |
|---|---|---|---|
| Example 2 | TM-2 | 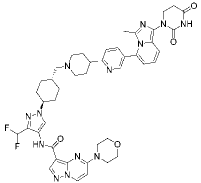 | LCMS (ESI) m/z: [M+1]=862.4. |
| Example 3 | TM-3 | 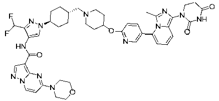 | LCMS (ESI) m/z: [M+1]=878.3. |
| Example 4 | TM-4 | 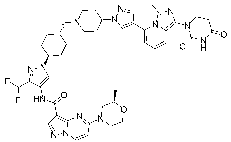 | LCMS (ESI) m/z: [M+1]=865.4. |
| Example 5 | TM-5 | 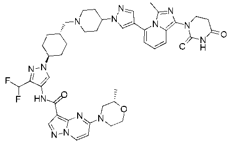 | LCMS (ESI) m/z: [M+1]=865.4. |
| Example 6 | TM-6 | 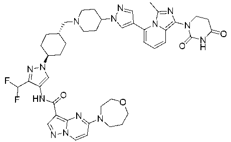 | LCMS (ESI) m/z: [M+1]=865.4. |
| Example 7 | TM-7 | 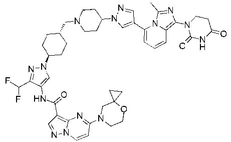 | LCMS (ESI) m/z: [M+1]=877.4. |
| Example 8 | TM-8 | 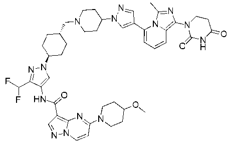 | LCMS (ESI) m/z: [M+1]=879.5. |
| Example 9 | TM-09 | 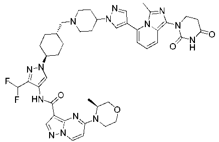 | LCMS (ESI) m/z: [M+1]=865.4. |
| Example 10 | TM-10 | 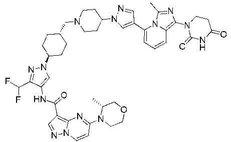 | LCMS (ESI) m/z: [M+1]=865.4. |
| Example 11 | TM-11 | 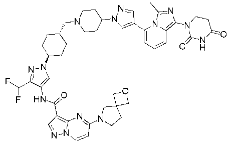 | LCMS (ESI) m/z: [M+1]=877.4. |
| Example 12 | TM-12 | 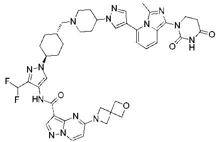 | LCMS (ESI) m/z: [M+1]=863.3. |
| Example 13 | TM-13 | 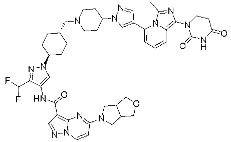 | LCMS (ESI) m/z: [M+1]=877.4. |
| Example 14 | TM-14 | 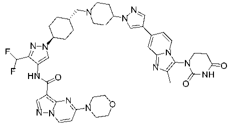 | LCMS (ESI) m/z: [M+1]=851.4. |
| Example 15 | TM-15 | 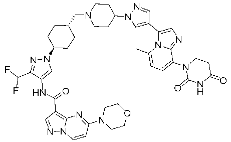 | LCMS (ESI) m/z: [M+1]=851.3. |
| Example 16 | TM-16 | 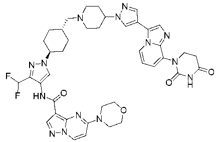 | LCMS (ESI) m/z: [M+1]=837.3. |
| Example 17 | TM-17 | 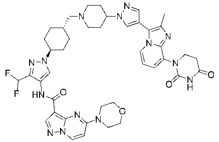 | LCMS (ESI) m/z: [M+1]=851.4. ¹H NMR (400 MHz, DMSO-d₆) δ 10.46 (s, 1H), 9.40 (s, 1H), 8.81 (d, J=7.9 Hz, 1H), 8.55-8.18 (m, 4H), 7.80 (s, 1H), 7.23 (d, J=7.2 Hz, 1H), 7.12-6.78 (m, 3H), 4.33-4.14 (m, 2H), 3.86 (t, J=6.7 Hz, 2H), 3.78 (br, 4H), 3.72 (br, 4H), 2.98 (d, J=8.2 Hz, 2H), 2.78 (t, J=6.6 Hz, 2H), 2.38 (s, 3H), 2.30-1.54 (m, 11H), 1.16-1.00 (m, 2H). |
| Example 18 | TM-18 | 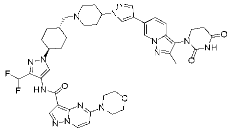 | LCMS (ESI) m/z: [M+1]=851.4. |
| Example 19 | TM-19 | 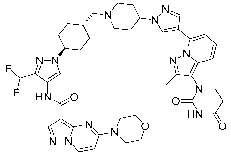 | LCMS (ESI) m/z: [M+1]=851.3. |
| Example 20 | TM-20 | 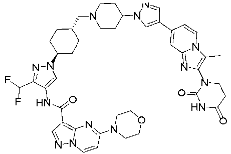 | LCMS (ESI) m/z: [M+1]=851.4. |
| Example 21 | TM-21 | 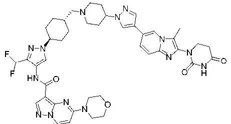 | LCMS (ESI) m/z: [M+1]=851.4. |
| Example 22 | TM-22 | 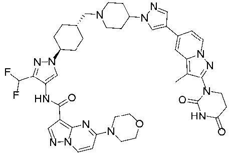 | LCMS (ESI) m/z: [M+1]=851.4. |
| Example 23 | TM-23 | 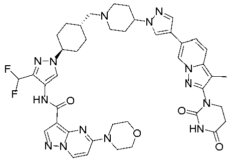 | LCMS (ESI) m/z: [M+1]=851.5. |
| Example 24 | TM-24 | 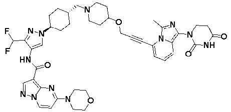 | LCMS (ESI) m/z: [M+1]=839.4. |

### Example 25: Synthesis of TM-25

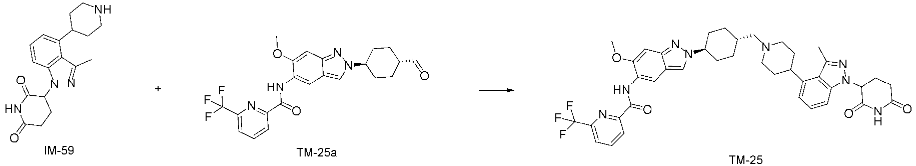

The IM-59 (23 mg) was dissolved in DMF/THF (0.25 mL/1 mL), the reaction mixture was cooled to -10°C, TM-24a (30 mg, method derived from WO2020/264499) and TEA (10 mg) were added, and the mixture was stirred under argon protection for 0.5 h. Then, NaBH(OAc)₃ (21 mg) was added to the reaction mixture. The reaction mixture was stirred for 3 h, quenched with water, and extracted with ethyl acetate. The organic layers were combined, washed with saturated brine, then dried over anhydrous sodium sulfate, concentrated, and subjected to reverse-phase preparative HPLC, to give 15 mg of the target compound. LCMS (ESI) m/z: [M+1]=757.34. ¹H NMR (400 MHz, Methanol-d₄) δ 8.77 (s, 1H), 8.47 (d, *J*=7.9 Hz, 1H), 8.31 (t, *J*=7.7 Hz, 1H), 8.20 (s, 1H), 8.05 (d, *J*=7.9 Hz, 1H), 7.46-7.33 (d, *J*=9.9 Hz, 2H), 7.13-7.03 (m, 2H), 5.65 (dd, *J*=11.2, 4.8 Hz, 1H), 4.51-4.37 (m, 1H), 4.06 (s, 3H),3.84-3.61 (m, 3H),3.38-3.33 (m, 2H), 3.22-3.08 (m, 2H), 2.98-2.69 (m, 7H), 2.41-1.98 (m, 10H), 1.67-1.04 (m, 4H).

### Example 26: Synthesis of TM-26

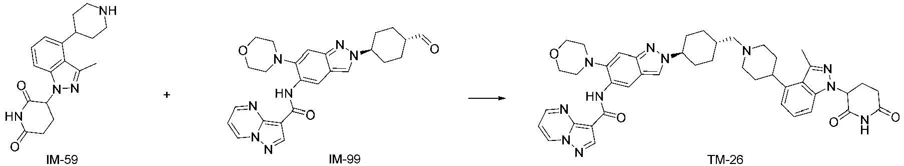

Preparation method for the TM-26 is similar to that for the TM-25. LCMS (ESI) m/z: [M+1]=784.5.

### Example 27: Synthesis of TM-27

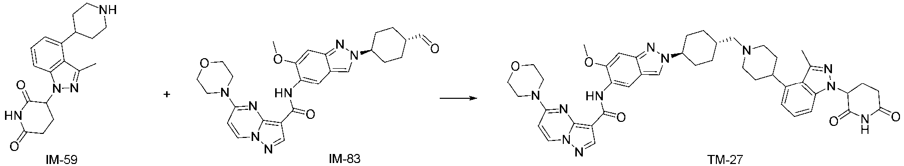

Preparation method for the TM-27 is similar to that for the TM-25. LCMS (ESI) m/z: [M+1]=814.6.

### Example 28: Synthesis of TM-28

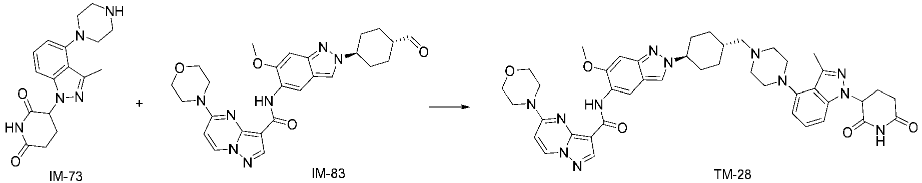

Preparation method for the TM-28 is similar to that for the TM-25. LCMS (ESI) m/z: [M+1]=815.5.

Synthesis in Examples 29 to 114: preparation methods for TM-29 to TM-114 in the Examples are similar to the preparation for the TM-25, as shown in Table 17.

**Table 17 Compound No. and structure**

| Example No. | Compound No. | Structure | Characterization |
|---|---|---|---|
| Example 29 | TM-29 | 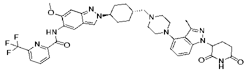 | LCMS (ESI) m/z: [M+1]=758.4. ¹H NMR (400 MHz, Methanol-*d*₄) δ 8.76 (s, 1H), 8.46 (d, *J*=7.8 Hz, 1H), 8.30 (t, *J*=7.9 Hz, 1H), 8.19 (s, 1H), 8.04 (d, *J*=7.8 Hz, 1H), 7.33 (t, *J*=7.9 Hz, 1H), 7.24 (d, *J*=8.2 Hz, 1H), 7.07 (s, 1H), 6.77 (d, *J*=7.2 Hz, 1H), 5.62 (dd, *J*=11.9, 5.1 Hz, 1H), 4.51-4.36 (m, 1H), 4.05 (s, 3H), 2.97-2.67 (m, 8H), 2.40-2.22 (m, 3H), 2.19-1.86 (m, 5H), 1.44-1.24 (m, 3H). |
| Example 30 | TM-30 | 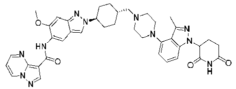 | LCMS (ESI) m/z: [M+1]=730.3. ¹H NMR (400 MHz, DMSO-*d*₆) δ 11.04 (s, 1H), 10.53 (s, 1H), 9.37 (dd, *J*=7.0, 1.6 Hz, 1H), 8.96 (dd, *J*=4.2, 1.6 Hz, 1H), 8.70 (s, 1H), 8.68 (s, 1H), 8.28 (s, 1H), 7.35 (dd, *J*=7.0, 4.2 Hz, 1H), 7.31-7.21 (m, 1H), 7.17 (d, *J*=8.4 Hz, 1H), 7.10 (s, 1H), 6.63 (d, *J*=7.3 Hz, 1H), 5.68 (dd, *J*=12.0, 5.3 Hz, 1H), 4.41-4.32 (m, 1H), 4.02 (s, 3H), 3.03 (br, 4H), 2.91-2.79 (m, 1H), 2.78-2.57 (m, 6H), 2.30-2.11 (m, 5H), 2.04-1.84 (m, 5H), 1.68 (br, 2H), 1.20-1.09 (m, 3H). |
| Example 31 | TM-31 | 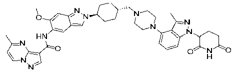 | LCMS (ESI) m/z: [M+1]=744.4. |
| Example 32 | TM-32 | 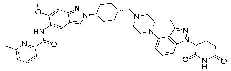 | LCMS (ESI) m/z: [M+1]=704.4. ¹H NMR (400 MHz, DMSO-*d*₆) δ 11.04 (s, 1H), 10.70 (s, 1H), 8.70 (s, 1H), 8.30 (s, 1H), 8.14-7.81 (m, 2H), 7.55 (dd, *J*=7.0, 1.6 Hz, 1H), 7.31-7.21 (m, 1H), 7.17 (d, *J*=8.4 Hz, 1H), 7.13 (s, 1H), 6.63 (d, *J*=7.2 Hz, 1H), 5.68 (dd, *J*=11.8, 5.0 Hz, 1H), 4.56-4.12 (m, 1H), 4.00 (s, 3H), 3.03 (br, 4H), 2.93-2.76 (m, 1H), 2.78-2.55 (m, 9H), 2.34-2.09 (m, 5H), 2.06-1.82 (m, 4H), 1.75-1.62 (br, 1H), 1.21-1.07 (m, 2H). |
| Example 33 | TM-33 | 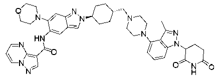 | LCMS (ESI) m/z: [M+1]=785.4. ¹H NMR (400 MHz, DMSO-*d*₆) δ 11.03 (s, 1H), 10.67 (s, 1H), 9.38 (dd, *J*=7.0, 1.5 Hz, 1H), 8.97 (dd, *J*=4.1, 1.5 Hz, 1H), 8.75 (s, 1H), 8.72 (s, 1H), 8.32 (s, 1H), 7.45 (s, 1H), 7.36 (dd, *J*=7.0, 4.2 Hz, 1H), 7.30-7.21 (m, 1H), 7.17 (d, *J*=8.4 Hz, 1H), 6.63 (d, *J*=7.2 Hz, 1H), 5.68 (dd, *J*=11.8, 5.0 Hz, 1H), 4.47-4.34 (m, 1H), 3.98-3.82 (m, 4H), 3.13-2.96 (s, 4H), 2.95-2.88 (m, 4H), 2.87-2.79 (m, 1H), 2.77-2.56 (m, 8H), 2.36-2.10 (m, 5H), 2.04-1.85 (m, 4H), 1.76-1.62 (m, 1H), 1.21-1.06 (m, 3H). |
| Example 34 | TM-34 | 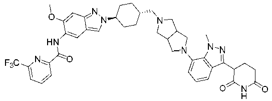 | LCMS (ESI) m/z: [M+1]=784.3. |
| Example 35 | TM-35 | 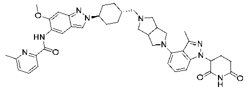 | LCMS (ESI) m/z: [M+1]=730.4. ¹H NMR (400 MHz, DMSO-*d*₆) δ 11.04 (s, 1H), 10.70 (s, 1H), 8.69 (s, 1H), 8.29 (s, 1H), 8.02-7.94 (m, 2H), 7.55 (dd, *J*=6.9, 1.5 Hz, 1H), 7.26-7.16 (m, 1H), 7.16-7.08 (m, 2H), 6.59 (d, *J*=7.3 Hz, 1H), 5.67 (dd, *J*=11.8, 5.0 Hz, 1H), 4.42-4.30 (m, 1H), 3.99 (s, 3H), 3.30-3.20 (m, 2H), 3.07-2.78 (m, 6H), 2.77-2.53 (m, 10H), 2.32 (br, 2H), 2.30-2.08 (m, 3H), 2.04-1.81 (m, 4H), 1.68-1.53 (m, 1H), 1.21-1.03 (m, 3H). |
| Example 36 | TM-36 | 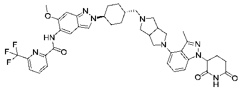 | LCMS (ESI) m/z: [M+1]=784.3. ¹H NMR (400 MHz, DMSO-*d*₆) δ 11.04 (s, 1H), 10.50 (s, 1H), 8.68 (s, 1H), 8.46 (d, *J*=7.7 Hz, 1H), 8.40 (t, *J*=7.7 Hz, 1H), 8.33 (s, 1H), 8.21 (d, *J*=7.6 Hz, 1H), 7.31-7.05 (m, 3H), 6.70-6.53 (m, 1H), 5.67 (dd, *J*=11.7, 4.9 Hz, 1H), 4.43-4.31 (m, 1H), 3.98 (s, 3H), 3.10-2.78 (m, 8H), 2.79-2.54 (m, 7H), 2.41-2.27 (m, 2H), 2.25-2.08 (m, 3H), 2.03-1.82 (m, 4H), 1.67-1.51 (m, 1H), 1.30-1.08 (m, 3H). |
| Example 37 | TM-37 | 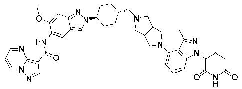 | LCMS (ESI) m/z: [M+1]=756.3. ¹H NMR (400 MHz, DMSO-*d*₆) δ 11.02 (br, 1H), 10.53 (s, 1H), 9.37 (dd, *J*=7.0, 1.6 Hz, 1H), 8.95 (dd, *J*=4.2, 1.6 Hz, 1H), 8.70 (s, 1H), 8.67 (s, 1H), 8.27 (s, 1H), 7.34 (dd, *J*=7.0, 4.2 Hz, 1H), 7.26-7.16 (m, 1H), 7.12 (d, *J*=8.4 Hz, 1H), 7.09 (s, 1H), 6.58 (d, *J*=7.3 Hz, 1H), 5.66 (dd, *J*=11.9, 5.0 Hz, 1H), 4.40-4.28 (m, 1H), 4.02 (s, 3H), 3.31-3.21 (m, 2H), 3.00-2.78 (m, 5H), 2.77-2.61 (m, 5H), 2.57-2.52 (m, 3H), 2.28 (d, *J*=7.1 Hz, 2H), 2.24-2.08 (m, 3H), 2.04-1.82 (m, 5H), 1.63-1.50 (m, 1H), 1.19-1.05 (m, 2H). |
| Example 38 | TM-38 | 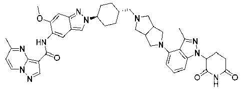 | LCMS (ESI) m/z: [M+1]=770.4. |
| Example 39 | TM-39 | 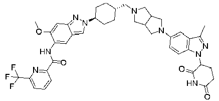 | LCMS (ESI) m/z: [M+1]=784.4. |
| Example 40 | TM-40 | 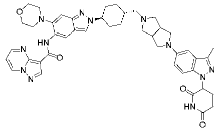 | LCMS (ESI) m/z: [M+1]=811.41. ¹H NMR (400 MHz, DMSO-*d*₆) δ 11.00 (s, 1H), 10.66 (s, 1H), 9.38 (dd, *J*=7.0, 1.6 Hz, 1H), 8.97 (dd, *J*=4.2, 1.6 Hz, 1H), 8.74 (s, 1H), 8.72 (s, 1H), 8.30 (s, 1H), 7.44 (s, 1H), 7.42-7.31 (m, 2H), 7.03 (dd, *J*=9.2, 2.1 Hz, 1H), 6.76 (s, 1H), 5.62 (dd, *J*=11.7, 5.1 Hz, 1H), 4.43-4.29 (m, 1H), 3.95-3.84 (m, 4H), 3.51-3.44 (m, 2H), 3.04-2.97 (m, 2H), 2.97-2.79 (m, 7H), 2.73-2.57 (m, 5H), 2.41 (s, 3H), 2.27 (d, *J*=7.1 Hz, 2H), 2.24-2.07 (m, 3H), 2.04-1.82 (m, 5H), 1.63-1.51 (m, 1H), 1.19-0.99 (m, 2H). |
| Example 41 | TM-41 | 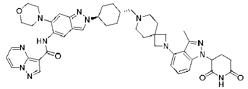 | LCMS (ESI) m/z: [M+1]=825.53. ¹H NMR (400 MHz, DMSO-*d*₆) δ 11.03 (s, 1H), 10.67 (s, 1H), 9.39 (dd, *J*=7.0, 1.6 Hz, 1H), 8.98 (dd, *J*=4.2, 1.6 Hz, 1H), 8.75 (s, 1H), 8.72 (s, 1H), 8.32 (s, 1H), 7.45 (s, 1H), 7.36 (dd, *J=*7.0, 4.2 Hz, 1H), 7.15 (t, *J*=7.90 Hz, 1H), 6.87 (d, *J*=8.3 Hz, 1H), 6.09 (d, *J*=7.7 Hz, 1H), 5.62 (dd, *J*=12.1, 5.0 Hz, 1H), 4.44-4.33 (m, 1H), 3.98-3.83 (m, 4H), 3.77 (s, 3H), 2.96-2.78 (m, 5H), 2.75-2.63 (m, 3H), 2.55 (s, 3H), 2.43-2.31 (m, 3H), 2.22-2.08 (m, 5H), 2.02-1.86 (m, 5H), 1.82-1.75 (m, 4H), 1.70-1.58 (m, 1H), 1.18-1.00 (m, 2H). |
| Example 42 | TM-42 | 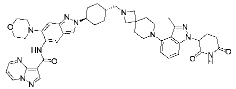 | LCMS (ESI) m/z: [M+1]=825.53. ¹H NMR (400 MHz, DMSO-*d*₆) δ 11.04 (s, 1H), 10.67 (s, 1H), 9.39 (dd, *J*=7.0, 1.6 Hz, 1H), 8.97 (dd, *J*=4.2, 1.6 Hz, 1H), 8.75 (s, 1H), 8.72 (s, 1H), 8.32 (s, 1H), 7.45 (s, 1H), 7.36 (dd, *J*=7.0, 4.2 Hz, 1H), 7.28-7.19 (m, 1H), 7.15 (d, *J*=8.5 Hz, 1H), 6.60 (d, *J*=7.3 Hz, 1H), 5.67 (dd, *J*=12.0, 5.2 Hz, 1H), 4.45-4.33 (m, 1H), 3.95-3.85 (m, 4H), 3.13-3.02 (m, 4H), 3.00-2.77 (m, 9H), 2.75-2.64 (m, 2H), 2.60 (s, 3H), 2.44-2.35 (m, 2H), 2.24-2.08 (m, 4H), 1.99-1.83 (m, 7H), 1.49-1.36 (m, 1H), 1.21-0.98 (m, 2H). |
| Example 43 | TM-43 | 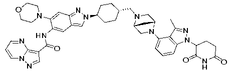 | LCMS (ESI) m/z: [M+1]=797.17. ¹H NMR (400 MHz, DMSO-*d*₆) δ 11.03 (s, 1H), 10.66 (s, 1H), 9.38 (dd, *J*=7.0, 1.6 Hz, 1H), 8.97 (dd, *J*=4.2, 1.6 Hz, 1H), 8.74 (s, 1H), 8.72 (s, 1H), 8.31 (s, 1H), 7.44 (s, 1H), 7.36 (dd, *J*=7.0, 4.2 Hz, 1H), 7.17 (t, *J*=7.7 Hz, 1H), 6.98 (dd, *J*=8.4, 2.2 Hz, 1H), 6.51 (dd, *J*=7.4, 4.7 Hz, 1H), 5.64 (dd, *J*=11.7, 5.1 Hz, 1H), 4.46-4.32 (m, 1H), 4.18-4.11 (br, 1H), 3.94-3.82 (m, 4H), 3.58-3.46 (m, 2H), 2.96-2.87 (m, 4H), 2.86-2.65 (m, 3H), 2.60 (s, 3H), 2.50-2.38 (m, 2H), 2.25-2.06 (m, 4H), 2.06-1.74 (m, 6H), 1.54-1.41 (br, 2H), 1.21-1.04 (m, 3H). |
| Example 44 | TM-44 | 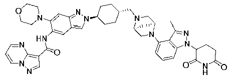 | LCMS (ESI) m/z: [M+1]=797.82. ¹H NMR (400 MHz, DMSO-*d*₆) δ 11.03 (s, 1H), 10.67 (s, 1H), 9.38 (dd, *J*=7.0, 1.6 Hz, 1H), 8.97 (dd, *J*=4.2, 1.6 Hz, 1H), 8.74 (s, 1H), 8.72 (s, 1H), 8.31 (s, 1H), 7.44 (s, 1H), 7.36 (dd, *J*=7.0, 4.2 Hz, 1H), 7.17 (t, *J*=7.8 Hz, 1H), 6.98 (d, *J*=8.4 Hz, 1H), 6.60-6.43 (m, 1H), 5.64 (dd, *J*=11.6, 5.2 Hz, 1H), 4.45-4.33 (m, 1H), 4.18-4.11 (br, 1H), 3.94-3.84 (m, 4H), 3.57-3.46 (m, 2H), 2.98-2.84 (m, 4H), 2.87-2.64 (m, 3H), 2.61 (s, 3H), 2.50-2.38 (m, 2H), 2.25-2.06 (m, 4H), 2.06-1.74 (m, 6H), 1.54-1.41 (br, 2H), 1.21-1.04 (m, 3H). |
| Example 45 | TM-45 | 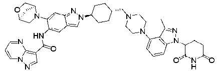 | LCMS (ESI) m/z: [M+1]=797.70. ¹H NMR (400 MHz, DMSO-*d*₆) δ 11.04 (s, 1H), 10.48 (s, 1H), 9.38 (dd, *J*=7.0, 1.6 Hz, 1H), 8.87 (dd, *J*=4.2, 1.6 Hz, 1H), 8.71 (s, 1H), 8.60 (s, 1H), 8.28 (s, 1H), 7.35 (dd, *J*=7.0, 4.2 Hz, 1H), 7.32 (s, 1H), 7.29-7.21 (m, 1H), 7.17 (d, *J*=8.3 Hz, 1H), 6.63 (d, *J*=7.2 Hz, 1H), 5.68 (dd, *J*=11.8, 5.2 Hz, 1H), 4.57 (s, 1H), 4.41-4.32 (m, 1H), 4.18 (s, 1H), 4.05 (d, *J*=7.3 Hz, 1H), 3.72 (d, *J*=5.6 Hz, 1H), 3.54 (d, *J*=9.5 Hz, 1H), 3.12-2.96 (m, 4H), 2.89-2.79 (m, 1H), 2.75-2.56 (m, 7H), 2.33-2.12 (m, 5H), 2.06-1.81 (m, 5H), 1.75-1.62 (m, 2H), 1.22-1.05 (m, 3H). |
| Example 46 | TM-46 | 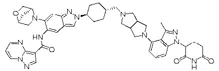 | LCMS (ESI) m/z: [M+1]=823.59. ¹H NMR (400 MHz, DMSO-*d*₆) δ 11.04 (br, 1H), 10.48 (s, 1H), 9.38 (dd, *J*=7.0, 1.6 Hz, 1H), 8.87 (dd, *J*=4.2, 1.6 Hz, 1H), 8.71 (s, 1H), 8.60 (s, 1H), 8.27 (s, 1H), 7.35 (dd, *J*=7.0, 4.2 Hz, 1H), 7.31 (s, 1H), 7.27-7.16 (m, 1H), 7.13 (d, *J*=8.4 Hz, 1H), 6.59 (d, *J*=7.3 Hz, 1H), 5.67 (dd, *J*=11.9, 5.1 Hz, 1H), 4.56 (s, 1H), 4.42-4.32 (m, 1H), 4.18 (s, 1H), 4.05 (d, *J*=7.3 Hz, 1H), 3.73-3.69 (m, 1H), 3.54 (d, *J*=9.6 Hz, 1H), 3.02 (d, *J*=8.3 Hz, 1H), 2.98-2.78 (m, 6H), 2.77-2.60 (m, 5H), 2.59-2.52 (m, 4H), 2.35-2.10 (m, 6H), 2.04-1.80 (m, 6H), 1.64-1.49 (m, 2H), 1.18-1.02 (m, 3H). |
| Example 47 | TM-47 | 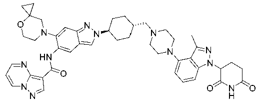 | LCMS (ESI) m/z: [M+1]=811.58. ¹H NMR (400 MHz, DMSO-*d*₆) δ 11.04 (s, 1H), 10.60 (s, 1H), 9.38 (dd, *J*=7.0, 1.6 Hz, 1H), 8.99 (dd, *J*=4.2, 1.6 Hz, 1H), 8.72 (s, 2H), 8.32 (s, 1H), 7.46 (s, 1H), 7.34 (dd, *J*=7.0, 4.2 Hz, 1H), 7.29-7.22 (m, 1H), 7.17 (d, *J*=8.4 Hz, 1H), 6.63 (d, *J*=7.1 Hz, 1H), 5.68 (dd, *J*=11.7, 4.9 Hz, 1H), 4.46-4.32 (m, 1H), 3.92 (s, 2H), 3.12-2.87 (m, 9H), 2.77-2.59 (m, 8H), 2.30-2.10 (m, 4H), 2.03-1.87 (m, 4H), 1.76-1.60 (m, 1H), 1.53-1.39 (m, 1H), 1.32-1.11 (m, 2H), 0.88-0.82 (m, 1H), 0.78-0.72 (m, 2H), 0.53-0.46 (m, 2H). |
| Example 48 | TM-48 | 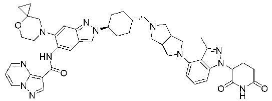 | LCMS (ESI) m/z: [M+1]=837.41. ¹H NMR (400 MHz, DMSO-*d*₆) δ 11.03 (s, 1H), 10.60 (s, 1H), 9.38 (dd, *J*=7.0, 1.6 Hz, 1H), 8.99 (dd, *J*=4.2, 1.6 Hz, 1H), 8.72 (s, 1H), 8.71 (s, 1H), 8.31 (s, 1H), 7.45 (s, 1H), 7.34 (dd, *J*=7.0, 4.2 Hz, 1H), 7.25-7.18 (m, 1H), 7.13 (d, *J*=8.5 Hz, 1H), 6.59 (d, *J*=7.4 Hz, 1H), 5.67 (dd, *J*=11.9, 5.1 Hz, 1H), 4.44-4.34 (m, 1H), 3.96-3.87 (m, 2H), 3.05-2.80 (m, 9H), 2.73-2.54 (m, 8H),2.34-2.08 (m, 5H), 2.02-1.85 (m, 5H), 1.68-1.40 (m, 2H), 1.20-1.07 (m, 2H), 0.89-0.83 (m, 1H), 0.78-0.71 (m, 2H), 0.53-0.46 (m, 2H). |
| Example 49 | TM-49 | 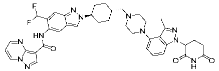 | LCMS (ESI) m/z: [M+1]=750.35. ¹H NMR (400 MHz, DMSO-*d*₆) δ 11.04 (s, 1H), 9.97 (s, 1H), 9.38 (dd, *J*=7.0, 1.6 Hz, 1H), 8.88 (dd, *J*=4.2, 1.5 Hz, 1H), 8.72 (s, 1H), 8.53 (s, 1H), 8.33 (s, 1H), 7.95 (s, 1H), 7.33 (dt, *J*=7.0, 3.6 Hz, 1H), 7.28-7.06 (m, 3H), 6.63 (d, *J*=7.3 Hz, 1H), 5.68 (dd, *J*=11.7, 5.1 Hz, 1H), 4.76-4.29 (m, 1H), 4.04 (s, 2H), 3.03 (br, 3H), 2.91-2.78 (m, 1H), 2.77-2.57 (m, 7H), 2.39-2.13 (m, 5H), 2.11-1.90 (m, 4H), 1.85-1.56 (m, 1H), 1.31-1.10 (m, 3H). |
| Example 50 | TM-50 | 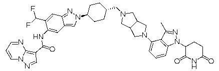 | LCMS (ESI) m/z: [M+1]=776.36. ¹H NMR (400 MHz, DMSO-*d*₆) δ 11.04 (s, 1H), 9.96 (s, 1H), 9.38 (dd, *J*=7.0, 1.6 Hz, 1H), 8.88 (dd, *J*=4.2, 1.6 Hz, 1H), 8.72 (s, 1H), 8.52 (s, 1H), 8.32 (s, 1H), 7.94 (s, 1H), 7.33 (dd, *J*=7.0, 4.2 Hz, 1H), 7.25-7.04 (m, 3H), 6.59 (d, *J*=7.3 Hz, 1H), 5.67 (dd, *J*=11.8, 5.0 Hz, 1H), 4.56-4.44 (m, 1H), 3.30-3.22 (m, 2H), 2.99-2.76 (m, 5H), 2.78-2.62 (m, 5H), 2.61-2.52 (m, 4H), 2.29 (d, *J*=7.1 Hz, 2H), 2.27-2.13 (m, 3H), 2.05-1.87 (m, 4H), 1.70-1.52 (m, 1H), 1.20-1.08 (m, 2H). |
| Example 51 | TM-51 | 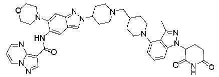 | LCMS (ESI) m/z: [M+1]=785.30. ¹H NMR (600 MHz, DMSO-*d*₆) δ 11.04 (s, 1H), 10.67 (s, 1H), 9.39 (dd, *J*=6.9, 1.3 Hz, 1H), 8.97 (dd, *J*=4.0, 1.3 Hz, 1H), 8.76 (s, 1H), 8.72 (s, 1H), 8.37 (s, 1H), 7.46 (s, 1H), 7.36 (dd, *J*=6.9, 4.2 Hz, 1H), 7.23 (t, *J*=7.8 Hz, 1H), 7.15 (d, *J*=8.4 Hz, 1H), 6.61 (d, *J*=7.4 Hz, 1H), 5.68 (dd, *J*=11.9, 5.1 Hz, 1H), 4.43 (br, 1H), 4.12-3.68 (m, 4H), 3.37-3.28 (m, 3H), 3.02 (br, 2H), 3.00-2.82 (m, 4H), 2.89-2.78 (m, 1H), 2.74-2.64 (m, 4H), 2.61 (s, 3H), 2.34-2.27 (m,, 2H), 2.24-2.17 (m, 1H), 2.17-2.05 (m, 5H), 1.94-1.86 (m, 2H), 1.77-1.68 (m, 1H), 1.45-1.34 (m, 2H). |
| Example 52 | TM-52 | 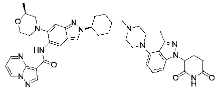 | LCMS (ESI) m/z: [M+1]=799.80. ¹H NMR (400 MHz, DMSO-*d*₆) δ 11.04 (s, 1H), 10.66 (s, 1H), 9.38 (dd, *J*=7.0, 1.6 Hz, 1H), 8.93 (dd, *J*=4.2, 1.6 Hz, 1H), 8.77 (s, 1H), 8.72 (s, 1H), 8.32 (s, 1H), 7.44 (s, 1H), 7.35 (dd, *J*=7.0, 4.2 Hz, 1H), 7.30-7.21 (m, 1H), 7.17 (d, *J*=8.3 Hz, 1H), 6.63 (d, *J*=7.3 Hz, 1H), 5.68 (dd, *J*=11.8, 5.0 Hz, 2H), 4.46-4.32 (m, 1H), 4.14-3.74 (m, 3H), 3.12-2.78 (m, 8H), 2.76-2.55 (m, 8H), 2.37-2.12 (m, 6H), 2.05-1.82 (m, 4H), 1.77-1.60 (m, 1H), 1.36-1.04 (m, 5H). |
| Example 53 | TM-53 | 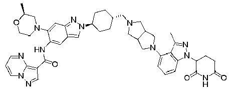 | LCMS (ESI) m/z: [M+1]=825.30. ¹H NMR (400 MHz, DMSO-*d*₆) δ 11.04 (s, 1H), 10.66 (s, 1H), 9.38 (dd, *J*=7.0, 1.3 Hz, 1H), 8.93 (dd, *J*=4.1, 1.3 Hz, 1H), 8.76 (s, 1H), 8.72 (s, 1H), 8.31 (s, 1H), 7.43 (s, 1H), 7.35 (dd, *J*=6.9, 4.2 Hz, 1H), 7.27-7.16 (m, 1H), 7.13 (d, *J*=8.4 Hz, 1H), 6.59 (d, *J*=7.3 Hz, 1H), 5.67 (dd, *J*=11.8, 5.1 Hz, 1H), 4.45-4.32 (m, 1H), 4.06-3.84 (m, 3H), 3.30-3.19 (m, 2H), 3.02-2.76 (m, 8H), 2.74-2.61 (m, 5H), 2.61-2.52 (m, 4H), 2.35-2.08 (m, 5H), 2.05-1.84 (m, 4H), 1.68-1.49 (m, 1H), 1.36-1.07 (m, 5H). |
| Example 54 | TM-54 | 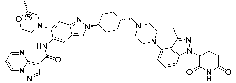 | LCMS (ESI) m/z: [M+1]=799.30. ¹H NMR (400 MHz, DMSO-*d*₆) δ 11.04 (s, 1H), 10.66 (s, 1H), 9.38 (dd, *J*=7.0, 1.6 Hz, 1H), 8.93 (dd, *J*=4.2, 1.6 Hz, 1H), 8.77 (s, 1H), 8.72 (s, 1H), 8.32 (s, 1H), 7.44 (s, 1H), 7.35 (dd, *J*=7.0, 4.2 Hz, 1H), 7.29-7.21 (m, 1H), 7.17 (d, *J=*8.3 Hz, 1H), 6.63 (d, *J*=7.2 Hz, 1H), 5.69 (dd, *J*=11.8, 5.0 Hz, 1H), 4.53-4.30 (m, 1H), 4.12-3.74 (m, 3H), 3.12-2.95 (m, 5H), 2.95-2.79 (m, 3H), 2.76-2.53 (m, 9H), 2.31-2.09 (m, 6H), 2.04-1.83 (m, 4H), 1.76-1.58 (m, 1H), 1.26-1.07 (m, 5H). |
| Example 55 | TM-55 | 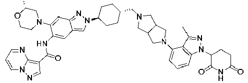 | LCMS (ESI) m/z: [M+1]=825.3. ¹H NMR (400 MHz, DMSO-*d*₆) δ 11.04 (s, 1H), 10.66 (s, 1H), 9.38 (dd, *J*=7.0, 1.3 Hz, 1H), 8.93 (d, *J*=2.7 Hz, 1H), 8.76 (s, 1H), 8.72 (s, 1H), 8.31 (s, 1H), 7.43 (s, 1H), 7.35 (dd, *J*=6.9, 4.2 Hz, 1H), 7.28-7.17 (m, 1H), 7.12 (d, *J*=8.4 Hz, 1H), 6.58 (d, *J*=7.3 Hz, 1H), 5.67 (dd, *J*=11.8, 5.0 Hz, 1H), 4.38-4.32 (m, 1H), 4.19-3.78 (m, 3H), 3.28-3.20 (m, 1H), 3.06-2.77 (m, 7H), 2.78-2.62 (m, 5H), 2.59-2.49 (m, 4H), 2.32-2.07 (m, 5H), 2.04-1.82 (m, 4H), 1.64-1.51 (m, 1H), 1.19-1.04 (m, 5H). |
| Example 56 | TM-56 | 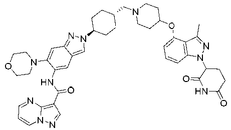 | LCMS (ESI) m/z: [M+1]=800.39. ¹H NMR (400 MHz, DMSO-d₆) δ 11.04 (s, 1H), 10.67 (s, 1H), 9.38 (dd, J=7.0, 1.6 Hz, 1H), 8.97 (dd, J=4.2, 1.6 Hz, 1H), 8.75 (s, 1H), 8.72 (s, 1H), 8.32 (s, 1H), 7.45 (s, 1H), 7.36 (dd, J=7.0, 4.2 Hz, 1H), 7.23 (t, J=8.0 Hz, 1H), 7.02 (d, J=8.5 Hz, 1H), 6.57 (d, J=7.8 Hz, 1H), 5.65 (dd, J=11.9, 5.1 Hz, 1H), 4.64 (br, 1H), 4.44-4.33 (m, 1H), 3.95-3.82 (m, 4H), 2.97-2.88 (m, 4H), 2.88-2.78 (m, 1H), 2.76-2.61 (m, 3H), 2.58 (s, 3H), 2.45-2.32 (m, 2H), 2.26-2.08 (m, 4H), 2.10-1.56 (m, 9H), 1.35-1.04 (m, 4H). |
| Example 57 | TM-57 | 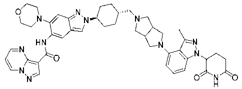 | LCMS (ESI) m/z: [M+1]=811.41. ¹H NMR (400 MHz, DMSO-*d*₆) δ 11.03 (s, 1H), 10.66 (s, 1H), 9.38 (dd, *J*=7.0, 1.4 Hz, 1H), 9.12-8.87 (m, 1H), 8.74 (s, 1H), 8.72 (s, 1H), 8.31 (s, 1H), 7.44 (s, 1H), 7.36 (dd, *J*=7.0, 4.2 Hz, 1H), 7.27-7.16 (m, 1H), 7.12 (d, *J*=8.4 Hz, 1H), 6.59 (d, *J*=7.4 Hz, 1H), 5.66 (dd, *J*=11.9, 5.0 Hz, 1H), 4.46-4.33 (m, 1H), 3.88 (d, *J*=4.7 Hz, 4H), 3.02-2.78 (m, 9H), 2.80-2.60 (m, 5H), 2.58-2.50 (m, 4H), 2.36-2.09 (m, 5H), 2.08-1.80 (m, 5H), 1.58-1.51 (m, 1H), 1.20-1.05 (m, 3H). |
| Example 58 | TM-58 | 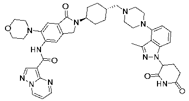 | LCMS (ESI) m/z: [M+1]=800.40. ¹HNMR (400 MHz, DMSO-*d*₆) δ11.04 (s.1H), 10.89 (s, 1H), 9.39 (dd, *J*=7.0, 1.6Hz, 1H), 8.99 (dd, *J*=4.2, 1.5Hz, 1H), 8.75 (s, 1H), 8.74 (s, 1H), 7.57 (s, 1H*),* 7.38 (dd, *J*=7.0, 4.2Hz, 1H), 7.24 (t, *J*=7.2 Hz,1H*)*,7.17 (d, *J*=8.3 Hz,1H),6.62 (d, *J*=7.1 Hz,1H),5.68 (dd, *J*=11.8, 5.0Hz, 1H), 4.43 (s, 2H), 4.00 (t, *J*=11.8 Hz,1H),3.94-3.82 (m, 4H), 3.15-2.80 (m, 10H), 2.77-2.55 (m, 8H), 2.28-2.16 (m, 3H), 1.98-1.85 (m, 2H), 1.83-1.71 (m, 2H), 1.73-1.48 (m, 3H), 1.16-0.98 (m, 2H). |
| Example 59 | TM-59 | 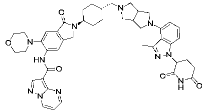 | LCMS (ESI) m/z: [M+1]=826.30. ¹H NMR (400 MHz, Chloroform-*d*) δ 10.91 (s, 1H), 9.13-8.64 (m, 4H), 8.20 (br, 1H), 7.72 (s, 1H),7.29-7.22 (m, 1H), 7.11 (dd, *J*=7.0, 4.2 Hz, 1H), 6.95 (d, *J*=8.4 Hz, 1H), 6.67 (d, *J*=7.5 Hz, 1H), 5.23 (dd, *J*=10.1, 5.1 Hz, 1H), 4.33 (s, 2H), 4.27-4.17 (m, 1H), 4.07-3.95 (m, 4H), 3.33-2.91 (m, 11 H), 2.87-2.77 (m, 2H), 2.73 (s, 3H), 2.67-2.34 (m, 5H), 2.15-1.83 (m, 6H), 1.70-1.52 (m, 4H), 0.92-0.78 (m, 2H). |
| Example 60 | TM-60 | 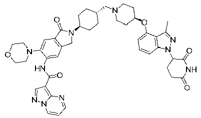 | LCMS (ESI) m/z: [M+1]=815.41. ¹H NMR (400 MHz, DMSO-*d*₆) δ 11.04 (s, 1H), 10.88 (s, 1H), 9.39 (dd, *J*=7.0, 1.2 Hz, 1H), 8.98 (dd, *J*=4.2, 1.4 Hz, 1H), 8.75 (s, 1H), 8.73 (s, 1H), 7.56 (s, 1H), 7.37 (dd, *J*=7.0, 4.2 Hz, 1H), 7.23 (t, *J*=8.0 Hz, 1H), 7.02 (d, *J*=8.5 Hz, 1H), 6.56 (d, *J*=7.8 Hz, 1H), 5.65 (dd, *J*=11.9, 5.0 Hz, 1H), 4.63 (br, 1H), 4.42 (s, 2H), 3.99 (t, *J*=11.8 Hz, 1H), 3.93-3.84 (m, 4H), 3.03-2.78 (m, 5H), 2.78-2.55 (m, 8H), 2.45-2.27 (m, 2H), 2.26-2.08 (m, 3H), 2.08-1.86 (m, 4H), 1.84-1.69 (m, 4H), 1.69-1.47 (m, 3H), 1.03 (q, *J*=11.5, 10.6 Hz, 2H). |
| Example 61 | TM-61 | 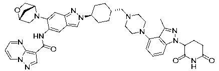 | LCMS (ESI) m/z: [M+1]=797.40. ¹H NMR (400 MHz, DMSO-*d*₆) δ 11.04 (s, 1H), 10.48 (s, 1H), 9.38 (dd, *J*=7.0, 1.6 Hz, 1H), 8.87 (dd, *J*=4.2, 1.7 Hz, 1H), 8.71 (s, 1H), 8.60 (s, 1H), 8.28 (d, *J*=0.9 Hz, 1H), 7.35 (dd, *J*=7.0, 4.2 Hz, 1H), 7.32 (s, 1H), 7.24 (dd, *J*=8.4, 7.2 Hz, 1H), 7.17 (d, *J*=8.4 Hz, 1H), 6.63 (d, *J*=7.2 Hz, 1H), 5.68 (dd, *J*=11.8, 5.1 Hz, 1H), 4.57 (s, 1H), 4.44-4.34 (m, 1H), 4.18 (s, 1H), 4.05 (d, *J*=7.3 Hz, 1H), 3.72 (d, *J*=7.3 Hz, 1H), 3.54 (d, *J*=9.6 Hz, 1H), 3.10-2.97 (m, 5H), 2.92-2.80 (m, 1H), 2.79-2.59 (m, 8H), 2.37-2.12 (m, 6H), 2.04-1.84 (m, 6H), 1.76-1.60 (m, 1H), 1.20-1.06 (m, 2H). |
| Example 62 | TM-62 | 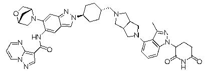 | LCMS (ESI) m/z: [M+1]=823.41. ¹H NMR (400 MHz, DMSO-*d₆*) δ 11.04 (s, 1H), 10.48 (s, 1H), 9.38 (dd, *J*=7.0, 1.6 Hz, 1H), 8.87 (dd, *J*=4.2, 1.7 Hz, 1H), 8.71 (s, 1H), 8.60 (s, 1H), 8.27 (d, *J*=0.8 Hz, 1H), 7.35 (dd, *J*=7.0, 4.2 Hz, 1H), 7.31 (s, 1H), 7.20 (dd, *J*=8.4, 7.3 Hz, 1H), 7.13 (d, *J*=8.4 Hz, 1H), 6.59 (d, *J*=7.3 Hz, 1H), 5.67 (dd, *J*=11.9, 5.1 Hz, 1H), 4.56 (s, 1H), 4.43-4.31 (m, 1H), 4.18 (s, 1H), 4.05 (d, *J*=7.3 Hz, 1H), 3.77-3.67 (m, 1H), 3.54 (d, *J*=9.6 Hz, 1H), 3.17 (d, *J*=5.2 Hz, 1H), 3.02 (d, *J*=9.2 Hz, 1H), 2.95-2.79 (m, 5H), 2.78-2.60 (m, 5H), 2.59-2.52 (m, 4H), 2.29 (d, *J*=7.1 Hz, 2H), 2.24-2.08 (m, 4H), 2.04-1.83 (m, 6H), 1.63-1.52 (m, 1H), 1.21-1.00 (m, 2H). |
| Example 63 | TM-63 | 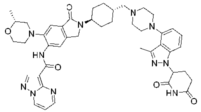 | LCMS (ESI) m/z: [M+1]=814.2. ¹H NMR (400 MHz, DMSO-*d₆*) δ11.05 (s, 1H), 10.88 (s, 1H), 9.39 (dt, *J* =7.0, 1.8 Hz, 1H), 8.95 (dt, *J* =4.3, 1.5 Hz, 1H), 8.86-8.65 (m, 2H), 7.55 (s, 1H), 7.40-7.35 (m, 1H), 7.27-7.21 (m, 1H), 7.20-7.15 (m, 1H), 6.62 (d, *J* =7.3 Hz, 1H), 5.69 (dd, *J* =11.8, 5.1 Hz, 1H), 4.43 (s, 2H), 4.06-3.80 (m, 4H), 3.02 (s, 4H), 2.91 (d, *J* =10.6 Hz, 1H), 2.88-2.80 (m, 3H), 2.76-2.57 (m, 10H), 2.27-2.17 (m, 3H), 1.92 (d, *J* =12.4 Hz, 2H), 1.83-1.73 (m, 2H), 1.65-1.52 (m, 3H), 1.15-1.00 (m, 5H). |
| Example 64 | TM-64 | 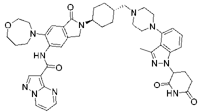 | LCMS (ESI) m/z: [M+1]=814.3. |
| Example 65 | TM-65 | 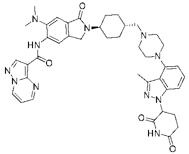 | LCMS (ESI) m/z: [M+1]=758.4. ¹H NMR (400 MHz, DMSO-*d*₆) δ 11.05 (s, 2H), 9.40-9.36 (m, 1H), 8.98-8.95 (m, 1H), 8.76-8.70 (m, 2H), 7.53 (s, 1H), 7.39-7.32 (m, 1H), 7.27-7.23 (m, 1H), 7.20-7.14 (m, 1H), 6.69-6.55 (m, 1H), 5.72-5.65 (m, 1H), 4.41 (s, 2H), 4.05-3.95 (m, 1H), 3.02 (s, 4H), 2.94-2.78 (m, 3H), 2.76 (s, 6H), 2.74-2.65 (m, 3H), 2.63-2.59 (m, 4H), 2.26-2.17 (m, 3H), 1.95-1.87 (m, 2H), 1.80-1.77 (m, 2H), 1.67-1.50 (m, 3H), 1.14-1.03 (m, 2H). |
| Example 66 | TM-66 | 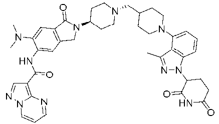 | LCMS (ESI) m/z: [M+1]=758.3. ¹H NMR (400 MHz, DMSO-d₆) δ 11.08 (s, 1H), 11.05 (s, 1H), 9.38 (dd, J=7.0, 1.7 Hz, 1H), 8.98 (dd, J=4.2, 1.7 Hz, 1H), 8.73 (d, J=5.8 Hz, 2H), 7.54 (s, 1H), 7.36 (dd, J=7.0, 4.2 Hz, 1H), 7.23 (dd, J=8.4, 7.3 Hz, 1H), 7.15 (d, J =8.4 Hz, 1H), 6.61 (d, J=7.4 Hz, 1H), 5.67 (dd, J=11.8, 5.1 Hz, 1H), 4.45 (s, 2H), 4.10-3.95 (m, 1H), 3.33 (s, 2H), 2.98 (d, J=10.9 Hz, 2H), 2.86 (td, J=12.9, 6.4 Hz, 1H), 2.76 (s, 6H), 2.74-2.65 (m, 4H), 2.61 (s, 3H), 2.27 (d, J=7.0 Hz, 2H), 2.24-2.18 (m, 1H), 2.08-1.98 (m, 2H), 1.94-1.76 (m, 4H), 1.70 (d, J=11.6 Hz, 3H), 1.38 (q, J=10.7, 9.6 Hz, 2H). |
| Example 67 | TM-67 | 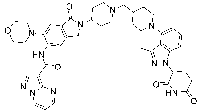 | LCMS (ESI) m/z: [M+1]=800.4. ¹H NMR (400 MHz, DMSO-d₆) δ 11.05 (s, 1H), 10.90 (s, 1H), 9.40 (dd, J=7.0, 1.6 Hz, 1H), 9.00 (dd, J=4.3, 1.7 Hz, 1H), 8.76 (s, 1H), 8.75 (s, 1H), 7.58 (s, 1H), 7.39 (dd, J=7.0, 4.2 Hz, 1H), 7.23 (dd, J=8.4, 7.3 Hz, 1H), 7.15 (d, J=8.4 Hz, 1H), 6.61 (d, J=7.3 Hz, 1H), 5.68 (dd, J=11.9, 5.1 Hz, 1H), 4.46 (s, 2H), 4.01 (s, 1H), 3.90 (t, J=4.5 Hz, 4H), 2.98 (s, 2H), 2.91 (t, J=4.5 Hz, 4H), 2.87-2.80 (m, 1H), 2.75-2.64 (m, 4H), 2.61 (s, 3H), 2.34-2.17 (m, 3H), 2.12-1.97 (m, 2H), 1.86 (dd, J=24.3, 11.8 Hz, 4H), 1.76- 1.65 (m, 3H), 1.45-1.33 (m, 2H), 1.23 (d, J=3.3 Hz, 2H). |
| Example 68 | TM-68 | 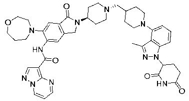 | LCMS (ESI) m/z: [M+1]=814.4. ¹H NMR (400 MHz, DMSO-d₆) δ 11.02 (d, J=18.6 Hz, 2H), 9.40 (dd, J=7.0, 1.6 Hz, 1H), 8.97 (dd, J=4.2, 1.7 Hz, 1H), 8.74 (d, J=4.9 Hz, 2H), 7.61 (s, 1H), 7.38 (dd, J=7.0, 4.2 Hz, 1H), 7.23 (dd, J=8.4, 7.3 Hz, 1H), 7.15 (d, J=8.3 Hz, 1H), 6.61 (d, J=7.3 Hz, 1H), 5.68 (dd, J=11.8, 5.1 Hz, 1H), 4.45 (s, 2H), 4.07-3.81 (m, 5H), 3.21-3.02 (m, 4H), 3.02 -2.77 (m, 4H), 2.76-2.64 (m, 4H), 2.61-2.55 (m, 4H), 2.30-2.12 (m, 3H), 2.10-1.98(m, 4H), 1.95-1.75 (m, 4H), 1.75-1.60(m, 3H), 1.45-1.30 m, 2H). |
| Example 69 | TM-69 | 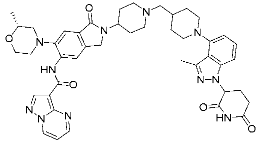 | LCMS (ESI) m/z: [M+1]=814.4. ¹H NMR (400 MHz, DMSO-d₆) δ 11.04 (s, 1H), 10.89 (s, 1H), 9.40 (dd, J=7.0, 1.6 Hz, 1H), 8.96 (dd, J=4.3, 1.7 Hz, 1H), 8.76 (d, J=7.6 Hz, 2H), 7.56 (s, 1H), 7.38 (dd, J=7.0, 4.2 Hz, 1H), 7.23 (dd, J=8.4, 7.3 Hz, 1H), 7.15 (d, J=8.4 Hz, 1H), 6.61 (d, J=7.3 Hz, 1H), 5.68 (dd, J=11.7, 5.1 Hz, 1H), 4.46 (s, 2H), 4.10-3.85 (m, 4H), 3.40-3.35 (m, 2H), 3.03-2.78 (m, 6H), 2.76-2.56 (m, 8H), 2.30-2.15(m, 3H), 2.03 (t, J=11.4 Hz, 2H), 1.93-1.79 (m , 4H), 1.75-1.65 (m, 3H), 1.42-1.30(m, 2H), 1.13 (d, J=6.2 Hz, 3H). |
| Example 70 | TM-70 | 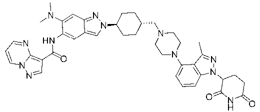 | LCMS (ESI) m/z: [M+1]=743.30. ¹H NMR (400 MHz, DMSO-*d*₆) δ 11.07 (s, 1H), 11.04 (s, 1H), 9.47-9.28 (m, 1H), 8.95 (d, *J*=2.7 Hz, 1H), 8.84 (s, 1H), 8.71 (s, 1H), 8.35 (s, 1H), 7.63 (s, 1H), 7.35 (dd, *J*=6.9, 4.2 Hz, 1H), 7.24 (t, *J*=7.8 Hz, 1H), 7.17 (d, *J*=8.4 Hz, 1H), 6.63 (d, *J*=7.2 Hz, 1H), 5.69 (dd, *J*=11.7, 4.9 Hz, 1H), 4.49-4.33 (m, 1H), 4.01-3.80 (m, 3H), 3.56 (t, *J*=10.2 Hz, 1H), 3.28-3.21 (m, 2H), 3.12-2.78 (m, 6H), 2.76-2.56 (m, 7H), 2.40-2.11 (m, 4H), 2.08-1.86 (m, 4H), 1.78-1.59 (m, 1H), 1.25-1.09 (m, 2H), 0.75 (d, *J*=6.0 Hz, 2H). |
| Example 71 | TM-71 | 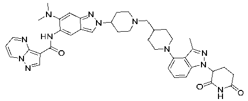 | LCMS (ESI) m/z: [M+1]=743.3. ¹H NMR (400 MHz, DMSO-*d*₆) δ 11.05 (s, 1H), 10.86 (s, 1H), 9.37 (dd, *J*=7.0, 1.7 Hz, 1H), 8.95 (dd, *J*=4.2, 1.7 Hz, 1H), 8.73 (s, 1H), 8.71 (s, 1H), 8.34 (s, 1H), 7.38 (s, 1H), 7.33 (dd, *J*=7.0, 4.2 Hz, 1H), 7.23 (t, *J*=7.9 Hz, 1H), 7.15 (d, *J*=8.3 Hz, 1H), 6.61 (d, *J*=7.2 Hz, 1H), 5.68 (dd, *J*=11.8, 5.1 Hz, 1H), 4.40 (s, 1H), 3.01 (s, 1H), 2.84-2.60 (m, 15H), 2.37-1.99 (m, 10H), 1.95-1.65 (m, 3H), 1.47-1.32 (m, 2H), 0.90-0.83 (m, 1H). |
| Example 72 | TM-72 | 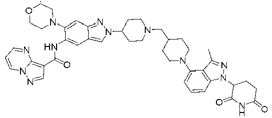 | LCMS (ESI) m/z: [M+1]=799.4. ¹H NMR (400 MHz, DMSO-d₆) δ11.05 (s, 1H), 10.67 (s, 1H), 9.38 (dd, J=7.0, 1.6 Hz, 1H), 8.93 (dd, J=4.2, 1.7 Hz, 1H), 8.75 (d, J=19.8 Hz, 2H), 8.36 (d, J=0.8 Hz, 1H), 7.44 (s, 1H), 7.35 (dd, J=7.0, 4.3 Hz, 1H), 7.23 (dd, J=8.4, 7.3 Hz, 1H), 7.15 (d, J=8.3 Hz, 1H), 6.60 (d, J=7.3 Hz, 1H), 5.68 (dd, J=11.8, 5.1 Hz, 1H), 4.41 (s, 1H), 4.06-3.81 (m, 3H), 3.33-3.30 (m, 2H), 3.06-2.91 (m, 3H), 2.91-2.75 (m, 3H), 2.75-2.52 (m, 8H), 2.29 (d, J=7.1 Hz, 2H), 2.26-2.18 (m, 1H), 2.18-2.05 (m, 6H), 1.95-1.85 (m, 2H), 1.80-.1.65(m, 1H), 1.45-1.30 (m, 2H), 1.12 (d, J=6.2 Hz, 3H). |
| Example 73 | TM-73 | 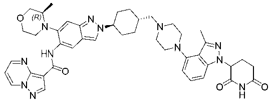 | LCMS (ESI) m/z: [M+1]=799.40. ¹H NMR (600 MHz, DMSO-*d*₆) δ 11.07 (s, 1H), 11.04 (s, 1H), 9.38 (dd, *J*=7.0, 1.5 Hz, 1H), 8.95 (dd, *J*=4.1, 1.5 Hz, 2H), 8.84 (s, 1H), 8.71 (s, 1H), 8.35 (s, 1H), 7.63 (s, 1H), 7.35 (dd, *J*=6.9, 4.2 Hz, 1H), 7.24 (t, *J*=7.9 Hz, 1H), 7.17 (d, *J*=8.4 Hz, 1H), 6.63 (d, *J*=7.3 Hz, 1H), 5.69 (dd, *J*=11.9, 5.1 Hz, 1H), 4.56-4.30 (m, 1H), 3.98-3.79 (m, 3H), 3.56 (t, *J*=10.3 Hz, 1H), 3.28-3.22 (m, 1H), 3.03 (br, 4H), 2.97-2.79 (m, 3H), 2.76-2.57 (m, 7H), 2.37-2.13 (m, 5H), 2.09-1.83 (m, 4H), 1.74-1.63 (m, 1H), 1.20-1.10 (m, 2H), 0.75 (d, *J*=6.1 Hz, 3H). |
| Example 74 | TM-74 | 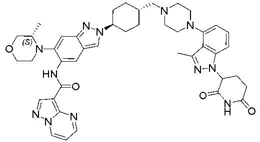 | LCMS (ESI) m/z: [M+1]=799.40. ¹H NMR (400 MHz, DMSO-*d*₆) δ 11.07 (s, 1H), 11.04 (s, 1H), 9.43-9.28 (m, 1H), 8.95 (d, *J*=2.7 Hz, 1H), 8.84 (s, 1H), 8.71 (s, 1H), 8.35 (s, 1H), 7.63 (s, 1H), 7.35 (dd, *J*=6.9, 4.2 Hz, 1H), 7.24 (t, *J*=7.8 Hz, 1H), 7.17 (d, *J*=8.4 Hz, 1H), 6.63 (d, *J*=7.2 Hz, 1H), 5.69 (dd, *J*=11.7, 4.9 Hz, 1H), 4.48-4.35 (m, 1H), 4.01-3.79 (m, 3H), 3.56 (t, *J*=10.2 Hz, 1H), 3.28-3.21 (m, 2H), 3.14-2.78 (m, 7H), 2.79-2.56 (m, 7H), 2.39-2.09 (m, 6H), 2.09-1.84 (m, 4H), 1.78-1.61 (m, 1H), 1.28-1.05 (m, 2H), 0.75 (d, *J*=6.0 Hz, 3H). |
| Example 75 | TM-75 | 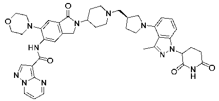 | LCMS (ESI) m/z: [M+1]=785.40. |
| Example 76 | TM-76 | 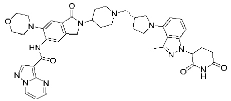 | LCMS (ESI) m/z: [M+1]=785.40. |
| Example 77 | TM-77 | 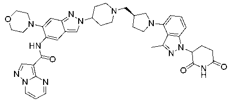 | LCMS (ESI) m/z: [M+1]=771.40. |
| Example 78 | TM-78 | 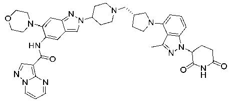 | LCMS (ESI) m/z: [M+1]=771.40. |
| Example 79 | TM-79 | 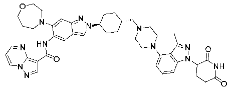 | LCMS (ESI) m/z: [M+1]=799.40. ¹H NMR (400 MHz, DMSO-*d*₆) δ 11.05 (s, 1H), 10.77 (s, 1H), 9.37 (dd, *J*=7.0, 1.7 Hz, 1H), 8.95 (dd, *J*=4.2, 1.6 Hz, 1H), 8.73 (s, 1H), 8.72 (s, 1H), 8.31 (s, 1H), 7.48 (s, 1H), 7.35 (dd, *J*=7.0, 4.2 Hz, 1H), 7.24 (t, *J*=7.8 Hz, 1H), 7.17 (d, *J*=8.4 Hz, 1H), 6.63 (d, *J*=7.3 Hz, 1H), 5.68 (dd, *J*=11.8, 5.1 Hz, 1H), 4.45-4.34 (m, 1H), 4.00-3.80 (m, 4H), 3.23-2.95 (m, 9H), 2.91-2.79 (m, 1H), 2.77-2.56 (m, 8H), 2.29-2.08 (m, 7H), 2.04-1.83 (m, 4H), 1.75-1.62 (m, 1H), 1.20-1.07 (m, 2H). |
| Example 80 | TM-80 | 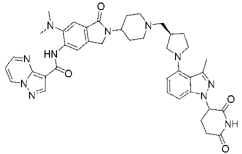 | LCMS (ESI) m/z: [M+1]=744.40. |
| Example 81 | TM-81 | 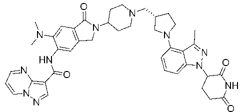 | LCMS (ESI) m/z: [M+1]=744.40. |
| Example 82 | TM-82 | 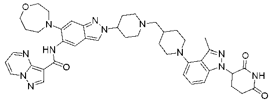 | LCMS (ESI) m/z: [M+1]=799.40. ¹H NMR (400 MHz, DMSO-d₆) δ 11.05 (s, 1H), 10.77 (s, 1H), 9.38 (dd, J=7.0, 1.7 Hz, 1H), 8.95 (dd, J=4.2, 1.7 Hz, 1H), 8.73 (d, J=5.1 Hz, 2H), 8.36 (d, J=0.9 Hz, 1H), 7.49 (t, J=0.8 Hz, 1H), 7.36 (dd, J=7.0, 4.2 Hz, 1H), 7.23 (dd, J=8.4, 7.3 Hz, 1H), 7.15 (d, J=8.4 Hz, 1H), 6.61 (d, J=7.3 Hz, 1H), 5.68 (dd, J=11.8, 5.1 Hz, 1H), 4.42 (s, 1H), 3.92 (t, J=6.1 Hz, 2H), 3.90-3.80 (m, 2H), 3.19-3.13 (m, 2H), 3.13-3.08 (m, 2H), 3.02 (s, 2H), 2.85 (ddd, J=18.2, 13.5, 5.2 Hz, 1H), 2.77-2.66 (m, 4H), 2.61 (s, 3H), 2.37-2.26 (m, 2H), 2.20 (dd, J=9.8, 5.3 Hz, 1H), 2.10 (q, J=10.0, 7.9 Hz, 7H), 1.91 (d, J=12.6 Hz, 2H), 1.74 (d, J=11.6 Hz, 1H), 1.64-1.53 (m, 1H), 1.48-1.32 (m, 4H). |
| Example 83 | TM-83 | 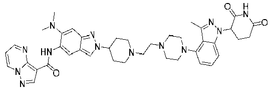 | LCMS (ESI) m/z: [M+1]=758.30. |
| Example 84 | TM-84 | 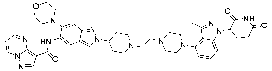 | LCMS (ESI) m/z: [M+1]=800.40. |
| Example 85 | TM-85 | 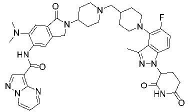 | LCMS (ESI) m/z: [M+1]=776.4. |
| Example 86 | TM-86 | 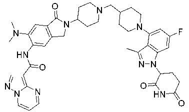 | LCMS (ESI) m/z: [M+1]=776.4. |
| Example 87 | TM-87 | 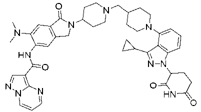 | LCMS (ESI) m/z: [M+1]=784.4. |
| Example 88 | TM-88 | 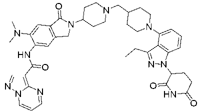 | LCMS (ESI) m/z: [M+1]=772.4. |
| Example 89 | TM-89 | 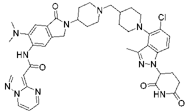 | LCMS (ESI) m/z: [M+1]=792.3. |
| Example 90 | TM-90 | 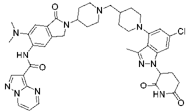 | LCMS (ESI) m/z: [M+1]=792.3. |
| Example 91 | TM-91 | 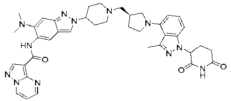 | LCMS (ESI) m/z: [M+1]=728.4. |
| Example 92 | TM-92 | 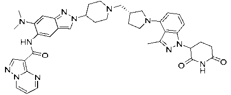 | LCMS (ESI) m/z: [M+1]=728.4. |
| Example 93 | TM-93 | 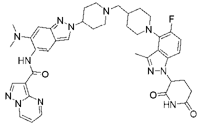 | LCMS (ESI) m/z: [M+1]=761.4. |
| Example 94 | TM-94 | 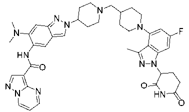 | LCMS (ESI) m/z: [M+1]=761.4. |
| Example 95 | TM-95 | 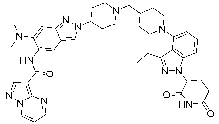 | LCMS (ESI) m/z: [M+1]=757.4. |
| Example 96 | TM-96 | 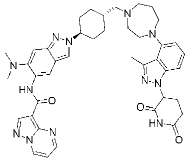 | LCMS (ESI) m/z: [M+1]=757.4. ¹H NMR (400 MHz, DMSO-*d*₆) δ 11.04 (s, 1H), 10.85 (s, 1H), 9.36 (dd, *J*=7.0, 1.7 Hz, 1H), 8.95 (dd, *J*=4.2, 1.7 Hz, 1H), 8.71 (s, 1H), 8.70 (s, 1H), 8.29 (d, *J*=0.9 Hz, 1H), 7.36 (s, 1H), 7.33 (dd, *J*=7.0, 4.2 Hz, 1H), 7.21 (dd, *J*=8.4, 7.4 Hz, 1H), 7.12 (d, *J*=8.3 Hz, 1H), 6.68 (d, *J*=7.4 Hz, 1H), 5.66 (dd, *J*=11.9, 5.1 Hz, 1H), 4.43-4.33 (m, 1H), 3.32-3.26 (m, 2H), 2.88-2.65 (m, 14H), 2.62 (s, 3H), 2.37 (d, *J*=7.0 Hz, 2H), 2.24-2.11 (m, 3H), 2.03-1.82 (m, 6H), 1.67-1.55 (m, 1H), 1.19-1.07 (m, 2H). |
| Example 97 | TM-97 | 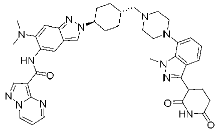 | LCMS (ESI) m/z: [M+1]=743.3. |
| Example 98 | TM-98 | 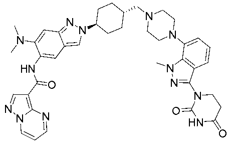 | LCMS (ESI) m/z: [M+1]=743.3. |
| Example 99 | TM-99 | 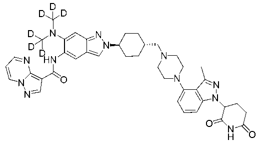 | LCMS (ESI) m/z: [M+1]=749.4. |
| Example 100 | TM-100 | 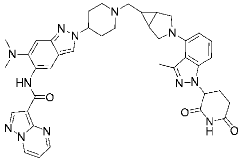 | LCMS (ESI) m/z: [M+1]=741.3. |
| Example 101 | TM-101 | 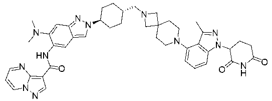 | LCMS (ESI) m/z: [M+1]=783.4. ¹H NMR (400 MHz, DMSO-*d*₆) δ 11.04 (s, 1H), 10.85 (s, 1H), 9.36 (dd, *J*=7.0, 1.6 Hz, 1H), 8.95 (dd, *J*=4.2, 1.7 Hz, 1H), 8.71 (s, 1H), 8.70 (s, 1H), 8.28 (d, *J*=0.9 Hz, 1H), 7.36 (s, 1H), 7.33 (dd, *J*=7.0, 4.2 Hz, 1H), 7.22 (t, *J*=7.8 Hz, 1H), 7.15 (d, *J*=8.4 Hz, 1H), 6.60 (d, *J*=7.2 Hz, 1H), 5.67 (dd, *J*=11.8, 5.1 Hz, 1H), 4.41-4.28 (m, 1H), 3.51 (m, 4H), 3.01 (br, 4H), 2.97-2.80 (m, 5H), 2.76 (s, 6H), 2.73-2.65 (m, 2H), 2.60 (s, 3H), 2.38-2.28 (m, 2H), 2.23-2.06 (m, 4H), 1.96-1.78 (m, 8H), 1.45-1.36 (m, 1H), 1.19-1.06 (m, 2H). |
| Example 102 | TM-102 | 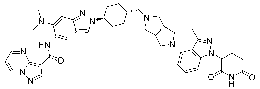 | LCMS (ESI) m/z: [M+1]=769.3. ¹H NMR (400 MHz, DMSO) δ 11.05 (s, 1H), 10.85 (s, 1H), 9.37 (dd, J=7.0, 1.7 Hz, 1H), 8.95 (dd, J=4.2, 1.7 Hz, 1H), 8.72 (s, 1H), 8.70 (s, 1H), 8.29 (s, 1H), 7.37 (s, 1H), 7.33 (dd, J=7.0, 4.2 Hz, 1H), 7.22 (t, J=7.8 Hz, 1H), 7.18-7.07 (m, 1H), 6.64 (d, J=20.8 Hz, 1H), 5.68 (dd, J=12.0, 5.0 Hz, 1H), 4.38 (t, J=10.7 Hz, 1H), 3.31-3.16 (m, 5H), 2.71 (d, J=43.2 Hz, 17H), 2.25-1.82 (m, 8H), 1.62 (s, 1H), 1.17 (s, 3H). |
| Example 103 | TM-103 | 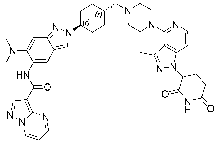 | LCMS (ESI) m/z: [M+1]=744.7. |
| Example 104 | TM-104 | 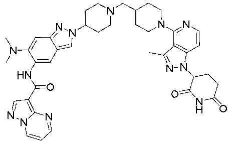 | LCMS (ESI) m/z: [M+1]=744.4. |
| Example 105 | TM-105 | 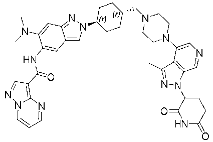 | LCMS (ESI) m/z: [M+1]=744.4. ¹H NMR (400 MHz, DMSO-d₆) δ 11.11 (s, 1H), 10.85 (s, 1H), 9.36 (dd, *J*=7.0, 1.6 Hz, 1H), 8.95 (dd, *J*=4.2, 1.6 Hz, 1H), 8.73-8.67 (m, 3H), 8.29 (s, 1H), 7.81 (s, 1H), 7.37 (s, 1H), 7.33 (dd, *J*=7.0, 4.2 Hz, 1H), 5.87 (dd, *J*=11.6, 5.0 Hz, 1H), 4.39 (t, *J*=10.5 Hz, 1H), 3.13 (br, 4H), 2.89-2.55 (m, 15H), 2.36-2.23 (m, 3H), 2.21-2.10 (m, 2H), 2.04-1.81 (m, 4H), 1.76-1.60 (m, 1H), 1.20-1.08 (m, 2H). |
| Example 106 | TM-106 | 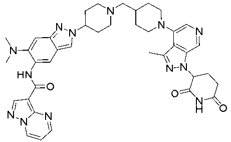 | LCMS (ESI) m/z: [M+1]=744.4. ¹H NMR (400 MHz, DMSO-d₆) δ 11.12 (s, 1H), 10.86 (s, 1H), 9.36 (dd, *J*=7.0, 1.7 Hz, 1H), 8.95 (dd, *J*=4.2, 1.7 Hz, 1H), 8.72 (s, 1H), 8.70 (s, 1H), 8.67 (s, 1H), 8.34 (s, 1H), 7.79 (s, 1H), 7.37 (s, 1H), 7.33 (dd, *J*=7.0, 4.2 Hz, 1H), 5.86 (dd, *J*=11.6, 5.0 Hz, 1H), 4.58 (t, *J*=5.5 Hz, 1H), 4.40 (s, 1H), 3.51 (s, 8H), 3.49-3.39 (m, 3H), 3.08-2.97 (m, 1H), 2.90-2.70 (m, 8H), 2.63 (s, 3H), 2.35-2.24 (m, 2H), 2.21-2.05 (m, 5H), 1.97-1.87 (m, 2H), 1.82-1.67 (m, 1H), 1.47-1.33 (m, 2H). |
| Example 107 | TM-107 | 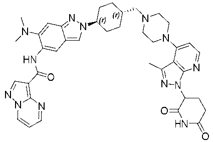 | LCMS (ESI) m/z: [M+1]=744.4. |
| Example 108 | TM-108 | 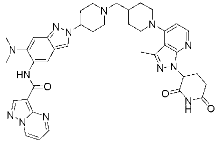 | LCMS (ESI) m/z: [M+1]=744.4. |
| Example 109 | TM-109 | 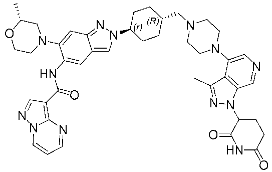 | LCMS (ESI) m/z: [M+1]=800.8. ¹H NMR (400 MHz, DMSO-*d*₆) δ 11.12 (s, 1H), 10.66 (s, 1H), 9.38 (dd, *J*=7.0, 1.6 Hz, 1H), 8.93 (dd, *J*=4.2, 1.7 Hz, 1H), 8.77 (s, 1H), 8.72 (s, 1H), 8.70 (s, 1H), 8.32 (s, 1H), 7.81 (s, 1H), 7.44 (s, 1H), 7.35 (dd, *J*=7.0, 4.2 Hz, 1H), 5.87 (dd, *J*=11.7, 5.0 Hz, 1H), 4.46-4.36 (m, 1H), 4.09-3.71 (m, 3H), 3.13 (br, 4H), 3.04-2.53 (m, 14H), 2.34-2.23 (m, 3H), 2.20-2.10 (m, 2H), 2.06-1.86 (m, 4H), 1.72 (br, 1H), 1.12 (d, *J*=6.3 Hz, 5H). |
| Example 110 | TM-110 | 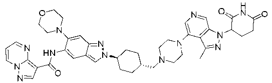 | LCMS (ESI) m/z: [M+1]=786.80. ¹H NMR (400 MHz, DMSO-*d*₆) δ11.11 (s, 1H), 10.67 (s, 1H), 9.38 (dd, *J*=7.0, 1.6 Hz, 1H), 8.97 (dd, *J*=4.3, 1.6 Hz, 1H), 8.76 (s, 1H), 8.72 (s, 1H), 8.32 (s, 1H), 7.81 (s, 1H), 7.74-7.63 (m, 1H), 7.45 (s, 1H), 7.36 (dd, *J*=7.0, 4.2 Hz, 1H), 5.87 (dd, *J*=11.6, 5.0 Hz, 1H), 4.41 (t, *J*=11.7 Hz, 1H), 4.13 (dd, *J*=5.8, 3.4 Hz, 1H), 3.89 (t, *J*=4.5 Hz, 4H), 3.13 (s, 4H), 2.92 (t, *J*=4.5 Hz, 4H), 2.86-2.59 (m, 8H), 2.34-2.25 (m, 3H), 2.20-2.08 (m, 2H), 2.06-1.84 (m, 5H), 1.75-1.60 (m, 1H), 1.25-1.08 (m, 2H). |
| Example 111 | TM-111 | 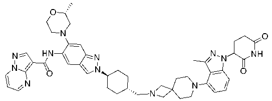 | LCMS (ESI) m/z: [M+1]=839.94. ¹H NMR (400 MHz, DMSO-*d*₆) δ 11.04 (s, 1H), 10.65 (s, 1H), 9.38 (dd, *J*=7.0, 1.7 Hz, 1H), 8.93 (dd, *J*=4.3, 1.6 Hz, 1H), 8.75 (s, 1H), 8.71 (s, 1H), 8.29 (s, 1H), 7.43 (s, 1H), 7.35 (dd, *J*=7.0, 4.2 Hz, 1H), 7.22 (t, *J*=7.8 Hz, 1H), 7.16 (d, *J*=8.4 Hz, 1H), 6.60 (d, *J*=7.3 Hz, 1H), 5.67 (dd, *J*=11.8, 5.1 Hz, 1H), 4.41 (br, 1H), 4.09-3.78 (m, 4H), 3.20-2.77 (m, 11H), 2.78-2.54 (m, 8H), 2.28-2.13 (m, 3H), 2.12-1.81 (m, 8H), 1.70-1.40 (m, 2H), 1.11 (d, *J*=6.3 Hz, 3H). |
| Example 112 | TM-112 | 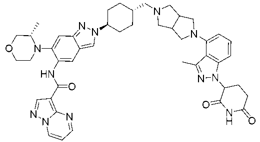 | LCMS (ESI) m/z: [M+1]=825.7. ¹H NMR (600 MHz, DMSO) δ 11.07 (s, 2H), 9.38 (dd, J=7.0, 1.6 Hz, 1H), 8.95 (dd, J=4.1, 1.7 Hz, 1H), 8.83 (s, 1H), 8.71 (s, 1H), 8.34 (s, 1H), 7.63 (s, 1H), 7.35 (dd, J=7.0, 4.2 Hz, 1H), 7.20 (t, J=7.9 Hz, 1H), 7.12 (d, J=8.4 Hz, 1H), 6.59 (d, J=7.4 Hz, 1H), 5.64 (dd, J=11.8, 4.5 Hz, 1H), 4.46-4.34 (m, 1H), 3.95 (dd, J=11.2, 3.1 Hz, 1H), 3.91-3.81 (m, 2H), 3.56 (t, J=10.7, 2H), 3.28-3.22 (m, 2H), 2.97-2.51 (m, 17H), 2.29 (d, J=7.2 Hz, 2H), 2.23-2.16 (m, 1H), 2.14 (d, J=11.8 Hz, 2H), 1.99 (d, J=13.3 Hz, 2H), 1.91 (dd, J=17.6, 7.9 Hz, 2H), 1.62-1.55 (m, 1H), 1.13 (dd, J=25.1, 12.2 Hz, 2H), 0.75 (d, J=6.1 Hz, 3H). |
| Example 113 | TM-113 | 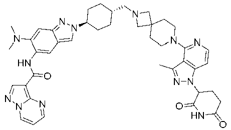 | LCMS (ESI) m/z: [M+1]=784.89. ¹H NMR (400 MHz, DMSO-*d*₆) δ 10.85 (s, 1H), 9.36 (dd, *J*=7.0, 1.6 Hz, 1H), 8.94 (dd, *J*=4.2, 1.6 Hz, 1H), 8.71 (s, 1H), 8.70 (s, 1H), 8.28 (s, 2H), 7.89 (d, *J*=6.0 Hz, 1H), 7.36 (s, 1H), 7.33 (dd, *J*=7.0, 4.2 Hz, 1H), 7.08 (d, *J*=6.1 Hz, 1H), 5.69 (dd, *J*=12.0, 5.0 Hz, 1H), 4.41-4.29 (m, 1H), 3.21 (s, 5H), 3.09 (s, 4H), 2.92-2.66 (m, 8H), 2.56 (s, 3H), 2.40 (d, *J*=6.3 Hz, 2H), 2.27-2.05 (m, 3H), 1.96-1.78 (m, 7H), 1.49-1.37 (m, 1H), 1.20-1.07 (m, 2H). |
| Example 114 | TM-114 | 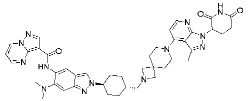 | LCMS (ESI) m/z: [M+1]=784.36. |

### Example 115: Synthesis of TM-115

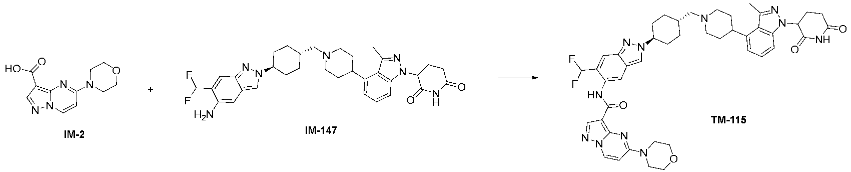

Preparation method for the TM-115 is similar to that for the TM-1. LCMS (ESI) m/z: [M+1]=834.4.

Synthesis in Examples 116 to 126: preparation methods for TM-116 to TM-126 in the Examples are similar to the preparation for the TM-25, as shown in Table 18.

**Table 18 Compound No. and structure**

| Example No. | Compound No. | Structure | Characterization |
|---|---|---|---|
| Example 116 | TM-116 | 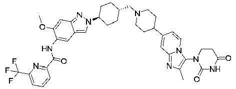 | LCMS (ESI) m/z: [M+1]=839.4. |
| Example 117 | TM-117 | 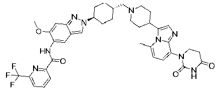 | LCMS (ESI) m/z: [M+1]=839.4. |
| Example 118 | TM-118 | 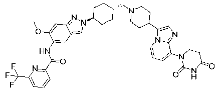 | LCMS (ESI) m/z: [M+1]=839.4. |
| Example 119 | TM-119 | 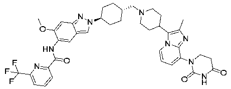 | LCMS (ESI) m/z: [M+1]=839.4. ¹H NMR (400 MHz, Methanol-*d*₄) δ 8.76 (s, 1H), 8.47 (d, *J*=7.9 Hz, 1H), 8.42 (dd, *J*=7.0, 1.1 Hz, 1H), 8.30 (t, *J*=7.9 Hz, 1H), 8.19 (d, *J*=0.9 Hz, 1H), 8.04 (dd, *J*=7.8, 1.0 Hz, 1H), 7.27 (dd, *J*=7.3, 1.0 Hz, 1H), 7.07 (s, 1H), 6.95 (t, *J*=7.1 Hz, 1H), 4.57 (s, 3H), 4.47-4.34 (m, 1H), 3.87 (t, *J*=6.8 Hz, 2H), 3.23-3.11 (m, 3H), 2.93 (t, *J*=6.8 Hz, 2H), 2.39 (d, *J*=6.9 Hz, 2H), 2.31-2.22 (m, 6H), 2.16-1.95 (m, 5H), 1.85 (d, *J*=8.3 Hz, 3H), 0.91-0.80 (m, 3H). |
| Example 120 | TM-120 | 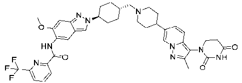 | LCMS (ESI) m/z: [M+1]=839.4. |
| Example 121 | TM-121 | 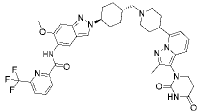 | LCMS (ESI) m/z: [M+1]=839.4. |
| Example 122 | TM-122 | 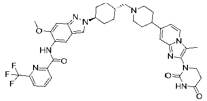 | LCMS (ESI) m/z: [M+1]=839.4. |
| Example 123 | TM-123 | 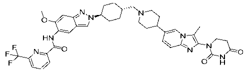 | LCMS (ESI) m/z: [M+1]=839.4. |
| Example 124 | TM-124 | 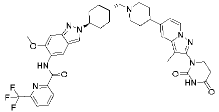 | LCMS (ESI) m/z: [M+1]=839.4. |
| Example 125 | TM-125 | 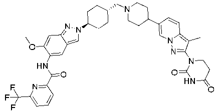 | LCMS (ESI) m/z: [M+1]=839.4. |
| Example 126 | TM-126 | 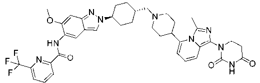 | LCMS (ESI) m/z: [M+1]=839.4. |

**The following test examples are used to illustrate the beneficial effects of the compounds of the present invention.**

The positive compound used in the invention is derived from patent No. WO2020113233 of Kymera Therapeutics, Inc., is synthesized by the applicant itself, and has a structure below:

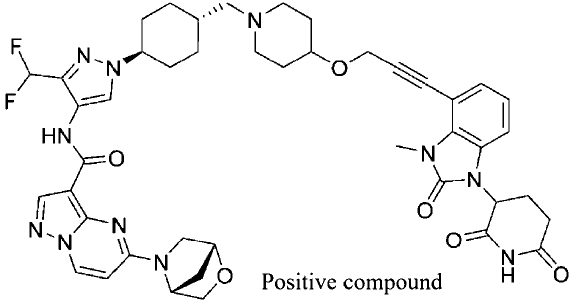

### Test Example 1: Inhibitory Effects of compounds of the present invention against secretion levels of TNFα in human THP-1 cells

This experiment was carried out in RPMI 1640 medium containing 10% FBS/1% penicillin/streptomycin, with each of the compounds at an initial test concentration of 1 uM.

Compound DMSO was dissolved to form a stock solution, which was diluted with the culture medium to a 3× working concentration. The diluted solution was added to a 96-well plate with 100 µL per well. THP1 cells in a logarithmic growth phase were counted, and diluted to a concentration of 2x10⁶/mL. The diluted cells were added to the above compound-containing 96-well plate with 100 µL per well, fully mixed, and incubated in an incubator with 5% CO2 at 37°C for 1 h. Then, LPS was further added until a final concentration of 1 ng/mL, and the cells were further incubated in the incubator with 5% CO2 at 37°C for additional 5 h. Then, after centrifugation at 1,000 rpm for 1 min, 16 µL of the supernatant was detected respectively using a TNFα ELISA kit. An OD450 value was read, and converted to a TNFα concentration based on a standard curve, to calculate an EC₅₀ value by fitting a dose-effect curve using GraphPad 5.0.

### Test Example 2: Inhibitory effects of compounds of the present invention against secretion levels of IL-6 in human peripheral blood mononuclear cells (PBMC) in response to R848 stimulation

### (1) Experimental method:

This experiment was carried out in RPMI 1640 medium containing 10% FBS/1% penicillin/streptomycin, with each of the compounds at an initial test concentration of 1 µM. The compound DMSO was dissolved to form a stock solution, which was gradiently diluted 4-fold with the culture medium to a 4x working solution concentration. The diluted solution was added to a 96-well plate with 50 µL per well. PBMC (batch No.: HPP20062307, Sichuan Hope Bio-tech Co., Ltd.) cells were counted, and diluted to a concentration of 2×10⁶/mL. The diluted cells were added to the above compound-containing 96-well plate with 150 µL per well, fully mixed, and incubated in an incubator with 5% CO₂ at 37°C for 20 h. Then, R848 was further added until a final concentration of 2.5 µg/mL, and the cells were further incubated in the incubator with 5% CO₂ at 37°C for additional 24 h. Then, after centrifugation at 2,000 rpm for 4 min, the supernatant was diluted 110×, and detected using an IL-6 ELISA kit. An OD450 value was read, and converted to an IL-6 concentration based on a standard curve, to calculate an IC₅₀ value by fitting a dose-effect curve using GraphPad 5.0.

### (2) Experimental result

The experimental result is shown in Table 19 below.

**Table 19 Inhibitory effects of compounds against R848-induced secretion levels of IL-6 in PBMC cells**

| Compound No. | IC₅₀ (nM) |
|---|---|
| Positive compound | 38.45 (mean result of two tests) |
| TM-25 | 27.99 |
| TM-27 | 46.79 |
| TM-28 | 23.31 |
| TM-29 | 56.27 |
| TM-33 | 13.04 |
| TM-46 | 46.79 |
| TM-54 | 4.26 |
| TM-55 | 5.09 |
| TM-57 | 3.43 |
| TM-58 | 3.57 |
| TM-59 | 2.21 |
| TM-61 | 3.01 |
| TM-66 | 261.6 |

A median inhibition concentration, or known as a median inhibition rate, i.e., IC50, is a very important data in a standard curve of indirect competitive ELISA. Generally, the smaller the IC50 value is, the stronger the specificity of the antibody is.

The result in Table 19 shows that: the compounds of the present invention have obvious advantages in the inhibitory effects against the R848-induced secretion levels of IL-6 in PBMC cells.

### Test Example 3: Inhibitory effects of compounds of the present invention against secretion levels of IL-6 in human peripheral blood mononuclear cells (PBMC) in response to LPS+IL-1β stimulation

This experiment was carried out in RPMI 1640 medium containing 10% FBS/1% penicillin/streptomycin, with each of the compounds at an initial test concentration of 1 µM. The compound DMSO was dissolved to form a stock solution, which was gradiently diluted 5-fold with the culture medium to a 4× working solution concentration. The diluted solution was added to a 96-well plate with 50 µL per well. PBMC (batch No.: HPP20062307, Sichuan Hope Bio-tech Co., Ltd.) cells were counted, and diluted to a concentration of 2×10⁶/mL. The diluted cells were added to the above compound-containing 96-well plate with 150 µL per well, fully mixed, and incubated in an incubator with 5% CO₂ at 37°C for 24 h. Then, LPS and IL-1β were further added to final concentrations of 10 ng/mL and 20 ng/mL, respectively, and the cells were further incubated in the incubator with 5% CO₂ at 37°C for 20 h. Then, after centrifugation at 2,000 rpm for 4 min, the supernatant was diluted 110%, and detected using an IL-6 ELISA kit. An OD450 value was read, and converted to an IL-6 concentration based on a standard curve, to calculate an IC₅₀ value by fitting a dose-effect curve using GraphPad 5.0. The results are shown in Table 20.

**Table 20 Inhibitory effects of compounds against LPS+IL-1β-induced secretion levels of IL-6 in PBMC cells**

| Compound No. | IC₅₀ (nM) |
|---|---|
| Positive compound | 38.44 (mean result of two tests) |
| TM-26 | 12.22 |
| TM-27 | 9.53 |
| TM-30 | 3.69 |
| TM-32 | 22.24 |
| TM-33 | 10.23 |
| TM-35 | 29.54 |
| TM-36 | 4.987 |
| TM-37 | 4.99 |
| TM-39 | 115.2 |
| TM-40 | 138.2 |
| TM-45 | 5.48 |
| TM-47 | 45.8 |
| TM-48 | 47.79 |
| TM-49 | 16.53 |
| TM-50 | 3.81 |
| TM-51 | 9.15 |
| TM-52 | 10.46 |
| TM-53 | 38.45 |
| TM-54 | 6.29 |
| TM-55 | 7.63 |
| TM-56 | 5.49 |
| TM-57 | 8.67 |
| TM-58 | 3.53 |
| TM-59 | 3.57 |
| TM-60 | 4.40 |
| TM-61 | 7.06 |
| TM-62 | 11.93 |
| TM-63 | 0.90 |
| TM-65 | 7.15 |
| TM-66 | 19.35 |
| TM-67 | 52.77 |
| TM-68 | 3.23 |
| TM-69 | 22.85 |
| TM-70 | 1.18 |
| TM-71 | 24.93 |
| TM-72 | 6.82 |
| TM-73 | 43.78 |
| TM-74 | 29.41 |
| TM-79 | 39.02 |
| TM-82 | 36.46 |
| TM-96 | 7.18 |
| TM-101 | 1.17 |
| TM-102 | 5.94 |
| TM-103 | 0.72 |
| TM-105 | 34.25 |
| TM-106 | 54.93 |
| TM-109 | 39.21 |
| TM-113 | 0.62 |
| TM-114 | 4.79 |

A median inhibition concentration, or known as a median inhibition rate, i.e., IC50, is a very important data in a standard curve of indirect competitive ELISA. Generally, the smaller the IC50 value is, the stronger the specificity of the antibody is.

The result in Table 20 shows that: the compounds of the present invention have obvious advantages in the inhibitory effects against the LPS+IL-1β-induced secretion levels of IL-6 in PBMC cells.

### Test Example 4: Study on degradation level of IRAK4 in human peripheral blood mononuclear cells (PBMC) by compounds of the present invention

This experiment was carried out in RPMI 1640 medium containing 10% FBS/1% penicillin/streptomycin, with each of the compounds at an initial test concentration of 1 uM.

Frozen human PBMCs were unfrozen into the culture medium. Cells were cultured at a minimum of 2.5×10⁶ c/mL. The PBMCs were incubated at 37°C/5% CO2 and allowed to stand overnight. After overnight recovery, cell count/viability assessment was performed by trypan blue exclusion. The cells were adjusted to a density of 2.5×10⁶/mL, and then added to a 96-well plate, with 90 uL per well. Each of the compounds was dissolved in DMSO to form a stock solution, which was diluted with the culture medium to a 3× working concentration. The diluted solution was added to the above 96-well plate, with 10 µL per well. The cells were incubated in an incubator with 5% CO2 at 37°C for 20 h. At the end of the treatment, the cells were collected, centrifuged at 1,800 rpm for 5 min, washed with 1×PBS, and centrifuged at 1,800 rpm for 5 min. The cell pellets were frozen, stored at -80°C until further processing, and resuspended in a lysis buffer to generate a lysate. Protein quantification was operated using a BCA kit on a 26-well 4-12% Bis-Tris SDS-page gel with 20 µg of protein loaded per lane. A PVDF membrane was transferred using BioRad Mixed MW turbo program for 7 min, and blocked on a shaker at room temperature for one h. A primary antibody was incubated on a shaker at 4°C overnight. The membrane was washed with 3×TBST for 5 min each. A secondary antibody was added and incubated on a shaker at room temperature for one h. The membrane was washed with 3×TBST for 5 min each, and fully rinsed with deionized H₂O. The membrane was scanned using LI-COR Odyssey CLx, and wavebands were quantified using Image Studio Lite version 5.2 software.

### Test Example 5: Effects of compounds on IRAK4 protein degradation in THP1 cells

THP-1 cells were inoculated into a 96-well cell culture plate, with 5×10⁴ cells and 100 µL of a culture medium per well. The cell culture plate was cultured in an incubator with 5% CO₂ at 37°C overnight. 100 nl of a well-prepared compound stock solution and a compound-free DMSO stock solution were respectively added to cells per well. The cell culture plate was incubated in an incubator with 5% CO₂ at 37°C for 24 h. One protease inhibitor tablet and one phosphatase inhibitor tablet were added to 20 mL of a cell lysis buffer, and they were gently mixed until complete dissolution. DTT at a final concentration of 200 mM was added to a 4x sample preparation buffer to prepare a 4x sample preparation working solution. 20x electrophoretic buffer was diluted to 1x with ultrapure water. 20x transfer buffer was diluted to 1x with ultrapure water, and 20% methanol was added. 10x electrophoretic buffer was diluted to 1x with ultrapure water. IRAK4 protein primary antibody working solution: 20 µl of IRAK4 antibody added to 20 mL of a blocking solution; β-Actin primary antibody working solution: 2 µl of beta-actin (13E5) Rabbit mAb antibody added to 20 mL of a blocking solution. IRAK4 protein secondary antibody working solution: Donkey Anti-Goat IgG H&L (HRP) diluted 1/5,000 in a blocking solution; β-Actin secondary antibody working solution: Anti-rabbit IgG, HRP-linked antibody diluted 1/10,000 in a blocking solution. The cell culture plate was centrifuged at 3,000 rpm for 3 min, and most of the culture medium was carefully pipetted. The cell plate was inverted, and centrifuged at 300 rpm for 30 s. 45 µL of a lysis buffer was added to each well. The mixture was shaken at 300 rpm for 30 s, allowed to stand on ice for 30 min, and pipetted 30 times at 20 min. 6 µL of 4x sample preparation working solution was added to 18 µL of protein supernatant for sample preparation, and the mixture was heated at 70°C for 10 min. Remaining protein samples were stored at -80°C. In the precast gel, 8 µl of sample was loaded into each well for electrophoresis at a constant voltage of 120 V for about 60 min, transferred using a PVDF membrane at a constant current of 300 mA for 1 h, then blocked with a blocking solution at room temperature for 1 h, and incubated in a primary antibody working solution at 4°C overnight. The membrane was washed with 1×TBST buffer for 3×10 min. The secondary antibody working solution was incubated at room temperature for 1 h. The membrane was washed with 1×TBST buffer for 3×10 min, and a chromogenic solution was used for exposure and color development. The gray value of each stripe was calculated using ImagJ software for semi-quantitative analysis, and DC₅₀ was calculated using GraphPad Prism8.0, with the results as shown in Table 21.

**Table 21 Effects of compounds on IRAK4 protein degradation in THP1 cells**

| Compound No. | DC₅₀ (nM) |
|---|---|
| Positive compound | 3.46 |
| TM-55 | 4.81 |
| TM-57 | 3.12 |
| TM-66 | 0.57 |
| TM-96 | 0.58 |
| TM-101 | <0.1 |
| TM-102 | 0.9 |
| TM-105 | <0.1 |
| TM-106 | 0.044 |
| TM-109 | 4.67 |
| TM-113 | 1.06 |

DC50 (nM) is an indicator measuring the degradation activity of a compound, and the lower its value is, the stronger the degradation activity is.

The above results show that: the compounds of the present invention have better degradation effects in THP1 cells.

### Test Example 6: A pharmacodynamic experiment on a Lewis rat model of arthritis induced by complete Freund's adjuvant (CFA) is provided below.

### Experimental method:

(1) Animal model establishment: Male Lewis rats (approximately 100-125 g) were grouped, with 8 rats per group. The rats were randomly grouped into 6 groups: a normal control group, a model control group, 3 dose groups (low, moderate, and high) of the test compound (10 mg/kg, 30 mg/kg, and 100 mg/kg), and a positive control group (PF06650883, 30 mg/kg). Except for the normal group, a rat AA model was established in each experimental group on day 0 by injecting 0.08 ml of the complete Freund's adjuvant containing inactivated Mycobacterium tuberculosis (H37RA, 10 mg/ml) into left hind paws of the rats to establish the rat adjuvant arthritis model. The rats were treated with different doses of the test substance by oral administration from day 10 of modeling for 10 consecutive days. Joints of the rats were scored respectively on days 8, 11, 14, 17, 20, 23, and 26 after modeling, and diameters of their left and right hind paws were detected respectively to observe the effect of the drug on adjuvant arthritis in rats.

The arthritis evaluation indicators are as follows:
A. Joint scoring
   Limbs: five-point scale from 0 to 4: no erythema or redness (0); mild erythema or swelling with erythema or swelling at one of the front/back toe joints (1); erythema or swelling at more than one toe (2); swelling of the paw below the ankle or wrist joint (3); and swelling of the entire paw including the ankle joint (4). Four paws of the rats were scored respectively, with a highest score being 16. Their joints were scored on days 8, 11, 17, 14, 17, 20, 23, and 26 after modeling, and the results were recorded.
B. Ankle diameter measurement

Diameters from the inner side to the outer side of the left and right ankles and thicknesses of the limbs of the rats were measured with a vernier caliper before modeling and on days 8, 11, 17, 14, 17, 20, 23 and 26 after modeling, and the results were recorded. The measurement data of the test results were expressed as a mathematical mean plus or minus a standard deviation (mean±SD), and the SPSS11.0 software was used to test the difference between each dosing group and the control group.

### Test Example 7: Metabolic stability test

Blank incubation plates T60 and NCF60 were preheated for 10 min; liver microsome was diluted to 0.56 mg/mL in 100 mM phosphate buffer, 445 uL of the microsome working solution (0.56 mg/mL) was transferred to the preheated "incubation" plates T60 and NCF60, and then the incubation plates T60 and NCF60 were pre-incubated with continuous shaking at 37°C for 10 min. 54 µL of the liver microsome was transferred to a blank plate, then 6 µL of a NAPDH cofactor was added to the blank plate, and then 180 µL of a quenching solution was added to the blank plate; 5 µL of a composite working solution (100 µM) was added to each of the microsome-containing incubation plates (T60 and NCF60), and the mixture was thoroughly mixed 3 times; 50 µL of a buffer was added to the NCF60 plate, and the mixture was thoroughly mixed 3 times. Timing was started; the plates were incubated with shaking at 37°C for 60 min; and 180 µL of a quenching solution and 6 µL of the NAPDH cofactor were added to a quenching plate T0. The plates were ensured to be cooled to prevent evaporation. For the T60 plate, the mixture was thoroughly mixed 3 times, and 54 µL of the mixture was immediately transferred to the quenching plate at the time point of 0 min. Then, 44 µL of the NAPDH cofactor was added to the incubation plate (T60). Timing was started; and the plate was incubated with shaking at 37°C for 60 min. At 5, 15, 30, 45, and 60 min, 180 µL of the quenching solution was added to the quenching plate, mixed once, and then 60 µL of sample was continuously transferred from the plate T60 to the quenching plate at each time point.

For NCF60, the mixture was mixed once, and at the time point of 60 min, 60 µL of sample was transferred from the NCF60 incubator to the quenching solution-containing quenching plate. All sampling plates were shaken for 10 min, then centrifuged at 4,000 rpm at 4°C for 20 min. 80 µL of the supernatant was transferred to 240 µL of HPLC water, and mixed using a plate shaker for 10 min. Each bioanalytical plate was sealed and shaken for 10 min before LC-MS/MS analysis, with the results as shown in Table 22.

**Table 22 Metabolic stability of test substances in liver microsome**

| Compound | Species | |
|---|---|---|
| | Human liver microsome (t_{1/2}, min) | Mouse liver microsome (t_{1/2}, min) |
| Positive compound | 39.4 | 29.4 |
| TM-25 | 117.1 | 128.7 |
| TM-33 | 77.9 | 46.9 |
| TM-56 | 80.8 | 70.1 |

The results show that the compounds of the present invention have better stability in liver microsome than the positive compound (positive drug).

### Test Example 8: Therapeutic effects of compounds of the present invention on psoriasis model

Male BALB/c mice aged 6-8 weeks were randomly grouped into 15 groups or 6 groups after 1 week of adaptive feeding: control group, imiquimod model group (i.e., Model group), clobetasol propionate group (i.e., CLO group), the compounds of the present invention: TM-57 ICL-004192 (3 mg/kg, 10 mg/kg, 30 mg/kg), TM-109ICL-004240 (3 mg/kg, 10 mg/kg, 30 mg/kg, 60mg/kg), TM-101ICL-004250 (3 mg/kg, 10 mg/kg, 30 mg/kg, 60 mg/kg), with 5 mice per group, and the Control group, the imiquimod model group (i.e., the Model group), and the clobetasol propionate group (i.e., the CLO group). On day 0, each of the mice was unhaired on the back using a hair removal cream, with a hair removal area of 2.5 cm×1.5 cm. On the first day, IMQ cream was used for modeling, and a corresponding drug was administered by gavage for treatment. The compounds of the present invention were administered by gavage at corresponding doses twice daily in the morning and evening with an interval of 8 h between two administrations. 2 h after the first gavage on a same day, the IMQ cream was applied to the to-be-unhaired site on the back and the left ear of each of the mice, with doses of the IMQ cream administered to the to-be-unhaired site on the back and the left ear being 62.5 mg and 7.5 mg, respectively. 5 h after the IMQ cream was applied to the to-be-unhaired area on the back and the left ear of each of the mice in the CLO group, clobetasol propionate cream was applied once daily. The blank group and the IMQ group were administrated with a vehicle twice daily in the morning and evening with an interval of 8 h between two administrations. After an interval of 2 h after the first administration on a same day, vaseline and the IMQ cream were applied to the to-be-unhaired site on the back and the ears of each of the mice respectively on the same day for successive administration in 5 days.

Referring to the Psoriasis Area and Severity Index (PASI) scoring criteria, the psoriasis, erythema, and thickness of skin lesions on the back were scored from 0 to 4 every morning from day 1 of modeling, with the scoring criteria as shown in Table 1. The 3 scores were added up to obtain a total score, i.e., a total PASI score. The ear thickness of the left ear of the mouse was measured using a digital vernier caliper three times every morning from day 1, to obtain a mean value.

**Table 23 Psoriasis Area and Severity Index (PASI) scoring criteria**

| | 0 scores | 1 score | 2 scores | 3 scores | 4 scores |
|---|---|---|---|---|---|
| Psoriasis | No psoriasis on the surface | Surface of some skin lesions covered with psoriasis | Surface of most skin lesions covered with psoriasis | Skin lesions almost completely covered with psoriasis | Skin lesions completely covered with psoriasis |
| Erythema | No erythema | Light red | Red | Dark red | Purple red |
| Thickness | Skin lesion flush with normal skin | Skin lesion slightly above normal skin surface | Skin lesion moderately raised | Skin lesions thickened and obviously raised | Skin lesions highly thickened and obviously raised |

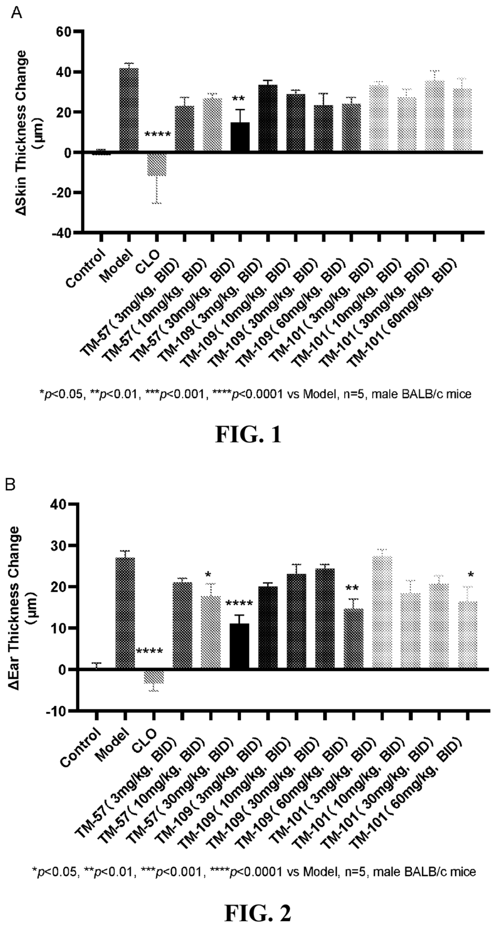

FIG. 1 shows a therapeutic effect of a compound of the present invention on IMQ-induced psoriatic skin thickening; and FIG. 2 shows a therapeutic effect of a compound of the present invention on IMQ-induced psoriatic ear thickening.

The results show that: the compound of the present invention has a significant therapeutic effect on the IMQ-induced psoriatic skin thickening and ear thickening.

In the therapeutic effect of the compound on the IMQ-induced psoriatic skin thickening shown in FIG. 1, compared with the Model group, the TM-57 (30 mg/kg, BID) has P<0.01.

In the therapeutic effect of the compound on the IMQ-induced psoriatic ear thickening as shown in FIG. 2, compared with the Model group, the TM-57 (10 mg/kg, BID) has P<0.05, the TM-57 (30 mg/kg, BID) has P<0.0001, the TM-109 (60mg/kg, BID) has P<0.01, and the TM-101 (60mg/kg, BID) has P<0.05.

### Test Example 9: In vivo metabolism test of drugs of the present invention

Pharmacokinetic experiment on ICR mice: in this experiment, two administration modes: oral intragastric administration and tail vein injection were selected. Each of the compounds of the present patented invention was administered at an oral dose of 5 mg/kg at an administration volume of 10 mL/kg. Right before use, the compound was accurately weighed, an appropriate amount of a drug was accurately weighed, and they were first dissolved in dimethyl sulfoxide (DMSO), to prepare a final volume of 5% DMSO, 10% Solutol, and 85% H₂O. Appropriate amounts of Solutol and H₂O were proportionally added sequentially, and fully mixed ultrasonically by vortexing, to prepare a clear drug solution at 0.5 mg/mL. ICL004-protodrug was administered by intravenous injection at a dose of 1 mg/kg at an administration volume of 10 mL/kg. Right before use, an appropriate amount of the drug was accurately weighed, to prepare a final volume of 20% PEG 400 and 80% Saline. Appropriate amounts of PEG 400 and Saline were proportionally added sequentially, and fully mixed ultrasonically by vortexing, to prepare a clear drug solution at 0.1 mg/mL.

12 ICR mice, each with body weight of 28-32 g, were randomly grouped into 2 groups. After fasting but with free access to water overnight, each mouse in the first group was administered by gavage. 100 µL of blood was sampled from its eye socket into an anticoagulant tube respectively at each time point of 15 min, 30 min, 1 h, 2 h, 4 h, 6 h, 8 h, and 24 h after administration (wherein 3 mice of a same batch were used at 15 min, 1 h, 4 h, and 8 h; and 3 mice of a same batch were used at 30 min, 2 h, 6 h, and 24 h). Within one hour, each of the blood samples was centrifuged at 10,000 rpm at 4°C for 20 min (the blood sample was stored on an ice box prior to the centrifugation), and the supernatant (i.e., the plasma) was stored in a refrigerator at -20°C for LC-MS/MS analysis. 100 µL of blood was sampled from the eye socket of each mouse in the second group into an anticoagulant tube respectively at each time point of 5 min, 15 min, 30 min, 1 h, 2 h, 4 h, 8 h, and 24 h after administration by intravenous injection (wherein 3 mice of a same batch were used at 5 min, 30 min, 2 h, and 8 h; and 3 mice of a same batch were used at 15 min, 1 h, 4 h, and 24 h). Within one h, each of the blood samples was centrifuged at 10,000 rpm at 4°C for 20 min (the sample was stored on an ice box prior to the centrifugation), and the supernatant, i.e., the plasma, was stored in a refrigerator at -20°C for LC-MS/MS analysis.

Pharmacokinetic experiment on SD rats: in this experiment, two administration modes: oral intragastric administration and tail vein injection were selected. Each of the compounds of the present patented invention was administered at an oral dose of 5 mg/kg at an administration volume of 10 mL/kg. Right before use, the compound was accurately weighed, an appropriate amount of a drug was accurately weighed, and they were first dissolved in dimethyl sulfoxide (DMSO), to prepare a final volume of 5% DMSO, 10% Solutol, and 85% H₂O. Appropriate amounts of Solutol and H₂O were proportionally added sequentially, and fully mixed ultrasonically by vortexing, to prepare a clear drug solution at 0.5 mg/mL. ICL004-protodrug was administered by intravenous injection at a dose of 1 mg/kg at an administration volume of 10 mL/kg. Right before use, an appropriate amount of the drug was accurately weighed, to prepare a final volume of 20% PEG 400 and 80% Saline. Appropriate amounts of PEG 400 and Saline were proportionally added sequentially, and fully mixed ultrasonically by vortexing, to prepare a clear drug solution at 0.1 mg/mL.

6 SD rats, each with body weight of 180-220 g, were randomly grouped into 2 groups. After fasting but with free access to water overnight, each mouse in the first group was administered by gavage. 200 µL of blood was sampled from its eye socket into an anticoagulant tube respectively at each time point of 15 min, 30 min, 1 h, 2 h, 4 h, 6 h, 8 h, and 24 h after administration. Within one h, each of the blood samples was centrifuged at 10,000 rpm at 4°C for 20 min (the sample was stored on an ice box prior to the centrifugation), and the supernatant (i.e., the plasma) was stored in a refrigerator at -20°C for LC-MS/MS analysis. 200 µL of blood was sampled from the eye socket of each mouse in the second group into an anticoagulant tube respectively at each time point of 5 min, 15 min, 30 min, 1 h, 2 h, 4 h, 8 h, and 24 h after administration by intravenous injection. Within one h, each of the blood samples was centrifuged at 10,000 rpm at 4°C for 20 min (the blood sample was stored on an ice box prior to the centrifugation), and the supernatant, i.e., the plasma, was stored in a refrigerator at -20°C for LC-MS/MS analysis. The results are shown in Table 24.

**Table 24 Pharmacokinetic data of compounds of the present invention**

| Compound | Parameters Parameters | | Dose (mg/kg) | AUCₐₗₗ (ng*h/mL) | AUC_{inf} (ng*h/mL) | Peak plasma concentration *C*ₘₐₓ (ng/ml) | Oral bioavailability *F*_{obs} (%) |
|---|---|---|---|---|---|---|---|
| TM-33 | BALB/c Mice | iv | 1.00 | 63065.60±1 1425.58 | 104470.75± 39342.92 | 6800.67±641.88 | |
| | | ig | 3.00 | 135385.09± 57153.54 | 288780.52± 128650.93 | 12411.33±3040.99 | 92.14 |
| | SD Rats | iv | 1.00 | 2248.59±54 7.02 | 2302.65±57 6.45 | 530.33±66.67 | |
| | | ig | 5.00 | 13829.46±7 748.72 | 10578.22±5 379.09 | 1101.67±573.70 | 91.88 |
| TM-56 | ICR Mice | iv | 1.00 | 523.76±51. 47 | 613.27±124. 89 | 81±8.66 | |
| | | ig | 5.00 | 2507.33±62 7.92 | 2819.01±51 4.60 | 219.67±90.47 | 91.93 |
| | SD Rats | iv | 1.00 | 1901.21±22 5.92 | 1984.24±24 2.35 | 477.00±137.18 | |
| | | ig | 5.00 | 1064.13±8. 84 | 1120.19 | 96.00±1.14 | 10.73 |
| TM-57 | BALB/c Mice | iv | 1.00 | 789.91±206 .64 | 795.40±206. 32 | 276.00±92.41 | |
| | | ig | 5.00 | 1768.21±71 7.84 | 1923.78±76 7.12 | 342.33±39.25 | 48.37 |
| | SD Rats | iv | 1.00 | 498.93±75. 21 | 525.73±56.3 3 | 225.00±57.82 | |
| | | ig | 5.00 | 579.63±231 .80 | 1225.02±78 0.91 | 79.67±20.74 | 46.60 |
| TM-109 | BALB/c Mice | iv | 1.00 | 3218.10±46 9.68 | 3221.69±16 8.57 | 605.33±57.42 | |
| | | ig | 5.00 | 10074.42±3 328.16 | 10145.03±3 331.52 | 1300.33±186.94 | 62.98 |
| | SD Rats | iv | 1.00 | 537.15±60. 78 | 776.30±365. 22 | 101.33±25.15 | |
| | | ig | 5.00 | 268.13±74. 60 | 305.51±118. 33 | 23.00±4.24 | 7.87 |
| TM-101 | BALB/c Mice | iv | 1.00 | 3389.06±26 7.86 | 3524.46±26 4.82 | 579.67±73.12 | |
| | | ig | 5.00 | 3371.54±10 45.96 | 3552.65±10 54.87 | 356.00±65.18 | 20.06 |
| | SD Rats | iv | 1.00 | 625.23±120 .37 | 673.55±110. 46 | 151.00±27.84 | |
| | | ig | 5.00 | 373.63±115 .53 | 435.21±136. 94 | 23.00±7.55 | 11.09 |

The results are as follows: the compounds of the present invention have excellent oral bioavailability in mice and rats.

To sum up, the present invention discloses a compound of formula I, which can effectively degrade IRAK4 or inhibit IRAK4 activity in other ways, and has very good application prospects in treating IRAK4-mediated diseases including, e.g., immune diseases (such as psoriasis, hidradenitis suppurativa, atopic dermatitis, rheumatoid arthritis, systemic lupus erythematosus, alcoholic liver disease, autoimmune liver disease, or acne), tumors (such as multiple myeloma, lymphocytic leukemia, and lymphoma), Alzheimer's disease, and fibrosis, thus providing a new choice for clinical selection and/or preparation of a medicament for treating a disease associated with the IRAK4 activity.

## Claims

1. A compound represented by formula I, or an enantiomer thereof, a diastereomer thereof, a racemate thereof, a mixture comprising the same, or a deuterated compound thereof, or a pharmaceutically acceptable salt thereof: 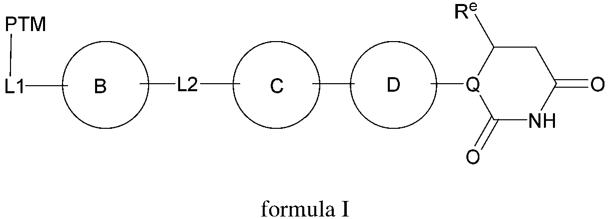 wherein
PTM is selected from 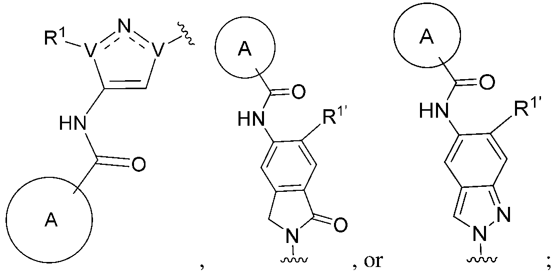
ring D is selected from 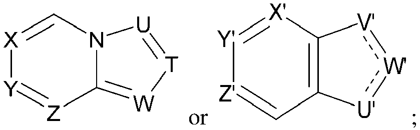
and when the ring D is selected from 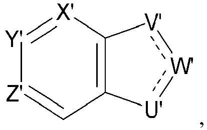 the PTM is not selected from 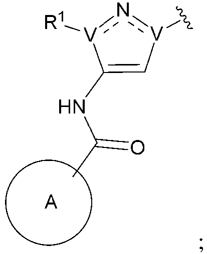
Q is selected from CR^{2a} or N;
V' is selected from C or N;
U' is selected from CR² or NR²;
W' is selected from CR³ or N;
X' is selected from C, CR⁴, or N;
Y' is selected from C, CR⁵, or N;
Z' is selected from C, CR⁶, or N;
U is selected from C, CR^{U}, or N;
W is selected from C, CR^{W}, or N;
T is selected from C, CR^{T}, or N;
X is selected from C, CR^{X}, or N;
Y is selected from C, CR^{Y}, or N;
Z is selected from C, CR^{Z}, or N;
-̅ -̅ -̅ represents a single bond or a double bond;
V is selected from C or N;
R¹ and R^{1'} are each independently selected from hydrogen, deuterium, halogen, cyano, substituted or unsubstituted C1-C6 linear or branched alkyl, substituted or unsubstituted C1-C6 linear or branched alkoxy, substituted or unsubstituted 4-10-membered heterocyclyl, substituted or unsubstituted C5-C12 bridged heterocyclyl, substituted or unsubstituted C3-C10 heterospirocyclyl, substituted or unsubstituted C1-C6 linear or branched alkylamino, substituted or unsubstituted 3-10-membered cycloalkyl, substituted or unsubstituted unsaturated 3-10-membered cyclic hydrocarbon radical, or substituted or unsubstituted C2-C6 linear or branched unsaturated hydrocarbon radical; wherein the substutuents are independently selected from deuterium, halogen, hydroxyl, cyano, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, or 4-10-membered heterocyclyl; and the heterocyclyl comprises 1-4 heteroatoms selected from oxygen, sulfur, and nitrogen;
ring A is independently selected from 0-4 R⁷-substituted: 5-10-membered heteroaromatic rings or 5-6-membered heteroaromatic ring-5-6-membered heteroaromatic rings; and the heteroaryl comprises 1-4 heteroatoms selected from oxygen, sulfur, and nitrogen;
R^{2a}, R², R³, R⁴, R⁵, R⁶, R^{U}, R^{W}, R^{T}, R^{X}, R^{Y}, R^{Z}, R^{e}, and R⁷ are each independently selected from hydrogen, deuterium, halogen, cyano, hydroxyl, amino, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, substituted or unsubstituted C1-C6 linear or branched alkanoyl, or substituted or unsubstituted 3-10-membered cycloalkanoyl; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, 5-10-membered heteroaromatic ring, 3-10-membered cycloalkyl, C1-C6 linear or branched alkyl-C(=O)-, or 3-10-membered cycloalkyl-C(=O)-; and the heterocyclyl, the bridged heterocyclyl, the heterospirocyclyl, and the heteroaromatic ring each comprise 1-4 heteroatoms selected from oxygen, sulfur, and nitrogen;
L1 is selected from N.A., 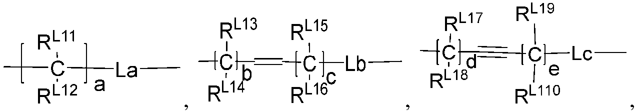 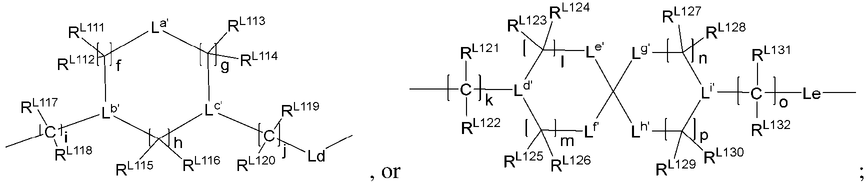
a, b, c, d, e, f, g, h, i, j, k, 1, m, n, o, and p are each independently selected from 0-6;
La, Lb, Lc, Ld, Le, L^{a'}, L^{e'}, L^{f'}, L^{g'}, and L^{h'} are each independently selected from N.A., O, S, - N(R⁸)-, -C(=O)-R⁹-, or -SO₂R¹⁰-; R⁸ is selected from hydrogen, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, -R¹¹COR¹²-, -R¹³OCOR¹⁴-, -R¹⁵COOR¹⁶-,-R¹⁷NR¹⁸COR¹⁹-, -R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴-, or 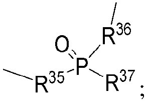 the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, or 5-10-membered heteroaromatic ring; the heterocyclyl, the bridged heterocyclyl, the heterospirocyclyl, and the heteroaromatic ring each comprise 1-4 heteroatoms selected from oxygen, sulfur, and nitrogen; the R⁸ can form a substituted or unsubstituted 3-10-membered ring with R^{L111}, R^{L112}, R^{L113}, R^{L114}, R^{L123}, R¹²⁴, R^{L125}, R^{L126}, R^{L127}, R^{L128}, R^{L129}, and R^{L130} respectively through C, N, O, and S; and the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-,-R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴-, or 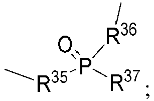
R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹, R³², R³³, R³⁴, R³⁵, R³⁶, and R³⁷ are each independently selected from N.A., hydrogen, deuterium, halogen, cyano, hydroxyl, amino, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, substituted or unsubstituted C1-C6 linear or branched alkanoyl, or substituted or unsubstituted 3-10-membered cycloalkanoyl; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, or 5-10-membered heteroaromatic ring; and the heterocyclyl, the bridged heterocyclyl, the heterospirocyclyl, and the heteroaromatic ring each comprise 1-4 heteroatoms selected from oxygen, sulfur, and nitrogen;
L^{b'}, L^{c'}, L^{d'}, and L^{i'} are each independently selected from N or CR³⁸;
R³⁸, R^{L11}, R^{L12}, R^{L13}, R^{L14}, R^{L15}, R^{L16}, R^{L17}, R^{L18}, R^{L19}, R^{L110}, R^{L111}, R^{L112}, R^{L113}, R^{L114}, R^{L115}, R^{L116}, R^{L117}, R^{L118}, R^{L119}, R^{L120}, R^{L121}, R^{L122}, R^{L123}, R^{L124}, R^{L25}, R^{L126}, R^{L127}, R^{L128}, R^{L129}, R^{L130}, R^{L131} and R^{L132} are each independently selected from hydrogen, deuterium, halogen, cyano, hydroxyl, amino, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino,-R¹¹COR¹²-, -R¹³OCOR¹⁴-, -R¹⁵COOR¹⁶-, -R¹⁷NR¹⁸COR¹⁹-, -R²⁰CONR²¹R²²-, -R²³SO₂R²⁴-,-R²⁵OSO₂R²⁶-, -R²⁷SO₂OR²⁸-, -R²⁹NR³⁰SO₂R³¹-, -R³²SO₂NR³³R³⁴-, or 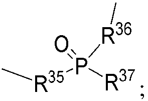
the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, or 5-10-membered heteroaromatic ring; the heterocyclyl, the bridged heterocyclyl, the heterospirocyclyl, and the heteroaromatic ring each comprise 1-4 heteroatoms selected from oxygen, sulfur, and nitrogen; the R³⁸ can form a substituted or unsubstituted 3-10-membered ring with the R^{L111}, R^{L112}, R^{L113}, R^{L114}, R^{L115}, R^{L116}, R^{L117}, R^{L118}, R^{L119}, R^{L120}, R^{L121}, R^{L122}, R^{L123}, R^{L124}, R^{L125}, R^{L126}, R^{L127}, R^{L128}, R^{L129}, R^{L130}, R^{L131}, and R^{L132} through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-,-R¹³OCOR¹⁴-, -R¹⁵COOR¹⁶-, -R¹⁷NR¹⁸COR¹⁹-, -R²⁰CONR²¹R²²-, -R²³SO₂R²⁴-, -R²⁵OSO₂R²⁶-,-R²⁷SO₂OR²⁸-, -R²⁹NR³⁰SO₂R³¹-, -R³²SO₂NR³³R³⁴-, or 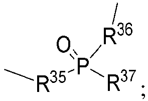 any two radicals among the R^{L111}, R^{L112}, R^{L113}, R^{L114}, R^{L115}, R^{L116}, R^{L117}, R^{L118}, R^{L119}, and R^{L120} can form a substituted or unsubstituted 3-12-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, -R²⁰CONR²¹R²²-, -R²³SO₂R²⁴-,-R²⁵OSO₂R²⁶-, -R²⁷SO₂OR²⁸-, -R²⁹NR³⁰SO₂R³¹-, -R³²SO₂NR³³R³⁴-, or 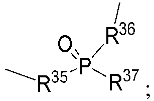 any two radicals among the R^{L121}, R^{L122}, R^{L123}, R^{L124}, R^{L125}, and R^{L126} can form a substituted or unsubstituted 3-10-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, -R²⁰CONR²¹R²²-, -R²³SO₂R²⁴-,-R²⁵OSO₂R²⁶-, -R²⁷SO₂OR²⁸-, -R²⁹NR³⁰SO₂R³¹-, -R³²SO₂NR³³R³⁴-, or 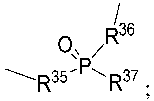 any two radicals among the R^{L127}, R^{L128}, R^{L129}, R^{L130}, R^{L131}, and R^{L132} can form a substituted or unsubstituted 3-10-membered ring through C, N, O, or S; and the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-, -R²⁰CONR²¹R²²-, -R²³SO₂R²⁴-,-R²⁵OSO₂R²⁶-, -R²⁷SO₂OR²⁸-, -R²⁹NR³⁰SO₂R³¹-, -R³²SO₂NR³³R³⁴-, or 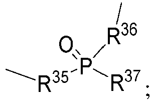
ring B is selected from N.A., substituted or unsubstituted C3-C12 cycloalkyl, substituted or unsubstituted C3-C12 heterocyclyl, substituted or unsubstituted C3-C12 bridged heterocyclyl, substituted or unsubstituted C3-C12 heterospirocyclyl, substituted or unsubstituted 6-10-membered aromatic ring, or substituted or unsubstituted 5-10-membered heteroaromatic ring; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-,-R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴- 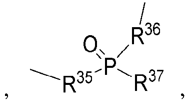 4-10-membered heterocyclyl, C5-C12 bridged heterocyclyl, C3-C10 heterospirocyclyl, 6-10-membered aromatic ring, or 5-10-membered heteroaromatic ring; and the heterocyclyl, the bridged heterocyclyl, the heterospirocyclyl, and the heteroaromatic ring each comprise 1-4 heteroatoms selected from oxygen, sulfur, and nitrogen;
L2 is selected from N.A., 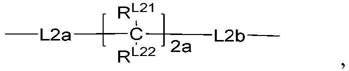 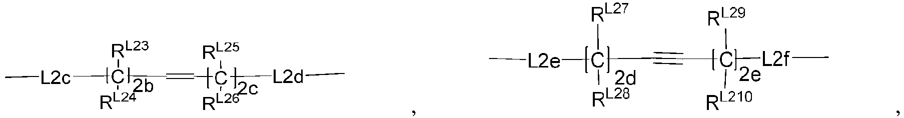 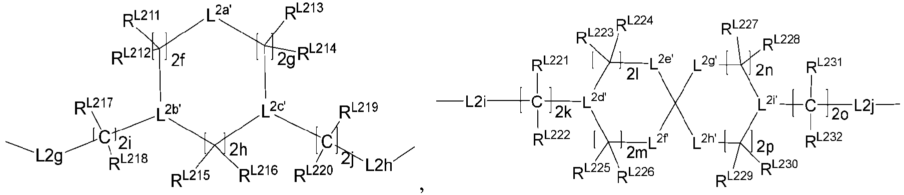 , or 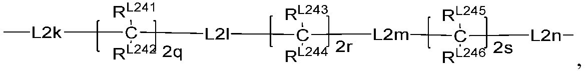
2a, 2b, 2c, 2d, 2e, 2f, 2g, 2h, 2i, 2j, 2k, 21, 2m, 2n, 2o, 2p, 2q, 2r, and 2s are each independently selected from 0-6;
L2a, L2b, L2c, L2d, L2e, L2f, L2g, L2h, L2i, L2j, L2k, L2l, L2m, L2n, L^{2a'}, L^{2e'}, L^{2f'}, L^{2g'}, and L^{2h'} are independently selected from N.A., O, S, NR³⁹, COR⁴⁰, or -SO₂R⁴¹;
L^{2b'}, L^{2c'}, L^{2d'}, and L^{2i'} are selected from N or CR⁴⁴;
R³⁹, R⁴⁰, R⁴¹, R⁴⁴, R^{L21}, R^{L22}, R^{L23}, R^{L24}, R^{L25}, R^{L26}, R^{L27}, R^{L28}, R^{L29}, R^{L210}, R^{L21}, R^{L22}, R^{L23}, R^{L24}, R^{L25}, R^{L26}, R^{L27}, R^{L28}, R^{L29}, R^{L210}, R^{L211}, R^{L212}, R^{L213}, R^{L214}, R^{L223}, R^{L224}, R^{L225}, R^{L226}, R^{L227}, R^{L228}, R^{L229}, R^{L230}, R^{L241}, R^{L242}, R^{L243}, R^{L244}, R^{L245}, and R^{L246} are each independently selected from hydrogen, substituted or unsubstituted 4-10-membered heterocyclyl, substituted or unsubstituted C5-C12 bridged heterocyclyl, substituted or unsubstituted C3-C10 heterospirocyclyl, substituted or unsubstituted 6-10-membered aromatic ring, substituted or unsubstituted 5-10-membered heteroaromatic ring, substituted or unsubstituted C1-C6 linear or branched alkyl, substituted or unsubstituted 3-10-membered cycloalkyl, substituted or unsubstituted unsaturated 3-10-membered cyclic hydrocarbon radical, substituted or unsubstituted C2-C6 linear or branched unsaturated hydrocarbon radical, substituted or unsubstituted C1-C6 linear or branched alkoxy, substituted or unsubstituted 3-10-membered cycloalkoxy, substituted or unsubstituted C1-C6 linear or branched alkylamino, substituted or unsubstituted 3-10-membered cycloalkylamino, -R¹¹COR¹²-,-R¹³OCOR¹⁴-, -R¹⁵COOR¹⁶-, -R¹⁷NR¹⁸COR¹⁹-, -R²⁰CONR²¹R²²-, -R²³SO₂R²⁴-, -R²⁵OSO₂R²⁶-,-R²⁷SO₂OR²⁸-, -R²⁹NR³⁰SO₂R³¹-, -R³²SO₂NR³³R³⁴-, or 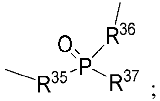 the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, C1-C6 linear or branched alkanoyl, 3-10-membered cycloalkanoyl, 4-10-membered heterocyclyl, C5-C12 bridged heterocyclyl, C3-C10 heterospirocyclyl, 6-10-membered aromatic ring, or 5-10-membered heteroaromatic ring; the heterocyclyl, the bridged heterocyclyl, heterospirocyclyl, and the heteroaromatic ring each comprise 1-4 heteroatoms selected from oxygen, sulfur, and nitrogen; the R³⁹ can form a substituted or unsubstituted 3-10-membered ring with the R^{L21}, R^{L22}, R^{L23}, R^{L24}, R^{L25}, R^{L26}, R^{L27}, R^{L28}, R^{L29}, R^{L210}, R^{L211}, R^{L212}, R^{L213}, R^{L214}, R^{L223}, R^{L224}, R^{L225}, R^{L226}, R^{L227}, R^{L228}, R^{L229}, and R^{L230} respectively through C, N, O, and S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-,-R¹⁷NR¹⁸COR¹⁹-, -R²⁰CONR²¹R²²-, -R²³SO₂R²⁴-, -R²⁵OSO₂R²⁶-, -R²⁷SO₂OR²⁸-, -R²⁹NR³⁰SO₂R³¹-, -R³²SO₂NR³³R³⁴-, or 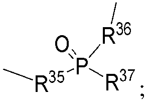 any two radicals among the R^{L211}, R^{L212}, R^{L213}, R^{L214}, R^{L215}, R^{L216}, R^{L217}, R^{L218}, R^{L219}, and R^{L220} can form a substituted or unsubstituted 3-12-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-,-R¹⁵COOR¹⁶-, -R¹⁷NR¹⁸COR¹⁹-, -R²⁰CONR²¹R²²-, -R²³SO₂R²⁴-, -R²⁵OSO₂R²⁶-, -R²⁷SO₂OR²⁸-,-R²⁹NR³⁰SO₂R³¹-, -R³²SO₂NR³³R³⁴-, or 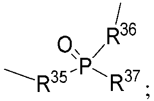 any two radicals among the R^{L221}, R^{L222}, R^{L223}, R^{L224}, R^{L225}, and R^{L226} can form a substituted or unsubstituted 3-10-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-,-R¹⁵COOR¹⁶-, -R¹⁷NR¹⁸COR¹⁹-, -R²⁰CONR²¹R²²-, -R²³SO₂R²⁴-, -R²⁵OSO₂R²⁶-, -R²⁷SO₂OR²⁸-,-R²⁹NR³⁰SO₂R³¹-, -R³²SO₂NR³³R³⁴-, or 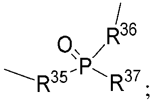 any two radicals among the R^{L227}, R^{L228}, R^{L229}, R^{L230}, R^{L231}, and R^{L232} can form a substituted or unsubstituted 3-10-membered ring through C, N, O, or S; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-,-R¹⁵COOR¹⁶-, -R¹⁷NR¹⁸COR¹⁹-, -R²⁰CONR²¹R²²-, -R²³SO₂R²⁴-, -R²⁵OSO₂R²⁶-, -R²⁷SO₂OR²⁸-,-R²⁹NR³⁰SO₂R³¹-, -R³²SO₂NR³³R³⁴-, or 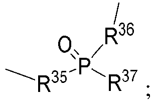 or the R⁴⁴ can form a substituted or unsubstituted 3-10-membered ring with the R^{L211}, R^{L212}, R^{L213}, R^{L214}, R^{L223}, R^{L224}, R^{L225}, R^{L226}, R^{L227}, R^{L228}, R^{L229}, R^{L230}, R^{L231}, and R^{L232} through C, N, O, or S; and the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, 3-10-membered cycloalkoxy, C1-C6 linear or branched alkylamino, 3-10-membered cycloalkylamino, -R¹¹COR¹²-, -R¹³OCOR¹⁴-, -R¹⁵COOR¹⁶-, -R¹⁷NR¹⁸COR¹⁹-,-R²⁰CONR²¹R²²-, -R²³SO₂R²⁴-, -R²¹OSO₂R²⁶-, -R²⁷SO₂OR²⁸-, -R²⁹NR³⁰SO₂R³¹-, -R³²SO₂NR³³R³⁴-, or 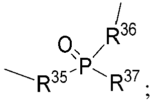
ring C is selected from N.A., substituted or unsubstituted C3-C12 cycloalkyl, substituted or unsubstituted C3-C12 heterocyclyl, substituted or unsubstituted C3-C12 bridged heterocyclyl, substituted or unsubstituted C3-C12 heterospirocyclyl, substituted or unsubstituted 6-10-membered aromatic ring, or substituted or unsubstituted 5-10-membered heteroaromatic ring; the substituents are independently selected from deuterium, halogen, cyano, hydroxyl, amino, C1-C6 linear or branched alkyl, C2-C6 linear or branched unsaturated hydrocarbon radical, C1-C6 linear or branched alkoxy, C1-C6 linear or branched haloalkyl, C2-C6 linear or branched unsaturated halohydrocarbon radical, C1-C6 linear or branched haloalkoxy, 3-10-membered cycloalkoxy, C1-C6 linear or branched alkylamino, 3-10-membered cycloalkylamino, -R¹¹COR¹²-, -R¹³OCOR¹⁴-,-R¹⁵COOR¹⁶-, -R¹⁷NR¹⁸COR¹⁹-, -R²⁰CONR²¹R²²-, -R²³SO₂R²⁴-, -R²⁵OSO₂R²⁶-, -R²⁷SO₂OR²⁸-,-R²⁹NR³⁰SO₂R³¹-, -R³²SO₂NR³³R³⁴-, 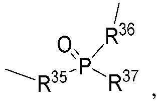 4-10-membered heterocyclyl, C5-C12 bridged heterocyclyl, C3-C10 heterospirocyclyl, 6-10-membered aromatic ring, or 5-10-membered heteroaromatic ring; and the heterocyclyl, the bridged heterocyclyl, the heterospirocyclyl, and the heteroaromatic ring each comprise 1-4 heteroatoms selected from oxygen, sulfur, and nitrogen;
wherein 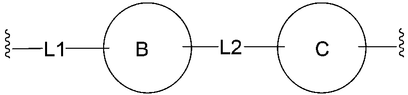 is preferably selected from: 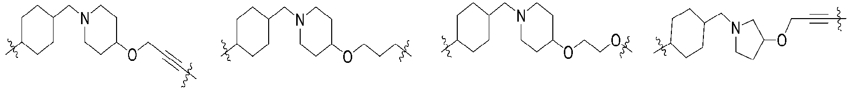 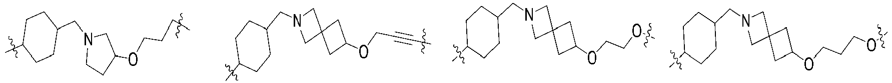 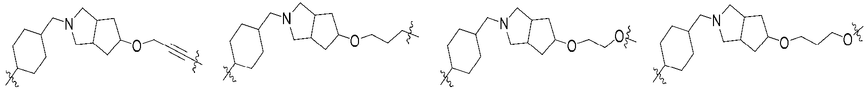 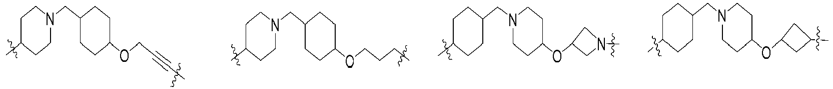 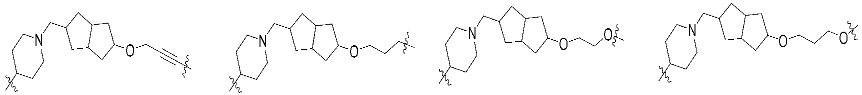 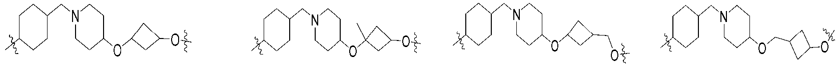 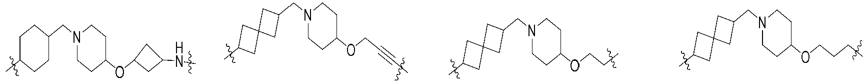 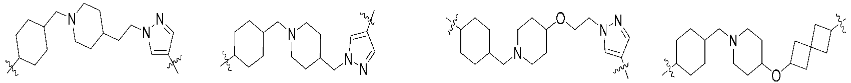 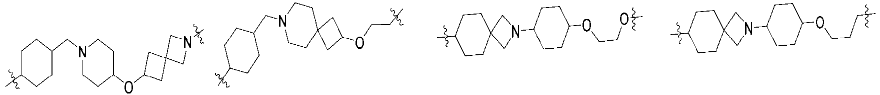 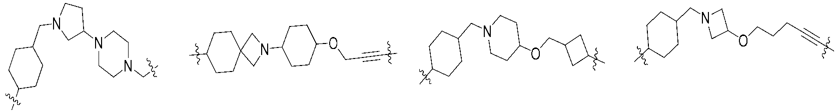 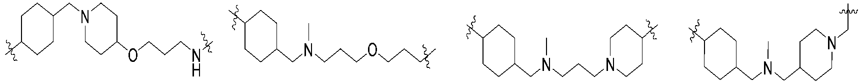 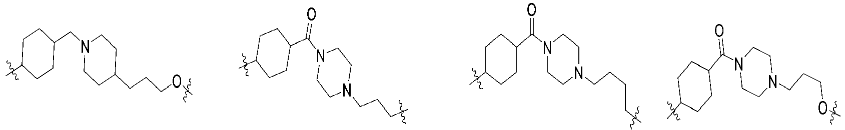 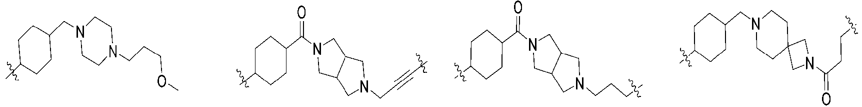 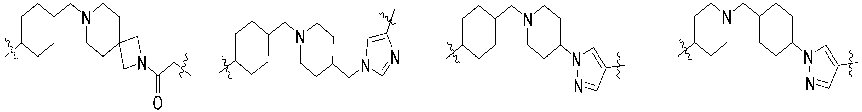 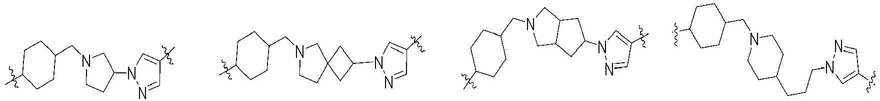 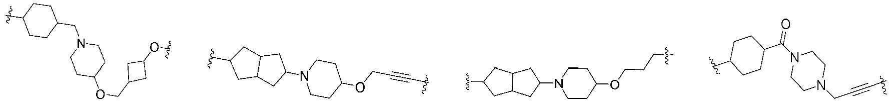 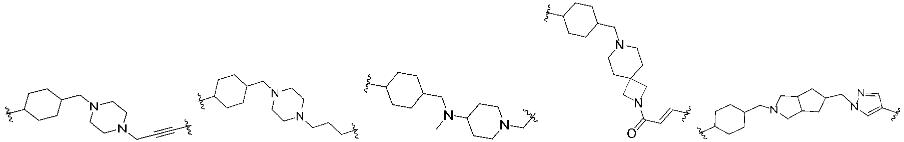 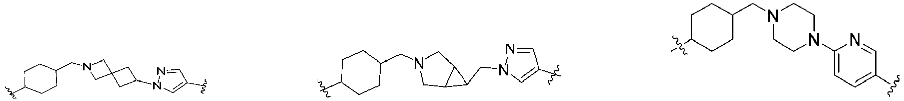 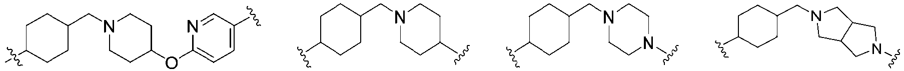 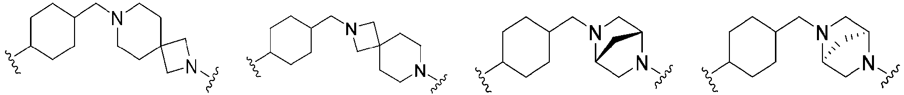 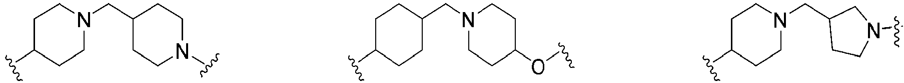 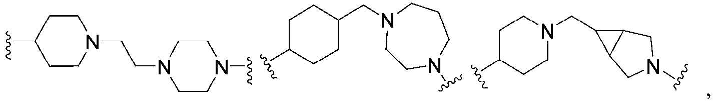 a single cis-trans isomer or a mixture thereof;
for example, 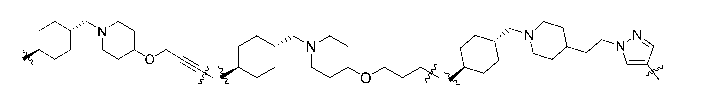 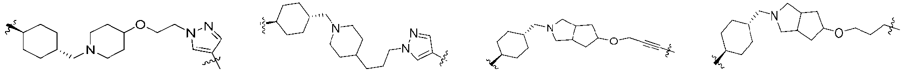 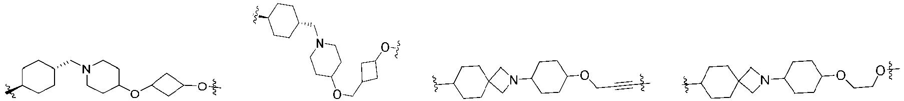 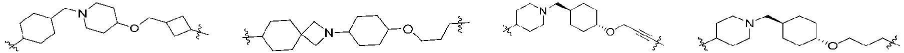 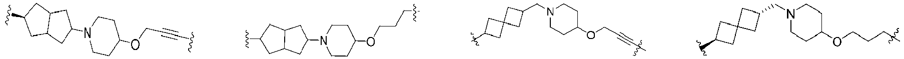 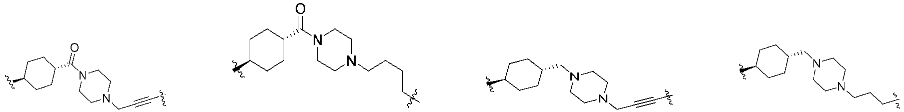 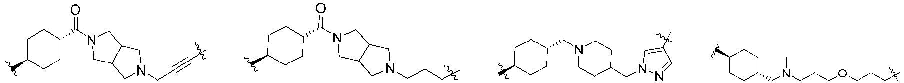 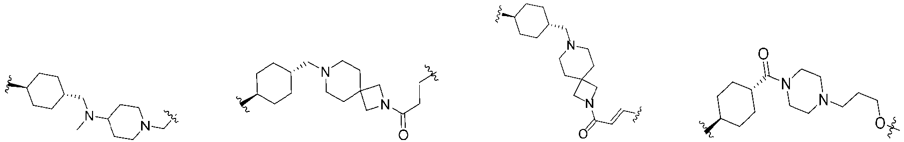 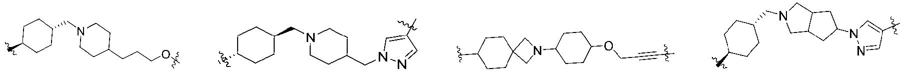 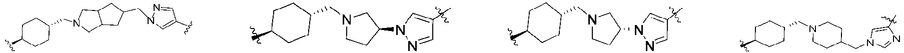 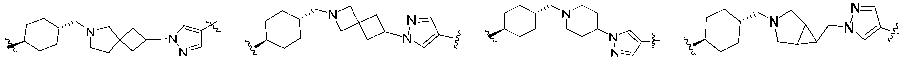 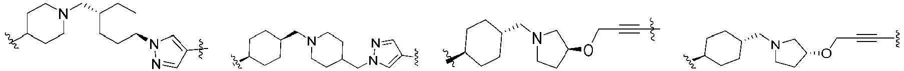 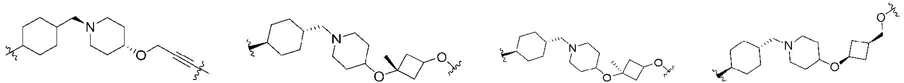 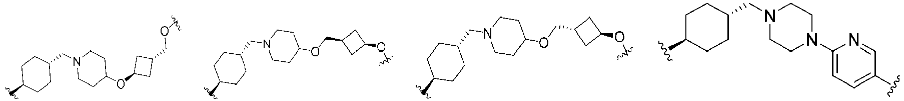 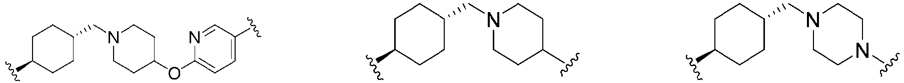 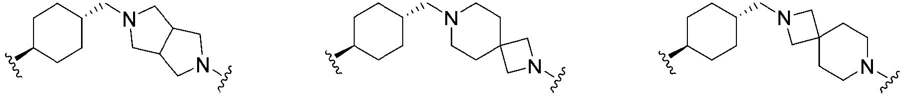 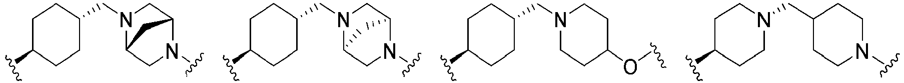 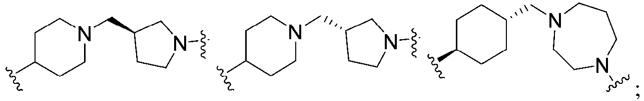
each of the halogen is independently fluorine, chlorine, bromine, or iodine;
C1-C6 alkyl among each of the substituted or unsubstituted C1-C6 linear or branched alkyl is independently methyl, ethyl, n-propyl, isopropyl, n-butyl, isobutyl, tert-butyl, sec-butyl, n-pentyl, isopentyl, neopentyl, and hexyl;
3-10-membered cycloalkyl among each of the substituted or unsubstituted 3-10-membered cycloalkyl is independently cyclopropyl, cyclobutyl, cyclopentyl, cyclobutyl, 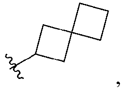 or 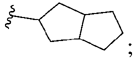
4-10-membered heterocyclyl among each of the substituted or unsubstituted 4-10-membered heterocyclyl is independently 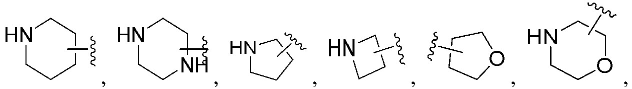 or 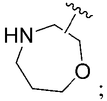 for example, 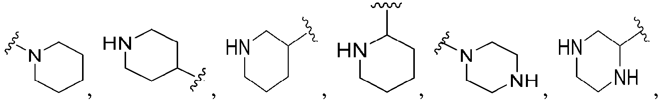 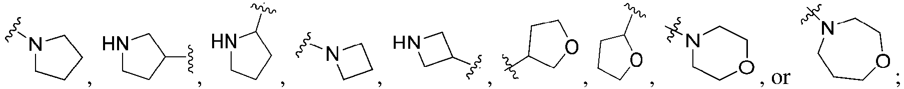
C3-C10 heterospirocyclyl among each of the substituted or unsubstituted C3-C10 heterospirocyclyl is independently 2-azaspirocyclo[3.3]heptyl, 7-azaspirocyclo[3.5]nonyl, 2-azaspirocyclo[3.5]nonyl, 2,7-diazaspirocyclo[3.5]nonyl, 6-azaspirocyclo[3.4]octyl, 4-oxa-7-azaspirocyclo[2.5]octyl, 5-oxa-8-azaspirocyclo[3.5]nonyl, 2-oxa-6-azaspirocyclo[3.3]heptyl, 2-oxa-6-azaspirocyclo[3.4]octyl, or 4,7-diazaspirocyclo[2.5]octyl; for example, 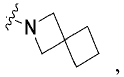 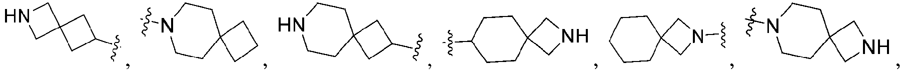 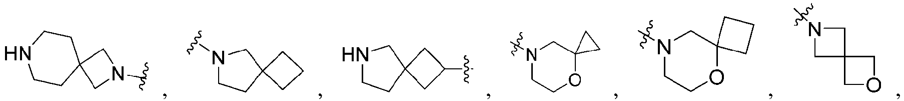 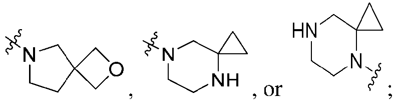
C5-C12 bridged heterocyclyl among each of the substituted or unsubstituted C5-C12 bridged heterocyclyl is independently octahydrocyclopenta[C]pyrrolyl, octahydropyrrolo[3,4-c]pyrrolyl, 3-azabicyclo[3.1.0]hexyl, 2-oxa-5-azabicyclo[2.2.1]heptyl, or 8-oxa-3-azabicyclo[3.2.1]octyl; for example, 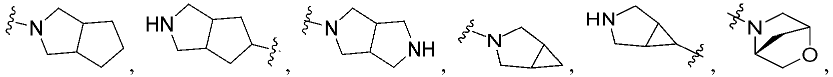 or 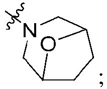 and
5-10-membered heteroaromatic ring among each of the substituted or unsubstituted 5-10-membered heteroaromatic ring is independently

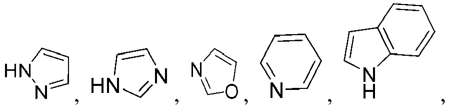

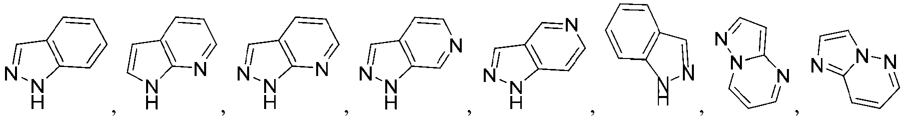

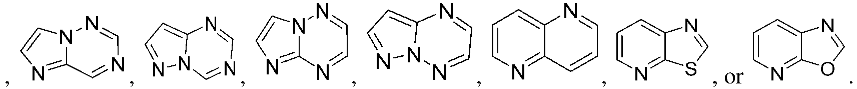

2. The compound according to claim 1, or the enantiomer thereof, the diastereomer thereof, the racemate thereof, the mixture comprising the same, or the deuterated compound thereof, or the pharmaceutically acceptable salt thereof, wherein: the compound represented by formula I is as shown in formula Ia: 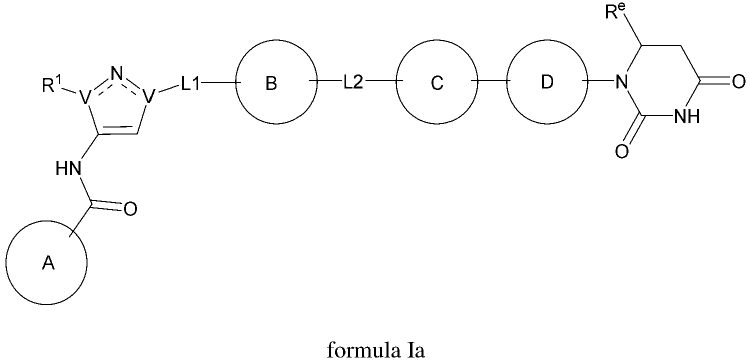 wherein
the ring D is selected from 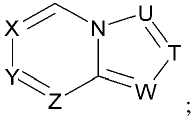
U is selected from N, W is selected from C or CR^{W}, and T is selected from C or CR^{T};
or the W is selected from N, the U is selected from C or CR^{U}, and the T is selected from C or CR^{T};
at most one of X, Y, and Z is N;
R¹ is selected from hydrogen, deuterium, halogen, cyano, or substituted or unsubstituted C1-C6 linear or branched alkyl; wherein the substituents are independently selected from deuterium, halogen, hydroxyl, C1-C6 linear or branched alkoxy, or 3-10-membered cycloalkoxy; and a number of the substituents is 1, 2, or 3; for example, the R¹ is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, or cyclopropoxymethyl; 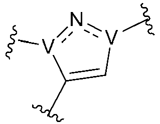 is 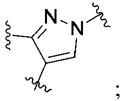
the R^{U}, R^{W}, R^{T}, R^{X}, R^{Y}, R^{Z}, and R^{e} are each independently selected from hydrogen, halogen, cyano, substituted or unsubstituted C1-C6 linear or branched alkyl, or substituted or unsubstituted C1-C6 linear or branched alkoxy; for example, H, F, or methyl;
the ring A is independently selected from rings below: 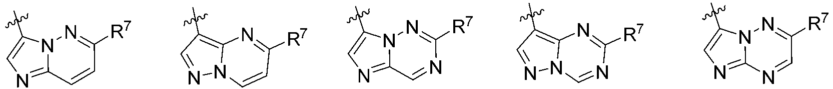 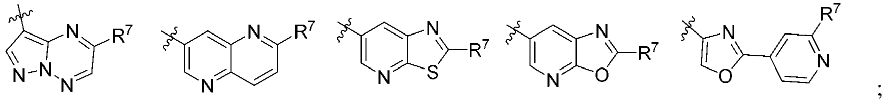 and
the R⁷ is selected from:

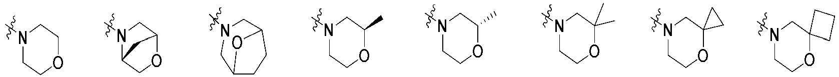

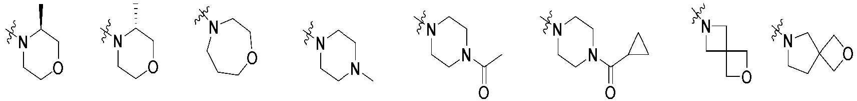

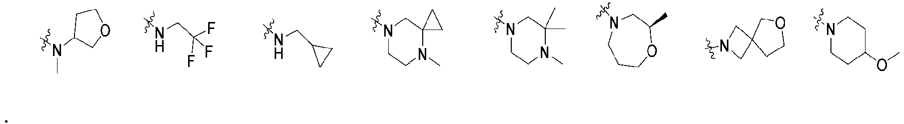

3. The compound according to claim 2, or the enantiomer thereof, the diastereomer thereof, the racemate thereof, the mixture comprising the same, or the deuterated compound thereof, or the pharmaceutically acceptable salt thereof, wherein:
the ring D is selected from: 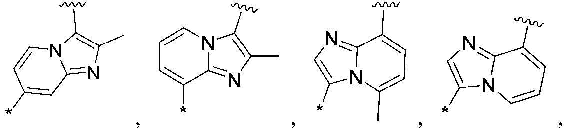 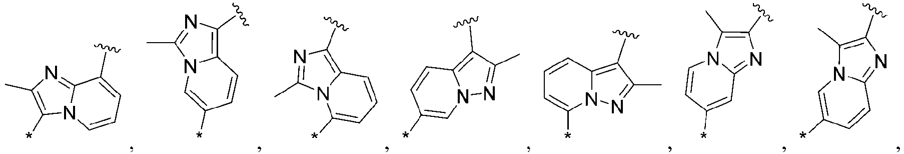 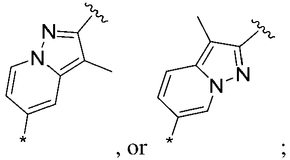 wherein * terminuses are each linked to the ring C; 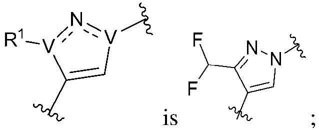
the ring A is selected from: 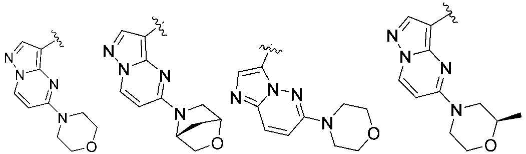 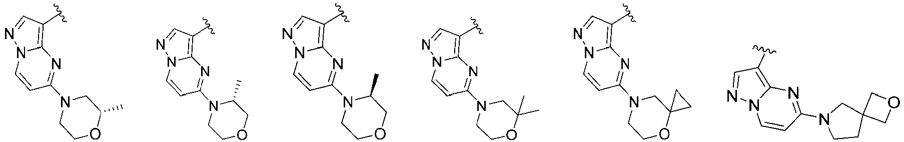 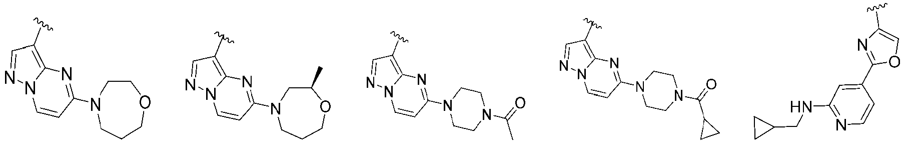 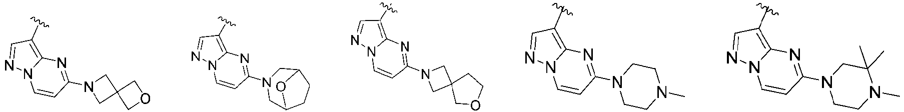 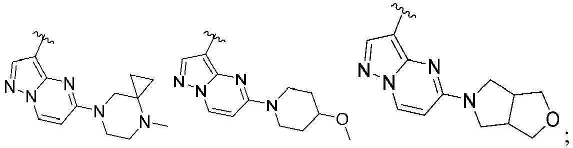 and
the R^{e} is hydrogen or methyl.

4. The compound according to claim 2, or the enantiomer thereof, the diastereomer thereof, the racemate thereof, the mixture comprising the same, or the deuterated compound thereof, or the pharmaceutically acceptable salt thereof, wherein: the compound represented by formula Ia is as shown below: 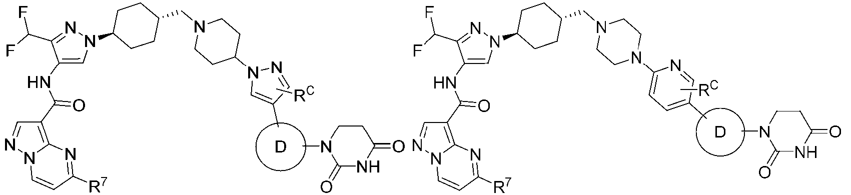 or 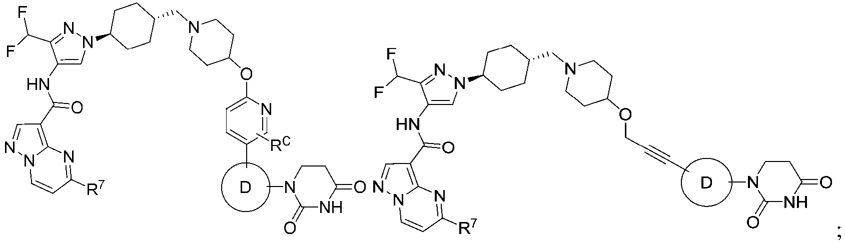 wherein
the R⁷ is selected from 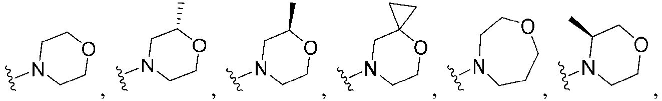 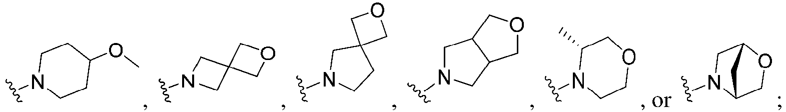 and
the R^{C} is selected from hydrogen, deuterium, halogen, cyano, hydroxyl, amino, C1-C6 linear or branched alkyl, C2-C6 linear or branched unsaturated hydrocarbon radical, C1-C6 linear or branched alkoxy, 3-10 membered cycloalkoxy, or C1-C6 linear or branched alkylamino.

5. The compound according to any one of claims 1 to 4, or the enantiomer thereof, the diastereomer thereof, the racemate thereof, the mixture comprising the same, or the deuterated compound thereof, or the pharmaceutically acceptable salt thereof, wherein: the compound is selected from compounds below:

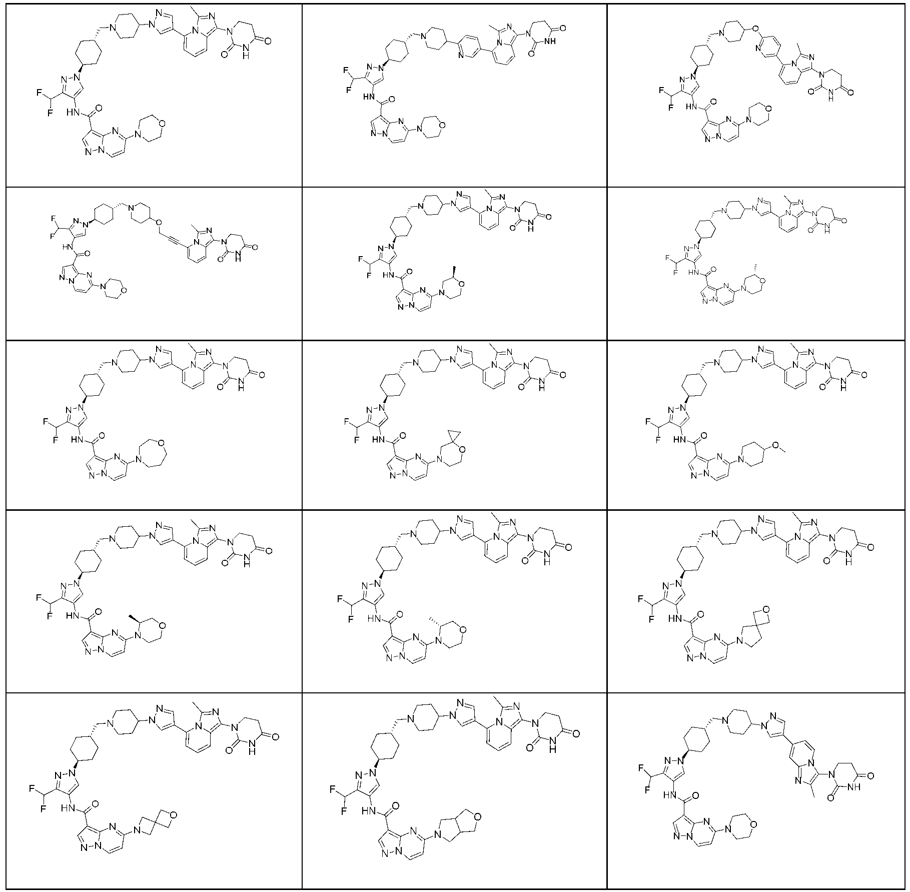

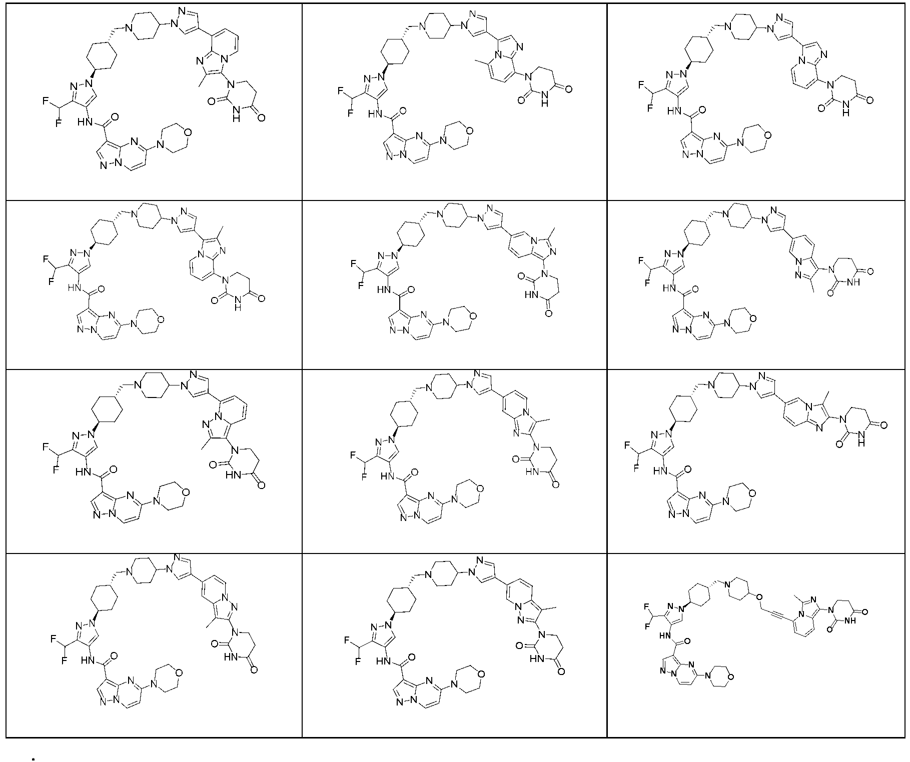

6. The compound according to claim 1, or the enantiomer thereof, the diastereomer thereof, the racemate thereof, the mixture comprising the same, or the deuterated compound thereof, or the pharmaceutically acceptable salt thereof, wherein: the compound represented by formula I is as shown in formula Ib or Ic: 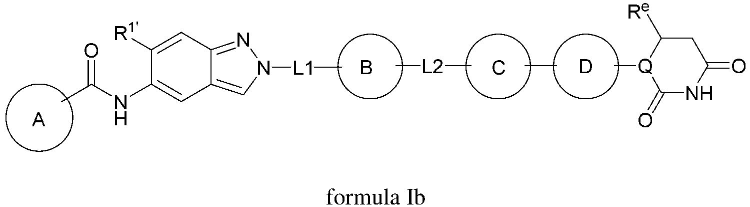 or 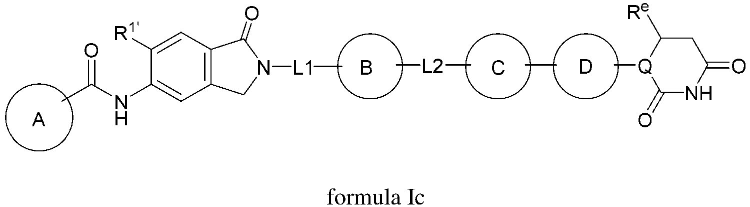 wherein
the ring D is selected from 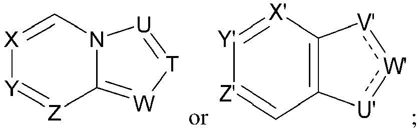
Q is selected from CR^{2a} or N;
U is selected from N, W is selected from C or CR^{W}, and T is selected from C or CR^{T};
or the W is selected from N, the U is selected from C or CR^{U}, and the T is selected from C or CR^{T};
at most one of X, Y, and Z is N;
V' is selected from N or C, U' is selected from CR² or NR², and W' is selected from CR³ or N;
X' is selected from CR⁴ or N; Y' is selected from CR⁵ or N; Z' is selected from C, CR⁶, or N; and at most one of X', Y', and Z' is N;
R^{1'} is selected from hydrogen, deuterium, halogen, cyano, substituted or unsubstituted C1-C6 linear or branched alkyl, substituted or unsubstituted C1-C6 linear or branched alkoxy, substituted or unsubstituted 4-10-membered heterocyclyl, substituted or unsubstituted C5-C12 bridged heterocyclyl, substituted or unsubstituted C3-C10 heterospirocyclyl, or substituted or unsubstituted C1-C6 linear or branched alkylamino; wherein the substutuents are independently selected from deuterium, halogen, hydroxyl, C1-C6 linear or branched alkyl, C1-C6 linear or branched alkoxy, 3-10-membered cycloalkoxy, 4-10-membered heterocyclyl, 3-10-membered cycloalkyl, C1-C6 linear or branched alkyl-C(=O)-, or 3-10-membered cycloalkyl-C(=O)-; and a number of the substituents is 1, 2, or 3;
for example, the R^{1'} is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, or cyclopropoxymethyl; and for another example, the R^{1'} is selected from: 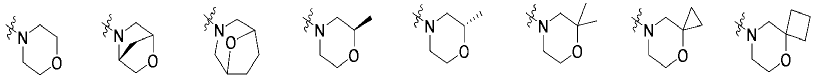 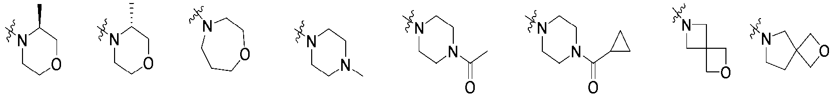 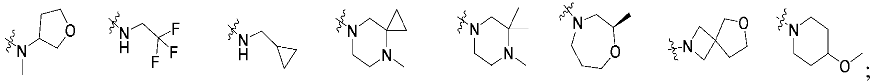
the R^{2a}, R², R³, R⁴, R⁵, R⁶, R^{U}, R^{W}, R^{T}, R^{X}, R^{Y}, R^{Z}, and R^{e} are each independently selected from hydrogen, halogen, cyano, substituted or unsubstituted C1-C6 linear or branched alkyl, substituted or unsubstituted C1-C6 linear or branched alkoxy, or substituted or unsubstituted 3-10-membered cycloalkyl; for example, H, F, Cl, cyclopropyl, cyclobutyl, ethyl, n-propyl, isopropyl, monofluoromethyl, difluoromethyl, trifluoromethyl, cyano, methoxy, ethoxy, or methyl;
the ring A is independently selected from rings below: 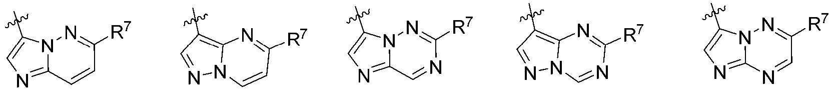 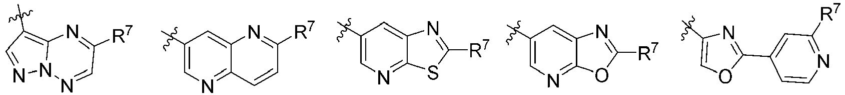 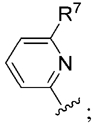
the R⁷ is selected from hydrogen, deuterium, halogen, cyano, substituted or unsubstituted C1-C6 linear or branched alkyl, substituted or unsubstituted C1-C6 linear or branched alkoxy, substituted or unsubstituted 4-10-membered heterocyclyl, substituted or unsubstituted C5-C12 bridged heterocyclyl, substituted or unsubstituted C3-C10 heterospirocyclyl, or substituted or unsubstituted C1-C6 linear or branched alkylamino; wherein the substutuents are independently selected from deuterium, halogen, hydroxyl, C1-C6 linear or branched alkyl, C1-C6 linear or branched alkoxy, 3-10-membered cycloalkoxy, 4-10-membered heterocyclyl, 3-10-membered cycloalkyl, C1-C6 linear or branched alkyl-C(=O)-, or 3-10-membered cycloalkyl-C(=O)-; and a number of the substituents is 1, 2, or 3;
for example, the R⁷ is selected from hydrogen, deuterium, halogen, cyano, methyl, ethyl, propyl, cyclopropyl, cyclobutyl, cyclopentyl, hydroxymethyl, trifluoromethyl, difluoromethyl, monofluoromethyl, methoxymethyl, ethoxymethyl, or cyclopropoxymethyl; and for another example, the R⁷ is selected from:

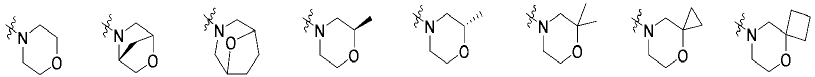

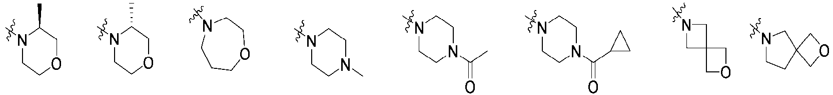

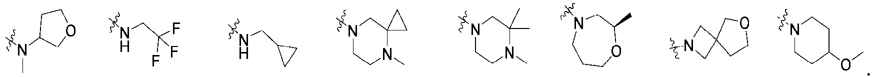

7. The compound according to claim 6, or the enantiomer thereof, the diastereomer thereof, the racemate thereof, the mixture comprising the same, or the deuterated compound thereof, or the pharmaceutically acceptable salt thereof, wherein:
the ring D is selected from: 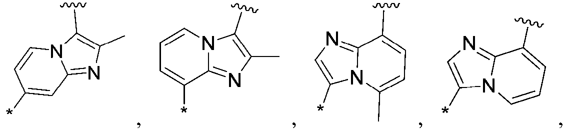 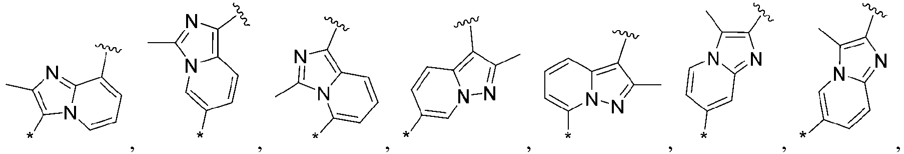 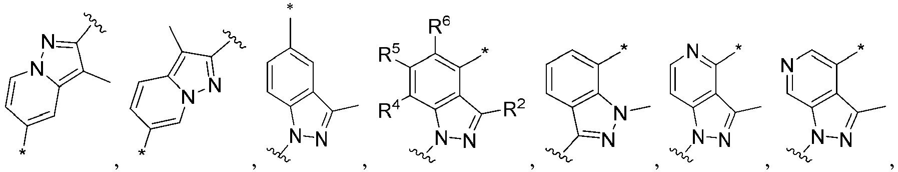 or 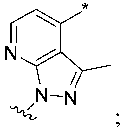 wherein * terminuses are each linked to the ring C; wherein the R², R⁴, R⁵, and R⁶ are each independently selected from hydrogen, halogen, cyano, substituted or unsubstituted C1-C6 linear or branched alkyl, substituted or unsubstituted C1-C6 linear or branched alkoxy, or substituted or unsubstituted 3-10-membered cycloalkyl; and preferably, the R², R⁴, R⁵, and R⁶ are each independently selected from H, F, Cl, cyclopropyl, cyclobutyl, ethyl, n-propyl, isopropyl, monofluoromethyl, difluoromethyl, trifluoromethyl, cyano, methoxy, ethoxy, or methyl; and
the ring A is selected from:

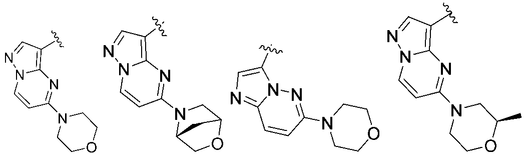

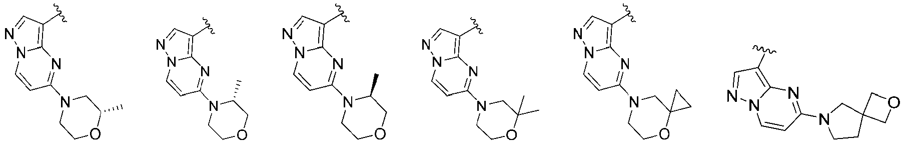

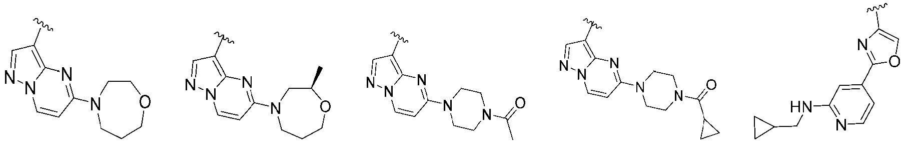

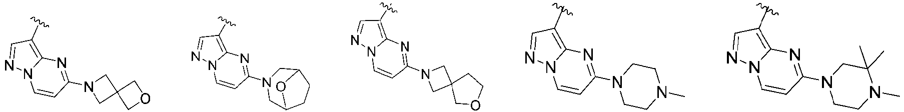

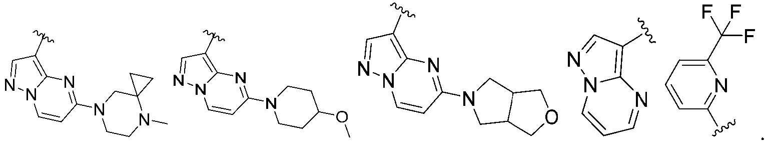

8. The compound according to claim 6, or the enantiomer thereof, the diastereomer thereof, the racemate thereof, the mixture comprising the same, or the deuterated compound thereof, or the pharmaceutically acceptable salt thereof, wherein: the compound represented by formula Ib is as shown below: 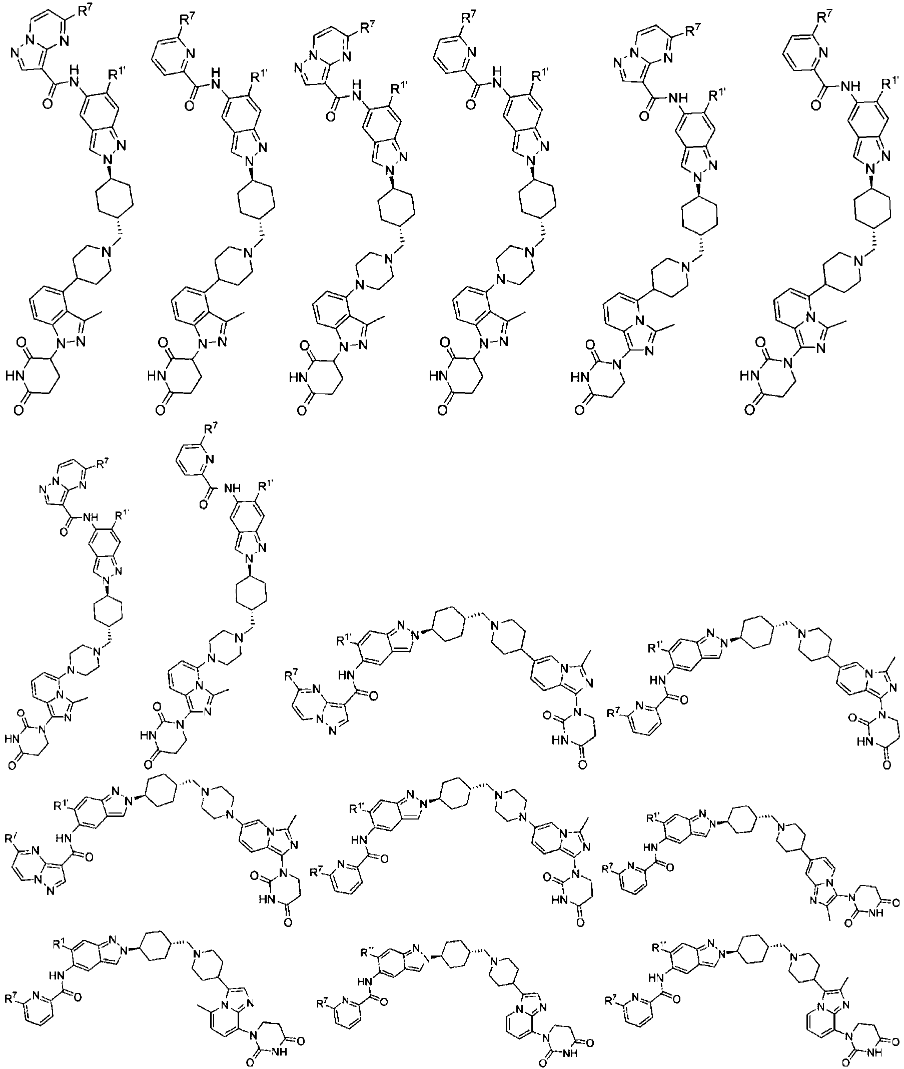 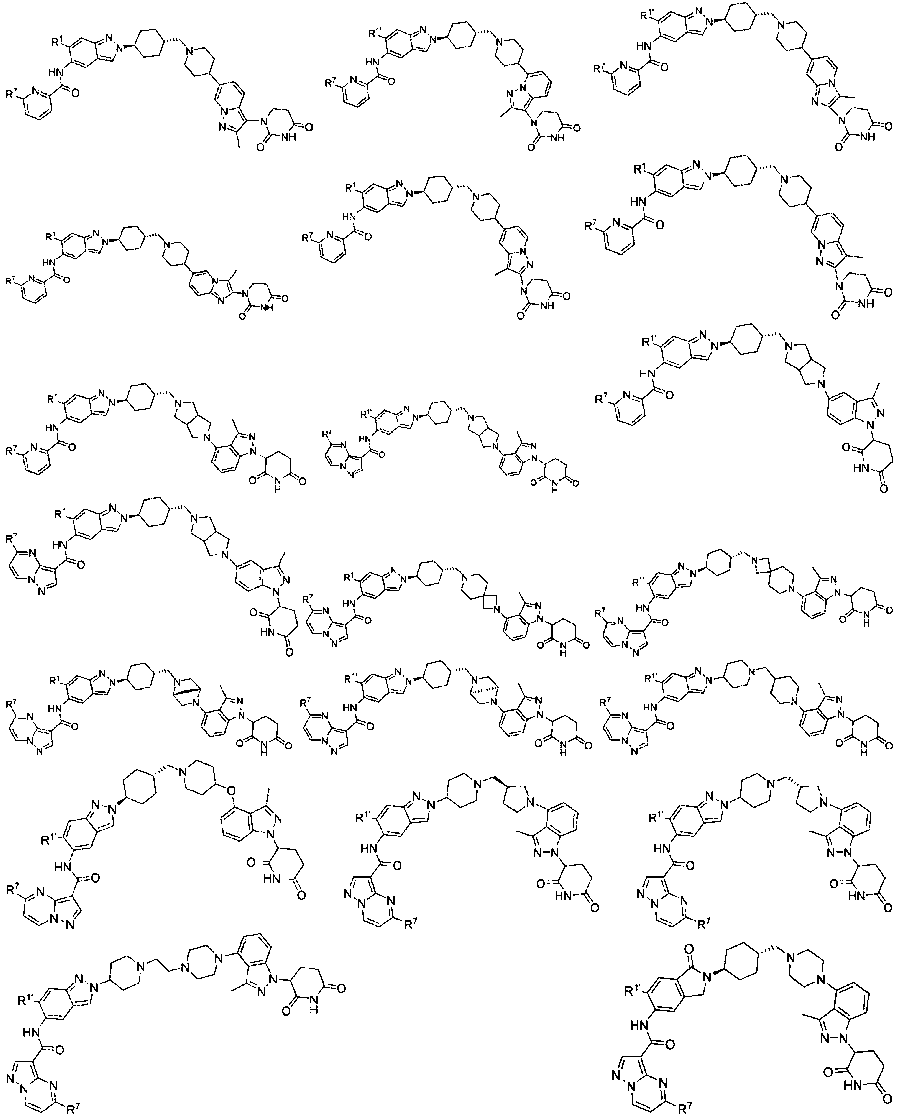 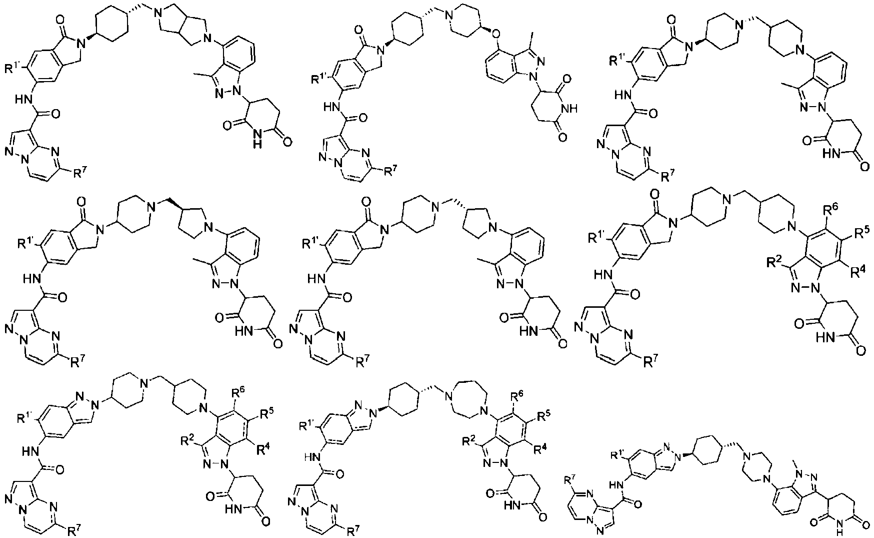 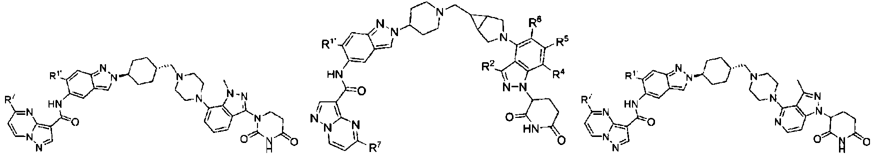 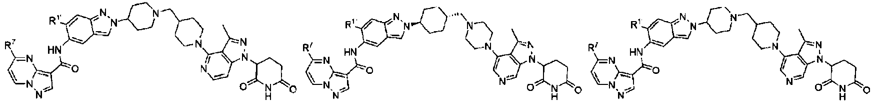 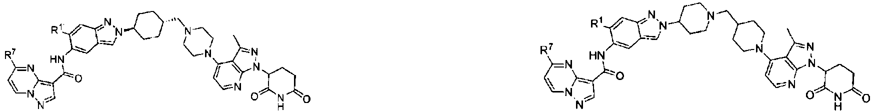 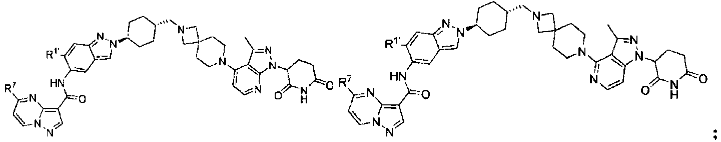 wherein
the R², R⁴, R⁵, and R⁶ are each independently selected from H, F, Cl, cyclopropyl, cyclobutyl, ethyl, n-propyl, isopropyl, monofluoromethyl, difluoromethyl, trifluoromethyl, cyano, methoxy, ethoxy, or methyl;
the R^{1'} is selected from hydrogen, deuterium, methyl, ethyl, propyl, hydroxymethyl, methoxy, ethoxy, trifluoromethyl, difluoromethyl, monofluoromethyl, methoxymethyl, amino, monomethylamino, dimethylamino, deuteromonomethylamino, deuterodimethylamino, 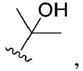 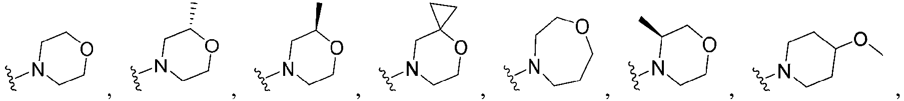 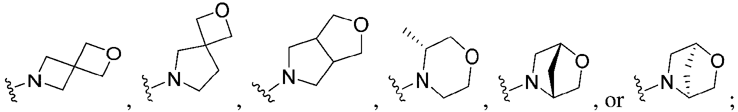 and
the R⁷ is selected from hydrogen, deuterium, methyl, ethyl, propyl, trifluoromethyl, difluoromethyl, monofluoromethyl, 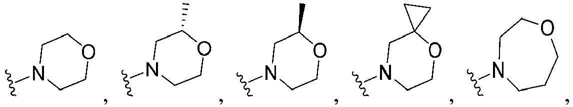 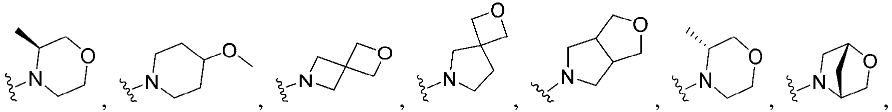 or

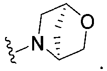

9. The compound according to any one of claims 6 to 8, or the enantiomer thereof, the diastereomer thereof, the racemate thereof, the mixture comprising the same, or the deuterated compound thereof, or the pharmaceutically acceptable salt thereof, wherein: the compound is selected from structures below:

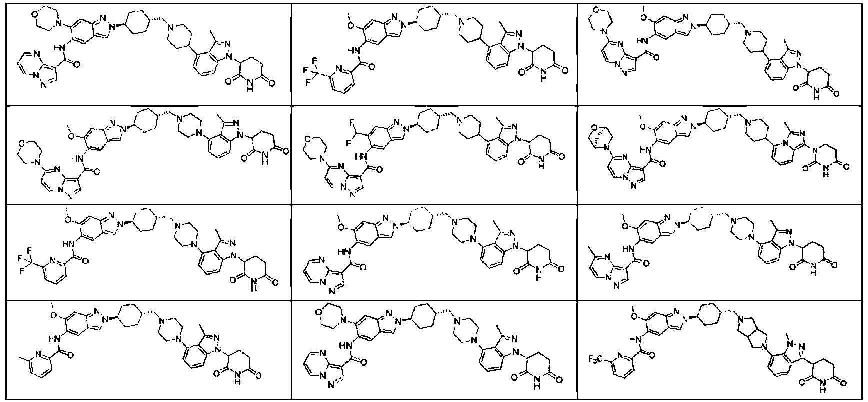

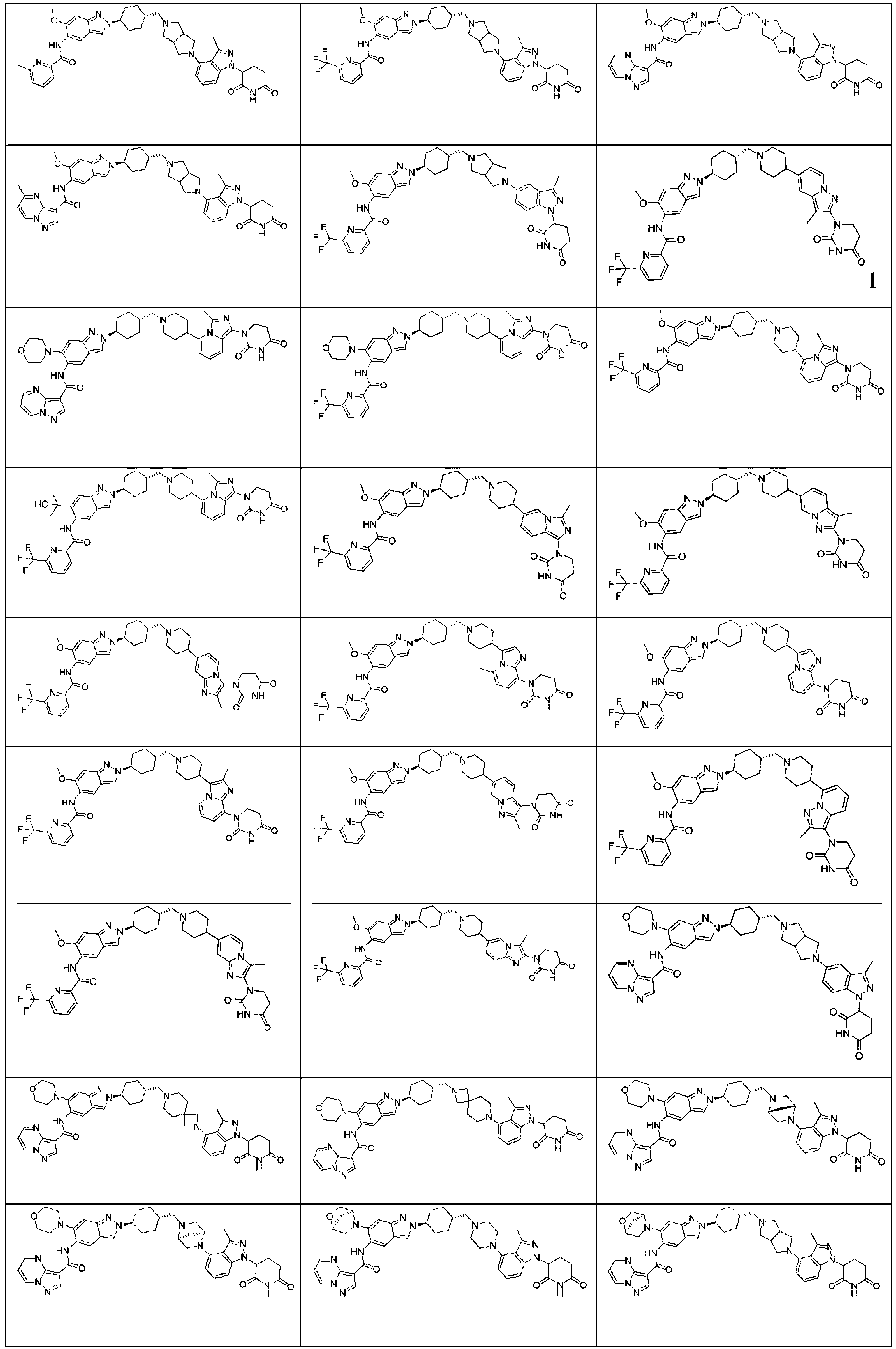

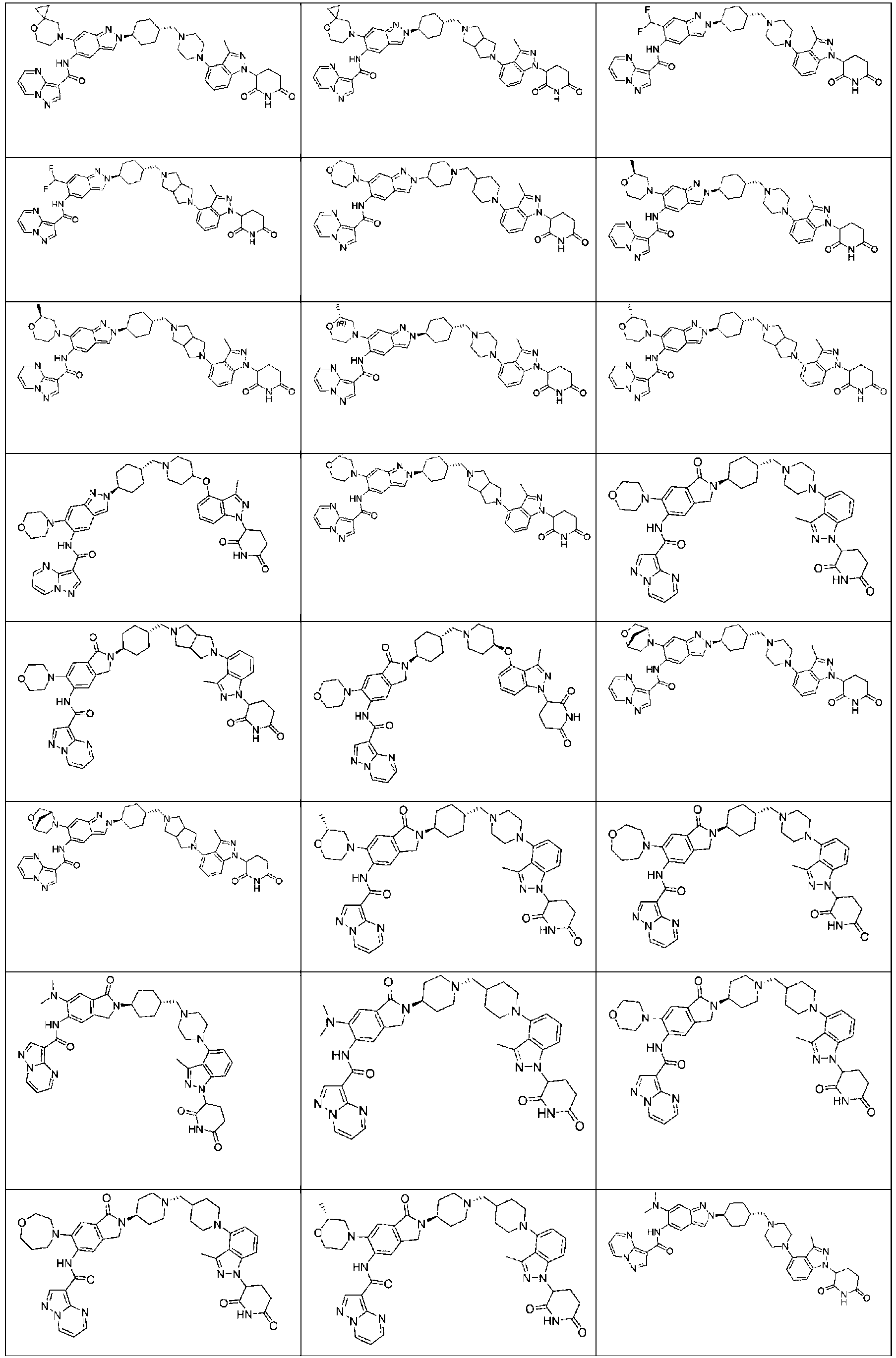

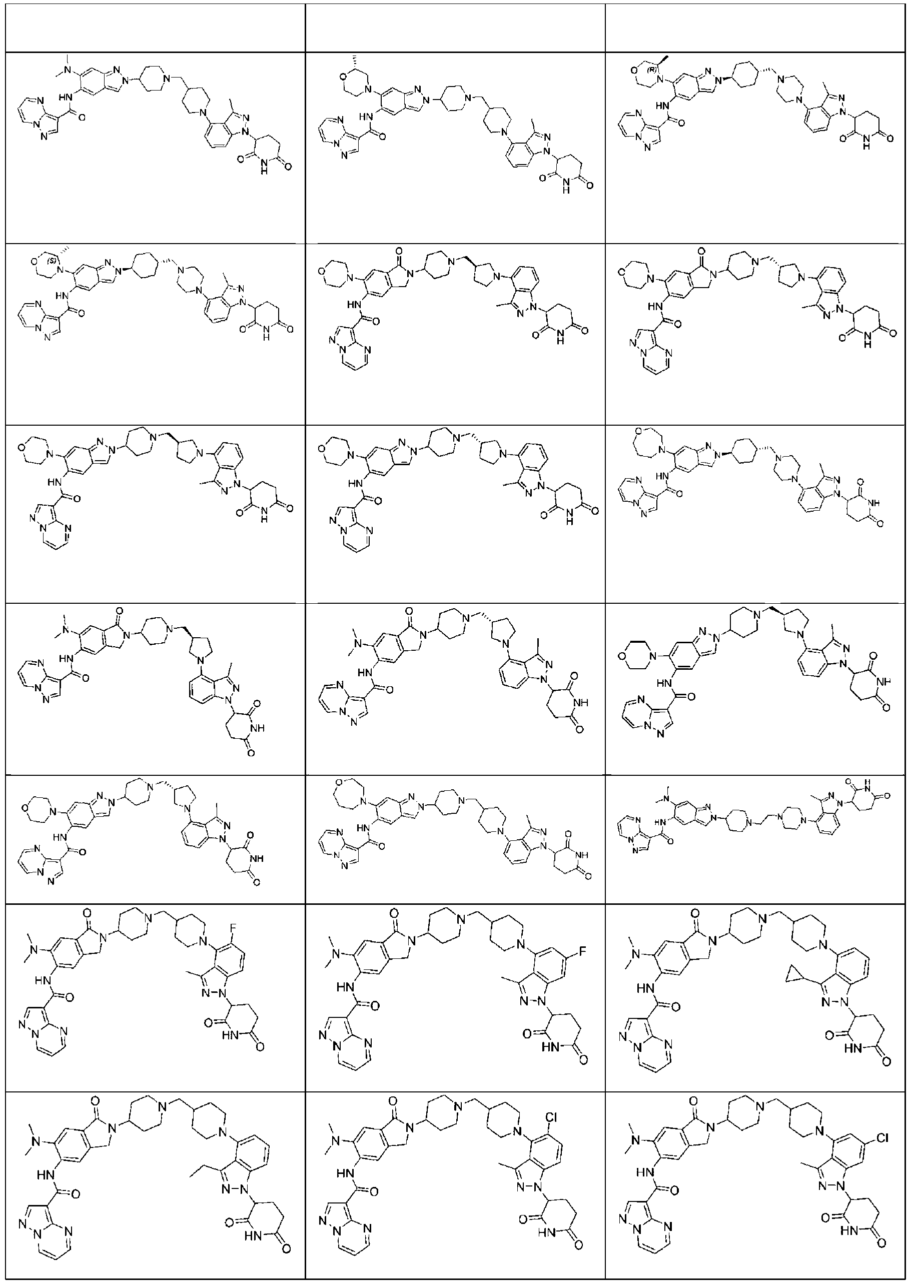

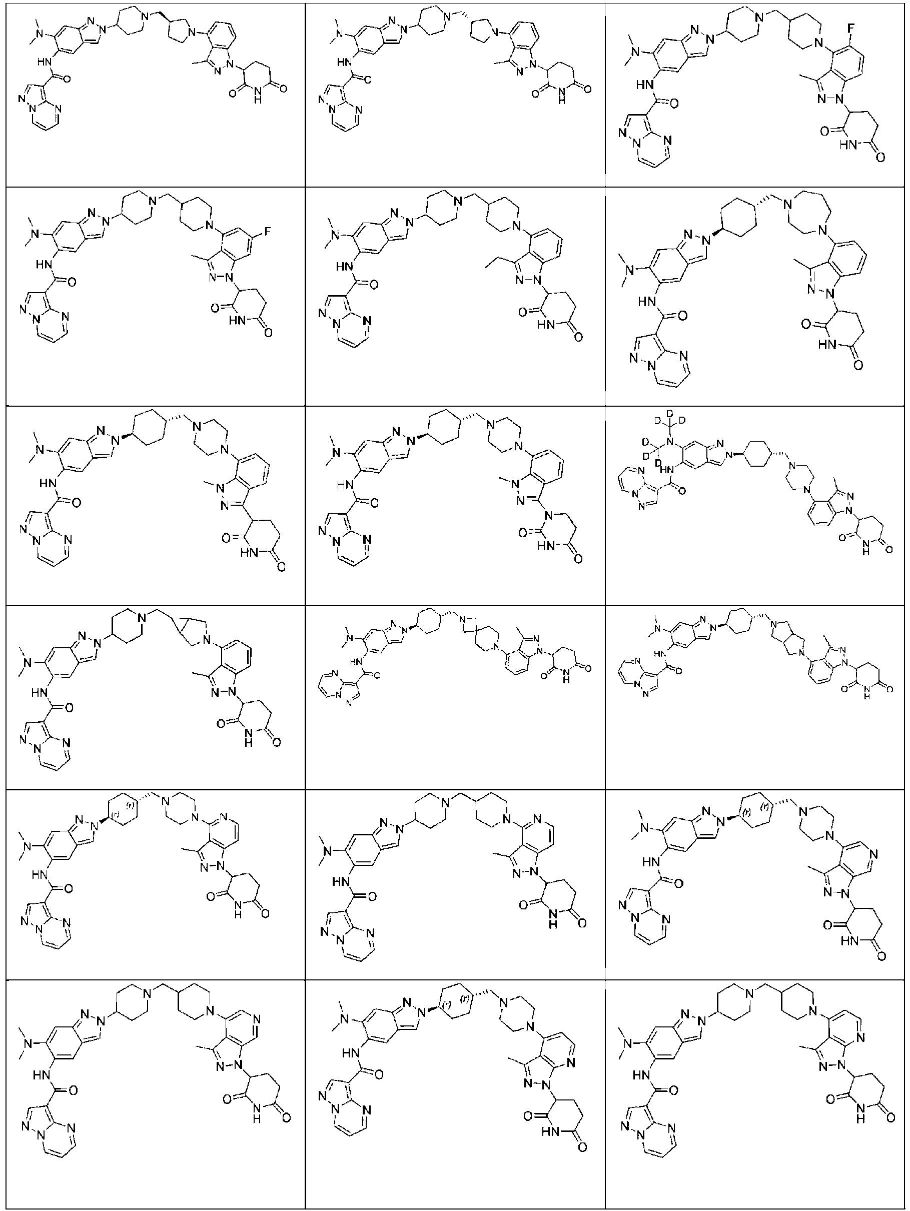

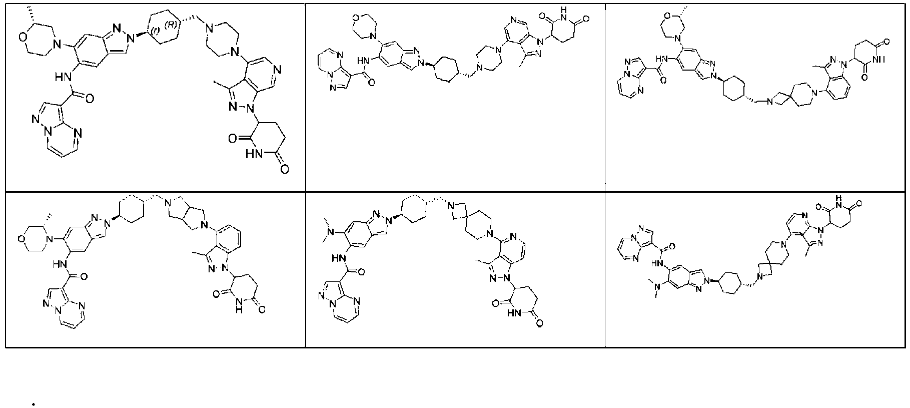

10. Use of the compound according to any one of claims 1 to 9, or the enantiomer thereof, the diastereomer thereof, the racemate thereof, the mixture comprising the same, or the pharmaceutically acceptable salt thereof for the manufacture of a medicament; the medicament being a medicament for treating and preventing one or more diseases associated with or mediated by an interleukin-1 receptor-associated kinase 4 (IRAK4) signaling pathway, an interleukin-6 (IL-6) receptor, and a tumor necrosis factor α (TNFα), or the medicament being a medicament for treating and/or preventing an autoimmune disease and/or a cancer or a proliferative disease.

11. The use according to claim 10, wherein:
the disease includes a cancer, a neurodegenerative disease, a viral disease, an autoimmune disease, an inflammatory disease, a genetic disease, a hormone-related disease, a metabolic disorder, an organ transplantation-related disease, an immunodeficiency disease, an osteoclastic disease, a proliferative disease, an infectious disease, thrombin-induced platelet aggregation, a liver disease, a lesion caused by T cell activation, and a cardiovascular disease;
the cancer or the proliferative disorder is selected from brain cancer, kidney cancer, liver cancer, bladder cancer, breast cancer, gastric cancer, ovarian cancer, colon cancer, rectal cancer, esophageal cancer, lung cancer, prostate cancer, pancreatic cancer, vaginal cancer, cervical cancer, testicular cancer, urogenital tract cancer, laryngeal cancer, skin cancer, bone cancer, thyroid cancer, sarcoma, glioblastoma, neuroblastoma, multiple myeloma, head and neck cancer, epidermoid carcinoma, large cell carcinoma, non-small cell lung cancer, lymphoma, Hodgkin's or non-Hodgkin's lymphoma, seminoma, melanoma, leukemia, diffuse large B-cell lymphoma, ABC DLBCL, chronic lymphocytic leukemia, chronic lymphocytic lymphoma, primary exudative lymphoma, Burkitt lymphoma/leukemia, acute lymphocytic leukemia, B-cell lymphocytic leukemia, lymphoplasmacytic lymphoma, Waldenström macroglobulinemia, splenic marginal zone lymphoma, plasmacytoma or intravascular large B cell lymphoma; epidermal hyperproliferative disease, psoriasis, prostatic hyperplasia, IL-1 driven disease, and MyD88 driven disease;
the MyD88-driven disease is selected from ABC DLBCL, Waldenström macroglobulinemia, Hodgkin's lymphoma, primary cutaneous T-cell lymphoma, and chronic lymphocytic leukemia;
the neurodegenerative disease is selected from Alzheimer's disease, Parkinson's disease, amyotrophic lateral sclerosis, Huntington's disease, cerebral ischemia, traumatic neurodegenerative disease, and graft-versus-host disease; and
the inflammatory disease is selected from ocular allergy, conjunctivitis, keratoconjunctivitis sicca, phlebitis-conjunctivitis, allergic rhinitis, hemolytic anemia, aplastic anemia, pure red cell anemia, idiopathic thrombocytopenic purpura, or acne; or another inflammatory disease caused by an autoimmune response is selected from systemic lupus erythematosus, rheumatoid arthritis, polychondritis, scleroderma, Wegener's granulomatosis, dermatomyositis, chronic active hepatitis, myasthenia gravis, Stephen-Johnson syndrome, idiopathic steatorrhea, ulcerative colitis, Crohn's disease or other autoimmune inflammatory bowel diseases, irritable bowel syndrome, celiaca, periodontitis, hyaline membrane disease, kidney disease, glomerular disease, alcoholic liver disease, endocrine ophthalmopathy, Graves disease, sarcoidosis, alveolar catarrh, chronic hypersensitivity pneumonia, multiple sclerosis, primary biliary cirrhosis, uveitis, Sjögren's syndrome, uveitis, keratoconjunctivitis, interstitial fibrosis, psoriatic arthritis, systemic juvenile idiopathic arthritis, nephritis, diverticulitis, interstitial cystitis, glomerulonephritis, pancreatitis, hereditary periodic fever syndrome, asthma, acute lung injury, acute respiratory distress syndrome, eosinophilia, hypersensitivity response, anaphylaxis, sinusitis, chronic obstructive pulmonary disease, lung disease, cystic fibrosis, appendicitis, atopic dermatitis, allergy, blepharitis, bronchiolitis, bronchitis, bursitis, cervicitis, cholangitis, cholecystitis, chronic graft rejection reaction, conjunctivitis, cystitis, dacryoadenitis, dermatitis, dermatomyositis, encephalitis, endocarditis, endometritis, enteritis, epididymitis, fasciitis, fibrositis, gastritis, gastroenteritis, allergic purpura, hepatitis, hidradenitis suppurativa, immunoglobulin A nephropathy, interstitial lung disease, laryngitis, mastitis, meningitis, myelitis, myocarditis, myositis, nephritis, oophoritis, orchitis, osteitis, tympanitis, pancreatitis, mumps, pericarditis, peritonitis, pharyngitis, pleurisy, phlebitis, pneumonia, polymyositis, enteritis, prostatitis, pyelonephritis, rhinitis, salpingitis, sinusitis, stomatitis, synovitis, tendinitis, tonsillitis, vaginitis, vasculitis, vulvitis, pelada, herpetiform dermatitis, subcutaneous dermatitis, vitiligo, hypersensitivity vasculitis, urticaria, bullous pemphigus, pemphigus vulgaris, pemphigus foliaceus, epidermolysis bullosa, acute and chronic gout, chronic gouty arthritis, psoriasis, psoriatic arthritis, rheumatoid arthritis, juvenile rheumatoid arthritis, or osteoarthritis.
